(19) Europäisches Patentamt / European Patent Office / Office européen des brevets

(11) EP 4 488 261 A1

(12) # EUROPEAN PATENT APPLICATION
published in accordance with Art. 153(4) EPC

(43) Date of publication:
**08.01.2025 Bulletin 2025/02**

(21) Application number: **23759323.1**

(22) Date of filing: **27.02.2023**

(51) International Patent Classification (IPC):
***C07C 271/20*** *(2006.01)* ***C07C 271/16*** *(2006.01)*
***C07C 271/12*** *(2006.01)* ***C07C 219/16*** *(2006.01)*
***C07C 229/10*** *(2006.01)* ***C07C 229/12*** *(2006.01)*
***C07D 207/02*** *(2006.01)* ***C07D 211/04*** *(2006.01)*
***A61K 9/127*** *(2006.01)* ***A61K 9/51*** *(2006.01)*
***A61K 47/18*** *(2017.01)* ***A61K 31/7088*** *(2006.01)*

(52) Cooperative Patent Classification (CPC):
**A61K 9/127; A61K 9/51; A61K 31/7088; A61K 47/18; C07C 219/16; C07C 229/10; C07C 229/12; C07C 271/12; C07C 271/16; C07C 271/20; C07D 207/02; C07D 211/04**

(86) International application number:
**PCT/CN2023/078449**

(87) International publication number:
**WO 2023/160702 (31.08.2023 Gazette 2023/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.02.2022 PCT/CN2022/078232**
**05.07.2022 PCT/CN2022/103830**
**29.12.2022 PCT/CN2022/143371**

(71) Applicant: **Shenzhen Shenxin Biotechnology Co., Ltd.**
**Shenzhen, Guangdong 518000 (CN)**

(72) Inventors:
- **LI, Linxian**
  **Shenzhen, Guangdong 518000 (CN)**
- **CHEN, Yonghao**
  **Shenzhen, Guangdong 518000 (CN)**
- **CHEN, Minghong**
  **Shenzhen, Guangdong 518000 (CN)**

(74) Representative: **Nederlandsch Octrooibureau**
**P.O. Box 29720**
**2502 LS The Hague (NL)**

Remarks:
The complete document including Reference Table(s) and the Sequence Listing(s) can be downloaded from the EPO website

(54) **AMINO LIPID COMPOUND, PREPARATION METHOD THEREFOR, COMPOSITION THEREOF AND USE THEREOF**

(57) The present disclosure relates to an amino lipid compound, a preparation method therefor, a composition thereof and the use thereof. Specifically, the present disclosure relates to an amino lipid compound represented by formula (I) or a pharmaceutically acceptable salt or stereoisomer thereof, and the use thereof in the formulation of a lipid nanoparticle for delivering an active ingredient. The present disclosure also relates to a composition containing the amino lipid compound, and in particular relates to a lipid nanoparticle and the use thereof.

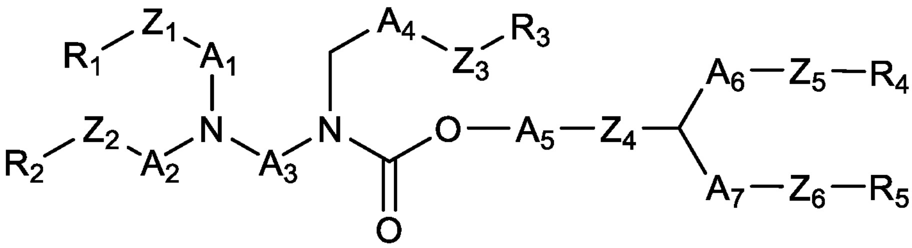

(I)

## Description

### Technical Field

The present disclosure relates to an amino lipid compound that can be used to prepare a lipid nanoparticle for delivering an active ingredient and a preparation method therefor. The present disclosure also relates to a composition, especially a lipid nanoparticle, containing the amino lipid compound, and use thereof.

### Background Art

Gene therapeutic agents deliver genes with specific genetic information to target cells by artificial means, and the expressed target proteins have regulatory, therapeutic and even curative effects on diseases caused by congenital or acquired gene defects, or gene series can interfere with or regulate the expression of related genes to achieve clinical therapeutic effects. However, gene therapeutic agents still face several challenges, including the fact that nucleic acids are difficult to be directly introduced into cells and are very easily degraded by nucleic acid-degrading enzymes in cytoplasm. Therefore, there is a need to develop more amino lipid compounds that can be used to deliver nucleic acid like active ingredients, as well as related preparation methods and applications, in order to promote gene therapeutic agents to achieve therapeutic and/or prophylactic purposes.

### Summary of the Invention

One aspect of the present disclosure provides an amino lipid compound represented by formula (I):

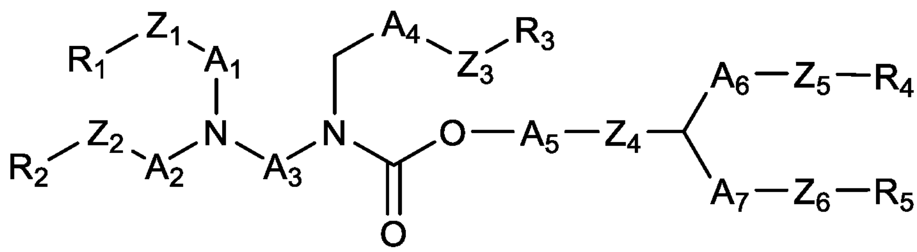

(I)

wherein $R_1$, $R_2$, $R_3$, $R_4$, $R_5$, $Z_1$, $Z_2$, $Z_3$, $Z_4$, $Z_5$, $Z_6$, $A_1$, $A_2$, $A_3$, $A_4$, $A_5$, $A_6$ and $A_7$ are each defined as follows.

Another aspect of the present disclosure provides a method for preparing the amino lipid compound.

Another aspect of the present disclosure provides a use of the amino lipid compound in the manufacture of a vehicle for an active ingredient.

Another aspect of the present disclosure provides a lipid nanoparticle comprising the amino lipid compound.

Another aspect of the present disclosure provides a composition comprising the amino lipid compound.

Another aspect of the present disclosure provides a use of the amino lipid compound, lipid nanoparticle, or composition in the manufacture of a medicament.

Another aspect of the present disclosure provides a use of the amino lipid compound, lipid nanoparticle, or composition in the manufacture of a medicament for nucleic acid transfer.

### Detailed Description of the Invention

<u>Definition</u>

Unless otherwise defined below, all technical and scientific terms used herein are intended to have the same meaning as commonly understood by one of ordinary skill in the art. Reference to a technique as used herein is intended to mean a technique as commonly understood in the art, including those variations that are apparent to those skilled in the art, or substitutions of equivalence technique. While it is believed that the following terms are well understood by those skilled in the art, the following definitions are set forth to better explain the present disclosure.

As used herein, the terms "comprising", "including", "having", "containing", or "involving" as well as other variations thereof, are inclusive or open-ended and do not exclude other non-recited elements or method steps.

As used herein, the term "hydrocarbyl" refers to the group remaining after the loss of one hydrogen atom from an aliphatic hydrocarbon, including straight or branched, saturated or unsaturated hydrocarbyl groups. Hydrocarbyl groups

include, but are not limited to, alkyl, alkenyl, and alkynyl groups. Preferably, a hydrocarbyl group has from 1 to 24 carbon atoms ($C_1$-$C_{24}$ hydrocarbyl), for example, 1, 2, 3, 4, 5, 6, 7, 8, 9, 10, 11, 12, 13, 14, 15, 16, 17, 18, 19 or 20 carbon atoms ($C_1$, $C_2$, $C_3$, ... $C_{17}$, $C_{18}$, $C_{19}$, or $C_{20}$ hydrocarbyl). Examples of hydrocarbyl groups include, but are not limited to, $C_1$-$C_{24}$ hydrocarbyl, $C_1$-$C_{20}$ hydrocarbyl, $C_1$-$C_{18}$ hydrocarbyl, $C_1$-$C_{16}$ hydrocarbyl, $C_1$-$C_{12}$ hydrocarbyl, $C_1$-$C_{10}$ hydrocarbyl, $C_1$-$C_8$ hydrocarbyl, $C_1$-$C_7$ hydrocarbyl, $C_1$-$C_6$ hydrocarbyl, $C_1$-$C_4$ hydrocarbyl, $C_1$-$C_3$ hydrocarbyl, $C_1$-$C_2$ hydrocarbyl, $C_2$-$C_8$ hydrocarbyl, $C_2$-$C_4$ hydrocarbyl, $C_4$-$C_8$ hydrocarbyl, $C_4$-$C_9$ hydrocarbyl, $C_5$-$C_8$ hydrocarbyl, $C_1$-$C_4$ hydrocarbyl, $C_2$-$C_8$ hydrocarbyl, $C_3$ hydrocarbyl, $C_4$ hydrocarbyl, $C_5$ hydrocarbyl, $C_6$ hydrocarbyl, $C_7$ hydrocarbyl, and $C_8$ hydrocarbyl. Unless expressly stated otherwise in this specification, hydrocarbyl is optionally substituted, and for the substituents, reference is made to the definition of "optionally substituted" below. In certain embodiments, the hydrocarbyl has no branches (i.e., is straight chain), one branch, two branches, or multiple branches.

As used herein, the term "hydrocarbylene" refers to a divalent group remaining after further loss of one hydrogen atom from the hydrocarbyl as defined above. Unless expressly stated otherwise in this specification, hydrocarbylene is also optionally substituted.

As used herein, the term "alkyl" is a straight or branched saturated monovalent hydrocarbyl. Preferably, an alkyl group has from 1 to 24 carbon atoms ($C_1$-$C_{24}$ alkyl), for example, 1, 2, 3, 4, 5, 6, 7, 8, 9, 10, 11, 12, 13, 14, 15, 16, 17, 18, 19 or 20 carbon atoms ($C_1$, $C_2$, $C_3$, ... $C_{17}$, $C_{18}$, $C_{19}$, or $C_{20}$ alkyl). Examples of alkyl groups include, but are not limited to, $C_1$-$C_{24}$ alkyl, $C_1$-$C_{20}$ alkyl, $C_1$-$C_{18}$ alkyl, $C_1$-$C_{16}$ alkyl, $C_1$-$C_{12}$ alkyl, $C_1$-$C_{10}$ alkyl, $C_1$-$C_8$ alkyl, $C_1$-$C_7$ alkyl, $C_1$-$C_6$ alkyl, $C_1$-$C_4$ alkyl, $C_1$-$C_3$ alkyl, $C_1$-$C_2$ alkyl, $C_2$-$C_8$ alkyl, $C_2$-$C_4$ alkyl, $C_4$-$C_8$ alkyl, $C_4$-$C_9$ alkyl, $C_5$-$C_8$ alkyl, $C_1$-$C_4$ alkyl, $C_2$-$C_8$ alkyl, methyl, ethyl, n-propyl, isopropyl, n-butyl, sec-butyl, isobutyl, tert-butyl, n-pentyl, n-hexyl, n-heptyl, n-octyl, n-nonyl, n-decyl, n-undecyl, n-dodecyl, n-tridecyl, and tridecan-7-yl. Unless explicitly stated otherwise in this specification, alkyl is optionally substituted.

As used herein, the term "alkylene" refers to a divalent group remaining after further loss of one hydrogen atom from the alkyl as defined above. Unless expressly stated otherwise in this specification, alkylene is also optionally substituted.

As used herein, the term "alkenyl" is a straight or branched monovalent hydrocarbyl containing one or more double bonds (C=C). Preferably, an alkenyl group has from 2 to 24 carbon atoms ($C_2$-$C_{24}$ alkenyl), for example, 2, 3, 4, 5, 6, 7, 8, 9, 10, 11, 12, 13, 14, 15, 16, 17, 18, 19 or 20 carbon atoms ($C_2$, $C_3$, ... $C_{17}$, $C_{18}$, $C_{19}$, or $C_{20}$ alkenyl), and has 1, 2, 3, 4, or more double bonds. Alkenyl groups include, but is not limited to, $C_2$-$C_{24}$ alkenyl, $C_2$-$C_{20}$ alkenyl, $C_2$-$C_{18}$ alkenyl, $C_2$-$C_{16}$ alkenyl, $C_2$-$C_{12}$ alkenyl, $C_2$-$C_{10}$ alkenyl, $C_2$-$C_8$ alkenyl, $C_2$-$C_7$ alkenyl, $C_2$-$C_6$ alkenyl, $C_2$-$C_4$ alkenyl, $C_2$-$C_3$ alkenyl, $C_4$-$C_8$ alkenyl, $C_4$-$C_9$ alkenyl, $C_5$-$C_8$ alkenyl having 1, 2, 3, 4 or more double bonds. Some more specific examples include, but are not limited to, ethenyl, propenyl, but-1-enyl, but-2-enyl, pent-1-enyl, pent-2-enyl, hex-1-enyl, hex-2-enyl, hex-3-enyl, hept-1-enyl, hept-2-enyl, hept-3-enyl, oct-1-enyl, oct-2-enyl, oct-3-enyl, non-1-enyl, non-2-enyl, and non-3-enyl. In some preferred embodiments, the alkenyl group has one double bond. Unless expressly stated otherwise in this specification, alkenyl is optionally substituted.

As used herein, the term "alkenylene" refers to a divalent group remaining after further loss of one hydrogen atom from the alkenyl as defined above. Unless expressly stated otherwise in the specification, alkenylene is also optionally substituted.

As used herein, the term "alkynyl" is a straight or branched monovalent alkynyl group containing one or more triple bonds (C≡C). Preferably, an alkynyl group has from 2 to 24 carbon atoms ($C_2$-$C_{24}$ alkynyl), for example, 2, 3, 4, 5, 6, 7, 8, 9, 10, 11, 12, 13, 14, 15, 16, 17, 18, 19 or 20 carbon atoms ($C_2$, $C_3$, ... $C_{17}$, $C_{18}$, $C_{19}$, or $C_{20}$ alkynyl), and having 1, 2, 3, 4, or more triple bonds. Alkynyl groups include, but are not limited to, $C_2$-$C_{24}$ alkynyl, $C_2$-$C_{20}$ alkynyl, $C_2$-$C_{18}$ alkynyl, $C_2$-$C_{16}$ alkynyl, $C_2$-$C_{12}$ alkynyl, $C_2$-$C_{10}$ alkynyl, $C_2$-$C_8$ alkynyl, $C_2$-$C_7$ alkynyl, $C_2$-$C_6$ alkynyl, $C_2$-$C_4$ alkynyl, $C_2$-$C_3$ alkynyl, $C_4$-$C_8$ alkynyl, $C_4$-$C_9$ alkynyl, $C_5$-$C_8$ alkynyl having 1, 2, 3, 4 or more triple bonds. Some more specific examples include, but are not limited to, ethynyl, propynyl, but-1-ynyl, but-2-ynyl, pent-1-ynyl, pent-2-ynyl, hex-1-ynyl, hex-2-ynyl, hex-3-ynyl, hept-1-ynyl, hept-2-ynyl, hept-3-ynyl, oct-1-ynyl, oct-2-ynyl, oct-3-ynyl, non-1-ynyl, non-2-alkynyl, and non-3-alkynyl. In some preferred embodiments, the alkynyl group has one triple bond. Unless explicitly stated otherwise in this specification, alkynyl is optionally substituted.

As used herein, the term "alkynylene" refers to a divalent group remaining after further loss of one hydrogen atom from the alkynyl as defined above. Unless explicitly stated otherwise in the specification, alkynylene is also optionally substituted.

As used herein, the terms "cyclohydrocarbyl", "cyclohydrocarbylene", and "hydrocarbon ring" refer to saturated (i.e., "cycloalkyl" and "cycloalkylene") or unsaturated (i.e., having one or more double bonds (cycloalkenyl) and/or triple bonds (cycloalkynyl) in the ring) monocyclic or polycyclic hydrocarbon rings having ring carbon atoms. In certain embodiments, "cyclohydrocarbyl", "cyclohydrocarbylene", and "hydrocarbon ring" have, for example, from 3 to 10, suitably from 3 to 8, more suitably from 3 to 6, such as from 5 to 6 or from 5 to 7, ring carbon atoms. "Cyclohydrocarbyl", "cyclohydrocarbylene", and "hydrocarbon ring" include, but are not limited to, cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclohexenyl(ene) (ring), etc. Unless expressly stated otherwise in this specification, cyclohydrocarbyl, cyclohydrocarbylene, and hydrocarbon rings are optionally substituted.

As used herein, the term "cycloalkyl" refers to a saturated monocyclic or polycyclic (such as bicyclic) hydrocarbon ring (e.g., monocyclic, such as cyclopropyl, cyclobutyl, cyclopentyl, cyclohexyl, cycloheptyl, cyclooctyl, cyclononyl, or bicyclic, including spirocyclic, fused, or bridged systems, such as bicyclo[1.1.1] pentyl, bicyclo[2.2.1]heptyl, bicyclo[3.2.1] octyl or bicyclo[5.2.0]nonyl, decalin, etc.). In certain embodiments, cycloalkyl has, for example, 3 to 10, such as 3-7, 5-6, or 5-7 carbon atoms. Unless expressly stated otherwise in this specification, cycloalkyl is optionally substituted.

As used herein, the term "heterohydrocarbyl" or its subordinate concepts (e.g., heteroalkyl, heteroalkenyl, heteroalkynyl, heteroaryl, etc.) refer to a stable straight, branched, or cyclic hydrocarbon radical or combination thereof, consisting of a specified number of carbon atoms and at least one heteroatom. Heteroatoms refer to atoms other than carbon and hydrogen. In certain embodiments, heterohydrocarbyl contains one, two, three, or more heteroatoms. In certain embodiments, heterohydrocarbyl contains one or more (e.g., 2 or 3) identical heteroatoms, or contains multiple (e.g., 2 or 3) different heteroatoms. Preferably, the heteroatom is selected from O, N and S. Examples of heterohydrocarbyl include, but are not limited to, $-CH_2-CH_2-O-CH_3$, $-CH_2-CH_2-CH_2-O-CH_2-CH_3$, $-CH_2-(CH_2)_3-O-(CH_2)_5-CH_3$, $-CH_2-CH_2-NH-CH_3$, $-CH_2-CH_2-N(CH_3)-CH_3$, $-CH_2-S-CH_2-CH_3$, $-CH_2-CH_2$, $-CH=CH-O-CH_3$, $-CH_2-CH=N-OCH_3$, $-CH=CH-N(CH_3)-CH_3$, and $-CH_2-NH-OCH_3$. Unless explicitly stated otherwise in this specification, heterohydrocarbyl or its subordinate concepts (such as heteroalkyl, heteroalkenyl, heteroalkynyl, heteroaryl, etc.) are optionally substituted.

As used herein, the term "heterohydrocarbylene" or its subordinate concepts (such as heteroalkylene, heteroalkenylene, heteroalkynylene, heteroarylene, etc.) refer to a divalent group remaining after further loss of one hydrogen atom from the heterohydrocarbyl as defined above. Unless explicitly stated otherwise in this specification, heterohydrocarbylene or its subordinate concepts (such as heteroalkylene, heteroalkenylene, heteroalkynylene, heteroarylene, etc.) are also optionally substituted.

As used herein, the term "heterocycle," "heterocyclyl," or "heterocyclylene" means a cyclic group having a cyclic structure and containing one or more heteroatoms in the ring-forming atoms. In certain embodiments, the ring-forming atoms include one or more heteroatoms which are the same or different. In certain embodiments, the one or more heteroatoms included in the ring-forming atoms are selected from N, O, and S. The "heterocycle", "heterocyclyl" or "heterocyclylene " as disclosed herein is saturated or unsaturated. In certain embodiments, "heterocycle", "heterocyclyl", or "heterocyclylene" comprises a monocyclic ring, a bicyclic ring, or a polycyclic ring. In certain embodiments, "heterocycle", "heterocyclyl", or "heterocyclylene" is a 4- to 10-membered heterocycle, e.g., 4- to 7-membered heterocycle, 5- to 7-membered heterocycle. Preferably, in certain embodiments, heterocycle is a 4- to 10-membered heterocycle which may be optionally substituted, wherein the ring-forming atoms contain 1, 2, 3, 4, 5, or 6 heteroatoms selected from N, O, and S. More preferably, heterocycle is a 4- to 7-membered saturated heterocycle which may be optionally substituted, wherein the ring-forming atoms contain 1, 2, 3 or 4 heteroatoms selected from N, O and S; more preferably, heterocycle is a 5- to 7-membered (e.g., 5- to 6-membered) saturated heterocycle which may be optionally substituted, wherein the ring-forming atoms contain 1, 2 or 3 heteroatoms selected from N, O and S. Examples of heterocycle include, but are not limited to, azetidine, oxetanyl, tetrahydrofuran, pyrrolidine, imidazolidine, pyrazolidine, tetrahydropyran, piperidine, morpholine, thiomorpholine, piperazine, and preferably pyrrolidine, piperidine, piperazine, and morpholine. The heterocycle may be optionally substituted with one or more substituents, and for the substituents, reference is made to the definition for "optionally substituted" below. Unless expressly stated otherwise in this specification, heterocycle, heterocyclyl, or heterocyclylene are optionally substituted.

As used herein, the terms "aryl" and "aromatic ring" refer to an all-carbon monocyclic or fused polycyclic aromatic group having a conjugated $\pi$-electron system. For example, as used herein, the terms "$C_{6-10}$ aryl(ene)" and "$C_{6-10}$ aromatic ring" mean an aromatic group containing 6 to 10 carbon atoms, such as phenyl (benzene ring) or naphthyl (naphthalene ring). Unless explicitly stated otherwise in this specification, aryl and aromatic ring are optionally substituted.

As used herein, the term "heteroaryl" or " heteroaryl ring" refers to a monocyclic, bicyclic or tricyclic aromatic ring system containing at least one heteroatom selected from N, O and S, for example, having 5, 6, 8, 9, 10, 11, 12, 13 or 14 ring atoms, especially containing 1 or 2 or 3 or 4 or 5 or 6 or 9 or 10 carbon atoms, and may additionally be benzo-fused in each case. For example, heteroaryl or heteroaryl ring may be selected from thienyl (ring), furyl (ring), pyrrolyl (ring), oxazolyl (ring), thiazolyl (ring), imidazolyl (ring), pyrazolyl (ring), isoxazolyl (ring), isothiazolyl (ring), oxadiazolyl (ring), triazolyl (ring), thiadiazolyl (ring), and the like, and the benzo derivatives thereof; or pyridyl (ring), pyridazinyl (ring), pyrimidinyl (ring), pyrazinyl (ring), triazinyl (ring), and the like, and the benzo derivatives thereof. Unless expressly stated otherwise in this specification, heteroaryl or heteroaryl ring is optionally substituted.

As used herein, the term "optionally substituted" means that one or more hydrogen atoms attached to an atom or group are independently unsubstituted or substituted with one or more (e.g., 1, 2, 3, or 4) substituents. The substituents are independently selected from, but are not limit to, deuterium (D), tritium (T), halogen, -OH, mercapto, cyano, $-CD_3$ , $C_1-C_6$ alkyl (preferably $C_1-C_3$ alkyl), $C_2-C_6$ alkenyl, $C_2-C_6$ alkynyl, cycloalkyl (preferably $C_3-C_8$ cycloalkyl), aryl, heterocyclyl (preferably 3- to 8-membered heterocyclyl), heteroaryl, aryl$C_1-C_6$ alkyl-, heteroaryl$C_1-C_6$ alkyl, $C_1-C_6$ haloalkyl, $-OC_1-C_6$ alkyl (preferably $-OC_1-C_3$ alkyl), $-OC_2-C_6$ alkenyl, $OC_1-C_6$ alkylphenyl, $C_1-C_6$ alkyl-OH (preferably $C_1-C_4$ alkyl-OH), $C_1-C_6$ alkyl-SH, $C_1-C_6$ alkyl-O-$C_1-C_6$ alkyl, $OC_1-C_6$ haloalkyl, $NH_2$ , $C_1-C_6$ alkyl-$NH_2$ (preferably $C_1-C_3$ alkyl-$NH_2$), -N($C_1-C_6$

alkyl)$_2$ (preferably -N($C_1$-$C_3$ alkyl)$_2$), -NH($C_1$-$C_6$ alkyl) (preferably -NH($C_1$-$C_3$ alkyl)), -N($C_1$-$C_6$ alkyl)($C_1$-$C_6$ alkylphenyl), -NH($C_1$-$C_6$ alkylphenyl), nitro, -C(O)-OH, -C(O)O$C_1$-$C_6$ alkyl (preferably -C(O)O$C_1$-$C_3$ alkyl), -CONRiRii (wherein Ri and Rii are H, D and $C_1$-$C_6$ alkyl, preferably $C_1$-$C_3$ alkyl), -NHC(O)($C_1$-$C_6$ alkyl), -NHC(O)(phenyl), -N($C_1$-$C_6$ alkyl)C(O)($C_1$-$C_6$ alkyl), -N($C_1$-$C_6$ alkyl)C(O)(phenyl), -C(O)$C_1$-$C_6$ alkyl, -C(O)heteroaryl (preferably -C(O)-5- to 7-membered heteroaryl), -C(O)$C_1$-$C_6$ alkylphenyl, -C(O)$C_1$-$C_6$ haloalkyl, -OC(O)$C_1$-$C_6$ alkyl (preferably -OC(O)$C_1$-$C_3$ alkyl), -S(O)$_2$-$C_1$-$C_6$ alkyl, -S(O)-$C_1$-$C_6$ alkyl, -S(O)$_2$-phenyl, -S(O)$_2$-$C_1$-$C_6$ haloalkyl, -S(O)$_2$NH$_2$, -S(O)$_2$NH ($C_1$-$C_6$ alkyl), -S(O)$_2$NH(phenyl), -NHS(O)$_2$($C_1$-$C_6$ alkyl), -NHS(O)$_2$(phenyl) and -NHS(O)$_2$($C_1$-$C_6$ haloalkyl), wherein each of the alkyl, cycloalkyl, phenyl, aryl, heterocyclyl, and heteroaryl is optionally further substituted with one or more substituents selected from halogen, -OH, -NH$_2$, cycloalkyl, 3- to 8-membered heterocyclyl, $C_1$-$C_4$ alkyl, $C_1$-$C_4$ haloalkyl-, -O$C_1$-$C_4$ alkyl, -$C_1$-$C_4$ alkyl-OH, -$C_1$-$C_4$ alkyl-O-$C_1$-$C_4$ alkyl, -O$C_1$-$C_4$ haloalkyl, cyano, nitro, -C(O)-OH, -C(O)O$C_1$-$C_6$ alkyl, -CON($C_1$-$C_6$ alkyl)$_2$, -CONH($C_1$ -$C_6$ alkyl), -CONH$_2$, -NHC(O)($C_1$-$C_6$ alkyl), -NH($C_1$-$C_6$ alkyl)C(O)($C_1$-$C_6$ alkyl), -SO$_2$($C_1$-$C_6$ alkyl), -SO$_2$ (phenyl), -SO$_2$($C_1$-$C_6$ haloalkyl), -SO$_2$NH$_2$, -SO$_2$NH($C_1$-$C_6$ alkyl), -SO$_2$NH(phenyl), -NHSO$_2$($C_1$-$C_6$ alkyl), -NHSO$_2$ (phenyl), and -NHSO$_2$($C_1$-$C_6$ haloalkyl). When an atom or group is substituted with a plurality of substituents, the plurality of substituents may be the same or different.

In certain embodiments, the substituents may be independently selected from, but are not limit to, a halogen (such as a chlorine, bromine, fluorine, or iodine), a carboxylic acid (such as -C(=O)-OH), an oxygen (such as =O), a sulfur (such as =S), a hydroxyl (such as -OH), an ester group (such as -C(=O)ORiii or -OC(=O)Riii), an aldehyde group (such as -C(=O)H), a carbonyl (such as -C(=O)Riii, or represented by C=O), an acyl halide (such as -C(=O)X, wherein X is selected from bromine, fluorine, chlorine, or iodine), a carbonic ester group (such as -OC(=O)ORiii), an alkoxy (such as -ORiii), an acetal (such as -C(ORiii)$_2$Riii, wherein each ORiii is the same or different and is an alkoxy group), a phosphate (such as $P(=O)_4^{3-}$), a thiol (such as -SH), a sulfoxide (such as -S(=O)Riii), a sulfinic acid (such as -S(=O)OH), a sulfonic acid (such as -S(=O)$_2$OH), a thioaldehyde (such as -C(=S)H), a sulfate (such as $S(=O)_4^{2-}$), a sulfonyl (such as -S(=O)$_2$Riii), a sulfinyl (such as -S(=O)Riii), an amide (such as -C(=O)N(Riii)$_2$ or -N(Riii)C(=O)Riii), an azido (such as -N3), a nitro (such as -NO$_2$), a cyano (such as -CN), an isocyano (such as -NC), an acyloxy (such as -OC(=O)Riii), an amino (such as -N(Riii)$_2$, -N(Riii)H or -NH$_2$), a carbamoyl (such as -OC(=O)N(Riii)$_2$, -OC(=O)N(Riii)H or -OC(=O)NH$_2$), a sulfonamide (such as -S(=O)$_2$N(Riii)$_2$, -S(=O)$_2$N(Riii)H, -S(=O)$_2$NH$_2$, -N(Riii)S(=O)$_2$Riii, -N(H)S(=O)$_2$Riii, -N(Riii)S(=O)$_2$H or -N(H)S(=O)$_2$H), an alkyl, an alkenyl, an alkynyl, a cyclohydrocarbyl (such as cycloalkyl, cycloalkenyl or cycloalkynyl), a heterocyclohydrocarbyl (such as heterocycloalkyl containing one or more heteroatoms selected from S, N, and O, or heterocyclenyl containing one or more heteroatoms selected from S, N, and O), an aryl (such as phenyl, or a fused ring group), a heteroaryl (such as an 8- to 10-membered bicyclic heteroaryl containing 1 to 4 heteroatoms independently selected from nitrogen, oxygen, or sulfur), -C(=O)SRiii, -C(=N-CN)N(Riii)$_2$, -C(=N-O-CH$_3$)N(Riii)$_2$, -C(=N-SO$_2$-NH$_2$)N(Riii)$_2$, -C(=CH-NO$_2$) N(Riii)$_2$, -OC(=O)N(Riii)$_2$, -CHN(Riii)N(Riii)$_2$, -C(=O)N(Riii)ORiii, -N(Riii)$_2$C(=O)ORiii, -OP(=O)(ORiii)$_2$, -P(=O)(ORiii)$_2$, -N(ORiii)C(=O)Riii, -N(ORiii)S(=O)$_2$Riii, -N(ORiii)C(=O)ORiii, -N(ORiii)C(=O)N(Riii)$_2$, -N(ORiii)C(=S)N(Riii)$_2$, -N(ORiii) C(NRiii)N(Riii)$_2$, -N(ORiii)C(CHRiii)N(Riii)$_2$. In any of the foregoing, Riii is a hydrogen, or alkyl, or alkenyl, or alkynyl, or heteroalkyl, or heteroalkenyl, or heteroalkynyl, as defined herein. In some embodiments, Riii is a hydrogen, or $C_1$-$C_{12}$ alkyl, or $C_1$-$C_{12}$ alkenyl, or $C_1$-$C_{12}$ alkynyl, or $C_1$-$C_{12}$ heteroalkyl, or $C_1$-$C_{12}$ heteroalkenyl, or $C_1$-$C_{12}$ heteroalkynyl, as defined herein.

In certain embodiments, the substituent itself may be further substituted with, for example, one or more substituents as defined herein. For example, the $C_1$-$C_6$ alkyl as a substituent may be further substituted with one or more substituents as define herein.

As use herein, the term "pharmaceutically acceptable salt" is a basic salt of an organic or inorganic acid, including, but are not limited to, hydrochloride, hydrobromide, hydroiodide, sulfate, phosphate, acetate, trifluoroacetate, thiocyanate, maleate, hydroxymaleate, glutarate, methanesulfonate, ethanesulfonate, benzenesulfonate, p-toluenesulfonate, benzoate, salicylate, phenylacetate, cinnamate, lactate, malonate, pivalate, succinate, fumarate, malate, mandelate, tartrate, gallate, gluconate, laurate, palmitate, pectate, picrate, citrate, or combinations thereof.

As use herein, that term "halo" or "halogen" group is defined to include F, Cl, Br, or I.

A numerical range stated herein should be understood to encompass the boundary values and any and all subranges contained therein. For example, a range of "1 to 10" should be understood to include not only the explicitly recited values of 1 and 10, but also any individual values in the range of 1 to 10 (e.g., 2, 3, 4, 5, 6, 7, 8, and 9) and subranges (e.g., 1 to 2, 1.5 to 2.5, 1 to 3, 1.5 to 3.5, 2.5 to 4, 3 to 4.5, etc.). This principle also applies to ranges that use only one value as a minimum or maximum.

As use herein, that term "isomer" means different compounds having the same molecular formula. "Stereoisomers" are isomers that differ only in the way the atoms are arranged in space. "Atropisomers" are stereoisomers resulting from hindered rotation about a single bond. "Enantiomers" are a pair of stereoisomers that are non-overlapping mirror images of each other. A mixture of any ratio of a pair of enantiomers may be referred to as a "racemic" mixture. "Diastereoisomers" are stereoisomers that have at least two asymmetric atoms and are not mirror-images of one another. "Tautomers" refer to isomeric forms of a compound that are in equilibrium with each other. The concentration of the isomeric form will depend on the environment in which the compound is found and may vary, for example, depending on

whether the compound is a solid or in an organic or aqueous solution.

In certain embodiments, "stereoisomers" may also include the E and Z isomers, or mixtures thereof, as well as the *cis* and *trans* isomers, or mixtures thereof.

Nucleic acids and/or polynucleotides useful in the present disclosure include a coding region encoding a polypeptide of interest, a 5'-UTR at the 5'-end of the coding region, a 3'-UTR at the 3'-end of the coding region. In some embodiments, the nucleic acid or polynucleotide further comprises at least one of a polyadenylation region and a Kozak sequence. In some embodiments, the nucleic acid or polynucleotide (e.g., mRNA) may also include a 5' cap structure. Any region of a nucleic acid may include one or more alternative nucleosides, such as 5-substituted uridine (e.g., 5-methoxyuridine), 1-substituted pseudouridine (e.g., 1-methyl-pseudouridine or 1-ethyl-pseudouridine), and/or 5-substituted cytidine (e.g., 5-methyl-cytidine).

The term "5'-UTR" or "5'-untranslated region" may be a RNA sequence in an mRNA that is located upstream of the coding sequence and is not translated into protein. The 5'-UTR in a gene typically begins at the transcription start site and ends at a nucleotide upstream of the translation start codon of the coding sequence. The 5' -UTR may contain an element that controls gene expression, such as a ribosome binding site, a 5' -terminal oligopyrimidine tract, and a translation initiation signal such as a Kozak sequence. The mRNA can be post-transcriptionally modified by the addition of a 5' cap. Thus, the 5' -UTR in mature mRNA can also refer to the RNA sequence between the 5' cap and the start codon. As used herein, that term "3' untranslated region" or "3'-UTR" can be a RNA sequence in an mRNA that is located upstream of the code sequence and is not translated into protein. The 3' -UTR in the mRNA is located between the stop codon of the coding sequence and the poly(A) sequence, for example, beginning at a nucleotide downstream of the stop codon and ending at a nucleotide upstream of the poly(A) sequence. The sequence of the 5'-UTR and/or 3'-UTR may be homologous or heterologous to the sequence of the coding region. The 3'-UTR may comprise a 3'-UTR derived from at least one gene of albumin gene, alpha-globin gene, beta-globin gene, tyrosine hydroxylase gene, lipoxygenase gene, and collagen alpha gene.

As used herein, the term "polyadenylation region", "poly(A) sequence" and "poly(A)tail" are used interchangeably. A naturally occurring poly(A) sequence typically consists of adenine ribonucleotides. Preferably, a "polyadenylation region" refers to a poly(A) sequence comprising nucleotides or nucleotide segments other than adenine ribonucleotides. The poly(A) sequence is usually located at the 3' end of the mRNA, such as at the 3' end (downstream) of the 3'-UTR. Poly-A region may have different lengths. In particular, in some embodiments, the poly-A region of the nucleic acid molecules of the present disclosure is at least 30 nucleotides in length; in some embodiments, the poly-A region of the nucleic acid molecules of the present disclosure is at least 80 nucleotides in length; and in some embodiments, the poly-A region of the nucleic acid molecules of the present disclosure is at least 100 nucleotides in length.

As used herein, the term "5' cap structure" is the 5' cap structure which is typically located at the 5' end of the mature mRNA. In some embodiments, the 5' cap structure is linked to the 5'-end of the mRNA by a 5'-5'-triphosphate bond. The 5' cap structure is typically formed from modified (e.g., methylated) ribonucleotides (especially from guanine nucleotide derivatives). For example, m7GpppN (cap 0, or "cap0", is a cap structure formed by the 5'-phosphate group of hnRNA interacting with the 5'-phosphate group of m7GTP under the action of guanylate transferase to form a 5',5'-phosphodiester bond), where N is the terminal 5' nucleotide of the nucleic acid carrying the 5'-cap structure. In some embodiments, the 5' cap structure includes, but is not limited to, cap 0, cap 1 (a cap structure formed by further methylation of the 2'-OH of the ribose on the first nucleotide of hnRNA on the basis of cap 0, or "cap 1"), cap 2 (a cap structure formed by further methylation of the 2'-OH of the ribose on the second nucleotide of hnRNA on the basis of cap 1, or "cap 2"), cap 4, cap 0 analog, cap 1 analog, cap 2 analog, or cap 4 analog.

<u>Amino lipid compound</u>

In one aspect, the present disclosure provides an amino lipid compound represented by the following formula (I):

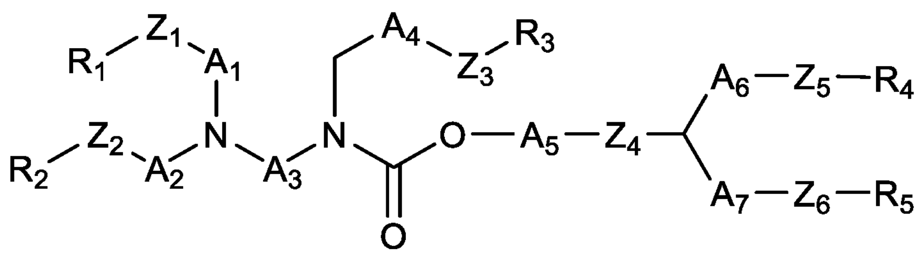

(I)

or a pharmaceutically acceptable salt or stereoisomer thereof,

wherein,

$Z_1$ , $Z_2$, $Z_3$, $Z_4$, $Z_5$, and $Z_6$ are each independently -CH(OH)-, -C=C-, -C≡C-, -O-, -C(=O)O-, -OC(=O)-, -N($R_6$)C(=O)-, -C(=O)N($R_6$)-, -N($R_6$)C(=O)N($R_6$)-, -OC(=O)N($R_6$)-, -N($R_6$)C(=O)O-, -C(=O)-, -C(=O)S-, -SC(=O)-, -S-S- or a bond;

$A_1$, $A_2$, $A_3$, $A_4$, $A_5$, $A_6$, and $A_7$ are each independently $C_1$-$C_{12}$ hydrocarbylene, cyclohydrocarbyl, phenyl, benzyl, heterocycle, or a bond;

$R_1$ and $R_2$ are each independently H or $C_1$-$C_{18}$ hydrocarbyl, or cyclohydrocarbyl, phenyl, benzyl, heterocycle; or $R_1$ and $R_2$, together with the nitrogen atom to which they are attached, form a 5- to 7-membered heterocycle;

$R_3$ is H or $C_1$-$C_{18}$ hydrocarbyl, or cyclohydrocarbyl, phenyl, benzyl, heterocycle;

$R_4$ and $R_5$ are each independently $C_1$-$C_{18}$ hydrocarbyl, or cyclohydrocarbyl, phenyl, benzyl, heterocycle;

$R_6$ is H or $C_1$-$C_{18}$ hydrocarbyl, or -OH, -O-optionally substituted $C_2$-$C_{18}$ hydrocarbyl, or -C=C-, -C≡C-optionally substituted $C_4$-$C_{18}$ hydrocarbyl;

preferably, $Z_5$ and $Z_6$ are each independently -O(C=O)-, -(C=O)O-, or a bond.

In some embodiments, the present disclosure provides an amino lipid compound of formula (I), or a pharmaceutically acceptable salt or stereoisomer thereof, as described above, wherein:
either $Z_5$ or $Z_6$ is -(C=O)O-, or each of them is -(C=O)O-.

In some embodiments, the present disclosure provides an amino lipid compound of formula (I), or a pharmaceutically acceptable salt or stereoisomer thereof, as described above, wherein:
either $Z_5$ or $Z_6$ is -O(C=O)-, or each of them is -O(C=O)-.

In some embodiments, the present disclosure provides an amino lipid compound of formula (I), or a pharmaceutically acceptable salt or stereoisomer thereof, as described above, wherein:
either $Z_1$ or $Z_2$ is a bond; or each of $Z_1$ and $Z_2$ is a bond.

In some embodiments, the present disclosure provides an amino lipid compound of formula (I), or a pharmaceutically acceptable salt or stereoisomer thereof, as described above, wherein:
either $A_1$ or $A_2$ is a bond, or each of them is a bond.

In some embodiments, the present disclosure provides an amino lipid compound of formula (I), or a pharmaceutically acceptable salt or stereoisomer thereof, as described above, wherein:
$Z_3$ is -(C=O)O- or -O(C=O)-; further, $R_4$ and $R_5$ are branched $C_3$-$C_{18}$ hydrocarbyl.

In another aspect, the present disclosure provides an amino lipid compound represented by the following formula (I):

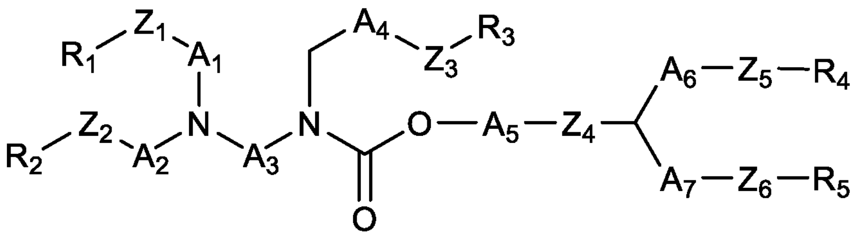

(I)

or a pharmaceutically acceptable salt or stereoisomer thereof,

wherein:

$Z_1$, $Z_2$, $Z_3$, $Z_4$, $Z_5$, and $Z_6$ are each independently -CH($OR_7$)-, -C=C-, -C≡C-, -O-, -C(=O)O-, -OC(=O)-, -N($R_6$)C(=O)-, -C(=O)N($R_6$)-, -N($R_6$)C(=O)N($R_6$)-, -OC(=O)N($R_6$)-, -N($R_6$)C(=O)O-, -C(=O)-, -C(=O)S-, -SC(=O)-, -S-S- or a bond;

$A_1$, $A_2$, $A_3$, $A_4$, $A_5$, $A_6$, and $A_7$ are each independently $C_1$-$C_{12}$ hydrocarbylene, $C_3$-$C_7$ cyclohydrocarbyl, phenyl, benzyl, 4- to 7-membered heterocycle, or a bond;

$R_1$ and $R_2$ are each independently H or $C_1$-$C_{18}$ hydrocarbyl, or $C_3$ -$C_7$ cyclohydrocarbyl, phenyl, benzyl, or 4- to 7-membered heterocycle; or $R_1$ and $R_2$ together with the nitrogen atom to which they are attached form a 4- to 7-membered heterocycle having said nitrogen atom and 0, 1, 2 or 3 additional heteroatoms independently selected from N, O and S in the ring, the heterocycle being optionally substituted with 1, 2, 3 or more substituents independently selected from $C_1$-$C_8$ alkyl, $C_1$-$C_8$ haloalkyl, -O-$C_1$-$C_8$ alkyl, -O-$C_1$-$C_8$ haloalkyl, halogen, OH, CN, nitro, $NH_2$, -NH($C_1$-$C_6$ alkyl), and -N($C_1$-$C_6$ alkyl)$_2$;

$R_3$ is H or $C_1$-$C_{18}$ hydrocarbyl, or $C_3$ -$C_7$ cyclohydrocarbyl, phenyl, benzyl, 4- to 7-membered heterocycle;

$R_4$ and $R_5$ are each independently $C_1$-$C_{18}$ hydrocarbyl, or $C_3$-$C_7$ cyclohydrocarbyl, phenyl, benzyl, or 4- to 7-membered heterocycle;

$R_6$ is H or $C_1$-$C_{18}$ hydrocarbyl, or -OH, -O-optionally substituted $C_2$-$C_{18}$ hydrocarbyl, or -C=C-, or -C≡C-optionally

substituted $C_4$-$C_{18}$ hydrocarbyl, or $C_1$-$C_{18}$ heterohydrocarbyl; and $R_7$ is H or $C_1$-$C_{12}$ hydrocarbyl.

In some embodiments, $R_6$ is H or $C_1$-$C_{18}$ hydrocarbyl, or -OH, -O-optionally substituted $C_2$-$C_{18}$ hydrocarbyl, or -C=C-, or -C≡C-optionally substituted $C_4$-$C_{18}$ hydrocarbyl.

In some embodiments, the present disclosure provides an amino lipid compound of formula (I), or a pharmaceutically acceptable salt or stereoisomer thereof, as described above, wherein $A_1$, $A_2$, $A_3$ , $A_4$, $A_5$, $A_6$ and $A_7$ are each independently $C_1$-$C_{12}$ alkylene, $C_2$-$C_{12}$ alkenylene having 1, 2, 3, 4 or more double bonds, $C_3$-$C_6$ cycloalkyl, phenyl, benzyl, 5- to 6-membered heterocycle or a bond. Preferably, $A_1$, $A_2$, $A_3$ , $A_4$, $A_5$, $A_6$ and $A_7$ are each independently $C_1$-$C_{12}$ alkylene, $C_2$-$C_{12}$ alkenylene having 1, 2, 3, 4, or more double bonds, or a bond. Preferably, $A_1$ and $A_2$ are each independently $C_1$-$C_6$ alkylene or a bond, more preferably $C_1$, $C_2$, $C_3$, $C_4$, $C_5$ alkylene or a bond, further preferably $C_1$ or $C_2$ alkylene or a bond. Preferably, $A_3$ is $C_1$-$C_6$ alkylene or a bond, more preferably $C_2$, $C_3$, $C_4$, or $C_5$ alkylene, further preferably $C_2$, $C_3$, or $C_4$ alkylene. Preferably, $A_4$ is $C_1$-$C_6$ alkylene or a bond, more preferably $C_1$, $C_2$, $C_3$, $C_4$, or $C_5$ alkylene or a bond, and further preferably $C_1$, $C_2$, $C_3$, or $C_4$ alkylene or a bond. Preferably, $A_5$ is $C_1$-$C_8$ alkylene or a bond, more preferably $C_1$, $C_2$, $C_3$, $C_4$, or $C_5$ alkylene or a bond, and further preferably $C_1$, $C_2$, $C_3$, or $C_4$ alkylene or a bond. Preferably, $A_6$ and $A_7$ are each independently $C_5$-$C_{12}$ alkylene, more preferably $C_6$-$C_{11}$ alkylene, and further preferably $C_7$, $C_8$, $C_9$, or $C_{10}$ alkylene.

In some embodiments as described above, $R_1$, $R_2$ are each independently H or $C_1$-$C_{18}$ alkyl, $C_2$-$C_{18}$ alkenyl having 1, 2, 3, 4 or more double bonds, or $C_3$-$C_6$ cycloalkyl, phenyl, benzyl, or 5- to 6-membered heterocycle, preferably $C_1$-$C_{18}$ alkyl, or $C_2$-$C_{18}$ alkenyl having 1, 2, 3, 4 or more double bonds; or

$R_1$ and $R_2$ together with the nitrogen atom to which they are attached form a 4- to 7-membered heterocycle having said nitrogen atom and 0, 1, 2 or 3 additional heteroatoms independently selected from N, O and S in the ring, the heterocycle being optionally substituted with 1, 2, 3 or more substituents independently selected from $C_1$-$C_8$ alkyl, $C_1$-$C_8$ haloalkyl, -O-$C_1$-$C_8$ alkyl, -O-$C_1$-$C_8$ haloalkyl, halogen, OH, CN, nitro, $NH_2$ , -NH($C_1$-$C_6$ alkyl), and -N($C_1$-$C_6$ alkyl)$_2$. In some preferred embodiments, $R_1$ and $R_2$ are each independently $C_1$-$C_6$ alkyl or a bond, preferably $C_1$, $C_2$, $C_3$, $C_4$, or $C_5$ alkyl or a bond, more preferably methyl, ethyl, n-propyl, or isopropyl. In other preferred embodiments, $R_1$ and $R_2$ together with the nitrogen atom to which they are attached form a 4- to 7-membered heterocycle, preferably 5- to 6-membered heterocycle, having said nitrogen atom in the ring.

In some embodiments as described above, $R_3$ is H, $C_1$-$C_{18}$ alkyl, $C_2$ -$C_{18}$ alkenyl having 1, 2, 3, 4 or more double bonds, or $C_3$-$C_6$ cycloalkyl, phenyl, benzyl, or 5- to 6-membered heterocycle. Preferably, $R_3$ is H, $C_1$-$C_{18}$ alkyl, or $C_2$-$C_{18}$ alkenyl having 1, 2, 3, 4 or more double bonds. Preferably, $R_3$ is $C_1$-$C_{16}$ alkyl, more preferably $C_1$-$C_{14}$ alkyl, and further preferably $C_1$, $C_2$, $C_3$, $C_4$, $C_5$, $C_6$, $C_7$, $C_8$, $C_9$ or $C_{10}$ alkyl.

In some embodiments as described above, $R_4$ and $R_5$ are each independently $C_1$-$C_{18}$ alkyl, $C_2$-$C_{18}$ alkenyl having 1, 2, 3, 4 or more double bonds, or $C_3$-$C_6$ cycloalkyl, phenyl, benzyl, or 5- to 6-membered heterocycle. Preferably, $R_4$ and $R_5$ are each independently $C_1$-$C_{18}$ alkyl, or $C_2$-$C_{18}$ alkenyl having 1, 2, 3, 4 or more double bonds. Preferably, $R_4$ and $R_5$ are each independently branched or straight $C_1$-$C_{18}$ alkyl, more preferably branched or straight $C_8$, $C_9$, $C_{10}$, $C_{11}$, $C_{12}$, $C_{13}$ or $C_{14}$ alkyl, further preferably branched $C_{12}$ or $C_{13}$ alkyl.

In some embodiments as described above, $R_6$ is H, $C_1$-$C_{18}$ alkyl, $C_2$-$C_{18}$ alkenyl having 1, 2, 3, 4 or more double bonds, -OH, -O-optionally substituted $C_2$-$C_{18}$ alkyl, -O-optionally substituted $C_2$-$C_{18}$ alkenyl having 1, 2, 3, 4 or more double bonds, -C=C-, -C=C-optionally substituted $C_4$-$C_{18}$ alkyl, or -C≡C-optionally substituted $C_4$-$C_{18}$ alkyl having 1, 2, 3, 4 or more double bonds, or $C_1$-$C_{18}$ heteroalkyl containing O, N or S, or $C_2$-$C_{18}$ heteroalkenyl containing O, N or S, or $C_2$-$C_{18}$ heteroalkynyl containing O, N, or S. Preferably, $R_6$ is H, $C_1$-$C_8$ alkyl, $C_2$-$C_{12}$ alkenyl having 1, 2 or 3 double bonds, -OH, -O-optionally substituted $C_2$-$C_{12}$ alkyl, -O-optionally substituted $C_2$-$C_{12}$ alkenyl having 1, 2 or 3 double bonds, -C=C-, -C=C-optionally substituted $C_4$-$C_{12}$ alkyl, or -C=C-optionally substituted $C_4$-$C_{12}$ alkyl having 1, 2 or 3 double bonds, or $C_1$-$C_8$ heteroalkyl containing O, N, or S, or $C_2$-$C_{12}$ heteroalkenyl containing O, N, or S, or $C_2$-$C_{12}$ heteroalkynyl containing O, N, or S.

In some embodiments as described above, $R_6$ is H, $C_1$-$C_{18}$ alkyl, $C_2$-$C_{18}$ alkenyl having 1, 2, 3, 4 or more double bonds, -OH, -O-optionally substituted $C_2$-$C_{18}$ alkyl, -O-optionally substituted $C_2$-$C_{18}$ alkenyl having 1, 2, 3, 4 or more double bonds, -C=C-, -C≡C-optionally substituted $C_4$-$C_{18}$ alkyl, or -C=C-optionally substituted $C_4$-$C_{18}$ alkyl having 1, 2, 3, 4 or more double bonds. Preferably, $R_6$ is H, $C_1$-$C_8$ alkyl, $C_2$-$C_{12}$ alkenyl having 1, 2 or 3 double bonds, -OH, -O-optionally substituted $C_2$-$C_{12}$ alkyl, -O-optionally substituted $C_2$-$C_{12}$ alkenyl having 1, 2 or 3 double bonds, -C=C-, -C=C-optionally substituted $C_4$-$C_{12}$ alkyl, or -C≡C-optionally substituted $C_4$-$C_{12}$ alkyl having 1, 2 or 3 double bonds.

In some embodiments as described above, $R_7$ is H, $C_1$-$C_{12}$ alkyl, or $C_2$-$C_{12}$ alkenyl having 1, 2, or 3 double bonds. Preferably, $R_7$ is H, or $C_1$-$C_{12}$ alkyl.

In some embodiments as described above, $Z_5$ and $Z_6$ are each independently -OC(=O)-, -C(=O)O-, or a bond.

In some embodiments as described above, at least one of $Z_1$ and $Z_2$ is a bond, preferably both $Z_1$ and $Z_2$ are bonds.

In some embodiments as described above, at least one of $A_1$ and $A_2$ is a bond, preferably both $A_1$ and $A_2$ are bonds.

In some embodiments as described above, $A_5$ is a bond.

In some embodiments as described above, at least one of $A_6$ and $A_7$ is a bond, preferably both $A_6$ and $A_7$ are bonds.

In some embodiments as described above, $Z_4$ is a bond.

In some embodiments as described above, $Z_3$ is -CH($OR_7$)-, -(C=O)O-, -O(C=O)-, or a bond.

In some preferred embodiments, the present disclosure provides an amino lipid compound of formula (I), or a pharmaceutically acceptable salt or stereoisomer thereof, as described above, wherein:

$Z_1$, $Z_2$, and $Z_4$ are each independently a bond;
$Z_3$ is -CH($OR_7$)-, -C(=O)O-, -OC(=O)-, or a bond;
$Z_5$ and $Z_6$ are each independently -C(=O)O- or -OC(=O)-;
$A_1$, $A_2$, and $A_5$ are each independently a bond;
$A_3$, $A_6$ and $A_7$ are each independently $C_1$-$C_{12}$ alkylene or $C_2$-$C_{12}$ alkenylene having 1, 2, 3, 4 or more double bonds;
$A_4$ is $C_1$-$C_{12}$ alkylene, $C_2$-$C_{12}$ alkenylene having 1, 2, 3, 4 or more double bonds, or a bond;
$R_1$ and $R_2$ are each independently $C_1$-$C_{18}$ alkyl, or $C_2$-$C_{18}$ alkenyl having 1, 2, 3, 4 or more double bonds; or $R_1$ and $R_2$ together with the nitrogen atom to which they are attached form a 4- to 7-membered heterocycle having said nitrogen atom and 0, 1, 2 or 3 additional heteroatoms independently selected from N, O and S in the ring, the heterocycle being optionally substituted with 1, 2, 3 or more substituents independently selected from $C_1$-$C_8$ alkyl, $C_1$-$C_8$ haloalkyl, -O-$C_1$-$C_8$ alkyl, -O-$C_1$-$C_8$ haloalkyl, halogen, OH, CN, nitro, $NH_2$ , -NH($C_1$-$C_6$ alkyl), and -N($C_1$-$C_6$ alkyl)$_2$;
$R_3$ is H, $C_1$-$C_{18}$ alkyl, or $C_2$-$C_{18}$ alkenyl having 1, 2, 3, 4 or more double bonds;
$R_4$ and $R_5$ are each independently $C_1$-$C_{18}$ alkyl, or $C_2$-$C_{18}$ alkenyl having 1, 2, 3, 4 or more double bonds; and
$R_7$ is H, $C_1$-$C_{12}$ alkyl, or $C_2$-$C_{12}$ alkenyl having 1, 2 or 3 double bonds, preferably H, or $C_1$-$C_{12}$ alkyl.

In some such embodiments, the present disclosure provides an amino lipid compound of formula (I), or a pharmaceutically acceptable salt or stereoisomer thereof, as described above, wherein:

$Z_3$ is a bond;
$A_4$ is a bond;
$R_1$ and $R_2$ are each independently $C_1$-$C_{18}$ alkyl, or $C_2$-$C_{18}$ alkenyl having 1, 2, 3, 4 or more double bonds; or $R_1$ and $R_2$ together with the nitrogen atom to which they are attached form a 4- to 7-membered heterocycle having said nitrogen atom and 0, 1 or 2 additional heteroatoms independently selected from N, O and S in the ring, the heterocycle being optionally substituted with 1, 2, or 3 substituents independently selected from $C_1$-$C_8$ alkyl, $C_1$-$C_8$ haloalkyl, -O-$C_1$-$C_8$ alkyl, -O-$C_1$-$C_8$ haloalkyl, halogen, OH, CN, nitro, $NH_2$ , -NH($C_1$-$C_6$ alkyl) and -N($C_1$-$C_6$ alkyl)$_2$; and
$R_3$ is $C_1$-$C_{18}$ alkyl, or $C_2$-$C_{18}$ alkenyl having 1, 2, 3, 4 or more double bonds, preferably $C_1$-$C_{18}$ alkyl.

In other such embodiments, the present disclosure provides an amino lipid compound of formula (I), or a pharmaceutically acceptable salt or stereoisomer thereof, as described above, wherein:

$Z_3$ is -CH($OR_7$)-; and
$R_1$ and $R_2$ are each independently $C_1$-$C_{18}$ alkyl, or $C_2$-$C_{18}$ alkenyl having 1, 2, 3, 4 or more double bonds, preferably $C_1$-$C_{18}$ alkyl.

In some embodiments as described above, $R_7$ is H. In some such embodiments, $A_4$ is a bond. In other embodiments, $A_4$ is $C_1$-$C_{12}$ alkylene, $C_2$-$C_{12}$ alkenylene having 1, 2, 3, 4 or more double bonds, preferably $C_1$-$C_{10}$ alkylene, more preferably $C_1$-$C_8$ alkylene, more preferably -$(CH_2)_{n4}$-, wherein n4 = 1, 2, 3, 4, 5, 6, 7 or 8. In some embodiments as described above, $R_3$ is $C_1$-$C_{18}$ alkyl, or $C_2$-$C_{18}$ alkenyl having 1, 2, 3, 4 or more double bonds, preferably $C_1$-$C_{18}$ alkyl.

In other embodiments as described above, $R_7$ is $C_1$-$C_{12}$ alkyl, or $C_2$-$C_{12}$ alkenyl having 1, 2, or 3 double bonds. Preferably, $R_7$ is $C_1$-$C_{12}$ alkyl. In some such embodiments, $R_3$ is H. In other embodiments, $R_3$ is $C_1$-$C_{18}$ alkyl, or $C_2$-$C_{18}$ alkenyl having 1, 2, 3, 4 or more double bonds. Preferably, $R_3$ is $C_1$-$C_{18}$ alkyl. In some embodiments as described above, $A_4$ is a bond. In other embodiments, $A_4$ is $C_1$-$C_{12}$ alkylene, or $C_2$-$C_{12}$ alkenylene having 1, 2, 3, 4 or more double bonds. Preferably, $A_4$ is $C_1$-$C_{10}$ alkylene, more preferably $C_1$-$C_8$ alkylene, more preferably -$(CH_2)_{n4}$-, wherein n4 = 1, 2, 3, 4, 5, 6, 7, or 8.

In other such embodiments, the present disclosure provides an amino lipid compound of formula (I), or a pharmaceutically acceptable salt or stereoisomer thereof, as described above, wherein:
$Z_3$ is -C(=O)O- or -OC(=O)-.

In some embodiments as described above, $Z_3$ is -C(=O)O-, wherein the C(=O) moiety is bonded to $A_4$; and $R_3$ is $C_1$-$C_{18}$ alkyl, or $C_2$-$C_{18}$ alkenyl having 1, 2, 3, 4 or more double bonds.

In some such embodiments, $A_4$ is a bond. In other embodiments, $A_4$ is $C_1$-$C_{12}$ alkylene, $C_2$-$C_{12}$ alkenylene having 1, 2, 3, 4 or more double bonds. Preferably, $A_4$ is $C_1$-$C_{10}$ alkylene, more preferably $C_1$-$C_8$ alkylene, more preferably $-(CH_2)_{n4}-$, wherein n4 = 1, 2, 3, 4, 5, 6, 7, or 8.

In some embodiments as described above, the hydrocarbylene or alkylene as defined for $A_1$, $A_2$, $A_3$, $A_4$, $A_5$, $A_6$, or $A_7$ is each independently $C_1$-$C_{10}$ alkylene. In other embodiments, the hydrocarbylene or alkenylene as defined for $A_1$, $A_2$, $A_3$, $A_4$, $A_5$, $A_6$, or $A_7$ is each independently $C_2$-$C_{10}$ alkenylene having 1, 2, 3, 4, or more double bonds.

In some embodiments as described above, $A_3$ is $C_1$-$C_8$ alkylene or $C_2$-$C_8$ alkenylene having 1 or 2 double bonds. Preferably, $A_3$ is $C_1$-$C_6$ alkylene or $C_2$-$C_6$ alkenylene having 1 double bond, more preferably $C_2$-$C_4$ alkylene or $C_2$-$C_4$ alkenylene having 1 double bond, and more preferably $-(CH_2)_2-$, $-(CH_2)_3-$ or $-(CH_2)_4-$.

In some embodiments as described above, $A_6$ and $A_7$ are each independently $C_1$-$C_{10}$ alkylene or $C_2$-$C_{10}$ alkenylene having 1, 2, 3, 4 or more double bonds. Preferably, $A_6$ and $A_7$ are each independently $C_5$-$C_{10}$ alkylene or $C_5$-$C_{10}$ alkenylene having 1, 2, 3, 4 or more double bonds, more preferably $C_6$-$C_9$ alkylene or $C_6$-$C_9$ alkenylene having 1, 2, 3, 4 or more double bonds, and more preferably $C_7$-$C_8$ alkylene, or $C_7$-$C_8$ alkenylene having 1, 2 or more double bonds.

In some embodiments as described above, the hydrocarbyl or alkyl as defined for $R_1$ or $R_2$ is $C_1$-$C_8$ alkyl, preferably $C_1$-$C_6$ alkyl, more preferably $C_1$-$C_4$ alkyl, and more preferably $C_1$-$C_2$ alkyl.

In other embodiments as described above, the heterocycle formed by $R_1$ and $R_2$ together with the nitrogen atom to which they are attached is 5- to 7-membered heterocycle having said nitrogen atom and 0, 1 or 2 additional heteroatoms independently selected from N, O and S in the ring, the heterocycle being optionally substituted with 1, 2, or 3 substituents independently selected from $C_1$-$C_6$ alkyl, $C_1$-$C_6$ haloalkyl, -O-$C_1$-$C_6$ alkyl, -O-$C_1$-$C_6$ haloalkyl, halogen, OH, CN, nitro, $NH_2$, -NH($C_1$-$C_6$ alkyl) and -N($C_1$-$C_6$ alkyl)$_2$. Preferably, the heterocycle is 5- to 6-membered heterocycle having said nitrogen atom and no additional heteroatom in the ring, the heterocycle being optionally substituted with 1, 2 or 3 substituents independently selected from $C_1$-$C_4$ alkyl, preferably $C_1$-$C_3$ alkyl. More preferably, the heterocycle is

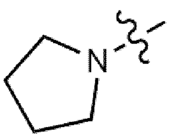 , 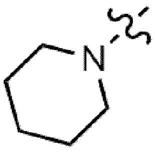 , or 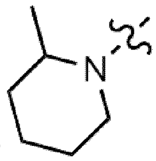 .

In some embodiments as described above, the hydrocarbyl or alkyl as defined for $R_3$ is $C_1$-$C_{12}$ alkyl, preferably $C_1$-$C_{10}$ alkyl, and more preferably $-(CH_2)_{n31}-CH_3$, or $-CH((CH_2)_{n32}-CH_3)-(CH_2)_{n33}-CH_3$, where n31 is 0, 1, 2, 3, 4, 5, 6, 7, or 8; n32 is 0, 1, 2, 3, 4, 5 or 6, preferably 0, 1, 2 or 3; and n33 is 0, 1, 2, 3, 4, 5, 6, 7 or 8, preferably 0, 1, 2, 3, 4, 5 or 6.

In some embodiments as described above, the hydrocarbyl or alkenyl as defined for $R_3$ is $C_2$-$C_{12}$ alkenyl having 1, 2, or 3 double bonds, preferably $C_2$-$C_{10}$ alkenyl having 1 double bond.

In some embodiments as described above, the hydrocarbyl or alkyl as defined for $R_7$ is $C_1$-$C_{10}$ alkyl, preferably $C_1$-$C_8$ alkyl, more preferably $-(CH_2)_{n7}-CH_3$, wherein n7 is 0, 1, 2, 3, 4, 5, 6, or 7.

In some embodiments as described above, the hydrocarbyl or alkenyl as defined for $R_7$ is $C_2$-$C_{10}$ alkenyl having 1, 2, or 3 double bonds, preferably $C_2$-$C_8$ alkenyl having 1 or 2 double bonds.

In some embodiments as described above, at least one of $Z_5$ and $Z_6$ is -C(=O)O-, preferably both $Z_5$ and $Z_6$ are -C(=O)O-, more preferably wherein the C(=O) moiety is attached to $A_6$ or $A_7$.

In some embodiments as described above, the hydrocarbyl or alkyl as defined for $R_4$ or $R_5$ is branched, preferably branched $C_3$-$C_{18}$ alkyl, more preferably branched $C_8$-$C_{18}$ alkyl, such as branched $C_{11}$-$C_{18}$ alkyl or branched $C_{13}$-$C_{15}$ alkyl, and preferably

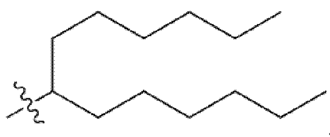 ,

or

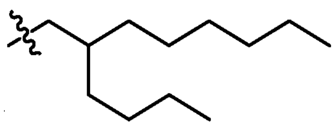 .

In some embodiments as described above, the hydrocarbyl or alkenyl as defined for $R_4$ or $R_5$ is branched,

preferably branched $C_3$-$C_{18}$ alkenyl, more preferably branched $C_8$-$C_{18}$ alkenyl, such as branched $C_{11}$-$C_{18}$ alkenyl or branched $C_{13}$-$C_{15}$ alkenyl, wherein said alkenyl groups have 1, 2, or 3 double bonds.

In some embodiments, the amino lipid compound represented by formula (I), or a pharmaceutically acceptable salt or stereoisomer thereof, the amino lipid compound having a structure represented by formula (II):

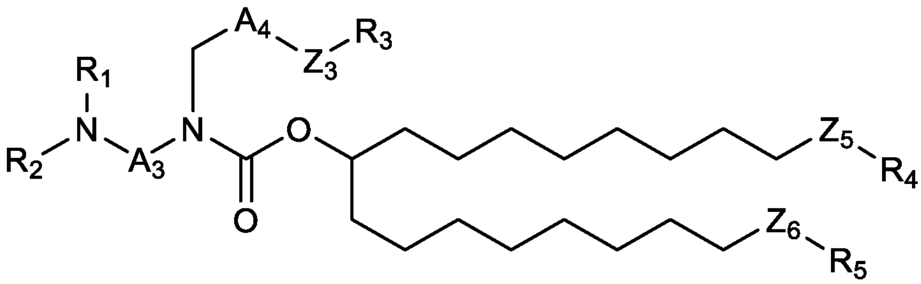

(II)

wherein,

$Z_3$, $Z_5$, and $Z_6$ are each independently -C(=O)O- or -OC(=O)-;
$A_3$ is $C_2$-$C_5$ alkylene;
$A_4$ is $C_1$-$C_5$ alkylene or a bond;
$R_1$, $R_2$ are each independently H or $C_1$-$C_3$ alkyl; or $R_1$ and $R_2$ together with the nitrogen atom to which they are attached form a 4- to 7-membered heterocycle having said nitrogen atom in the ring, the heterocycle being optionally substituted with 1, 2, 3 or more substituents independently selected from $C_1$-$C_8$ alkyl, $C_1$-$C_8$ haloalkyl, -O-$C_1$-$C_8$ alkyl, -O-$C_1$-$C_8$ haloalkyl, halogen, OH, CN, nitro, $NH_2$, -NH($C_1$-$C_6$ alkyl), and -N($C_1$-$C_6$ alkyl)$_2$;
$R_3$ is $C_1$-$C_{10}$ alkyl; and
$R_4$ and $R_5$ are each independently branched $C_1$-$C_{18}$ alkyl.

In some embodiments, the present disclosure provides an amino lipid compound represented by Formula (II), or a pharmaceutically acceptable salt or stereoisomer thereof, as described herein above, comprising one or more of the following features, where applicable.

In some embodiments, $Z_3$ is -C(=O)O-.

In some embodiments, $Z_5$ is -C(=O)O-.

In some embodiments, $Z_6$ is -C(=O)O-.

In some embodiments, $A_3$ is $C_3$-$C_4$ alkylene.

In some embodiments, $A_4$ is $C_1$-$C_2$ alkylene, $C_3$-$C_4$ alkylene, or a bond.

In some embodiments, $R_1$ is methyl, ethyl, n-propyl, or isopropyl.

In some embodiments, $R_2$ is methyl, ethyl, n-propyl, or isopropyl.

In some embodiments, $R_1$ and $R_2$ together with the nitrogen atom to which they are attached form 5- to 6-membered heterocycle having said nitrogen atom in the ring, the heterocycle being optionally substituted with 1, 2, 3 or more substituents independently selected from $C_1$-$C_8$ alkyl, $C_1$-$C_8$ haloalkyl, -O-$C_1$-$C_8$ alkyl, -O-$C_1$-$C_8$ haloalkyl, halogen, OH, CN, nitro, $NH_2$, -NH($C_1$-$C_6$ alkyl), and -N($C_1$-$C_6$ alkyl)$_2$, for example, substituted by methyl. In some embodiments, the heterocycle is

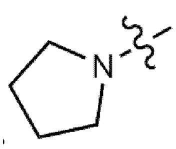 , 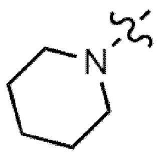 , or 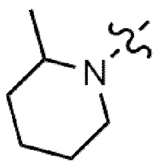 .

In some embodiments, $R_3$ is $C_1$, $C_2$, $C_3$, $C_4$, $C_5$, $C_6$, $C_7$, or $C_8$ alkyl, the alkyl being straight or branched.

In some embodiments, $R_4$ is branched $C_{13}$-$C_{15}$ alkyl. In some embodiments, $R_4$ is

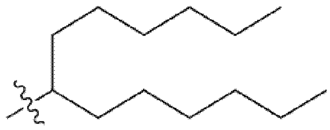 or 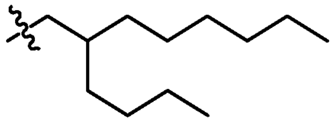 .

In some embodiments, $R_5$ is branched $C_{13}$-$C_{15}$ alkyl. In some embodiments, $R_5$ is

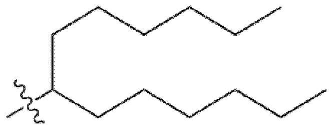 or 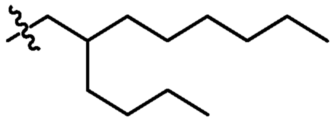 .

In various embodiments, the present disclosure provides an amino lipid compound of formula (I) or (II), or a pharmaceutically acceptable salt or stereoisomer thereof, as described above, wherein the amino lipid compound has one of the structures shown in Table 1 below.

Table 1

| No. | Structural formula | Molecular formula | Molecular weight |
|---|---|---|---|
| 108 | 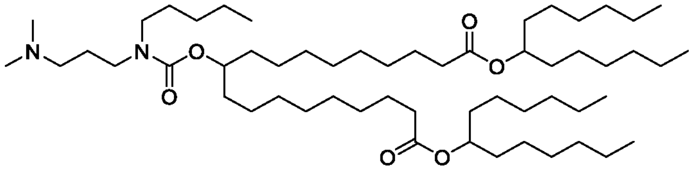 | $C_{56}H_{110}N_2O_6$ | 907.50 |
| 109 | 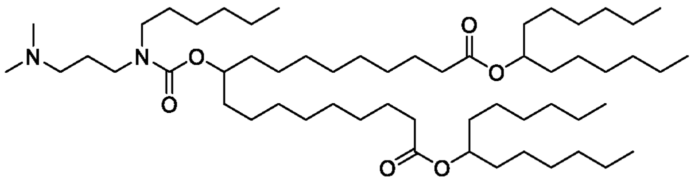 | $C_{57}H_{112}N_2O_6$ | 921.53 |
| 110 | 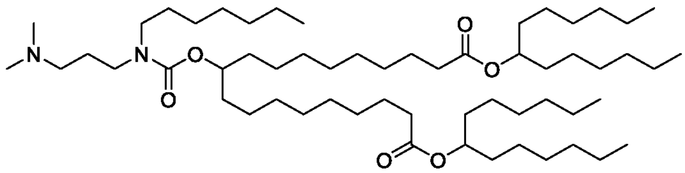 | $C_{58}H_{114}N_2O_6$ | 935.56 |
| 111 | 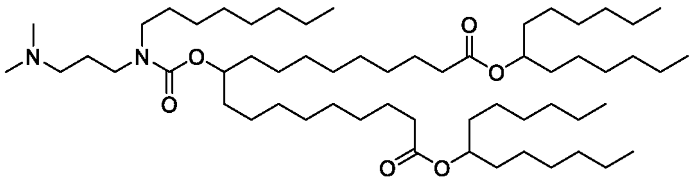 | $C_{59}H_{116}N_2O_6$ | 949.59 |
| 112 | 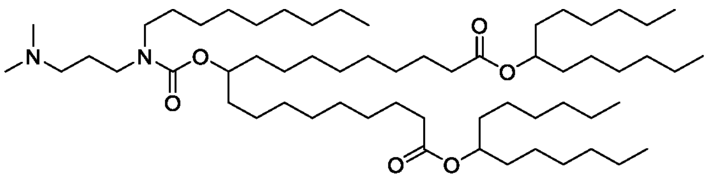 | $C_{60}H_{118}N_2O_6$ | 963.61 |
| 113 | 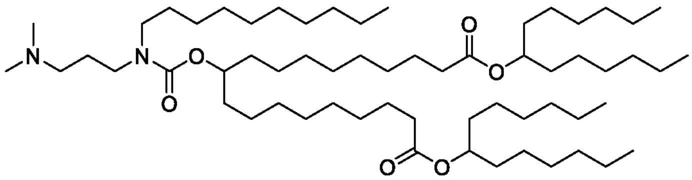 | $C_{61}H_{120}N_2O_6$ | 977.64 |

(continued)

| No. | Structural formula | Molecular formula | Molecular weight |
|---|---|---|---|
| 114 | 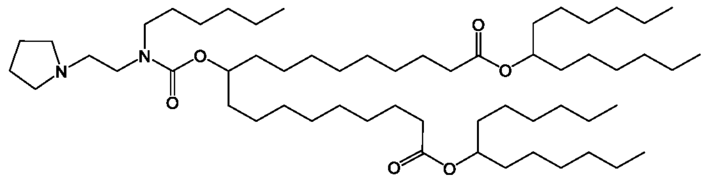 | $C_{58}H_{112}N_2O_6$ | 933.5 |
| 115 | 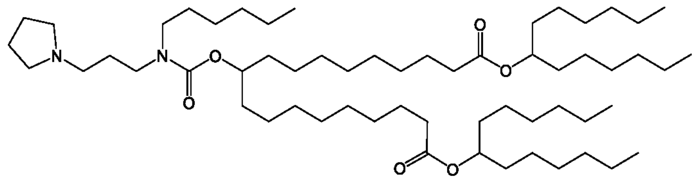 | $C_{59}H_{114}N_2O_6$ | 947.6 |
| 116 | 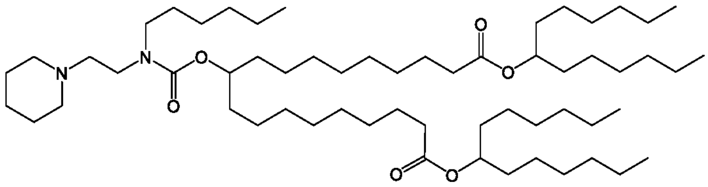 | $C_{59}H_{114}N_2O_6$ | 947.6 |
| 117 | 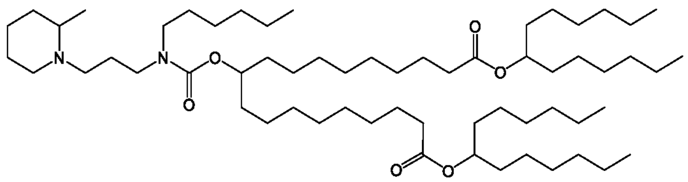 | $C_{61}H_{118}N_2O_6$ | 975.6 |
| 137 | 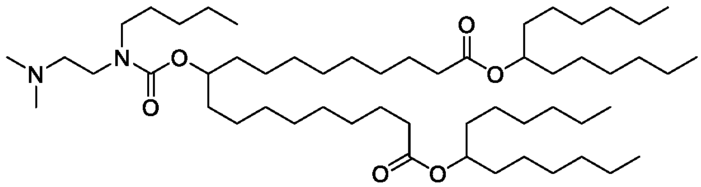 | $C_{55}H_{108}N_2O_6$ | 893.5 |
| 138 | 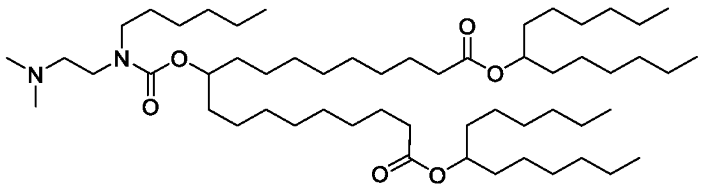 | $C_{56}H_{110}N_2O_6$ | 907.5 |
| 139 | 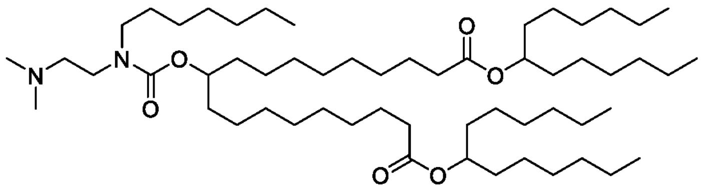 | $C_{57}H_{112}N_2O_6$ | 921.5 |
| 140 | 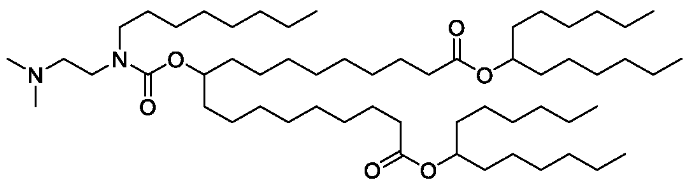 | $C_{58}H_{114}N_2O_6$ | 935.6 |

(continued)

| No. | Structural formula | Molecular formula | Molecular weight |
|---|---|---|---|
| 141 | 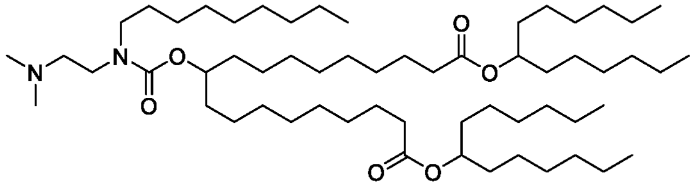 | $C_{59}H_{116}N_2O_6$ | 949.6 |
| 142 | 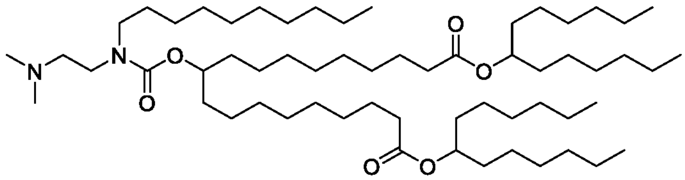 | $C_{60}H_{118}N_2O_6$ | 963.6 |
| 159 | 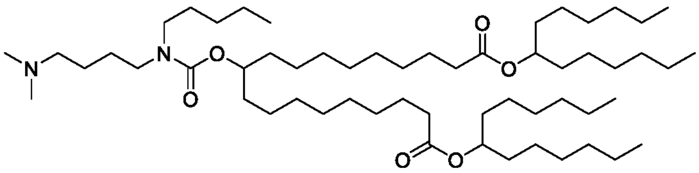 | $C_{57}H_{112}N_2O_6$ | 921.5 |
| 160 | 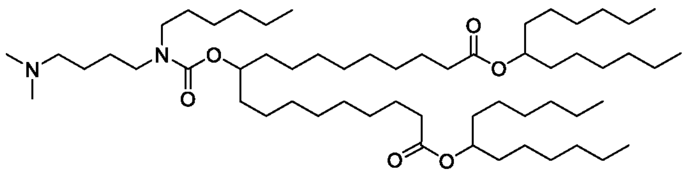 | $C_{58}H_{114}N_2O_6$ | 935.6 |
| 161 | 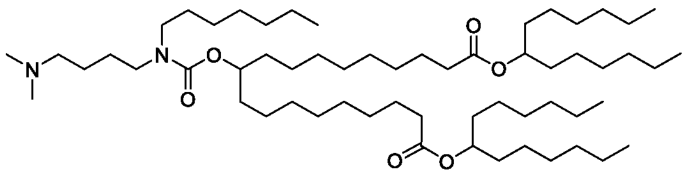 | $C_{59}H_{116}N_2O_6$ | 949.6 |
| 162 | 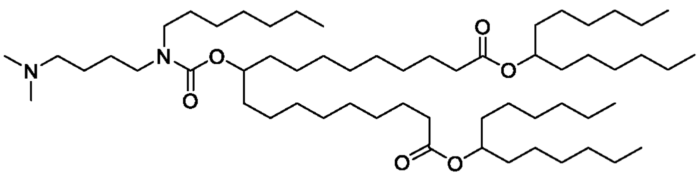 | $C_{60}H_{118}N_2O_6$ | 963.6 |
| 163 | 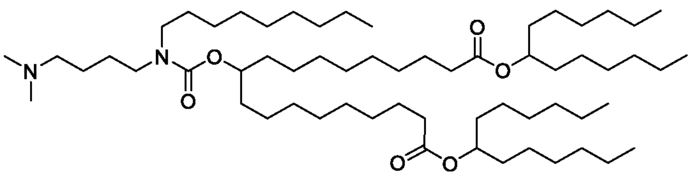 | $C_{61}H_{120}N_2O_6$ | 977.6 |
| 164 | 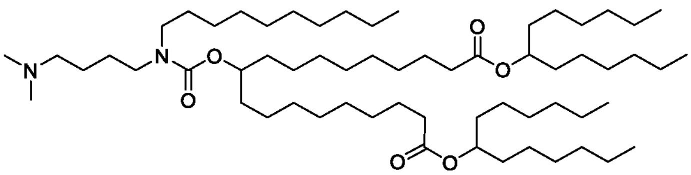 | $C_{62}H_{122}N_2O_6$ | 991.7 |

(continued)

| No. | Structural formula | Molecular formula | Molecular weight |
|---|---|---|---|
| 181 | 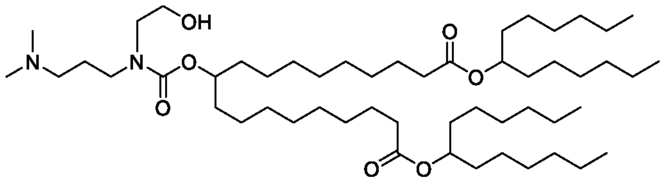 | $C_{53}H_{104}N_2O_7$ | 881.4 |
| 182 | 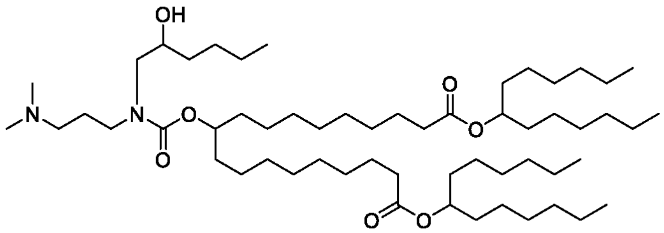 | $C_{57}H_{112}N_2O_7$ | 937.5 |
| 183 | 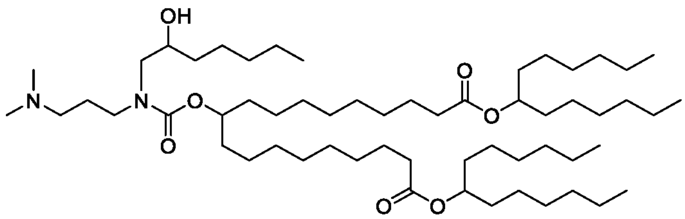 | $C_{58}H_{114}N_2O_7$ | 951.6 |
| 184 | 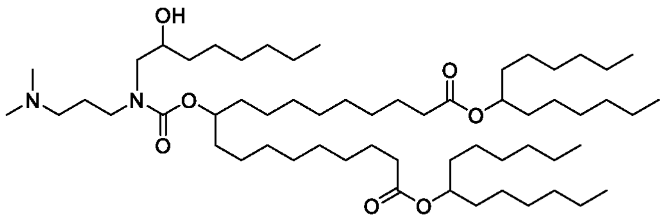 | $C_{59}H_{116}N_2O_7$ | 965.6 |
| 185 | 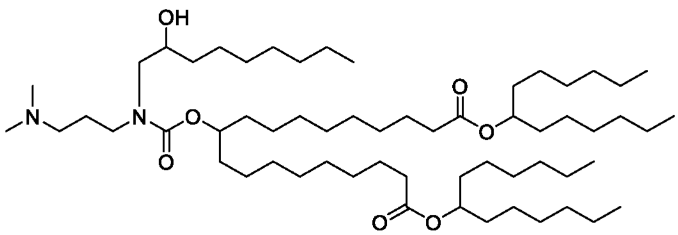 | $C_{60}H_{118}N_2O_7$ | 979.6 |
| 186 | 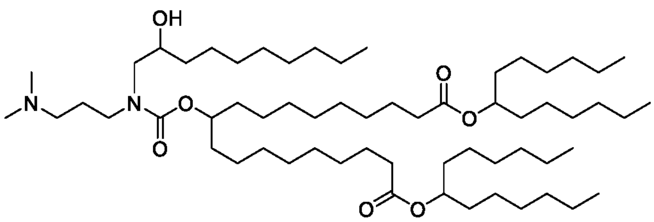 | $C_{61}H_{120}N_2O_7$ | 993.6 |
| 187 | 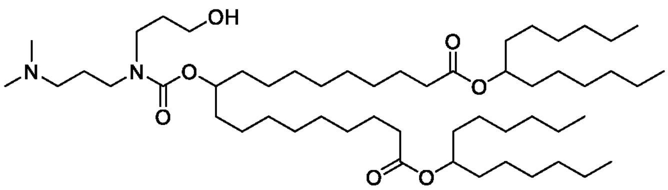 | $C_{54}H_{106}N_2O_7$ | 895.5 |

(continued)

| No. | Structural formula | Molecular formula | Molecular weight |
|---|---|---|---|
| 188 | 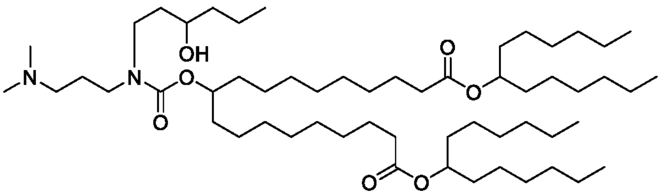 | $C_{57}H_{112}N_2O_7$ | 937.5 |
| 189 | 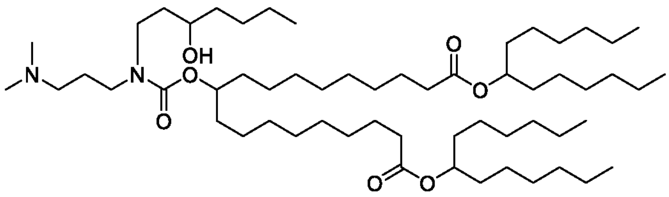 | $C_{58}H_{114}N_2O_7$ | 951.6 |
| 190 | 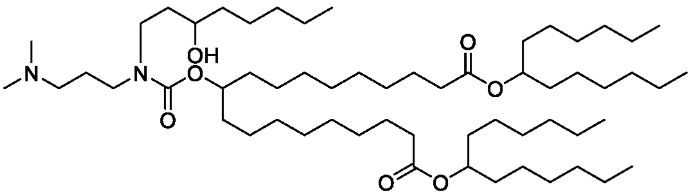 | $C_{59}H_{116}N_2O_7$ | 965.6 |
| 191 | 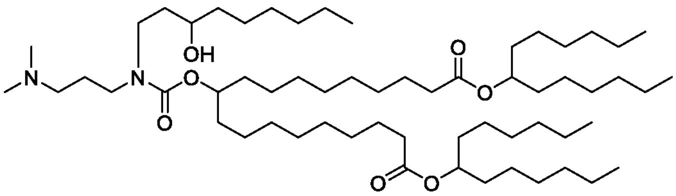 | $C_{60}H_{118}N_2O_7$ | 979.6 |
| 192 | 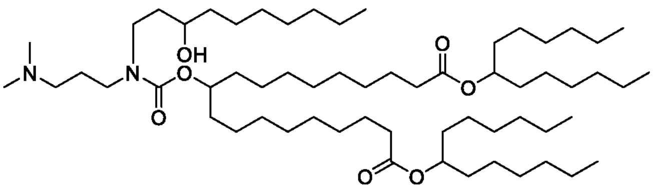 | $C_{61}H_{120}N_2O_7$ | 993.6 |
| 193 | 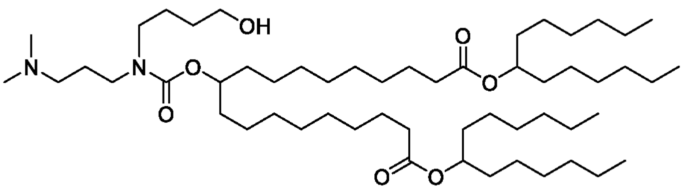 | $C_{55}H_{108}N_2O_7$ | 909.5 |
| 194 | 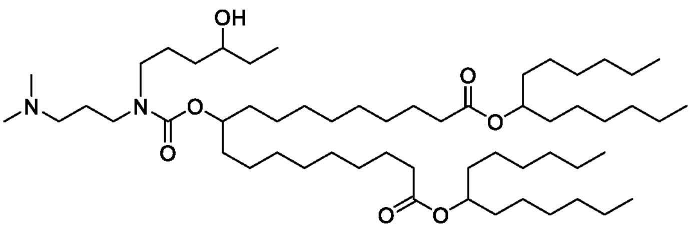 | $C_{57}H_{112}N_2O_7$ | 937.5 |

(continued)

| No. | Structural formula | Molecular formula | Molecular weight |
|---|---|---|---|
| 195 | 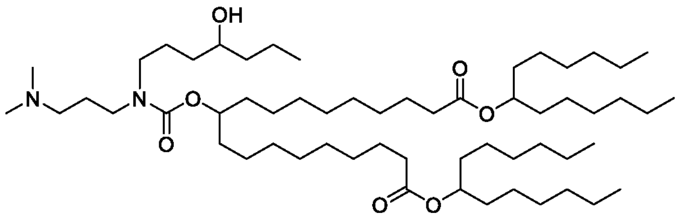 | $C_{58}H_{114}N_2O_7$ | 951.6 |
| 196 | 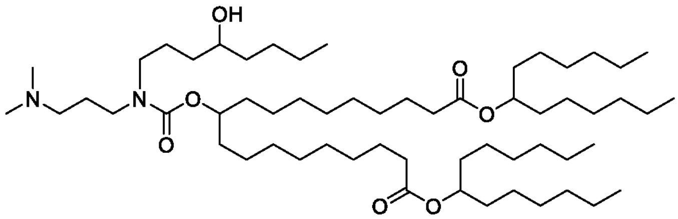 | $C_{59}H_{116}N_2O_7$ | 965.6 |
| 197 | 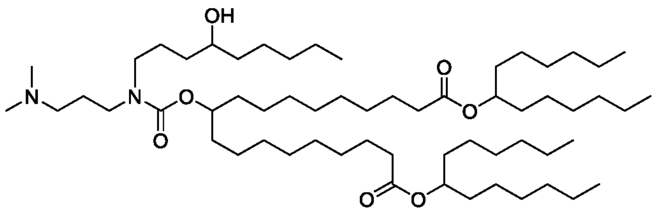 | $C_{60}H_{118}N_2O_7$ | 979.6 |
| 198 | 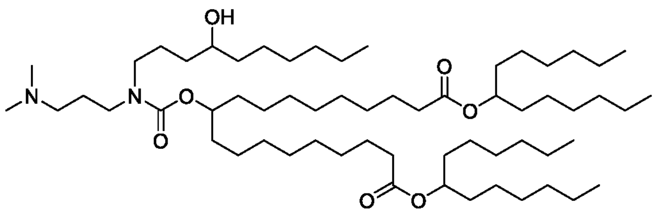 | $C_{61}H_{120}N_2O_7$ | 993.6 |
| 199 | 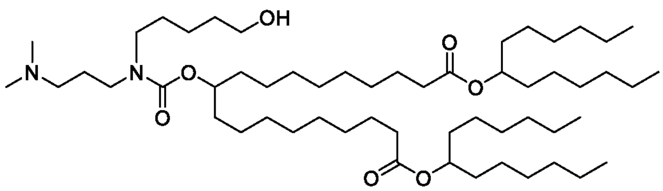 | $C_{56}H_{110}N_2O_7$ | 923.5 |
| 200 | 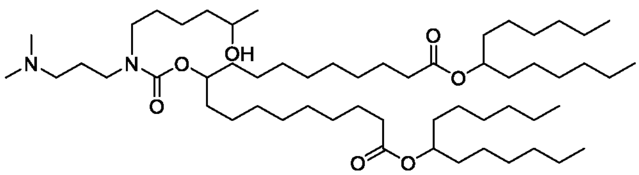 | $C_{57}H_{112}N_2O_7$ | 937.5 |
| 201 | 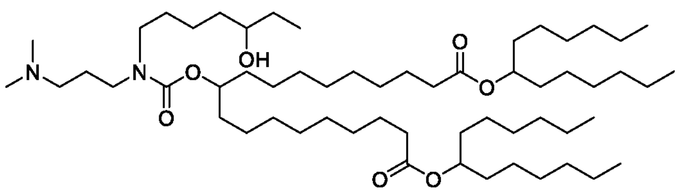 | $C_{58}H_{114}N_2O_7$ | 951.6 |

(continued)

| No. | Structural formula | Molecular formula | Molecular weight |
|---|---|---|---|
| 202 | 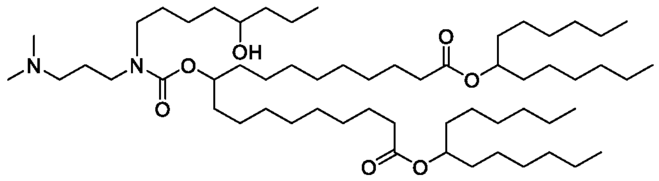 | $C_{59}H_{116}N_2O_7$ | 965.6 |
| 203 | 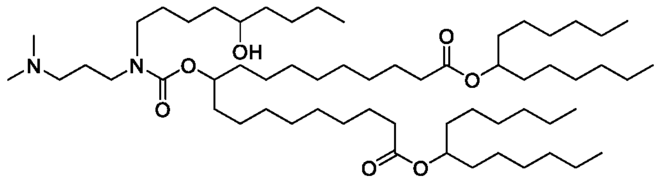 | $C_{60}H_{118}N_2O_7$ | 979.6 |
| 204 | 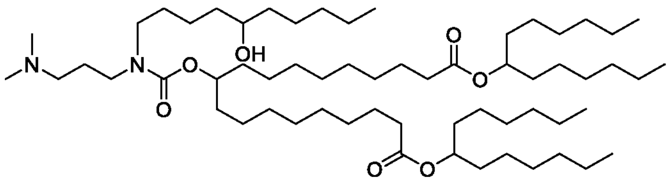 | $C_{61}H_{120}N_2O_7$ | 993.6 |
| 205 | 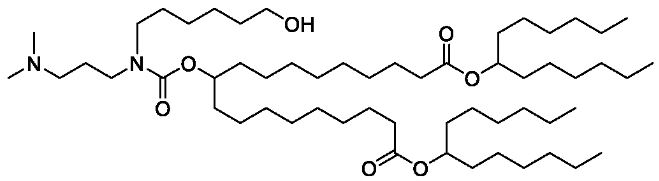 | $C_{57}H_{112}N_2O_7$ | 937.5 |
| 206 | 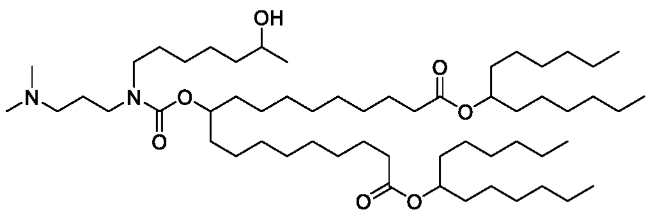 | $C_{58}H_{114}N_2O_7$ | 951.6 |
| 207 | 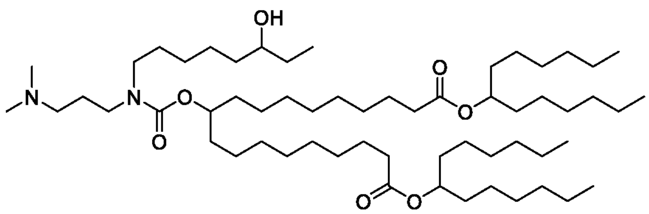 | $C_{59}H_{116}N_2O_7$ | 965.6 |
| 208 | 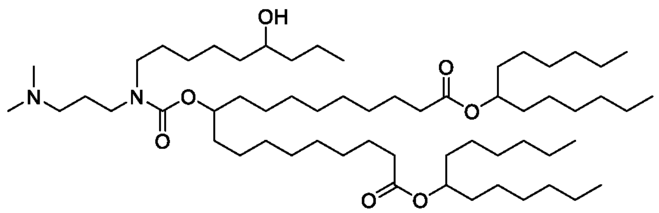 | $C_{60}H_{118}N_2O_7$ | 979.6 |
| 209 | 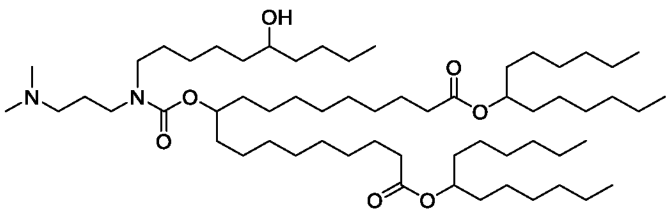 | $C_{61}H_{120}N_2O_7$ | 993.6 |

(continued)

| No. | Structural formula | Molecular formula | Molecular weight |
|---|---|---|---|
| 210 | 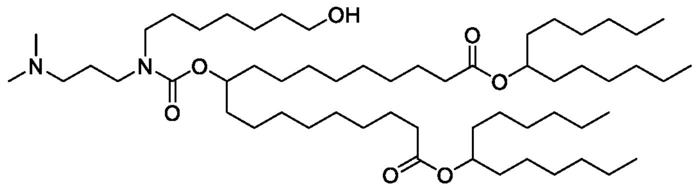 | $C_{58}H_{114}N_2O_7$ | 951.6 |
| 211 | 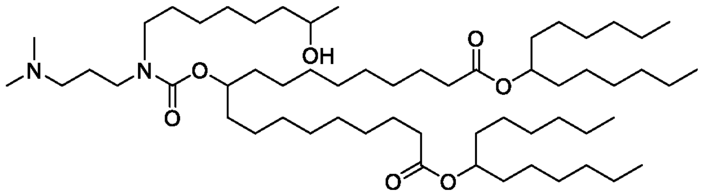 | $C_{59}H_{116}N_2O_7$ | 965.6 |
| 212 | 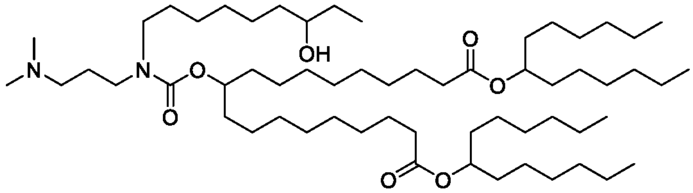 | $C_{60}H_{118}N_2O_7$ | 979.6 |
| 213 | 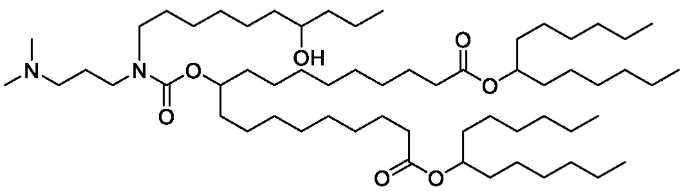 | $C_{61}H_{120}N_2O_7$ | 993.6 |
| 214 | 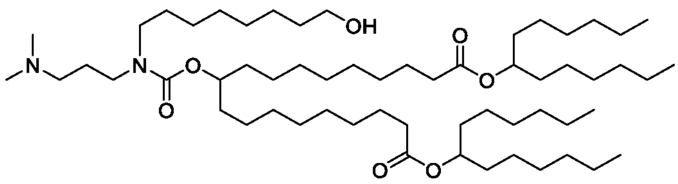 | $C_{59}H_{116}N_2O_7$ | 965.6 |
| 215 | 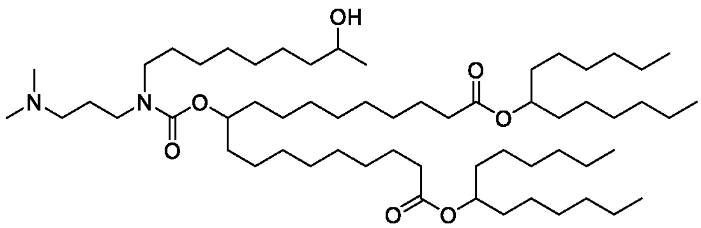 | $C_{60}H_{118}N_2O_7$ | 979.6 |
| 216 | 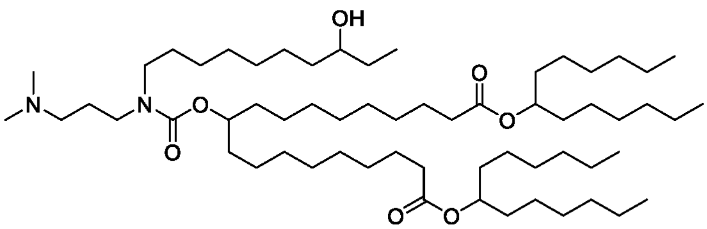 | $C_{61}H_{120}N_2O_7$ | 993.6 |

(continued)

| No. | Structural formula | Molecular formula | Molecular weight |
|---|---|---|---|
| 217 | 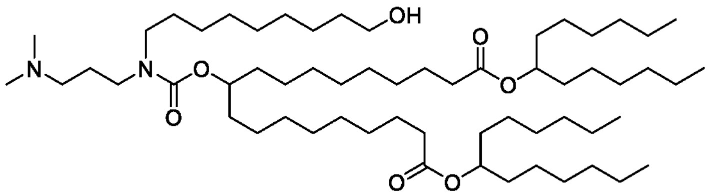 | $C_{60}H_{118}N_2O_7$ | 979.6 |
| 218 | 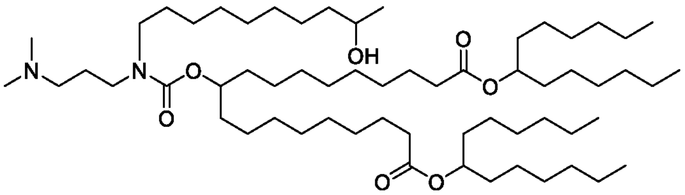 | $C_{61}H_{120}N_2O_7$ | 993.6 |
| 219 | 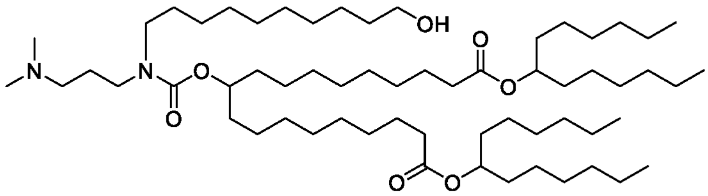 | $C_{61}H_{120}N_2O_7$ | 993.6 |
| 220 | 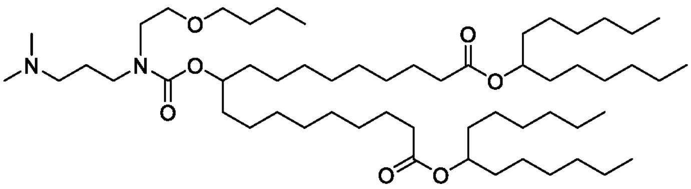 | $C_{57}H_{112}N_2O_7$ | 937.5 |
| 221 | 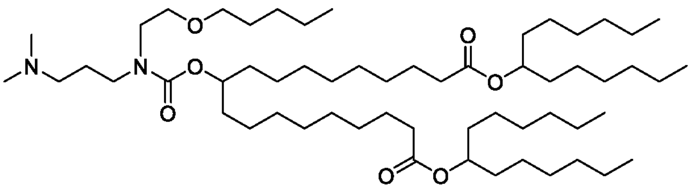 | $C_{58}H_{114}N_2O_7$ | 951.6 |
| 222 | 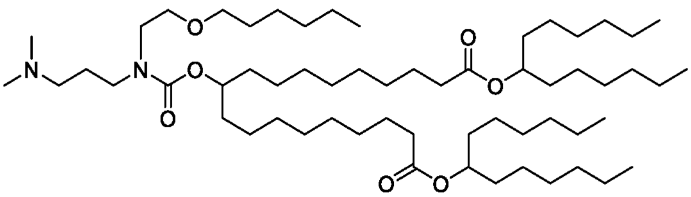 | $C_{59}H_{116}N_2O_7$ | 965.6 |
| 223 | 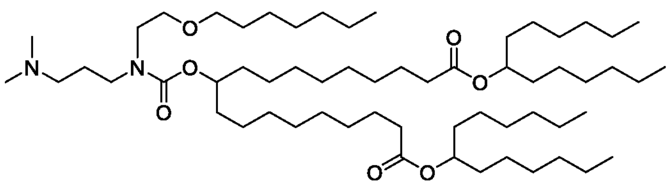 | $C_{60}H_{118}N_2O_7$ | 979.6 |
| 224 | 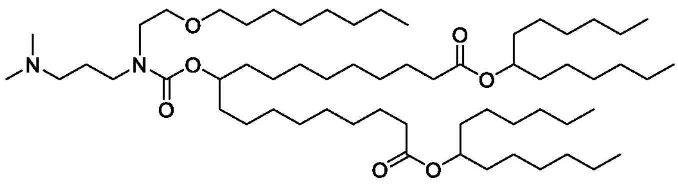 | $C_{61}H_{120}N_2O_7$ | 993.6 |

(continued)

| No. | Structural formula | Molecular formula | Molecular weight |
|---|---|---|---|
| 225 | 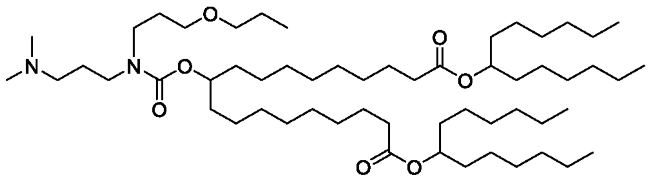 | $C_{57}H_{112}N_2O_7$ | 937.5 |
| 226 | 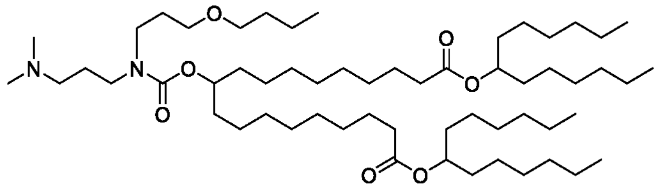 | $C_{58}H_{114}N_2O_7$ | 951.6 |
| 227 | 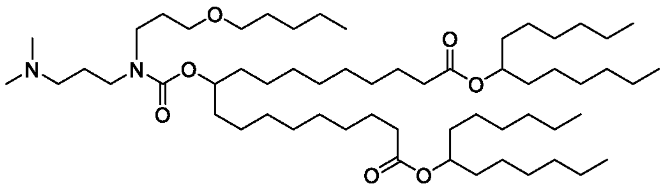 | $C_{59}H_{116}N_2O_7$ | 965.6 |
| 228 | 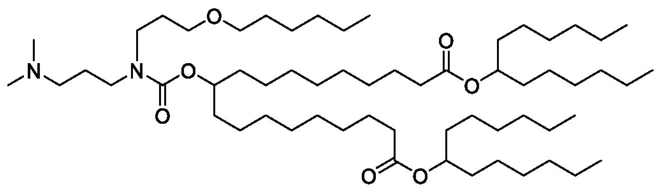 | $C_{60}H_{118}N_2O_7$ | 979.6 |
| 229 | 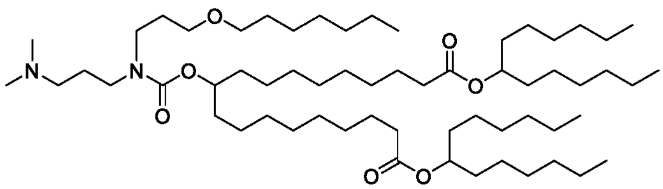 | $C_{61}H_{120}N_2O_7$ | 993.6 |
| 230 | 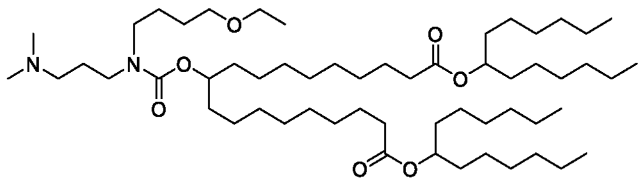 | $C_{57}H_{112}N_2O_7$ | 937.5 |
| 231 | 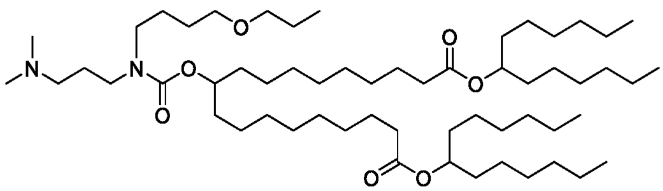 | $C_{58}H_{114}N_2O_7$ | 951.6 |
| 232 | 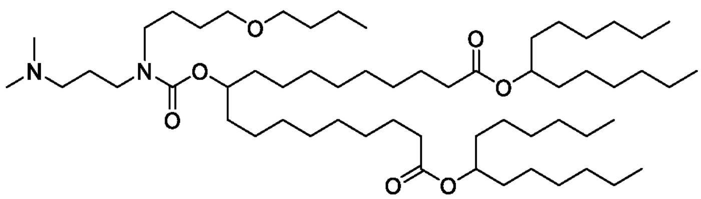 | $C_{59}H_{116}N_2O_7$ | 965.6 |

(continued)

| No. | Structural formula | Molecular formula | Molecular weight |
|---|---|---|---|
| 233 | 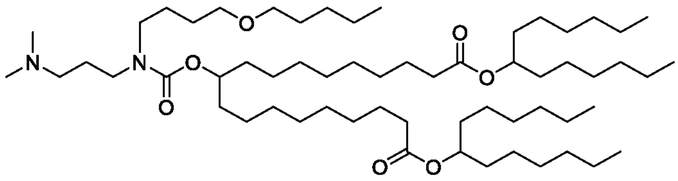 | $C_{60}H_{118}N_2O_7$ | 979.6 |
| 234 | 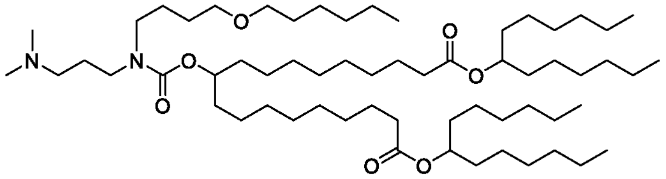 | $C_{61}H_{120}N_2O_7$ | 993.6 |
| 235 | 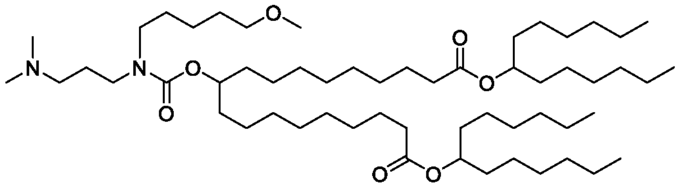 | $C_{57}H_{112}N_2O_7$ | 937.5 |
| 236 | 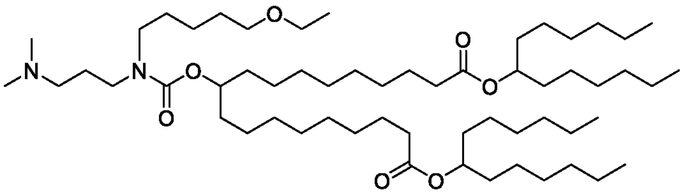 | $C_{58}H_{114}N_2O_7$ | 951.6 |
| 237 | 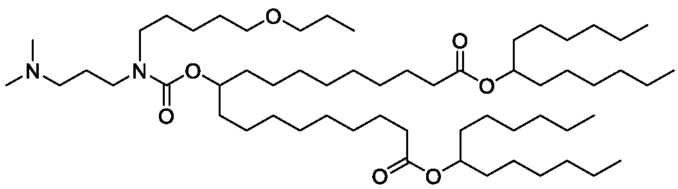 | $C_{59}H_{116}N_2O_7$ | 965.6 |
| 238 | 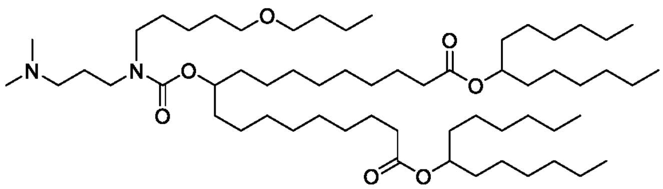 | $C_{60}H_{118}N_2O_7$ | 979.6 |
| 239 | 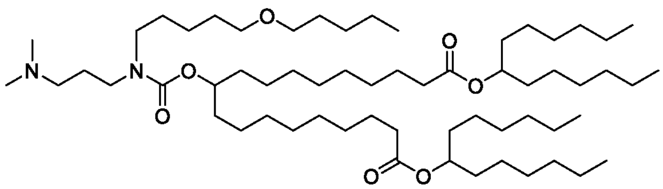 | $C_{61}H_{120}N_2O_7$ | 993.6 |
| 240 | 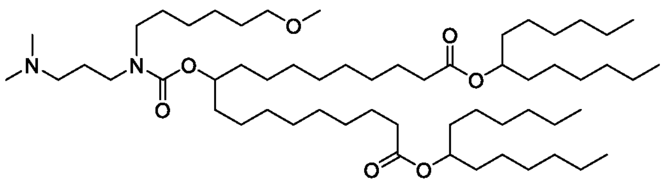 | $C_{58}H_{114}N_2O_7$ | 951.6 |

(continued)

| No. | Structural formula | Molecular formula | Molecular weight |
|---|---|---|---|
| 241 | 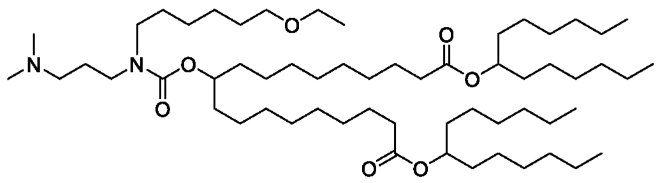 | $C_{59}H_{116}N_2O_7$ | 965.6 |
| 242 | 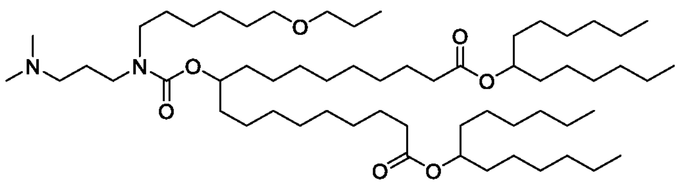 | $C_{60}H_{118}N_2O_7$ | 979.6 |
| 243 | 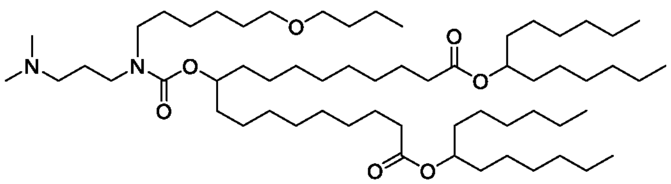 | $C_{61}H_{120}N_2O_7$ | 993.6 |
| 244 | 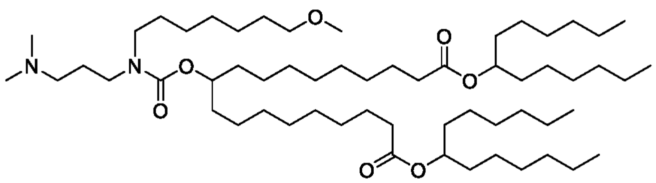 | $C_{59}H_{116}N_2O_7$ | 965.6 |
| 245 | 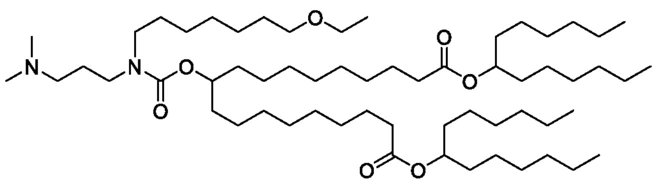 | $C_{60}H_{118}N_2O_7$ | 979.6 |
| 246 | 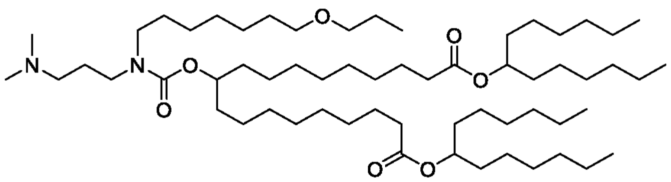 | $C_{61}H_{120}N_2O_7$ | 993.6 |
| 247 | 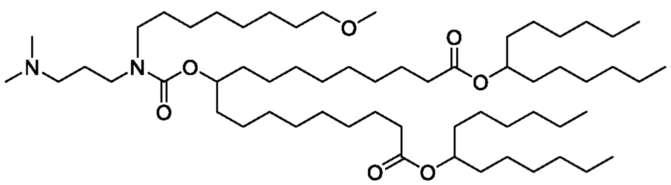 | $C_{60}H_{118}N_2O_7$ | 979.6 |
| 248 | 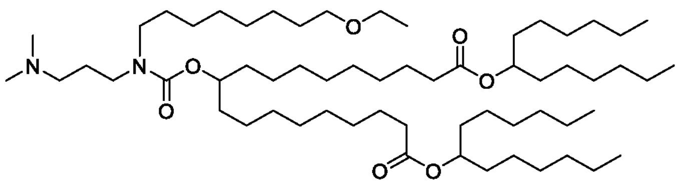 | $C_{61}H_{120}N_2O_7$ | 993.6 |

(continued)

| No. | Structural formula | Molecular formula | Molecular weight |
|---|---|---|---|
| 249 | 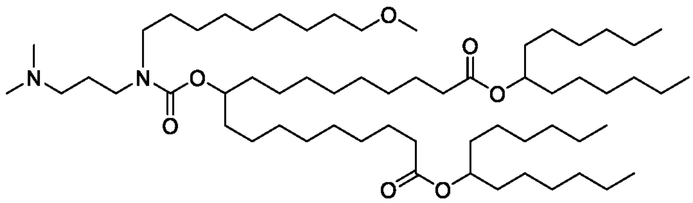 | $C_{61}H_{120}N_2O_7$ | 993.6 |
| 250 | 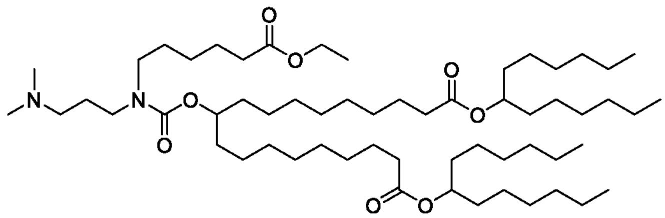 | $C_{59}H_{114}N_2O_8$ | 979.6 |
| 251 | 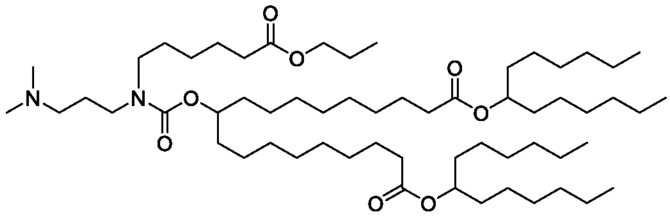 | $C_{60}H_{116}N_2O_8$ | 993.6 |
| 252 | 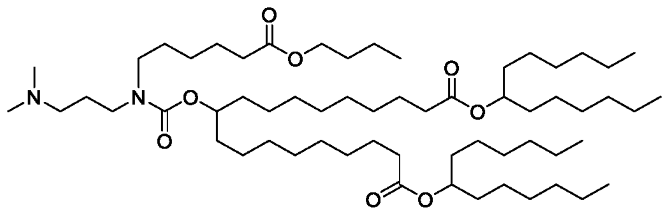 | $C_{61}H_{118}N_2O_8$ | 1007.6 |
| 253 | 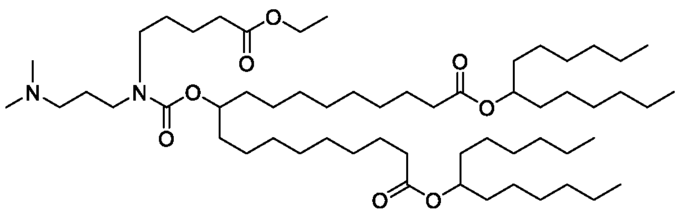 | $C_{58}H_{112}N_2O_8$ | 965.5 |
| 254 | 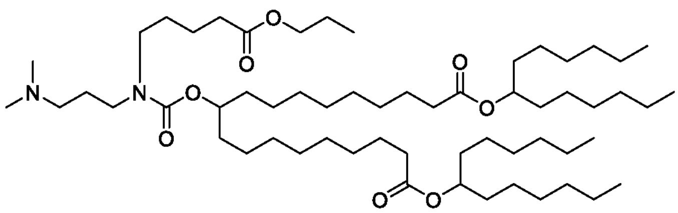 | $C_{59}H_{114}N_2O_8$ | 979.6 |
| 255 | 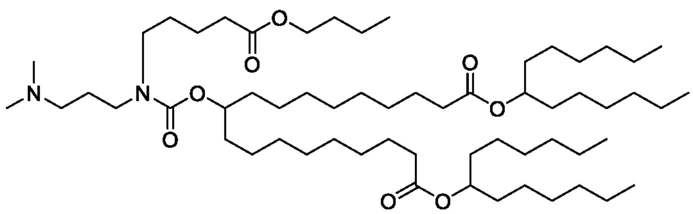 | $C_{60}H_{116}N_2O_8$ | 993.6 |

(continued)

| No. | Structural formula | Molecular formula | Molecular weight |
|---|---|---|---|
| 256 | 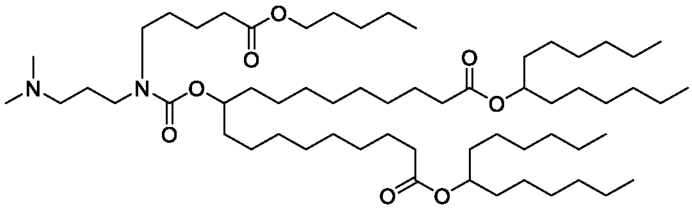 | $C_{61}H_{118}N_2O_8$ | 1007.6 |
| 257 | 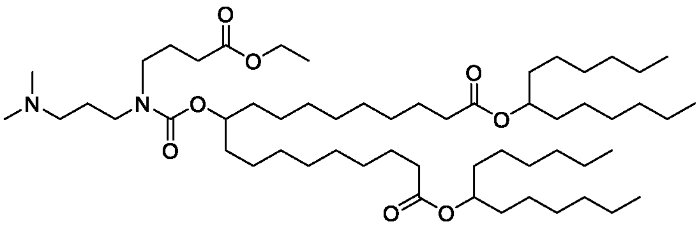 | $C_{57}H_{110}N_2O_8$ | 951.5 |
| 258 | 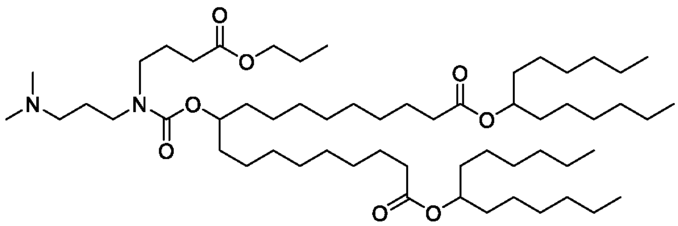 | $C_{58}H_{112}N_2O_8$ | 965.5 |
| 259 | 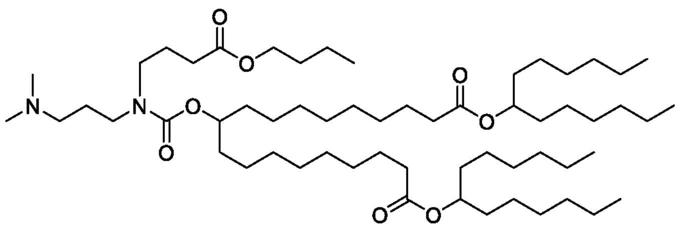 | $C_{59}H_{114}N_2O_8$ | 979.6 |
| 260 | 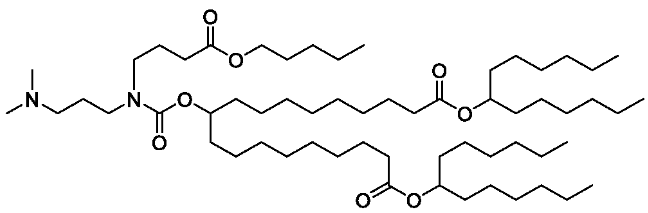 | $C_{60}H_{116}N_2O_8$ | 993.6 |
| 261 | 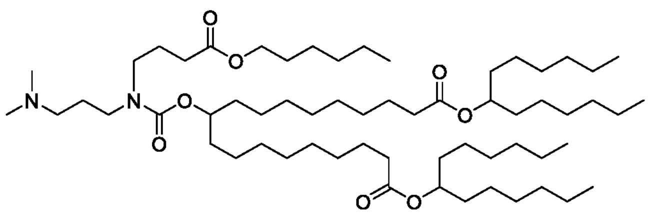 | $C_{61}H_{118}N_2O_8$ | 1007.6 |
| 262 | 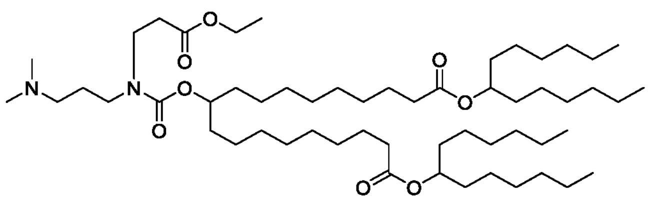 | $C_{56}H_{108}N_2O_8$ | 937.5 |

(continued)

| No. | Structural formula | Molecular formula | Molecular weight |
|---|---|---|---|
| 263 | 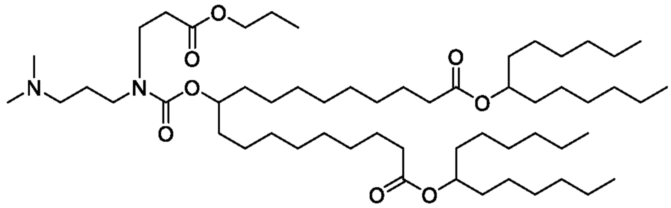 | $C_{57}H_{110}N_2O_8$ | 951.5 |
| 264 | 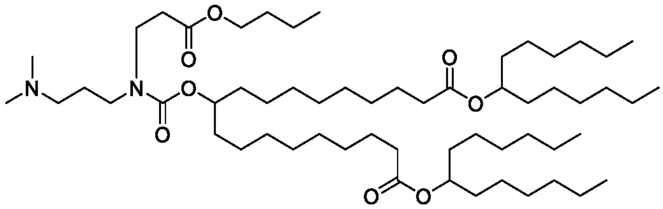 | $C_{58}H_{112}N_2O_8$ | 965.5 |
| 265 | 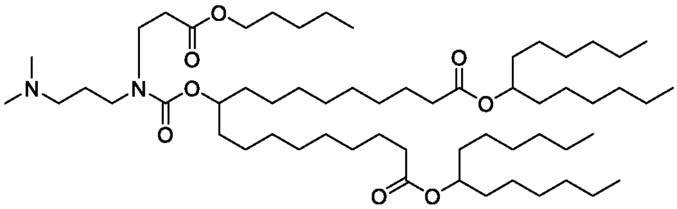 | $C_{59}H_{114}N_2O_8$ | 979.6 |
| 266 | 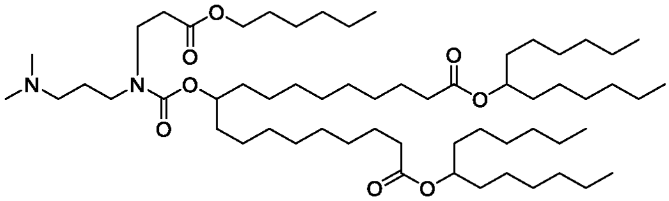 | $C_{60}H_{116}N_2O_8$ | 993.6 |
| 267 | 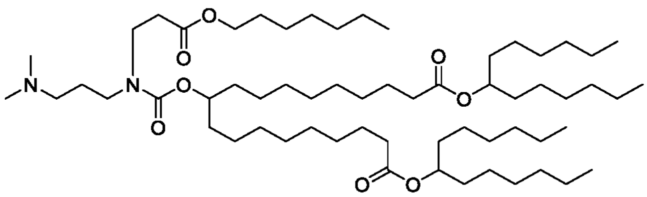 | $C_{61}H_{118}N_2O_8$ | 1007.6 |
| 268 | 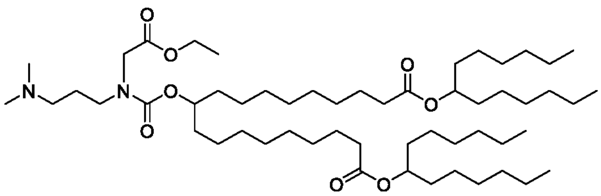 | $C_{55}H_{106}N_2O_8$ | 923.5 |
| 269 | 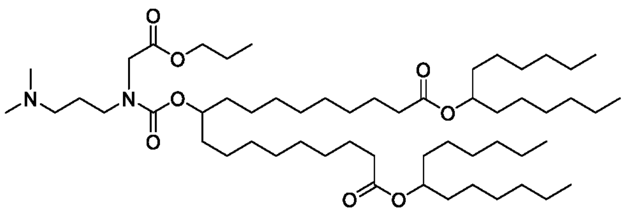 | $C_{56}H_{108}N_2O_8$ | 937.5 |

(continued)

| No. | Structural formula | Molecular formula | Molecular weight |
|---|---|---|---|
| 270 | 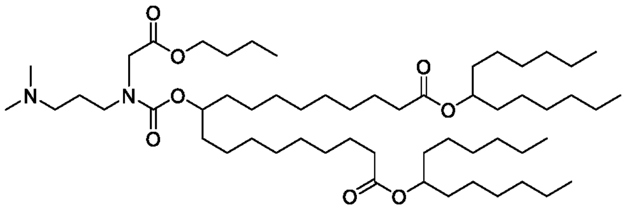 | $C_{57}H_{110}N_2O_8$ | 951.5 |
| 271 | 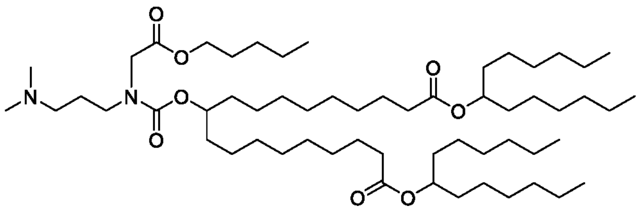 | $C_{58}H_{112}N_2O_8$ | 965.5 |
| 272 | 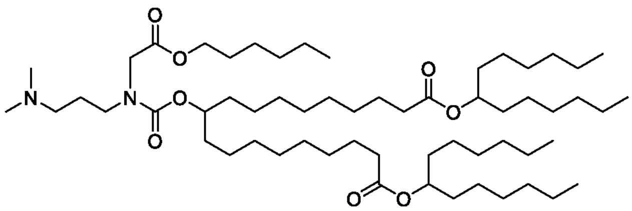 | $C_{59}H_{114}N_2O_8$ | 979.6 |
| 273 | 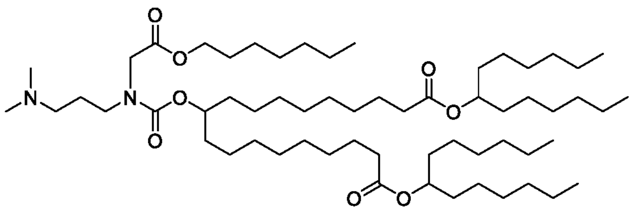 | $C_{60}H_{116}N_2O_8$ | 993.6 |
| 274 | 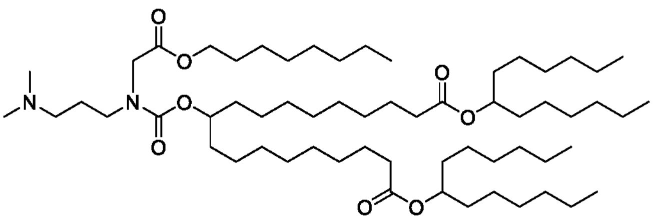 | $C_{61}H_{118}N_2O_8$ | 1007.6 |
| 275 | 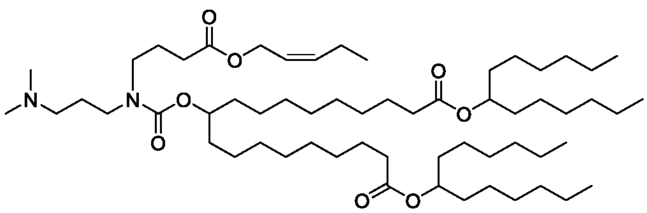 | $C_{60}H_{114}N_2O_8$ | 991.6 |
| 276 | 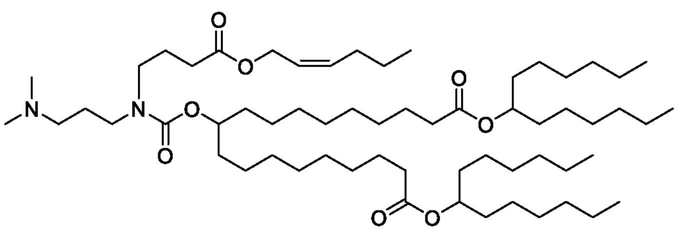 | $C_{61}H_{116}N_2O_8$ | 1005.6 |

(continued)

| No. | Structural formula | Molecular formula | Molecular weight |
|---|---|---|---|
| 277 | 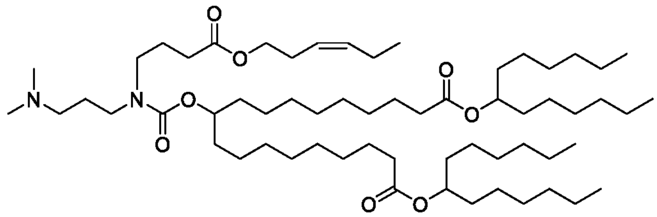 | $C_{61}H_{116}N_2O_8$ | 1005.6 |
| 278 | 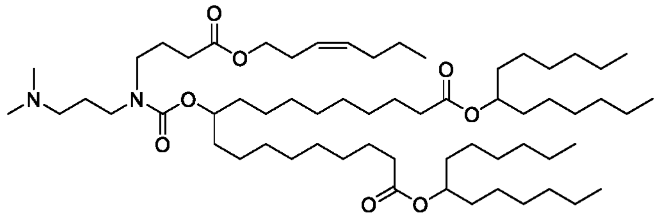 | $C_{62}H_{118}N_2O_8$ | 1019.6 |
| 279 | 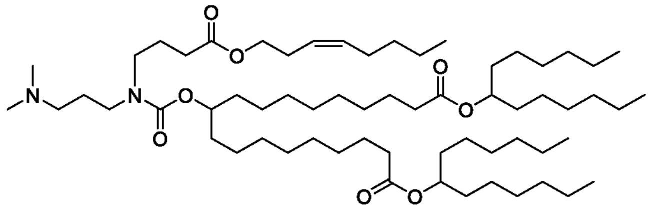 | $C_{63}H_{120}N_2O_8$ | 1033.7 |
| 280 | 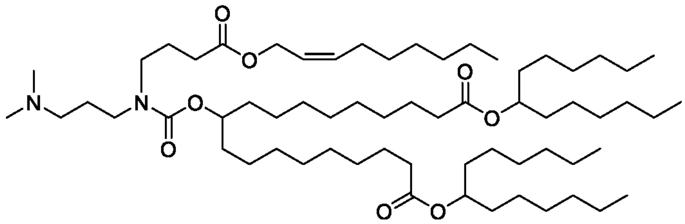 | $C_{64}H_{122}N_2O_8$ | 1047.7 |
| 281 | 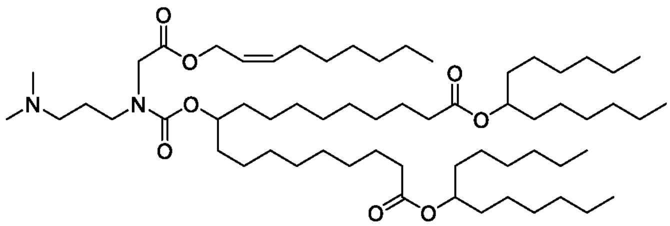 | $C_{62}H_{118}N_2O_8$ | 1019.6 |
| 282 | 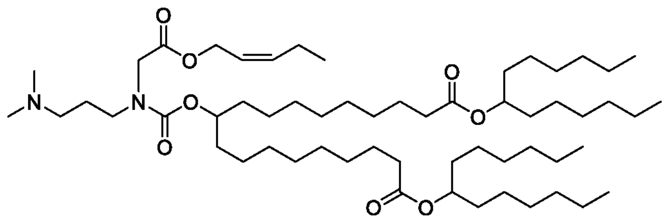 | $C_{58}H_{110}N_2O_8$ | 963.5 |
| 283 | 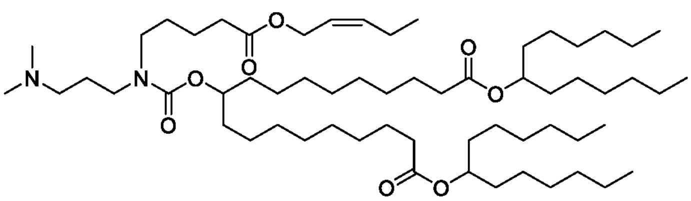 | $C_{61}H_{116}N_2O_8$ | 1005.6 |

(continued)

| No. | Structural formula | Molecular formula | Molecular weight |
|---|---|---|---|
| 284 | 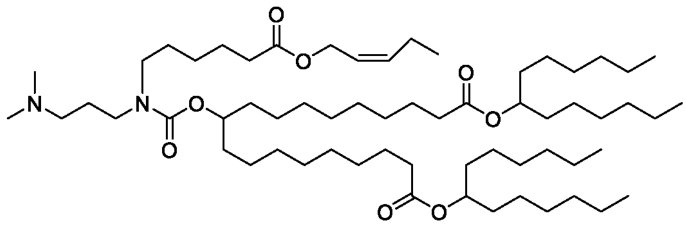 | $C_{62}H_{118}N_2O_8$ | 1019.6 |
| 285 | 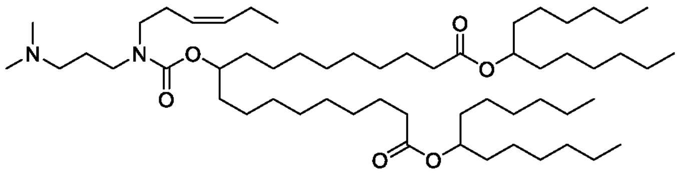 | $C_{57}H_{110}N_2O_6$ | 919.5 |
| 286 | 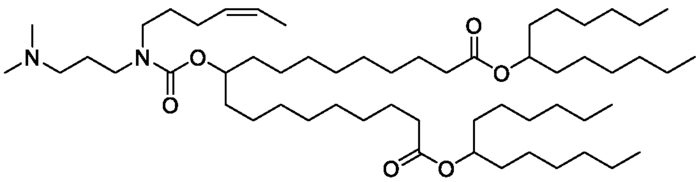 | $C_{57}H_{110}N_2O_6$ | 919.5 |
| 287 | 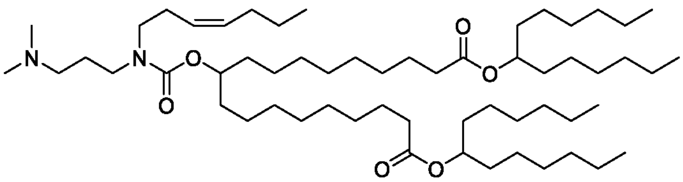 | $C_{58}H_{112}N_2O_6$ | 933.5 |
| 288 | 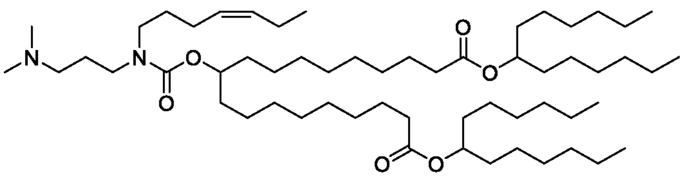 | $C_{58}H_{112}N_2O_6$ | 933.5 |
| 289 | 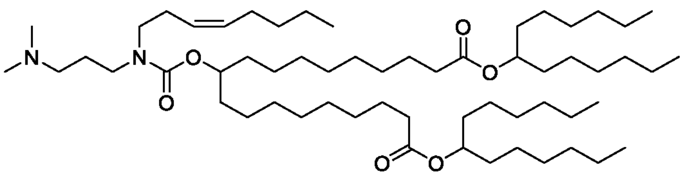 | $C_{59}H_{114}N_2O_6$ | 947.6 |
| 290 | 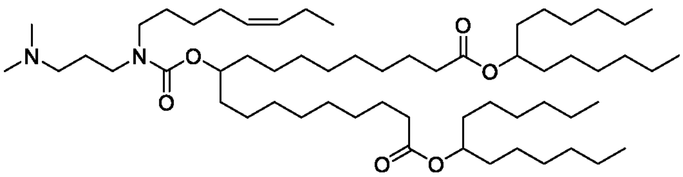 | $C_{59}H_{114}N_2O_6$ | 947.6 |
| 291 | 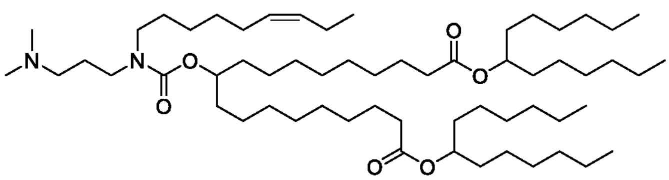 | $C_{60}H_{116}N_2O_6$ | 961.6 |

(continued)

| No. | Structural formula | Molecular formula | Molecular weight |
|---|---|---|---|
| 292 | 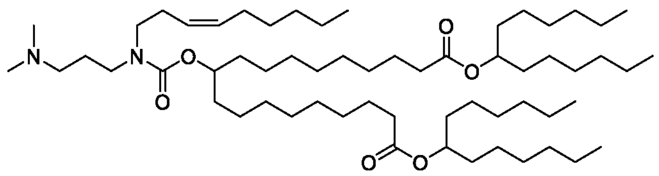 | $C_{60}H_{116}N_2O_6$ | 961.6 |
| 293 | 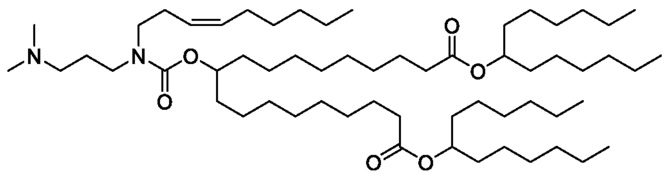 | $C_{60}H_{116}N_2O_6$ | 961.6 |
| 297 | 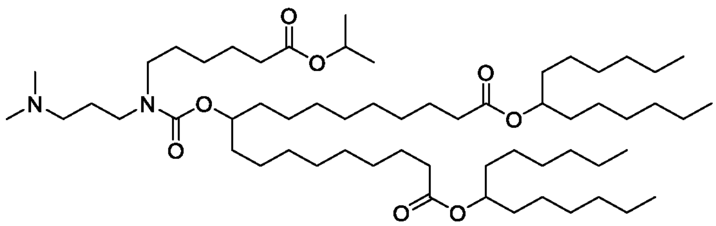 | $C_{60}H_{116}N_2O_8$ | 993.6 |
| 298 | 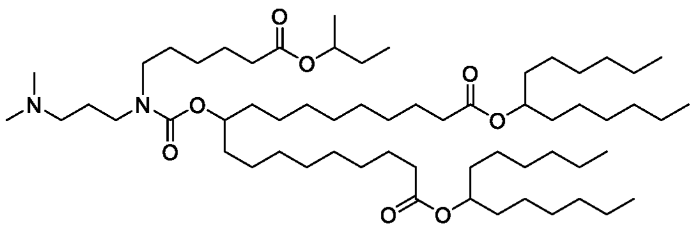 | $C_{61}H_{118}N_2O_8$ | 1007.6 |
| 299 | 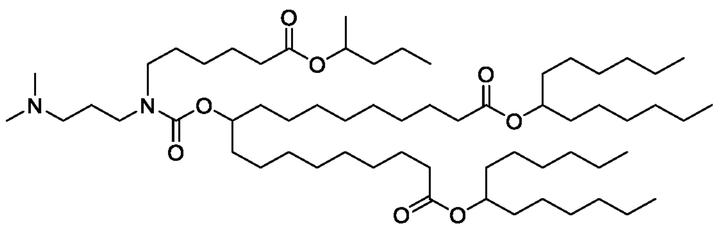 | $C_{62}H_{120}N_2O_8$ | 1021.7 |
| 300 | 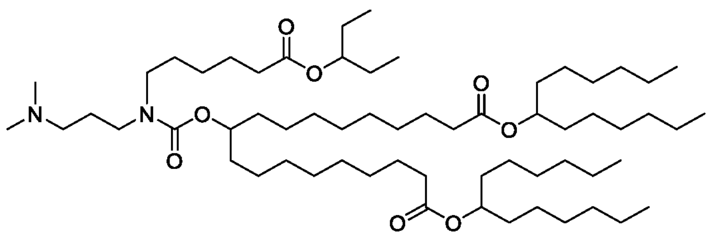 | $C_{62}H_{120}N_2O_8$ | 1021.7 |
| 301 | 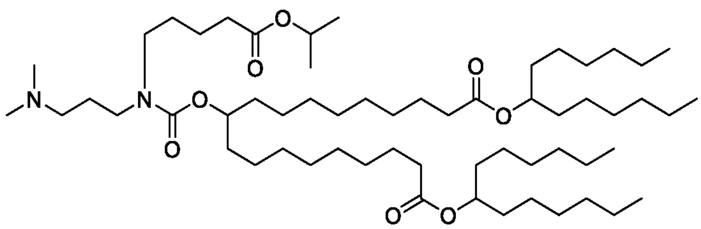 | $C_{59}H_{114}N_2O_8$ | 979.6 |

(continued)

| No. | Structural formula | Molecular formula | Molecular weight |
|---|---|---|---|
| 302 | 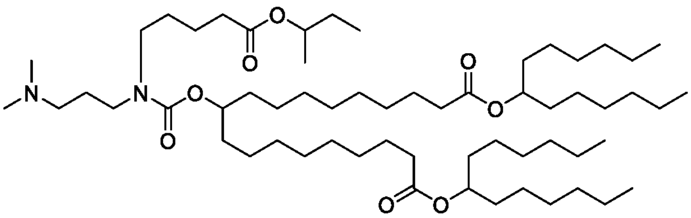 | $C_{60}H_{116}N_2O_8$ | 993.6 |
| 303 | 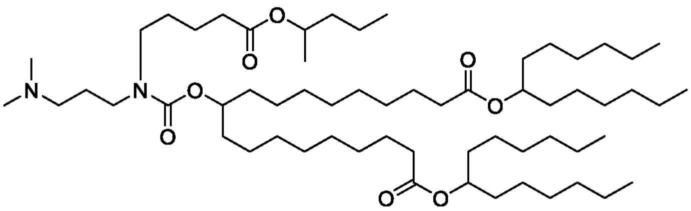 | $C_{61}H_{118}N_2O_8$ | 1007.6 |
| 304 | 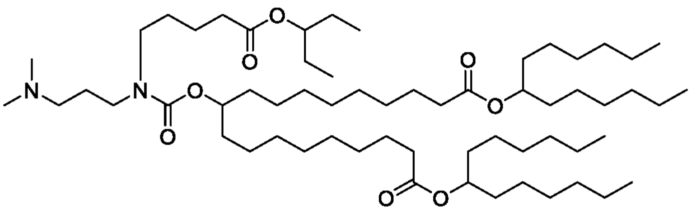 | $C_{61}H_{118}N_2O_8$ | 1007.6 |
| 305 | 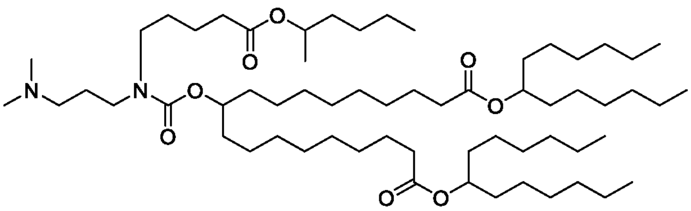 | $C_{62}H_{120}N_2O_8$ | 1021.7 |
| 306 | 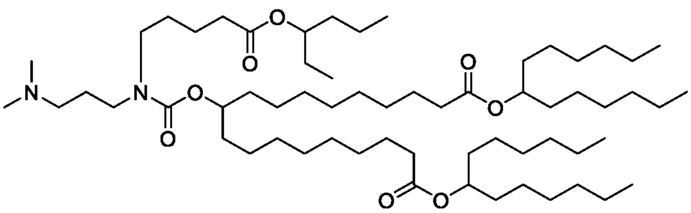 | $C_{62}H_{120}N_2O_8$ | 1021.7 |
| 307 | 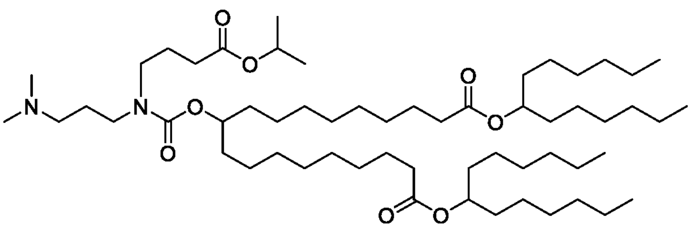 | $C_{58}H_{112}N_2O_8$ | 965.5 |
| 308 | 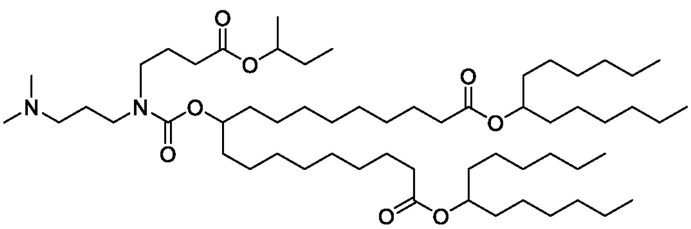 | $C_{59}H_{114}N_2O_8$ | 979.6 |

(continued)

| No. | Structural formula | Molecular formula | Molecular weight |
|---|---|---|---|
| 309 | 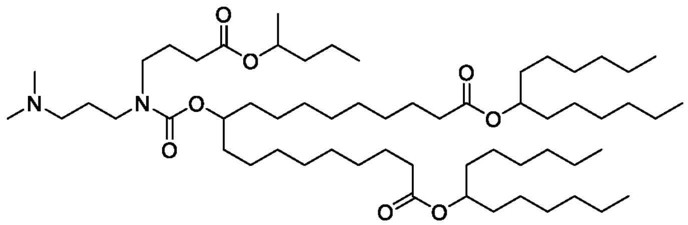 | $C_{60}H_{116}N_2O_8$ | 993.6 |
| 310 | 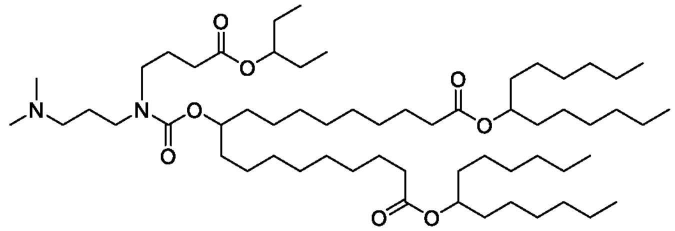 | $C_{60}H_{116}N_2O_8$ | 993.6 |
| 311 | 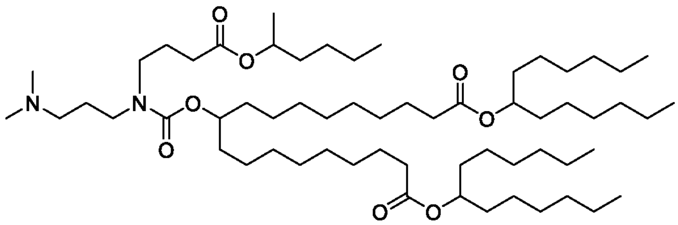 | $C_{61}H_{118}N_2O_8$ | 1007.6 |
| 312 | 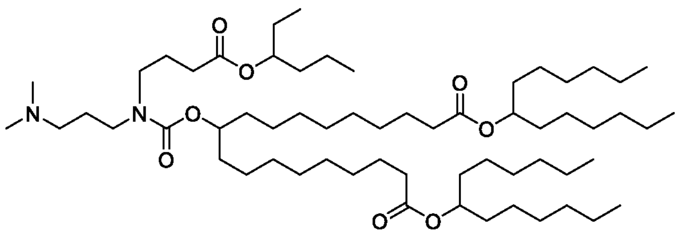 | $C_{61}H_{118}N_2O_8$ | 1007.6 |
| 313 | 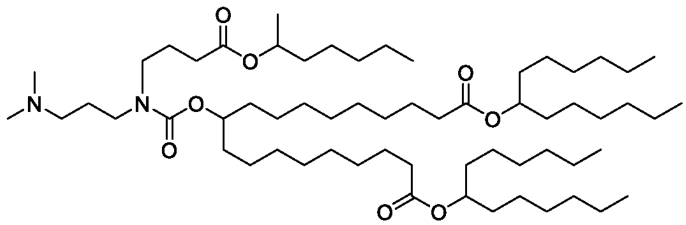 | $C_{62}H_{120}N_2O_8$ | 1021.7 |
| 314 | 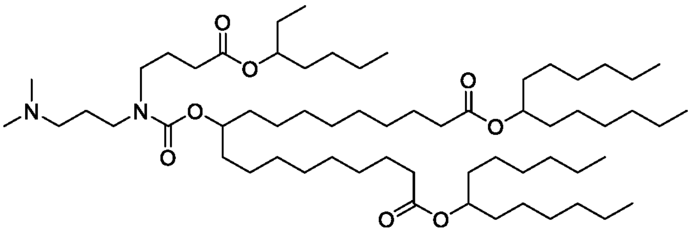 | $C_{62}H_{120}N_2O_8$ | 1021.7 |
| 315 | 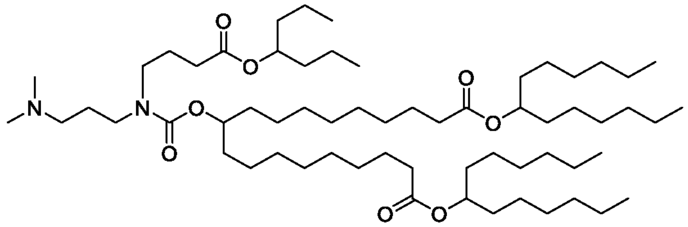 | $C_{62}H_{120}N_2O_8$ | 1021.7 |

(continued)

| No. | Structural formula | Molecular formula | Molecular weight |
|---|---|---|---|
| 316 | 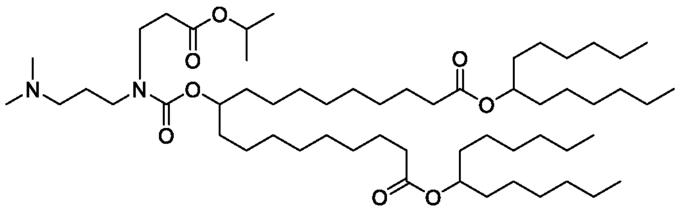 | $C_{57}H_{110}N_2O_8$ | 951.5 |
| 317 | 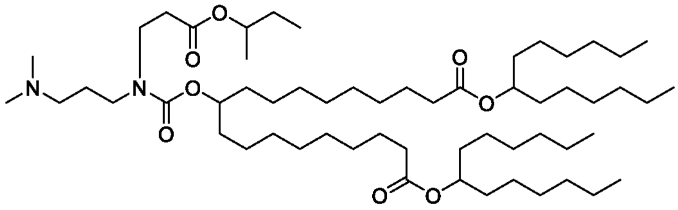 | $C_{58}H_{112}N_2O_8$ | 965.5 |
| 318 | 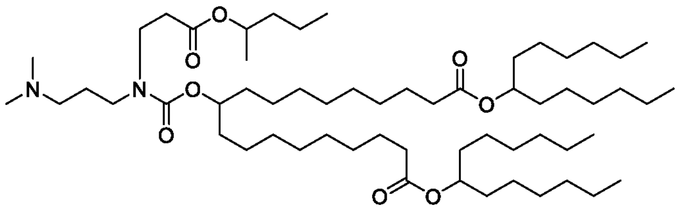 | $C_{59}H_{114}N_2O_8$ | 979.6 |
| 319 | 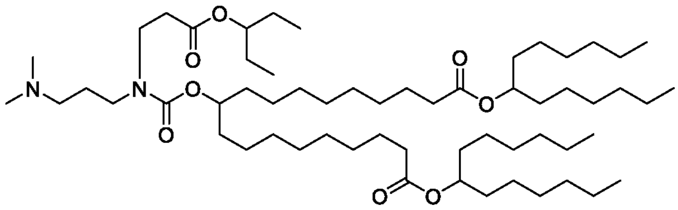 | $C_{59}H_{114}N_2O_8$ | 979.6 |
| 320 | 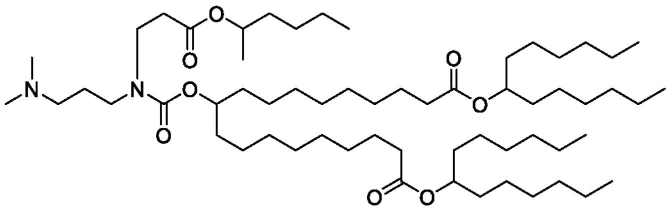 | $C_{60}H_{116}N_2O_8$ | 993.6 |
| 321 | 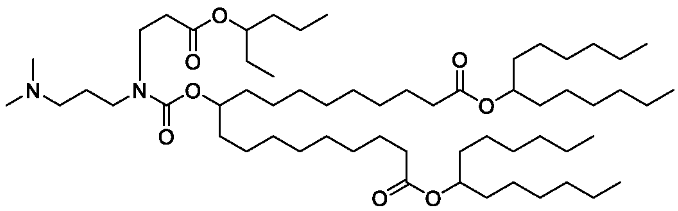 | $C_{60}H_{116}N_2O_8$ | 993.6 |
| 322 | 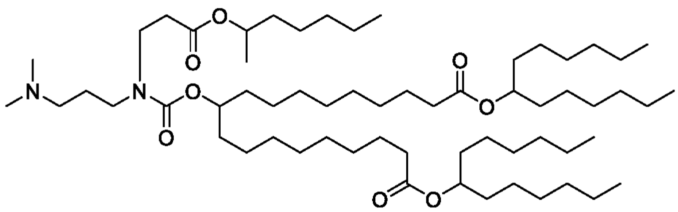 | $C_{61}H_{118}N_2O_8$ | 1007.6 |

(continued)

| No. | Structural formula | Molecular formula | Molecular weight |
|---|---|---|---|
| 323 | 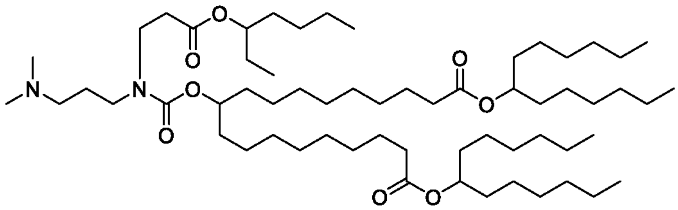 | $C_{61}H_{118}N_2O_8$ | 1007.6 |
| 324 | 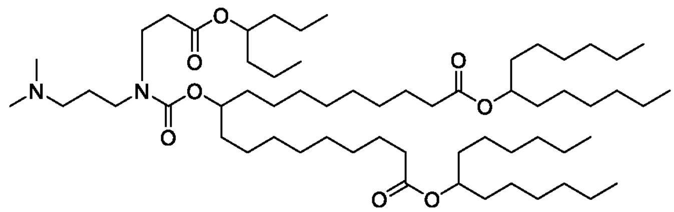 | $C_{61}H_{118}N_2O_8$ | 1007.6 |
| 325 | 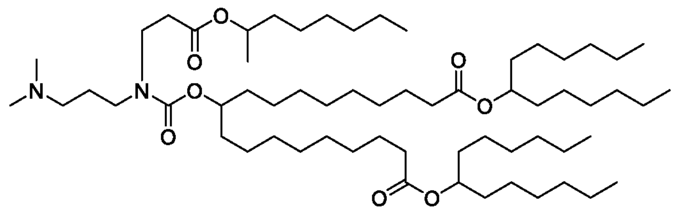 | $C_{62}H_{120}N_2O_8$ | 1021.7 |
| 326 | 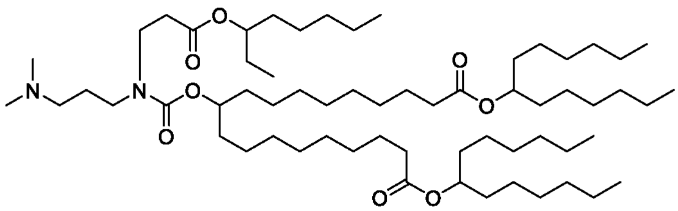 | $C_{62}H_{120}N_2O_8$ | 1021.7 |
| 327 | 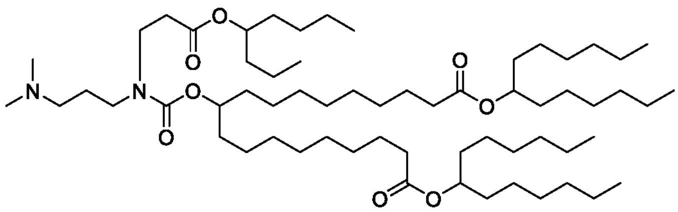 | $C_{62}H_{120}N_2O_8$ | 1021.7 |
| 328 | 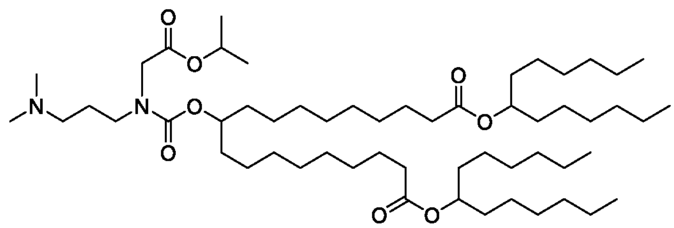 | $C_{56}H_{108}N_2O_8$ | 937.5 |
| 329 | 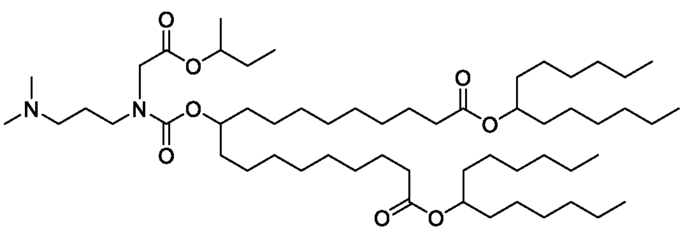 | $C_{57}H_{110}N_2O_8$ | 951.5 |

(continued)

| No. | Structural formula | Molecular formula | Molecular weight |
|---|---|---|---|
| 330 | 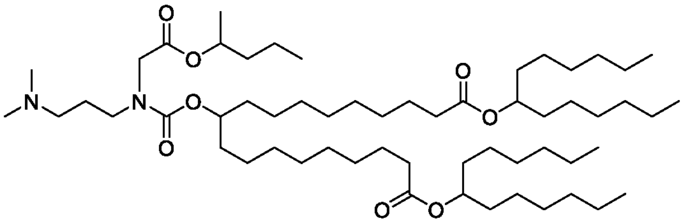 | $C_{58}H_{112}N_2O_8$ | 965.5 |
| 331 | 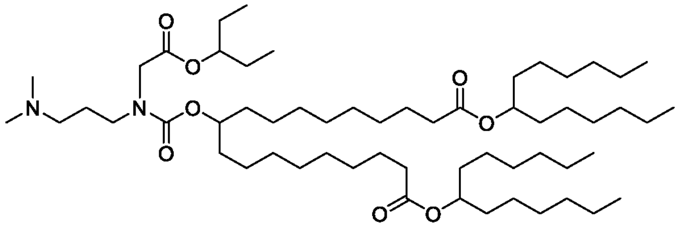 | $C_{58}H_{112}N_2O_8$ | 965.5 |
| 332 | 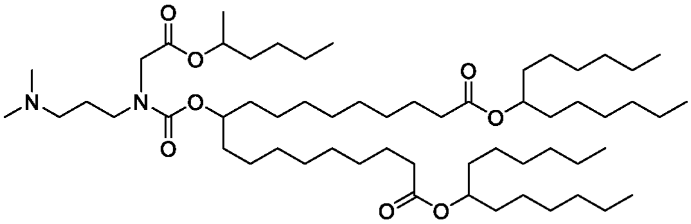 | $C_{59}H_{114}N_2O_8$ | 979.6 |
| 333 | 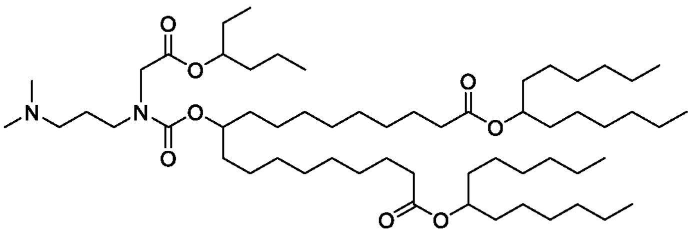 | $C_{59}H_{114}N_2O_8$ | 979.6 |
| 334 | 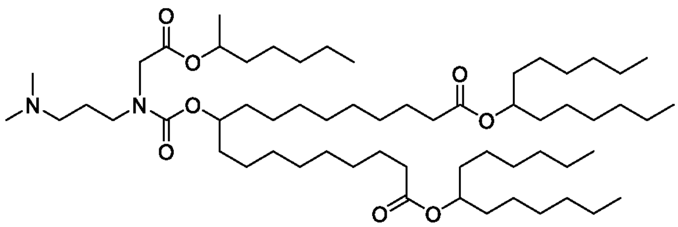 | $C_{60}H_{116}N_2O_8$ | 993.6 |
| 335 | 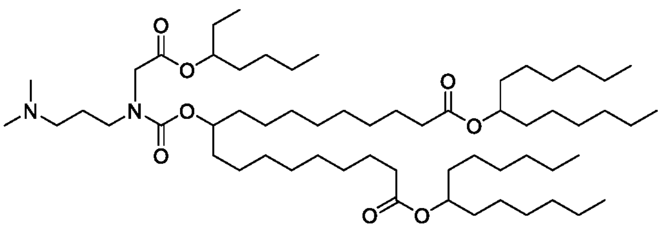 | $C_{60}H_{116}N_2O_8$ | 993.6 |
| 336 | 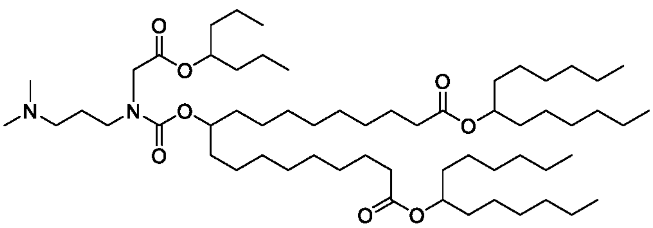 | $C_{60}H_{116}N_2O_8$ | 993.6 |

(continued)

| No. | Structural formula | Molecular formula | Molecular weight |
|---|---|---|---|
| 337 | 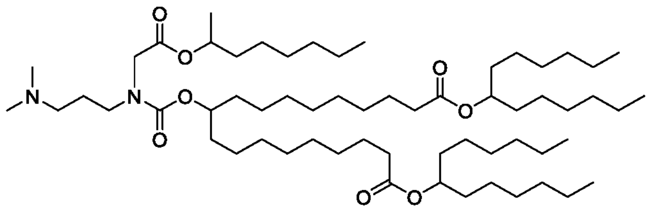 | $C_{61}H_{118}N_2O_8$ | 1007.6 |
| 338 | 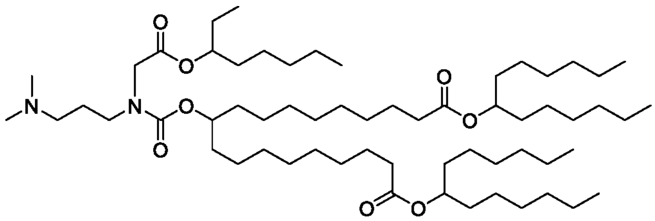 | $C_{61}H_{118}N_2O_8$ | 1007.6 |
| 339 | 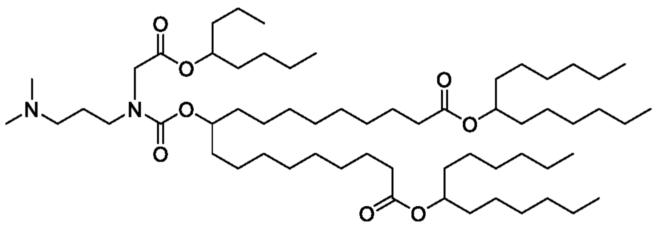 | $C_{61}H_{118}N_2O_8$ | 1007.6 |
| 340 | 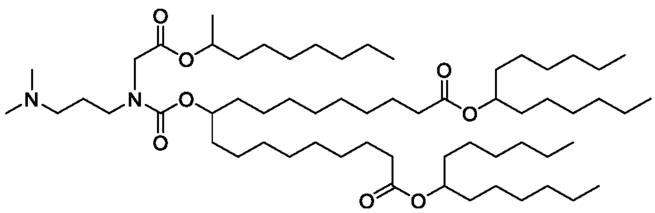 | $C_{62}H_{120}N_2O_8$ | 1021.7 |
| 341 | 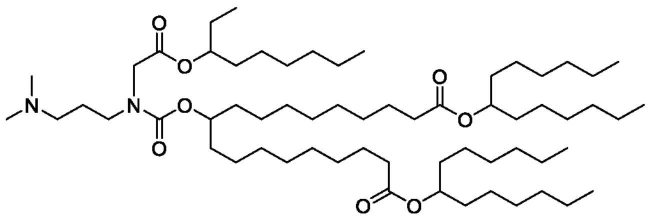 | $C_{62}H_{120}N_2O_8$ | 1021.7 |
| 342 | 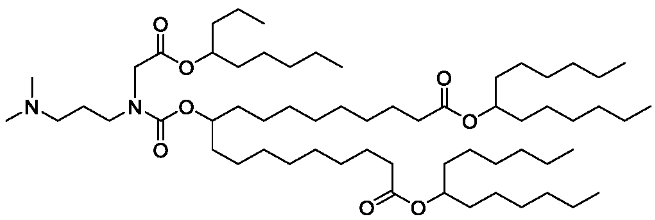 | $C_{62}H_{120}N_2O_8$ | 1021.7 |
| 343 | 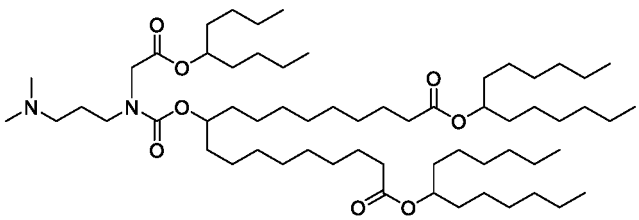 | $C_{62}H_{120}N_2O_8$ | 1021.7 |

(continued)

| No. | Structural formula | Molecular formula | Molecular weight |
|---|---|---|---|
| 358 | 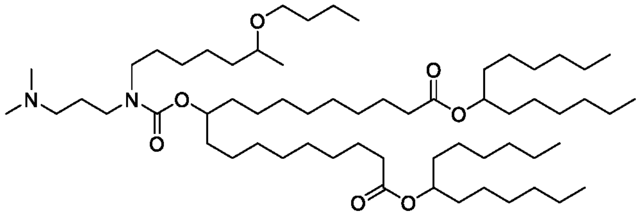 | $C_{61}H_{118}N_2O_8$ | 1007.6 |
| 359 | 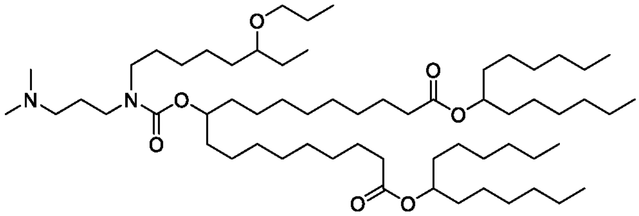 | $C_{61}H_{118}N_2O_8$ | 1007.6 |
| 360 | 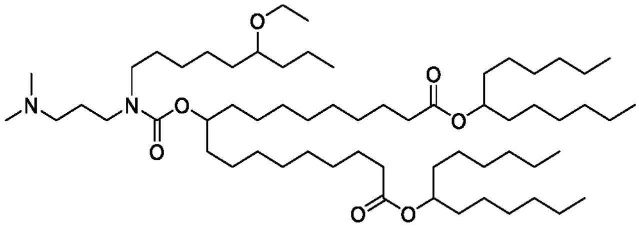 | $C_{61}H_{118}N_2O_8$ | 1007.6 |
| 361 | 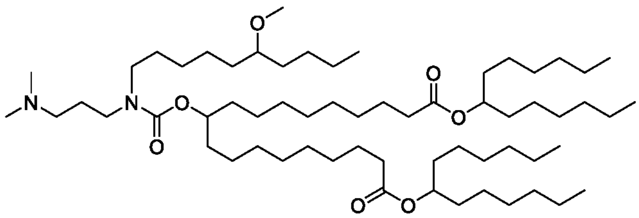 | $C_{60}H_{116}N_2O_8$ | 993.6 |
| 362 | 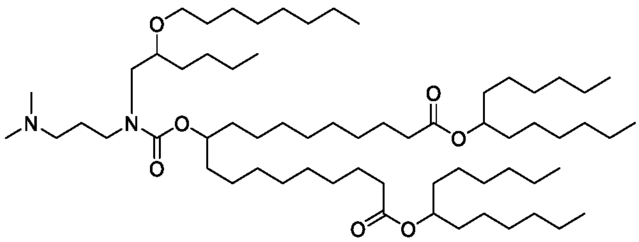 | $C_{65}H_{128}N_2O_8$ | 1049.8 |
| 363 | 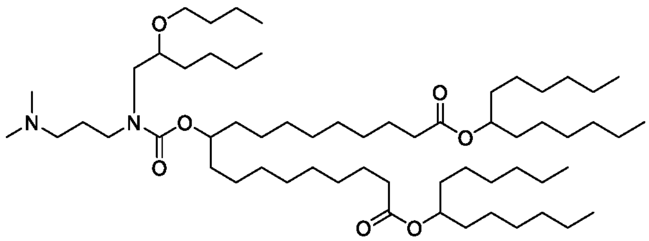 | $C_{60}H_{116}N_2O_8$ | 993.6 |
| 364 | 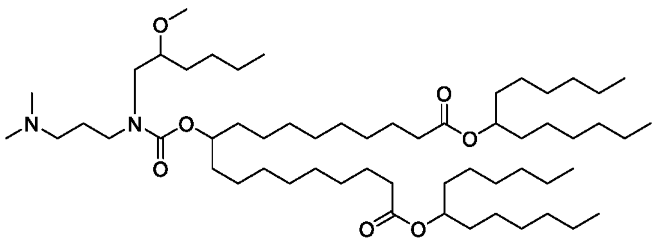 | $C_{58}H_{114}N_2O_8$ | 951.6 |

(continued)

| No. | Structural formula | Molecular formula | Molecular weight |
|---|---|---|---|
| 365 | 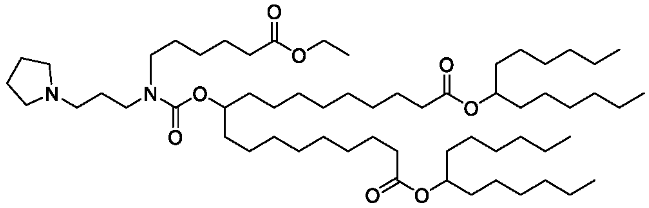 | $C_{61}H_{116}N_2O_8$ | 1005.61 |
| 366 | 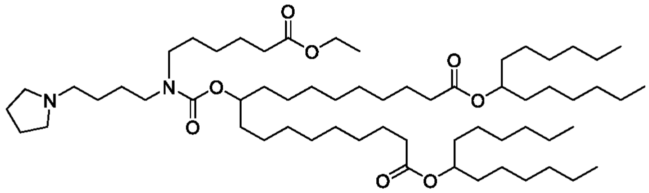 | $C_{62}H_{118}N_2O_8$ | 1019.53 |
| 367 | 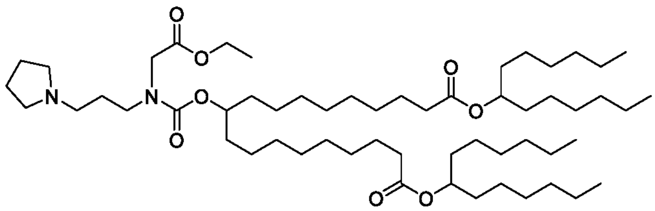 | $C_{57}H_{108}N_2O_8$ | 949.5 |
| 368 | 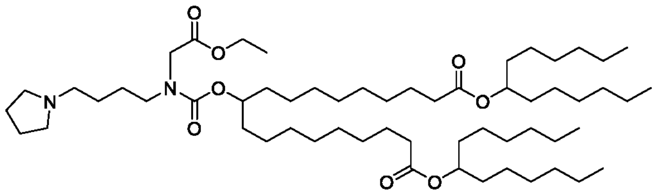 | $C_{58}H_{110}N_2O_8$ | 963.52 |
| 369 | 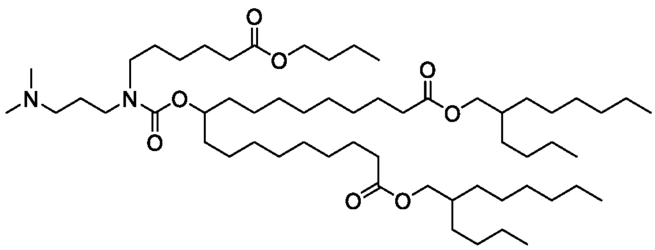 | $C_{59}H_{114}N_2O_8$ | 979.57 |
| 370 | 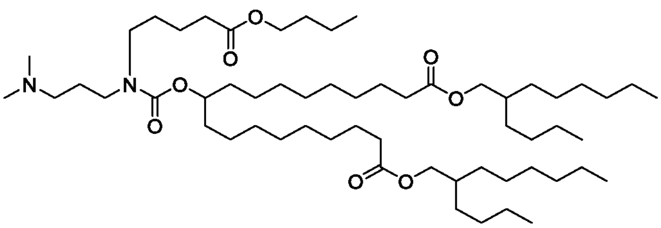 | $C_{58}H_{112}N_2O_8$ | 965.54 |
| 371 | 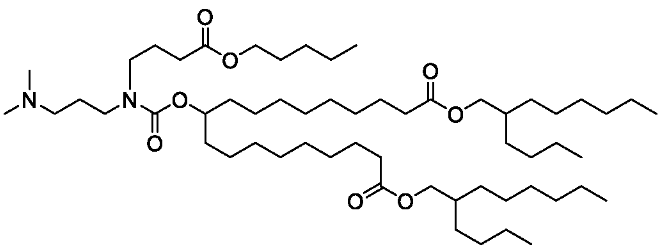 | $C_{58}H_{112}N_2O_8$ | 965.54 |

(continued)

| No. | Structural formula | Molecular formula | Molecular weight |
|---|---|---|---|
| 372 | 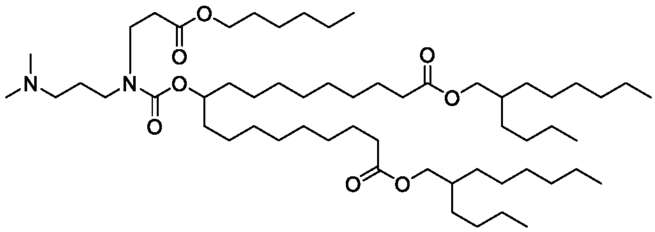 | $C_{58}H_{112}N_2O_8$ | 965.54 |
| 373 | 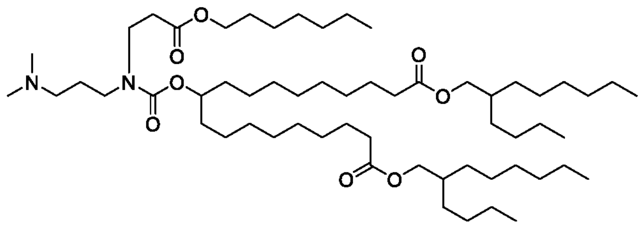 | $C_{59}H_{114}N_2O_8$ | 979.57 |
| 374 | 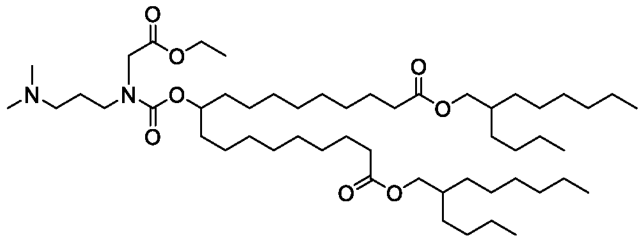 | $C_{53}H_{102}N_2O_8$ | 895.41 |
| 375 | 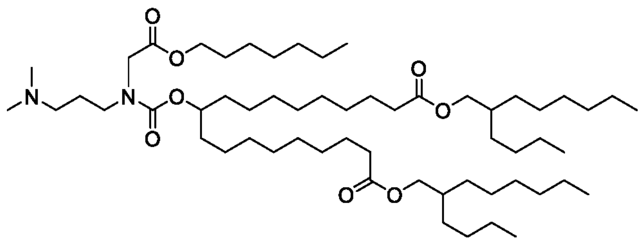 | $C_{58}H_{112}N_2O_8$ | 965.54 |
| 376 | 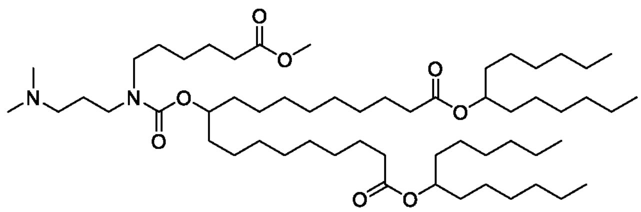 | $C_{58}H_{112}N_2O_8$ | 965.54 |
| 377 | 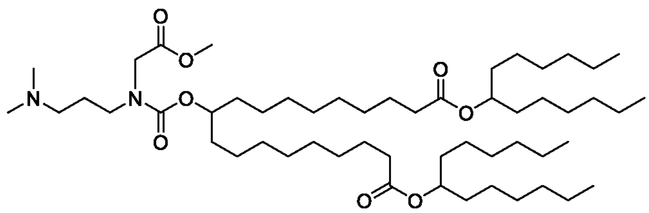 | $C_{54}H_{104}N_2O_8$ | 909.43 |
| 378 | 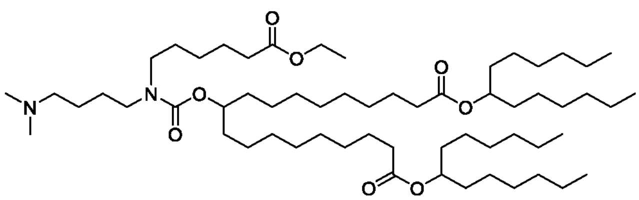 | $C_{60}H_{116}N_2O_8$ | 993.59 |

(continued)

| No. | Structural formula | Molecular formula | Molecular weight |
|---|---|---|---|
| 379 | 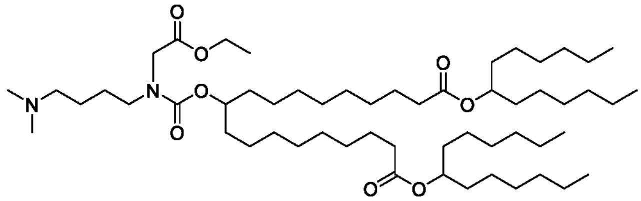 | $C_{56}H_{108}N_2O_8$ | 937.49 |

The amino lipid compounds of the present disclosure all have a hydrophobic characteristic due to the presence of long nonpolar residues and simultaneously a hydrophilic characteristic due to the amino group. Due to this amphiphilic characteristic, the amino lipid compounds of the present disclosure can be used to form a lipid nanoparticle, such as a lipid bilayer, a micelle, a liposome, and the like.

In the context of the present disclosure, the term "lipid nanoparticle" means a nanometer-sized material produced by introducing an amino lipid compound into an aqueous solution. The particle is in particular a lipid nanoparticle, a lipid bilayer vesicle (a liposome), a multilayer vesicle or a micelle.

In some preferred embodiments, the lipid nanoparticle is a liposome containing an amino lipid compound of the present disclosure. Within the scope of the present disclosure, a liposome is a microvesicle consisting of a bilayer of lipid amphipathic molecules encapsulating an aqueous compartment.

Liposome formation is not a spontaneous process. When a lipid is introduced into water, a lipid vesicle is firstly formed, thus forming a bilayer or a series of bilayers, each of which is separated by a water molecule. Liposomes can be formed by sonicating lipid vesicles in water.

In the context of the present disclosure, the term "lipid bilayer" means a thin film formed by two layers of lipid molecules. The term "micelle" means an aggregate of surfactant molecules dispersed in a liquid colloid. Typical micelles in an aqueous solution form aggregates with the hydrophilic head region upon contact with water, chelating the hydrophobic single tail region at the center of the micelle.

In one aspect, the present disclosure provides a use of the amino lipid compound of the present disclosure for the manufacture of a vehicle for an active ingredient. In some embodiments, the vehicle is in the form of a lipid nanoparticle, such as a lipid bilayer, micelle, liposome.

<u>Lipid nanoparticle (LNP)</u>

In another aspect, the present disclosure provides a lipid nanoparticle containing the amino lipid compound of the present disclosure and a pharmaceutically acceptable carrier, diluent or excipient.

In some embodiments, the lipid nanoparticle further contains one or more of a helper lipid, a structural lipid, and a PEG-lipid (polyethylene glycol-lipid).

In some further embodiments, the lipid nanoparticle further contains the helper lipid, the structural lipid, and the PEG-lipid.

In some embodiments, the lipid nanoparticle comprises the amino lipid compound in an amount (molar percent) of about 25.0% to 75.0%, such as about 25.0%-28.0%, 28.0%-32.0%, 32.0%-35.0%, 35.0%-40.0%, 40.0%-42.0%, 42.0%-45.0%, 45.0%-46.3%, 46.3%-48.0%, 48.0%-49.5%, 49.5%-50.0%, 50.0%-55.0%, 55.0%-60.0%, 60.0%-65.0%, or 65.0%-75.0%, based on the total amount of the amino lipid compound, the helper lipid, the structural lipid, and the PEG-lipid.

In some embodiments, the lipid nanoparticle comprises the helper lipid in an amount (molar percent) of about 5.0% to 45.0%, such as about 5.0%-9.0%, 9.0%-9.4%, 9.4%-10.0%, 10.0%-10.5%, 10.5%-11.0%, 11.0%-15.0%, 15.0%-16.0%, 16.0%-18.0%, 18.0%-20.0%, 20.0%-25.0%, 25.0%-33.5%, 33.5%-37.0%, 37.0%-40.0%, 40.0%-42.0%, or 42.0%-45.0%, based on the total amount of the amino lipid compound, the helper lipid, the structural lipid, and the PEG-lipid.

In some embodiments, the lipid nanoparticle comprises the structural lipid in an amount (molar percent) of about 0.0% to 50.0%, such as about 0.0%-10.0%, 10.0%-15.5%, 15.5%-18.5%, 18.5%-22.5%, 22.5%-23.5%, 23.5%-28.5%, 28.5%-33.5%, 33.5%-35.0%, 35.0%-36.5%, 36.5%-38.0%, 38.0%-38.5%, 38.5%-39.0%, 39.0%-39.5%, 39.5%-40.5%, 40.5%-41.5%, 41.5%-42.5%, 42.5%-42.7%, 42.7%-43.0%, 43.0%-43.5%, 43.5%-45.0%, 45.0%-46.5%, 46.5%-48.5%, or 46.5%-50.0%, based on the total amount of the amino lipid compound, the helper lipid, the structural lipid, and the PEG-lipid.

In some embodiments, the lipid nanoparticle comprises the PEG-lipid in an amount (molar percent) of about 0.5% to 5.0%, such as about 0.5%-1.0%, 1.0%-1.5%, 1.5%-1.6%, 1.6%-2.0%, 2.0%-2.5%, 2.5%-3.0%, 3.0%-3.5%,

3.5%-4.0%, 4.0%-4.5%, or 4.5%-5.0%, based on the total amount of the amino lipid compound, the helper lipid, the structural lipid, and the PEG-lipid.

In the context of the present disclosure, the helper lipid is a phospholipid. The phospholipids are generally semi-synthetic and may also be of natural origin or chemically modified. The phospholipids include, but are not limited to, DSPC (distearoyl phosphatidylcholine), DOPE (dioleoyl phosphatidylethanolamine), DOPC (dioleoyl lecithin), DOPS (dioleoyl phosphatidylserine), DSPG (1,2-octacosyl-sn-glycero-3-phospho-(1'-rac-glycerol)), DPPG (dipalmitoyl phosphatidylglycerol), DPPC (dipalmitoyl phosphatidylcholine), DGTS (1,2-dipalmitoyl-sn-glycero-3-O-4'-(N,N,N-trimethyl) homoserine), lysophospholipid, and the like. Preferably, the helper lipid is one or more selected from the group consisting of DSPC, DOPE, DOPC, and DOPS. In some embodiments, the helper lipid is DSPC and/or DOPE.

In the context of the present disclosure, the structural lipid is sterol, including but not limited to, cholesterol, cholesterol esters, steroid hormones, steroid vitamins, bile acid, cholesterin, ergosterol, β-sitosterol, oxidized cholesterol derivatives, and the like. Preferably, the structural lipid is at least one selected from cholesterol, cholesteryl esters, steroid hormones, steroid vitamins, and bile acid. In some embodiments, the structural lipid is cholesterol, preferably high purity cholesterol, particularly injection grade high purity cholesterol, such as CHO-HP.

In the context of the present disclosure, the PEG-lipid (polyethylene glycol-lipid) is a conjugate of polyethylene glycol and a lipid structure. Preferably, the PEG-lipid is selected from PEG-DMG and PEG-distearoyl phosphatidylethanolamine (PEG-DSPE), preferably PEG-DMG. Preferably, the PEG-DMG is a polyethylene glycol (PEG) derivative of 1,2-dimyristoyl-sn-glycerol. Preferably, the PEG has an average molecular weight of about 2,000 to 5,000, preferably about 2,000.

In some embodiments as described above, in the lipid nanoparticle, the molar ratio of the amino lipid compound of the present disclosure: helper lipid: structural lipid: PEG-lipid is about 45:10:42.5:2.5, or 45:11:41.5:2.5, or 42.0:10.5:45.0:2.5, or 42.0:16.0:39.5:2.5, or 40.0:16.0:41.5:2.5, or 40.0:18.0:39.5:2.5, or 35.0:16.0:46.5:2.5, or 35.0:25.0:36.5:3.5, or 28.0:33.5:35.0:3.5, or 32.0:37.0:40.5:0.5, or 35.0:40.0:22.5:2.5, or 40.0:42.0:15.5:2.5, or 40.0:20.0:38.5:1.5, or 45.0:15.0:38.5:1.5, or 55.0:5.0:38.5:1.5, or 60.0:5.0:33.5:1.5, or 45.0:20.0:33.5:1.5, or 50.0:20.0:28.5:1.5, or 55.0:20.0:23.5:1.5, or 60.0:20.0:18.5:1.5, or 40.0:15.0:43.5:1.5, or 50.0:15.0:33.5:1.5, or 55.0:15.0:28.5:1.5, or 60.0:15.0:23.5:1.5, or 40.0:10.0:48.5:1.5, or 45.0:10.0:43.5:1.5, or 55.0:10.0:33.5:1.5, or 40.0:5.0:53.5:1.5, or 45.0:5.0:48.5:1.5, or 50.0:5.0:43.5:1.5. In some such embodiments, the helper lipid is DOPE, and the structural lipid is CHO-HP.

In other embodiments as described above, in the lipid nanoparticle, the molar ratio of the amino lipid compound of the present disclosure: helper lipid: structural lipid: PEG-lipid is about 50.0: 10.0: 38.5: 1.5, or 50.0 : 9.0 : 38.0 : 3.0, or 49.5 : 10.0 : 39.0 : 1.5, or 48.0 : 10.0 : 40.5 : 1.5, or 46.3 : 9.4 : 42.7 : 1.6, or 45.0 : 9.0 : 43.0 : 3.0, or 45.0:11.0:41.5:2.5, or 42.0:10.5:45.0:2.5, or 42.0:16.0:39.5:2.5, or 40.0:16.0:41.5:2.5, or 40.0:18.0:39.5:2.5, or 35.0:40.0:22.5:2.5, or 40.0:20.0:38.5:1.5, or 45.0:15.0:38.5:1.5, or 55.0:5.0:38.5:1.5, or 60.0:5.0:33.5:1.5, or 45.0:20.0:33.5:1.5, or 50.0:20.0:28.5:1.5, or 55.0:20.0:23.5:1.5, or 60.0:20.0:18.5:1.5, or 40.0:15.0:43.5:1.5, or 50.0:15.0:33.5:1.5, or 55.0:15.0:28.5:1.5, or 60.0:15.0:23.5:1.5, or 40.0:10.0:48.5:1.5, or 45.0:10.0:43.5:1.5, or 55.0:10.0:33.5:1.5, or 40.0:5.0:53.5:1.5, or 45.0:5.0:48.5:1.5, or 50.0:5.0:43.5:1.5. In some such embodiments, the helper lipid is DSPC, and the structural lipid is CHO-HP.

In some embodiments, the lipid nanoparticle has the amino lipid compound of the present disclosure, helper lipid, structural lipid, and PEG-lipid in molar percent (%) as shown in Nos. 1-24 in Table 2 below, based on the total amount of the amino lipid compound, the helper lipid, the structural lipid, and the PEG-lipid:

Table 2

| Component \ No. | Molar percentage (%) | | | | | |
|---|---|---|---|---|---|---|
| | 1 | 2 | 3 | 4 | 5 | 6 |
| Amino lipid compound of the present disclosure | 48.0 | 45.0 | 42.0 | 42.0 | 40.0 | 40.0 |
| DSPC | 10.0 | 0.0 | 0.0 | 0.0 | 0.0 | 0.0 |
| DOPE | 0.0 | 11.0 | 10.5 | 16.0 | 16.0 | 18.0 |
| CHO-HP | 40.5 | 41.5 | 45.0 | 39.5 | 41.5 | 39.5 |
| PEG-lipid | 1.5 | 2.5 | 2.5 | 2.5 | 2.5 | 2.5 |
| Component \ No. | Molar percentage (%) | | | | | |
| | 7 | 8 | 9 | 10 | 11 | 12 |
| Amino lipid compound of the present disclosure | 35.0 | 35.0 | 28.0 | 32.0 | 35.0 | 40.0 |
| DOPE | 16.0 | 25.0 | 33.5 | 37.0 | 40.0 | 42.0 |
| CHO-HP | 46.5 | 36.5 | 35.0 | 40.5 | 22.5 | 15.5 |
| PEG-lipid | 2.5 | 3.5 | 3.5 | 0.5 | 2.5 | 2.5 |
| Component \ No. | Molar percentage (%) | | | | | |
| | 13 | 14 | 15 | 16 | 17 | 18 |
| Amino lipid compound of the present disclosure | 45.0 | 42.0 | 42.0 | 40.0 | 40.0 | 35.0 |
| DSPC | 11.0 | 10.5 | 16.0 | 16.0 | 18.0 | 40.0 |
| CHO-HP | 41.5 | 45.0 | 39.5 | 41.5 | 39.5 | 22.5 |
| PEG-lipid | 2.5 | 2.5 | 2.5 | 2.5 | 2.5 | 2.5 |
| Component \ No. | Molar percentage (%) | | | | | |
| | 19 | 20 | 21 | 22 | 23 | 24 |
| Amino lipid compound of the present disclosure | 50.0 | 50.0 | 49.5 | 46.3 | 45.0 | 45.0 |
| DSPC | 10.0 | 9.0 | 10.0 | 9.4 | 9.0 | 0.0 |
| DOPE | 0.0 | 0.0 | 0.0 | 0.0 | 0.0 | 10.0 |
| CHO-HP | 38.5 | 38.0 | 39.0 | 42.7 | 43.0 | 42.5 |
| PEG-lipid | 1.5 | 3.0 | 1.5 | 1.6 | 3.0 | 2.5 |

In some embodiments, the lipid nanoparticle has the amino lipid compound of the present disclosure, helper lipid, structural lipid, and PEG-lipid in molar percent (%) as shown in Nos. 25-42 in Table 3 below, based on the total amount of the amino lipid compound, the helper lipid, the structural lipid, and the PEG-lipid:

Table 3

| No. | Molar percentage (%) |
|---|---|

| Component | 25 | 26 | 27 | 28 | 29 | 30 |
|---|---|---|---|---|---|---|
| Amino lipid compound of the present disclosure | 40.0 | 45.0 | 55.0 | 60.0 | 45.0 | 50.0 |
| DSPC or DOPE | 20.0 | 15.0 | 5.0 | 5.0 | 20.0 | 20.0 |
| CHO-HP | 38.5 | 38.5 | 38.5 | 33.5 | 33.5 | 28.5 |
| PEG-lipid | 1.5 | 1.5 | 1.5 | 1.5 | 1.5 | 1.5 |
| No. / Component | Molar percentage (%) | | | | | |
| | 31 | 32 | 33 | 34 | 35 | 36 |
| Amino lipid compound of the present disclosure | 55.0 | 60.0 | 40.0 | 50.0 | 55.0 | 60.0 |
| DSPC or DOPE | 20.0 | 20.0 | 15.0 | 15.0 | 15.0 | 15.0 |
| CHO-HP | 23.5 | 18.5 | 43.5 | 33.5 | 28.5 | 23.5 |
| PEG-lipid | 1.5 | 1.5 | 1.5 | 1.5 | 1.5 | 1.5 |
| No. / Component | Molar percentage (%) | | | | | |
| | 37 | 38 | 39 | 40 | 41 | 42 |
| Amino lipid compound of the present disclosure | 40.0 | 45.0 | 55.0 | 40.0 | 45.0 | 50.0 |
| DSPC or DOPE | 10.0 | 10.0 | 10.0 | 5.0 | 5.0 | 5.0 |
| CHO-HP | 48.5 | 43.5 | 33.5 | 53.5 | 48.5 | 43.5 |
| PEG-lipid | 1.5 | 1.5 | 1.5 | 1.5 | 1.5 | 1.5 |

As described above, the lipid nanoparticle of the present disclosure may be used as a delivery vehicle for an active ingredient.

In some embodiments, the active ingredient includes a therapeutic and/or a prophylactic agent.

The term "therapeutic agent" or "prophylactic agent" refers to any agent that, when administered to a subject, has therapeutic, diagnostic, and/or prophylactic effects and/or elicits desired biological and/or pharmacological effects.

An "effective amount" or "therapeutically effective amount" refers to an amount of the amino lipid compound of the present disclosure or the lipid nanoparticle comprising the amino lipid compound of the present disclosure that is sufficient to effect treatment in a mammal (preferably a human), when administered to the mammal (preferably the human). The amount of the lipid nanoparticle of the present disclosure that constitutes a "therapeutically effective amount" will depend on the amino lipid compound, the condition and its severity, the mode of administration, and the age of the mammal to be treated, but may be routinely determined by one of ordinary skill in the art in light of their own knowledge and the present disclosure.

Preferably, the pharmaceutically active ingredient is a biologically active ingredient, which is a substance that has a biological effect when introduced into a cell or a host, for example, by stimulating an immune or inflammatory response, by exerting an enzymatic activity, by complementing a mutation, or the like. A biologically active ingredient includes, but is not limited to, a nucleic acid, a protein, a peptide, an antibody, a small molecule, and a mixture thereof.

Preferably, the biologically active ingredient is a nucleic acid.

In another preferred embodiment, the biologically active ingredient is an antineoplastic agent, an antibiotic, an immunomodulator, an anti-inflammatory agent, an agent acting on the central nervous system, a polypeptide, a polypeptoid, or a mixture thereof.

A lipid nanoparticle may be referred to as "a lipid nanoparticle drug" when it encapsulates the active ingredient in its internal aqueous space.

In the context of the present disclosure, the term "cell" is a generic term and includes the culture of individual cells, tissues, organs, insect cells, avian cells, fish cells, amphibian cells, mammalian cells, primary cells, continuous cell lines, stem cells, and/or genetically engineered cells (e.g., recombinant cells expressing a heterologous polypeptide or protein). Recombinant cells include, for example, cells expressing heterologous polypeptides or proteins (such as growth factors or

blood factors).

In some embodiments as described above, the lipid nanoparticle of the present disclosure further comprises a nucleic acid.

In some embodiments, the mass ratio of the amino lipid compound of the present disclosure to the nucleic acid in the lipid nanoparticle is about (5-30): 1, such as about (5-10): 1, (10-15): 1, (15-20): 1, (20-25): 1, or (25-30): 1, preferably about 10:1.

In some embodiments, the nucleic acid is selected from the group consisting of RNA, antisense oligonucleotide, and DNA.

In some embodiments, the RNA is selected from the group consisting of messenger RNA (mRNA), ribosomal RNA (rRNA), microRNA (miRNA), transfer RNA (tRNA), small interfering RNA (siRNA), small nuclear RNA (snRNA), small hairpin RNA (shRNA), single guide RNA (sgRNA), Cas9 mRNA or a mixture thereof.

In some embodiments, the messenger RNA (mRNA) encodes a polypeptide and/or protein of interest. Any naturally or non-naturally occurring or otherwise modified polypeptide is included. In some embodiments, the polypeptide and/or protein encoded by the mRNA may have therapeutic and/or prophylactic effects when expressed in a cell.

In some embodiments, the RNA is an siRNA that is capable of selectively decreasing the expression of a gene of interest or down-regulating the expression of the gene. For example, an siRNA may be selected such that a gene associated with a particular disease, disorder, or condition is silenced upon administration of a lipid nanoparticle comprising the siRNA to a subject in need thereof. An siRNA may comprise a sequence complementary to an mRNA sequence encoding a gene or protein of interest. In some embodiments, the siRNA may be an immunomodulatory siRNA.

In certain embodiments, the RNA is sgRNA and/or cas9 mRNA. The sgRNA and/or cas9 mRNA may be used as a gene editing tool. For example, the sgRNA-Cas9 complex can affect mRNA translation of cellular genes.

In some embodiments, the RNA is an shRNA or a vector or plasmid encoding the same. The shRNA may be produced inside the target cell after delivery of an appropriate construct into the nucleus. Constructs and mechanisms associated with shRNA are well known in the relevant art.

In some embodiments, the DNA is a plasmid.

In some embodiments, the lipid nanoparticle is used to transfer nucleic acids. In some embodiments, the lipid nanoparticle may be used for, for example, gene therapy, gene vaccination, protein replacement therapy, antisense therapy, or therapy by interfering RNA.

Pharmaceutical composition

In another aspect, the present disclosure provides a pharmaceutical composition comprising a lipid nanoparticle as described above and a pharmaceutically acceptable carrier, diluent, or excipient.

In some embodiments, the pharmaceutical composition further comprises a buffer solution. In some such embodiments, the buffer solution is selected from a phosphate buffer and a Tris buffer, preferably a phosphate buffer. In some embodiments, the buffer solution has a concentration of about 5 mmol/L to about 30 mmol/L, preferably about 10 mmol/L. In some embodiments, the buffer solution has a pH of about 6 to 8, preferably about 7 to 8, and more preferably about 7 to 7.5.

In some embodiments, the pharmaceutical composition further comprises a cryoprotectant. In some such embodiments, the cryoprotectant is selected from sucrose and trehalose, preferably sucrose. In some embodiments, the cryoprotectant has a concentration of about 50 mg/ml to 100 mg/ml.

In some embodiments as described above, the pharmaceutical composition further comprises a cryoprotectant. In some such embodiments, the cryoprotectant is selected from sucrose and trehalose, preferably sucrose. In some embodiments, the cryoprotectant has a concentration of about 50 mg/ml to 100 mg/ml.

Use

The lipid nanoparticle of the present disclosure has excellent properties of encapsulating biologically active ingredients. The lipid nanoparticle comprising biologically active ingredients can be used to deliver any of a variety of therapeutic agents into cells. The present disclosure includes use of the lipid nanoparticle as described above to deliver a biologically active ingredient into a cell. The present disclosure also provides a method of delivering a biologically active ingredient into a cell, tissue or organ, comprising contacting the lipid nanoparticle of the present disclosure comprising the biologically active ingredient with the cell, tissue or organ. This provides a subject with the possibility of new therapeutic treatment.

In some embodiments, the tissue or organ is selected from the group consisting of spleen, liver, kidney, lung, femur, ocular tissue, vascular endothelium in blood vessels, lymph, and tumor tissue.

Preferably, the cell is a mammalian cell; and further preferably, the mammalian cell is in a mammal. As used herein, a subject may be any mammal, preferably selected from the group consisting of mice, rats, pigs, cats, dogs, horses,

goats, cattle, and monkeys, etc. In some preferred embodiments, the subject is a human.

The present disclosure provides a method of producing a polypeptide and/or protein of interest in a mammalian cell, comprising contacting the cell with the lipid nanoparticle comprising mRNA encoding the polypeptide and/or protein of interest, upon contact of the cell with the lipid nanoparticle, the mRNA being able to be taken up into the cell and translated to produce the polypeptide and/or protein of interest.

In yet another aspect, the present disclosure provides a use of the amino lipid compound, lipid nanoparticle, or pharmaceutical composition of the present disclosure in the manufacture of a medicament. Preferably, the pharmaceutical composition is used for treatment and/or prevention of a disease.

In some embodiments, the disease is selected from the group consisting of rare diseases, infectious diseases, cancers, genetic diseases, autoimmune diseases, diabetes, neurodegenerative diseases, cardiovascular, renal vascular diseases, and metabolic diseases.

The medicaments are used for, for example, gene therapy, protein replacement therapy, antisense therapy, or therapy by interfering RNA, and gene vaccination.

In some embodiments, the cancers are selected from one or more of lung cancer, stomach cancer, liver cancer, esophageal cancer, colon cancer, pancreatic cancer, brain cancer, lymphoma, blood cancer, or prostate cancer. In some embodiments, the genetic disorders are selected from one or more of hemophilia, thalassemia, and Gaucher's disease.

In some embodiments, the gene vaccination is preferably used to treat and/or prevent cancer, allergy, toxicity, and pathogen infection. In some embodiments, the pathogen is selected from one or more of viruses, bacteria, or fungi.

The present disclosure provides a method of treating a disease or condition in a mammal in need thereof, comprising administering to the mammal a therapeutically effective amount of the lipid nanoparticle as described above.

Preferably, the disease or disorder is selected from the group consisting of rare diseases, infectious diseases, cancers, genetic diseases, autoimmune diseases, diabetes, neurodegenerative diseases, cardiovascular, renal vascular diseases, and metabolic diseases.

In yet another aspect, the present disclosure provides a use of the amino lipid compound, lipid nanoparticle, or pharmaceutical composition of the disclosure in the manufacture of a medicament for nucleic acid transfer. In some embodiments, the nucleic acid is selected from the group consisting of RNA, antisense oligonucleotide, and DNA. In some embodiments, the RNA is selected from the group consisting of messenger RNA (mRNA), ribosomal RNA (rRNA), microRNA (miRNA), transfer RNA (tRNA), small interfering RNA (siRNA), small nuclear RNA (snRNA), small hairpin RNA (shRNA), single guide RNA (sgRNA), Cas9 mRNA, or a mixture thereof. In some embodiments, the DNA is a plasmid.

Preparation method

In yet another aspect, the present disclosure also provides a general synthetic process for preparing the amino lipid compound of formula (I) of the present disclosure, as follows:

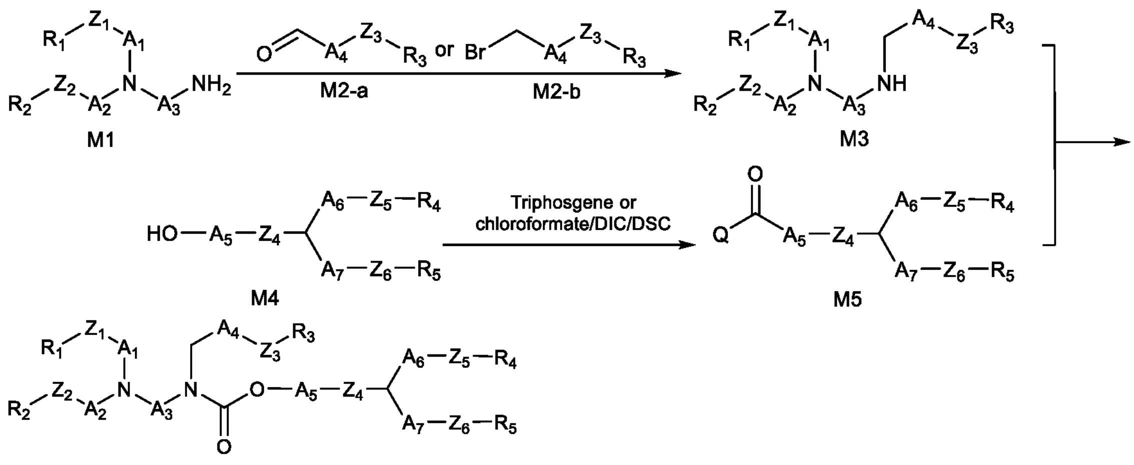


(I)

wherein,

$Z_1$, $Z_2$, $Z_3$, $Z_4$, $Z_5$, $Z_6$, $A_1$, $A_2$,$A_3$, $A_4$, $A_5$, $A_6$, $A_7$, $R_1$, $R_2$, $R_3$, $R_4$, $R_5$ and $R_6$ have the same meanings as defined above for the amino lipid compound of formula (I).

Specifically, the method comprises:

(1) subjecting M1 and M2-a to reductive amination reaction to obtain M3, or subjecting M1 and M2-b to alkylation

reaction to obtain M3;
(2) reacting M4 with an acylating agent (or triphosgene, chloroformate, DIC, DSC) to obtain M5; and
(3) reacting M5 with M3 in the presence of a base (e.g., triethylamine or pyridine, DMAP) to obtain the amino lipid compound of formula (I).

In yet another aspect, the lipid nanoparticle or composition of the present disclosure may be prepared according to methods known in the art. For example, the method may comprise the following steps:

(1) formulating: formulating a suitable aqueous phase; and formulating an organic phase comprising the amino lipid compound of the present disclosure and optionally a helper lipid, a structural lipid, and/or a PEG-lipid;
(2) encapsulation: mixing a suitable amount of the aqueous phase with the organic phase;
(3) dialysis: optionally dialyzing the mixture of step (2); and
(4) sterilization: optionally sterilizing the product of step (3), for example, by means of a sterilizing filter, such as a 0.22 μm microporous membrane.

In some embodiments, the lipid nanoparticle or composition of the present disclosure comprising a nucleic acid, particularly mRNA may be prepared by a method comprising the following steps:

(1) formulating: formulating an aqueous phase comprising the nucleic acid; and formulating an organic phase (e.g., an ethanol phase) comprising the amino lipid compound of the present disclosure and optionally a helper lipid, a structural lipid, and/or a PEG-lipid;
(2) encapsulation: mixing a suitable amount of the aqueous phase with the organic phase;
(3) dialysis: optionally dialyzing the mixture of step (2);
(4) sterilization: optionally sterilizing the product of step (3), for example, by means of a sterilizing filter, such as a 0.22 μm microporous membrane.

The present disclosure also includes the following embodiments:

Embodiment 1. An amino lipid compound having the structure of formula (I) or a pharmaceutically acceptable salt or a stereoisomer thereof:

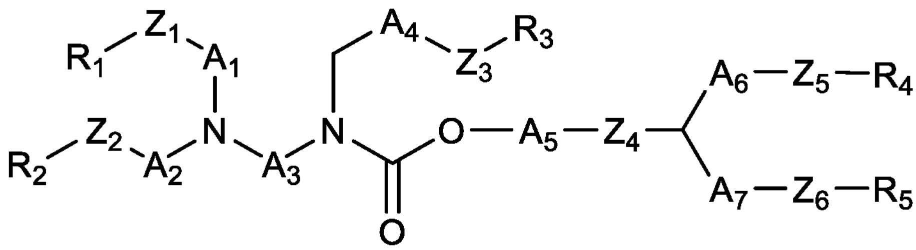

(I)

wherein,

$Z_1$, $Z_2$, $Z_3$, $Z_4$, $Z_5$, and $Z_6$ are each independently -CH(OH)-, -C=C-, -C=C-, -O-, -C(=O)O-, -OC(=O)-, -N($R_6$)C(=O)-, -C(=O)N($R_6$)-, -N($R_6$)C(=O)N($R_6$)-, -OC(=O)N($R_6$)-, -N($R_6$)C(=O)O-, -C(=O)-, -C(=O) S-, -SC(=O)-, -S-S- or a bond;
$Z_3$ is -CH(OH)-, -C=C-, -C = C-, -O-, -C(=O)O-, -OC(=O)-, -N($R_6$)C(=O)-, -C(=O)N($R_6$)-, -N($R_6$)C(=O)N($R_6$)-, -OC(=O)N($R_6$)-, -N($R_6$)C(=O)O-, -C(=O)-, -C(=O) S-, -SC(=O)-, -S-S- or a bond;
$A_1$, $A_2$, $A_3$, $A_4$, $A_5$, $A_6$, and $A_7$ are each independently $C_1$-$C_{12}$ hydrocarbylene, cyclohydrocarbyl, phenyl, benzyl, heterocycle, or a bond;
$R_1$ and $R_2$ are each independently H or $C_1$-$C_{18}$ hydrocarbyl, or cyclohydrocarbyl, phenyl, benzyl, heterocycle; or $R_1$ and $R_2$, together with the nitrogen atom to which they are attached, form a 5- to 7-membered heterocycle;
$R_3$ is H or $C_1$-$C_{18}$ hydrocarbyl or cyclohydrocarbyl, phenyl, benzyl, heterocycle;
$R_4$ and $R_5$ are each independently $C_1$-$C_{18}$ hydrocarbyl, or cyclohydrocarbyl, phenyl, benzyl, heterocycle;
$R_6$ is H or $C_1$-$C_{18}$ hydrocarbyl, or -OH, -O-optionally substituted $C_2$-$C_{18}$ hydrocarbyl, or -C=C-, -C=C-optionally substituted $C_4$-$C_{18}$ hydrocarbyl.

Embodiment 2. The amino lipid compound of embodiment 1, wherein $Z_5$ and $Z_6$ are each independently -OC(=O)-, -C(=O)O-, or a bond.

Embodiment 3. The amino lipid compound of any one of embodiments 1 to 2, wherein one of $Z_5$ and $Z_6$ is -C(=O)O-.

Embodiment 4. The amino lipid compound of embodiments 1 to 2, wherein both $Z_5$ and $Z_6$ are -C(=O)O-.

Embodiment 5. The amino lipid compound of any one of embodiments 1 to 2, wherein one of $Z_5$ and $Z_6$ is -OC(=O)-.

Embodiment 6. The amino lipid compound of embodiments 1 to 2, wherein both $Z_5$ and $Z_6$ are -OC(=O)-.

Embodiment 7. The amino lipid compound of any one of embodiments 1 to 2, wherein one of $Z_1$ and $Z_2$ is a bond.

Embodiment 8. The amino lipid compound of embodiments 1 to 2, wherein both $Z_1$ and $Z_2$ are bonds.

Embodiment 9. The amino lipid compound according to any one of embodiments 1 to 2, wherein one of $A_1$ and $A_2$ is a bond.

Embodiment 10. The amino lipid compound of embodiments 1 to 2, wherein both $A_1$ and $A_2$ are bonds.

Embodiment 11. The amino lipid compound according to any one of embodiments 1 to 2, wherein $Z_3$ is -C(=O)O- or -OC(=O)-.

Embodiment 12. The amino lipid compound of embodiment 11, wherein $R_4$ and $R_5$ are branched $C_3$-$C_{18}$ hydrocarbyl groups.

Embodiment 13. A lipid nanoparticle comprising the amino lipid compound according to any one of embodiments 1 to 12 and a pharmaceutically acceptable carrier, diluent or excipient.

Embodiment 14. A pharmaceutical composition comprising the amino lipid compound of any one of embodiments 1 to 12 and a pharmaceutically acceptable carrier, diluent or excipient.

Embodiment 15. Use of the amino lipid compound according to any one of embodiments 1 to 12, the lipid nanoparticle according to embodiment 13, and the pharmaceutical composition according to embodiment 14 in the manufacture of a medicament for gene therapy, gene vaccination, antisense therapy, or therapy by interfering RNA.

Embodiment 16. The use according to embodiment 15, wherein the gene therapy is useful for the treatment of cancers and genetic diseases.

Embodiment 17. The use according to embodiment 16, wherein the cancers are selected from one or more of lung cancer, stomach cancer, liver cancer, esophageal cancer, colon cancer, pancreatic cancer, brain cancer, lymphatic cancer, blood cancer, or prostate cancer; and the genetic diseases are selected from one or more of hemophilia, thalassemia and Gaucher's disease.

Embodiment 18. The use according to embodiment 17, wherein the gene vaccination is useful for the treatment of cancer, allergy, toxicity, and pathogen infection.

Embodiment 19. The use according to embodiment 18, wherein the pathogen is selected from one or more of a virus, a bacterium or a fungus.

Embodiment 20. Use of the lipid nanoparticle according to embodiment 13 or the amino lipid compound according to any one of embodiments 1 to 12 in the manufacture of a medicament for nucleic acid transfer, wherein the nucleic acid is RNA, messenger RNA (mRNA), antisense oligonucleotide, DNA, plasmid, ribosomal RNA (rRNA), microRNA (miRNA), transfer RNA (tRNA), small interfering RNA (siRNA), and small nuclear RNA (snRNA).

## Beneficial effect

The amino lipid compound of the present disclosure can form a vehicle, such as a lipid nanoparticle, with excellent properties of encapsulating biologically active ingredients, can be used for delivering biologically active ingredients, especially water-insoluble drugs or active ingredients that are easily decomposed or degraded (such as nucleic acids), and improving its bioavailability and efficacy, or transfection efficiency (for nucleic acids), or safety, or preference for a particular organ or tissue.

## Brief description of the drawings

FIG. 1 shows the results of ALT enzyme activity test 12 h after *in vivo* delivery of the lipid nanoparticle in Experimental Example 4.

FIG. 2 shows the results of Elispot cell immunity test for spleen lymphocytes of mice with representative amino lipid compounds in Experimental Example 5.

FIG. 3 shows the results of Elispot cell immunity test for spleen lymphocytes of mice with representative amino lipid compounds in Experimental Example 5.

FIG. 4 shows the results of Elispot cell immunity test for spleen lymphocytes of mice with representative amino lipid compounds in Experimental Example 5.

FIG. 5 shows the IgG titer of serum from mice immunized for 14 days with LNP containing IN002.5.1 mRNA

encapsulated with representative amino lipid compounds in Experimental Example 6.
FIG. 6 shows the IgG titer of serum from mice immunized for 14 days with LNP containing N002.5.1 mRNA encapsulated with representative amino lipid compounds in Experimental Example 6.

In order to make the purposes, technical solutions, and advantages of the present disclosure clearer, the present disclosure is described below with reference to specific examples. The follow examples are merely illustrative of the present disclosure and are not intended to be limiting.

**Examples**

The following examples are provided for purposes of illustration and not limitation.
The experimental methods for which specific conditions are not specified in the examples are usually under conventional conditions or conditions as recommended by the manufacturer of the raw material or commodity; and the reagents of unspecified origin are generally conventional reagents commercially available.
The abbreviations used in the examples have the following meanings:

| | |
|---|---|
| DSC | N,N'-disuccinimidyl carbonate; |
| TLC | Thin layer chromatography; |
| EA | Ethyl acetate; |
| DCM | Dichloromethane; |
| TEA | Triethylamine; |
| PMA | Phosphomolybdic acid; |
| THF | Tetrahydrofuran; |
| DMF | N,N-dimethylformamide; |
| EDCI | 1-(3-dimethylaminopropyl)-3-ethylcarbodiimide hydrochloride; |
| DMAP | 4-dimethylaminopyridine; |
| TEA 3HF | Triethylamine trihydrofluoride; |
| M-DMG-2000 | Methoxy PEG Dimyristoyl-rac-glycero; |
| h | Hour |
| min | Minute. |

**Example 1: Synthesis of Amino Lipid Compound 108**

(1) Synthesis of Compound 108-M3

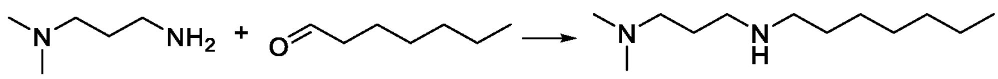

M1 108-M2 108-M3

N, N-dimethylaminopropylamine (108-M1) (3.2 g, 31.53 mmol), heptanal (108-M2) (3 g, 26.26 mmol), ethanol (15 mL), and palladium on carbon (0.15 g) were added to a 25 mL single-necked flask. The flask was transferred to a 500 mL autoclave, replaced with nitrogen for three times, then filled with hydrogen to 2.0 MPa, then deflated to 0.5 MPa, repeated for three times, and finally filled with hydrogen to 1.0 MPa. The mixture was reacted at room temperature for about 3 h.
TLC was used for monitoring, using EA: n-hexane = 1:5 for developing, and a Rf = 0.8 for the starting material heptanal was detected; after developing with methanol: DCM = 1:1 and fumigating with iodine, a Rf = 0.2 for the product (108-M3) and a Rf = 0.1 for the starting material N,N-dimethylaminopropylamine (108-M1) were detected. After the reaction was completed, the reaction mixture was subjected to suction filtration, recovery of palladium on carbon, and concentration of the filtrate to obtain 4.6 g crude 108-M3. The crude 108-M3 was purified by silica gel column chromatography, eluted with EA: methanol = 3:1 to obtain 3.30 g 108-M3 with a yield of 52%.

(2) Synthesis of Compound M5

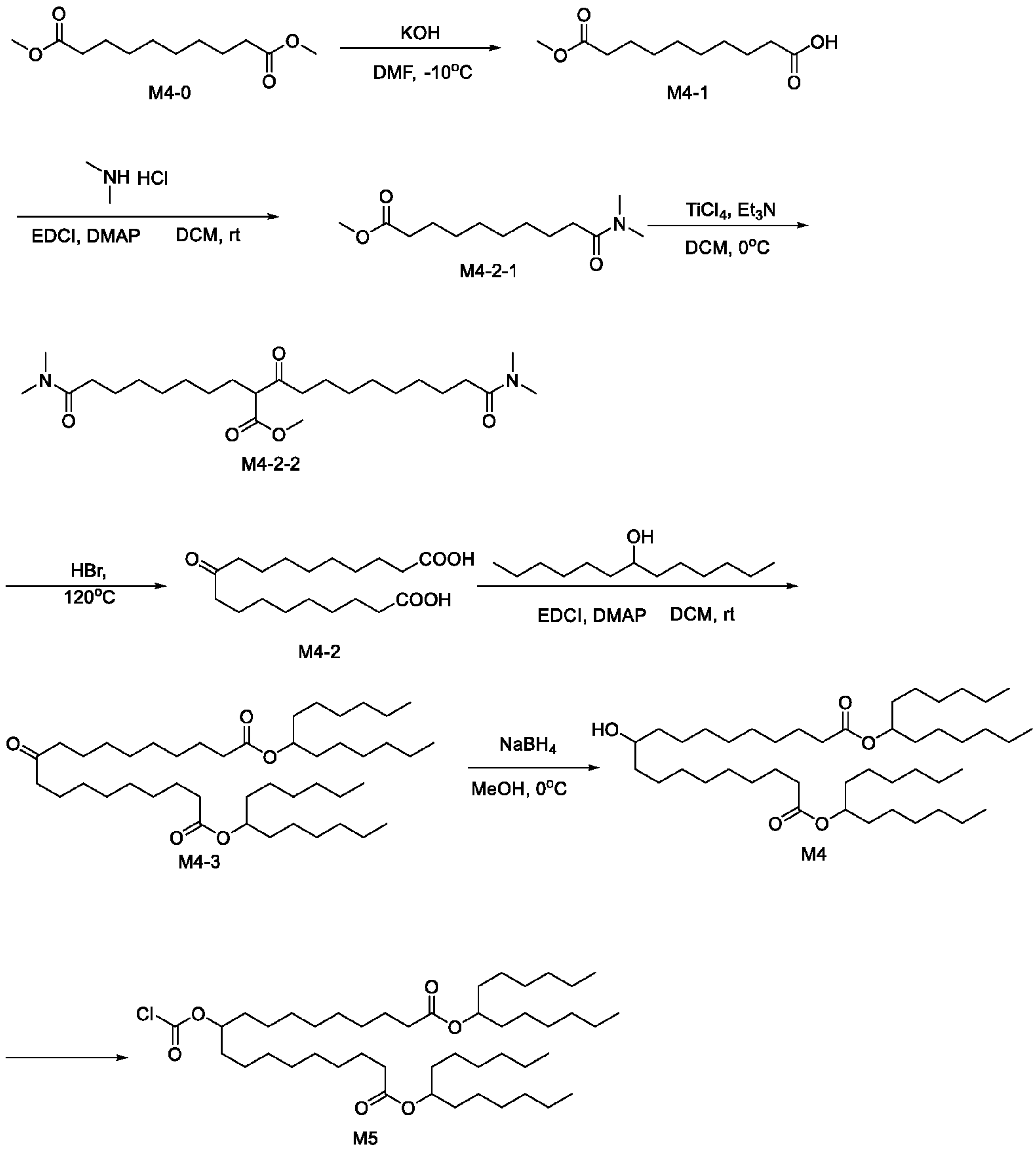


1) Synthesis of Compound M4-1

Dimethyl sebacate (M4-0) (170 g, 738.2 mmol) was added to a 2 L single-necked round bottom flask, and 850 ml DMF was added and cooled to -10°C, added with 46 g potassium hydroxide powder while stirring, and reacted at -10°C for 20 h. The reaction solution was diluted with 1000 mL water, and extracted with 500 mL EA, and the solvent was removed by evaporation to obtain a crude product, which was purified by silica gel column chromatography, eluted with EA: n-hexane = 1:5 to obtain 80.0 g monomethyl sebacate (M4-1) with a yield of 50%.

2) Synthesis of Compound M4-2

M4-1 (120 g, 555 mmol), dimethylamine hydrochloride (59 g), EDCI (149 g), and DMAP (6.8 g) were sequentially added to a 2 L single-necked round-bottom flask, dissolved in 1.2 L DCM, and well stirred, and pyridine (123 g) was finally added, and stirred at room temperature overnight. 1 L water was added to dilute, and pH was adjusted to 3 with diluted hydrochloric acid. The organic phase was separated, and the aqueous phase was extracted with 500 mL DCM. The organic phases were combined, and the solvent was removed by evaporation to obtain 126 g crude methyl 10-(dimethylamino)-10-oxodecanoate (M4-2-1), which was directly used in the next reaction.

Crude M4-2-1 (126 g) was added to a 1L four-necked flask, dissolved in DCM (600 ml), cooled to 0°C under the

protection of nitrogen. Titanium tetrachloride (118 g) was added dropwise, then TEA (73.4g) was added dropwise, followed by reacting at 0°C for 2 h. 600 mL water was slowly added for quenching with stirring at 0°C. The organic phase was separated, and the aqueous phase was extracted with 300 ml DCM. The organic phases were combined, and the solvent was removed by evaporation to obtain 133 g methyl 12-(dimethylamino)-2-(8-dimethylamino)-8-oxooctyl)-3,12-dioxodecanoate (M4-2-2), which was directly used in the next reaction without further purification.

M4-2-2 (133 g) was added in a 1L single-necked flask, then added with hydrobromic acid (500 ml, 48% aqueous solution) and refluxed at 120°C overnight. The mixture was cooled to room temperature with stirring, then stirred at -10°C for 20 min, and filtered with suction. The filter cake was washed with about 100 mL water to obtain 120 g crude 10-oxononadecanedioic acid (M4-2).

Crude M4-2 (120 g) and acetonitrile (1 L) were added to a 2L single-necked flask and heated to 85 °C and refluxed for 0.5 h. After cooled to room temperature, the mixture was stirred at -10°C for 20 min, and filtered with suction. The filter cake was rinsed with about 100 mL cold acetonitrile, and transferred to a flask, and the residual solvent was removed by evaporation to obtain 45g M4-2 as an off-white solid.

$^1H$ NMR (600 MHz, DMSO-d6) $\delta$ 11.94 (brs, 1H), 2.37 (t, J = 7.3, 4H), 2.18 (t, J = 7.5, 4H), 1.50-11.41 (m, 8H), 1.24-1.19 (m, 16H).

LC-MS (ESI): (M-1) calculated 341.23, found 341.4.

3) Synthesis of Compound M4-3

EDCI (16.8 g), DCM (100 ml), triethylamine (8.86 g), DMAP (1.78 g), 7-tridecanol (10.5 g), and M4-2 (10.0 g, 29.2 mol) were sequentially added to a 500 mL round-bottom flask, and stirred at room temperature overnight. By TLC monitoring, the conversion of 7-tridecanol was almost complete (developing with EA: n-hexane = 1:25, staining with PMA, Rf = 0.4 for the starting material 7-tridecanol, Rf = 0.5 for the product DTN-T), and the reaction was ended. 200 mL DCM and 300 mL water were added for extraction, the liquid was separated, the organic phase was collected, and the solvent was removed by evaporation to obtain 30 g yellow oily crude product, which was purified by silica gel column chromatography, eluted with EA: n-hexane = 1:25 to obtain 15.72 g M4-3 with a yield of 85%.

$^1H$ NMR (600 MHz, $CDCl_3$) $\delta$ 4.88-4.84 (m, 2H), 2.37 (t, J = 7.5, 4H), 2.27 (t, J = 7.5, 4H), 1.64-1.48 (m, 16H), 1.31-1.26 (m, 48H), 0.87 (t, J = 6.9, 12H).

4) Synthesis of Compound M4

M4-3 (30.0 g, 42.4 mmol) was added to a 1L single-necked flask, dissolved in methanol (300 ml), and cooled to 0°C. Sodium borohydride (1.7 g) was slowly added with stirring, and reacted at 0°C for 1 h after addition, then adjusted to pH 6 with diluted hydrochloric acid. The solvent was removed by evaporation. 300 mL DCM and 300 mL water were added for extraction, the organic phase was collected, and the solvent was removed by evaporation to obtain 42.7 g crude product, which was purified by silica gel column chromatography, eluted with EA: n-hexane = 1:15 to obtain 24.6 g M4.

$^1H$ NMR (600 MHz, $CDCl_3$) $\delta$ 4.89-4.84 (m, 2H), 3.58-3.56 (m, 1H), 2.27 (t, J = 7.5, 4H), 1.62-1.59 (m, 4H), 1.50-1.26 (m, 64H), 0.87 (t, J = 6.9, 12H).

5) Synthesis of Compound M5

M4 (24 g, 33.8 mmol), toluene (240 ml), and TEA (5.1 g) were sequentially added to a 500 ml single-necked flask, stirring was started at room temperature, and triphosgene (15 g) was slowly added. After the addition, the temperature was raised to 70 °C to react for 2 h. Samples were taken for monitoring (TLC, EA: n-hexane = 1:9), staining with PMA, Rf = 0.2 for the starting material and Rf = 0.8 for the product) until the reaction was completed.

The reaction solution was cooled to room temperature, filtered with suction. The filter cake was washed with toluene. The filtrate was collected, and the solvent was removed by evaporation. The residue was purified by silica gel column chromatography, and eluted with EA: n-hexane = 1: 5-25 to obtain 21.6 g M5 as a light yellow transparent oil with a yield of 83%.

$^1H$ NMR (600 MHz, $CDCl_3$) $\delta$ 4.90-4.85 (m, 3H), 2.28 (t, J = 7.5, 4H), 1.62-1.59 (m, 4H), 1.65-1.59 (m, 8H), 1.51-1.49 (m, 8H), 1.37-1.22 (m, 52H), 0.88 (t, J = 7.1, 12H).

(3) Synthesis of Amino Lipid Compound 108

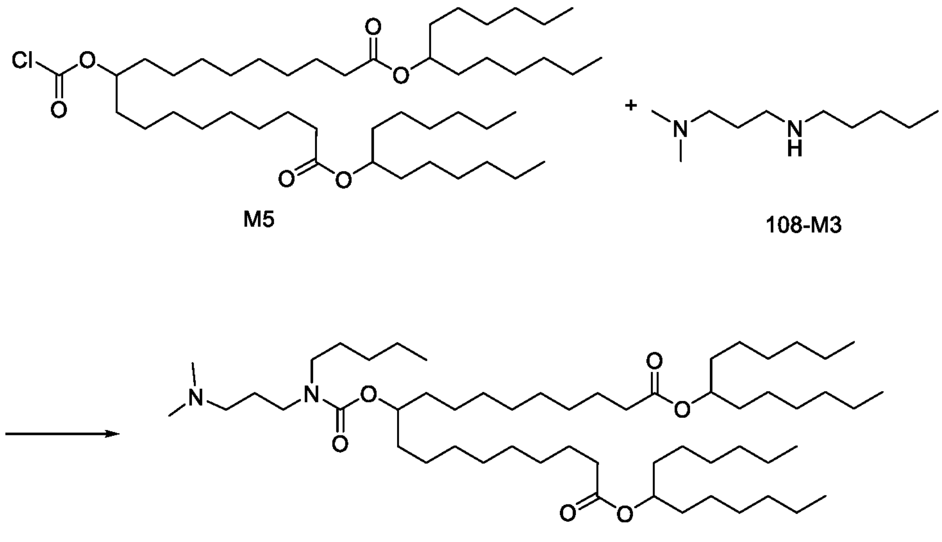

M5

108-M3

108

108-M3 (266 mg, 1.55 mmol), THF (10 ml), and potassium carbonate (178.3 mg) were sequentially added to a 50 ml single-necked flask, stirred and cooled to 0°C. M5 (1.0 g, 1.29 mmol) and THF (10 ml) were added through a constant pressure dropping funnel, with M5 being slowly added dropwise to the single-necked flask over about 10 min. After dropwise addition was completed, the mixture was stirred at 0°C for 5 min, and then reacted at room temperature. After 1.5 h, the reaction was monitored by TLC (EA: n-hexane = 30:1, developed with PMA, the product and the starting material 108-M3 were at the origin, and compound 108-M3 had a Rf = about 0.5) until the reaction was completed. 100 ml water and 100 ml EA were added to the reaction solution for extraction, the organic phase was collected, and the aqueous phase was extracted with 100 ml EA for three times. The organic phases were combined, dried with anhydrous sodium sulfate, and the solvent was removed by evaporation to obtain 1.1 g crude product, which was purified by silica gel column chromatography, eluted with EA: n-hexane = 1:5 to obtain 630 mg amino lipid compound 108, as a colorless oil with a yield of 53.8% and a purity of 97.41%.

$^1H$ NMR (600 MHz, $CDCl_3$) δ 4.88-4.84 (m, 2H), 4.74 (m, 1H), 3.25-3.17 (m, 4H), 2.26 (t, J = 7.5, 6H), 2.21 (s, 6H), 1.70 (m, 2H), 1.62-1.58 (m, 4H), 1.50-1.49 (m, 14H), 1.30-1.25 (m, 56H), 0.90-0.86 (m, 15H).

LC-MS (ESI): (M + H) calculated 907.8, found 908.2.

**Example 2: Synthesis of Amino Lipid Compound 109**

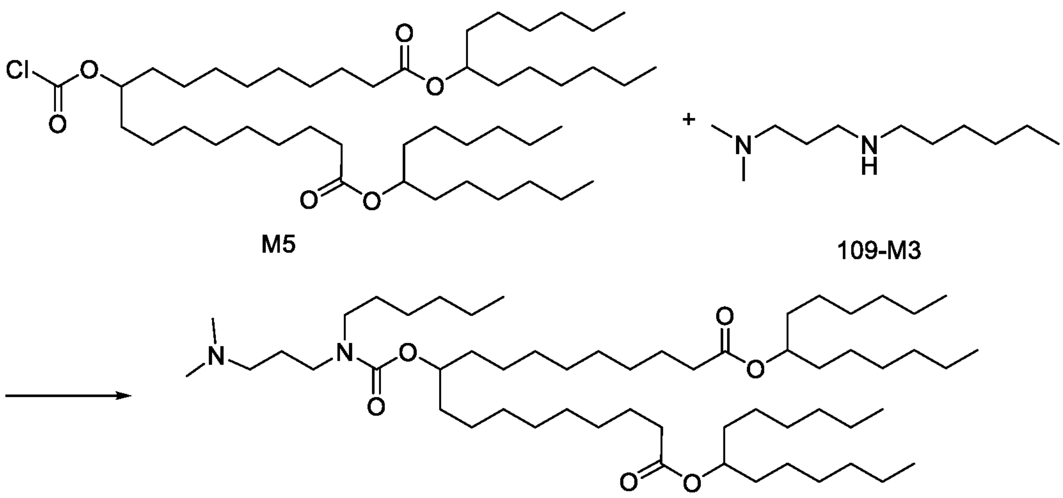

M5

109-M3

109

According to the general synthetic process, 190 mg amino lipid compound 109, as a colorless oil with a yield of

21% and a purity of 94.94%, was prepared from compound M5 (1.0 g, 1.29 mmol) and 109-M3 (1.55 mmol).

$^1$H NMR (600 MHz, $CDCl_3$) δ 4.88-4.84 (m,2H), 4.74 (m,1H), 3.25-3.17 (m,4H), 2.27 (t,J=7.5,6H), 2.22(s,6H), 1.70 (m,2H), 1.72-1.69 (m,4H), 1.63-1.58 (m,14H), 1.33-1.26 (m,58H), 0.87 (t,J=7.0,15H).

LC-MS (ESI): (M + H) calculated 921.9, found 922.2.

**Example 3: Synthesis of Amino Lipid Compound 110**

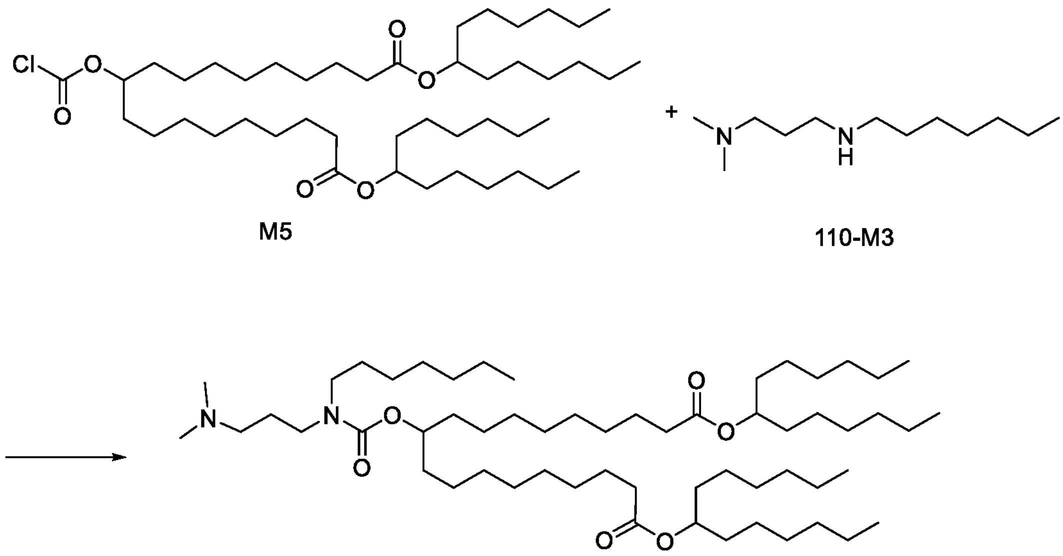

M5

110-M3

110

According to the general synthetic process, 640 mg amino lipid compound 110, as a colorless oil with a yield of 60% and a purity of 97.71%, was prepared from compound M5 (1.0 g, 1.29 mmol) and 110-M3 (1.55 mmol).

$^1$H NMR (600 MHz, $CDCl_3$) δ 4.88-4.84 (m,2H), 4.74 (m,1H), 3.25-3.17 (m,4H), 2.27 (t,J=7.5,6H), 2.22 (s,6H), 1.72-1.70 (m,2H), 1.63-1.58 (m,4H), 1.51-1.50 (m,14H), 1.28-1.26 (m,60H), 0.88-0.86 (m,15H).

LC-MS (ESI): (M + H) calculated 935.9, found 936.3.

**Example 4: Synthesis of Amino Lipid Compound 111**

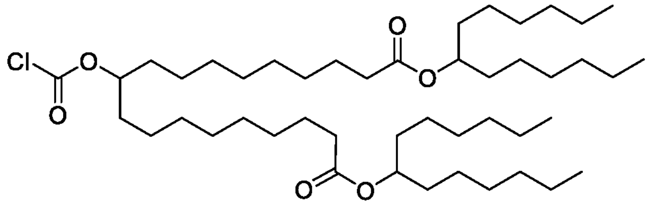

M5

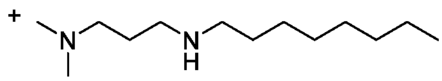

111-M3

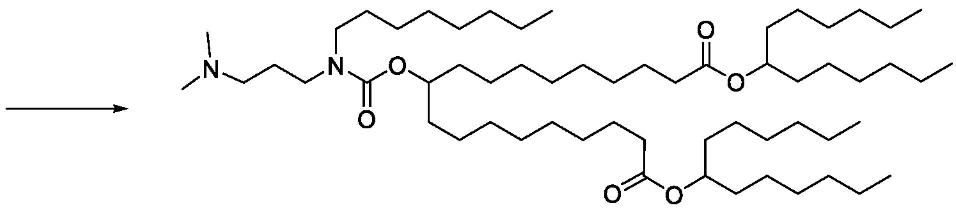

111

According to the general synthetic process, 510 mg compound 111, as a colorless oil with a yield of 47% and a purity of 97.18%, was prepared from compound M5 (1.0 g, 1.29 mmol) and 111-M3 (1.55 mmol).

$^{1}H$ NMR (600 MHz, $CDCl_3$) $\delta$ 4.88-4.84 (m,2H), 4.73 (m,1H), 3.25-3.16 (m,4H), 2.26 (t,J=7.5,6H), 2.21 (s,6H), 1.70 (m,2H), 1.62-1.57 (m,4H), 1.50-1.49 (m,14H), 1.27-1.25 (m,62H), 0.88-0.86 (m,15H).

LC-MS (ESI): (M + H) calculated 949.9, found 950.3.

**Example 5: Synthesis of Amino Lipid Compound 112**

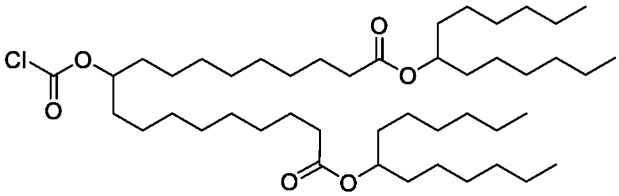

M5

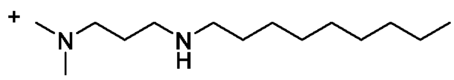

112-M3

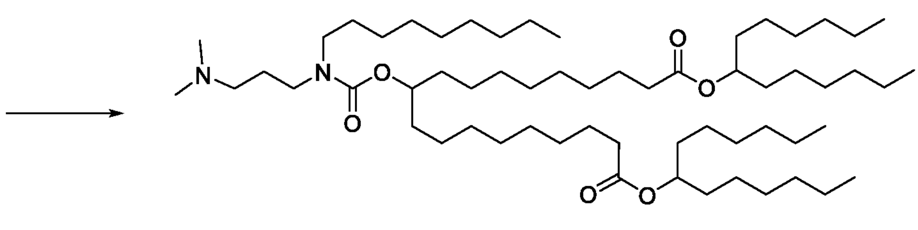

112

According to the general synthetic process, 760 mg amino lipid compound 112, as a colorless oil with a yield of 69% and a purity of 94.69%, was prepared from compound M5 (1.0 g, 1.29 mmol) and 112-M3 (1.55 mmol).

$^{1}H$ NMR (600 MHz, $CDCl_3$) $\delta$ 4.88-4.84 (m,2H), 4.73 (m,1H), 3.25-3.16 (m,4H), 2.26 (t,J=7.5,6H), 2.21 (s,6H), 1.71-1.69 (m,2H), 1.62-1.58 (m,4H), 1.50-1.49 (m,14H), 1.27-1.25 (m,64H), 0.88-0.86 (m,15H).

LC-MS (ESI): (M + H) calculated 963.9, found 964.3.

**Example 6: Synthesis of Amino Lipid Compound 113**

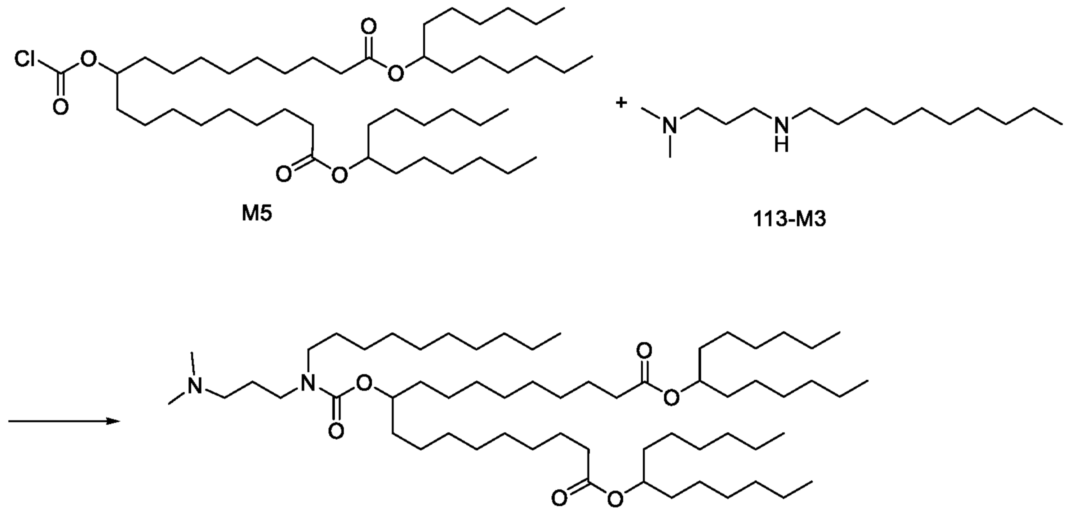

M5

113-M3

113

According to the general synthetic process, 640 mg amino lipid compound 113, as a colorless oil with a yield of 58% and a purity of 98.04%, was prepared from compound M5 (1.0 g, 1.29 mmol) and 113-M3 (1.55 mmol).

$^1$H NMR (600 MHz, $CDCl_3$) δ 4.89-4.85 (m,2H), 4.74 (m,1H), 3.26-3.17 (m,4H), 2.27 (t,J=7.5,6H), 2.22 (s,6H), 1.71-1.70 (m,2H), 1.63-1.58 (m,4H), 1.51-1.50 (m,14H), 1.28-1.25 (m,66H), 0.89-0.86 (m,15H).

LC-MS (ESI): (M + H) calculated 977.9, found 978.4.

**Example 7: Synthesis of Amino Lipid Compound 114**

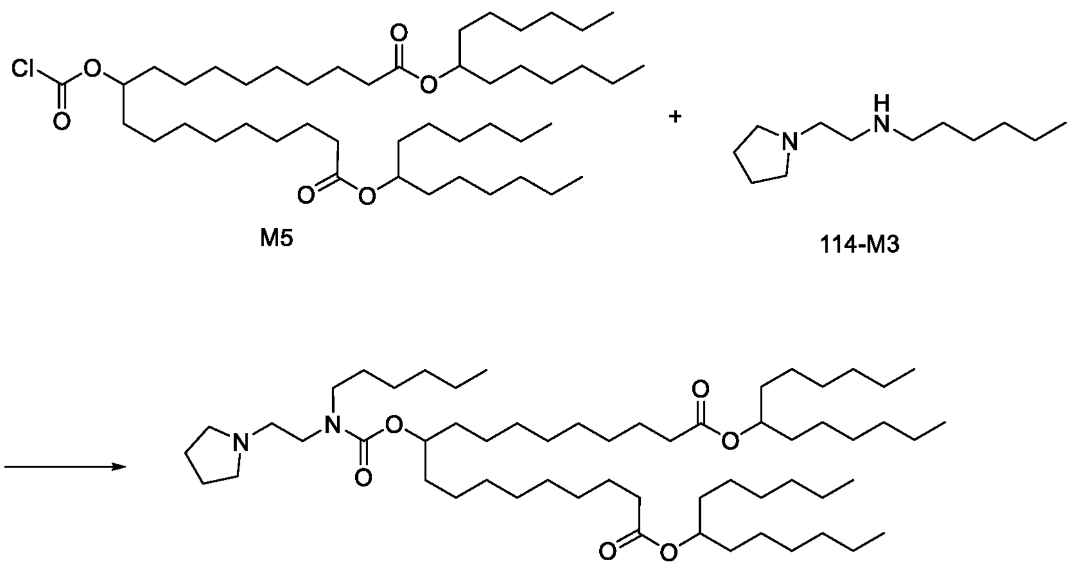

M5

114-M3

114

According to the general synthetic process, 270 mg amino lipid compound 114, as a colorless oil with a yield of 22% and a purity of 75.59%, was prepared from compound M5 (1.0 g, 1.29 mmol) and 114-M3 (1.55 mmol).

$^1$H NMR (600 MHz, $CDCl_3$) δ4.89-4.84 (m, 2H),4.74-4.72 (m, 1H), 3.40-3.33 (m, 2H), 3.24-3.19 (m, 2H), 2.63-2.53 (m, 6H), 2.27 (t, J=7.5, 4H),1.78-1.74 (m, 4H), 1.63-1.58 (m, 4H), 1.51-1.50 (m, 14H), 1.33-1.26 (m, 58H), 0.89-0.86 (m, 15H).

LC-MS (ESI): (M + H) calculated 933.86, found 934.3.

**Example 8: Synthesis of Amino Lipid Compound 115**

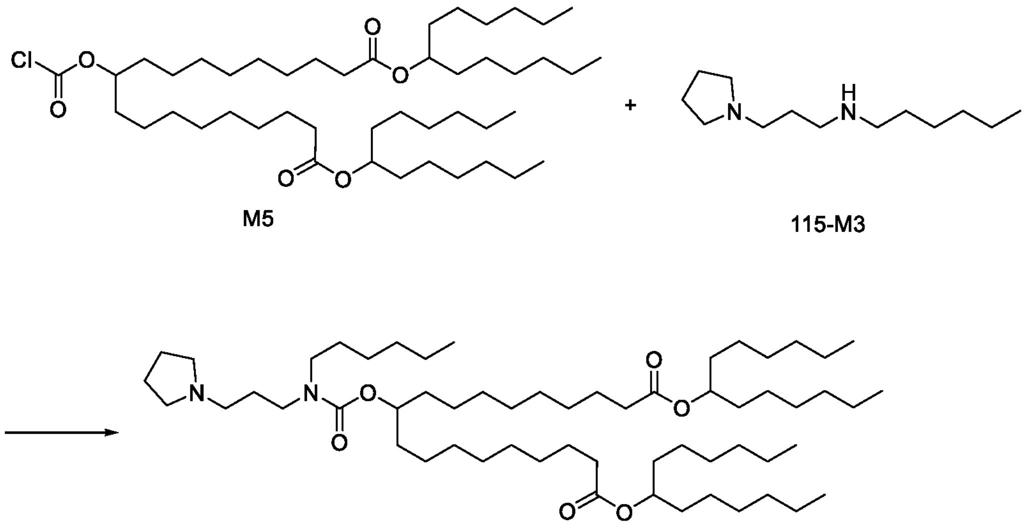

M5

115-M3

115

According to the general synthetic process, 468 mg amino lipid compound 115, as a colorless oil with a yield of 38% and a purity of 77.72%, was prepared from compound M5 (1.0 g, 1.29 mmol) and 115-M3 (1.55 mmol).

$^1$H NMR (600 MHz, $CDCl_3$) δ4.88-4.84 (m, 2H),4.74-4.72 (m, 1H), 3.28-3.16 (m, 4H), 2.48-2.43 (m, 6H), 2.27 (t, J = 7.5, 4H),1.77-1.74 (m, 6H), 1.63-1.58 (m, 4H), 1.50-1.49 (m, 14H), 1.33-1.26 (m, 58H), 0.88-0.86 (m, 15H).

LC-MS (ESI): (M + H) calculated 947.87, found 948.3.

**Example 9: Synthesis of Amino Lipid Compound 117**

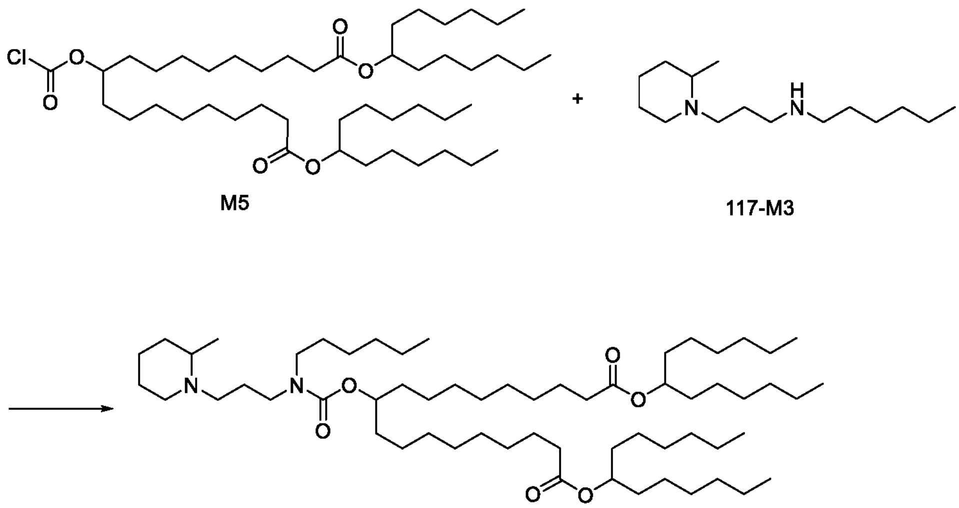

M5

117-M3

117

According to the general synthetic process, 140 mg amino lipid compound 117, as a colorless oil with a yield of 13% and a purity of 97.21%, was prepared from compound M5 (1.0 g, 1.29 mmol) and 117-M3 (1.55 mmol).

$^1$H NMR (600 MHz, $CDCl_3$) δ4.88-4.84 (m, 2H),4.73 (m, 1H), 3.21-3.16 (m, 4H), 2.84 (m, 1H), 2.65 (m, 1H), 2.31-2.25 (m, 6H), 2.11 (m, 1H), 1.69-1.58 (m, 9H), 1.50-1.49 (m, 15H), 1.33-1.26 (m, 60H), 1.04 (d, J = 6.1, 3H), 0.88-0.86 (m, 15H).

LC-MS (ESI): (M + H) calculated 975.9, found 976.4.

**Example 10: Synthesis of Amino Lipid Compound 137**

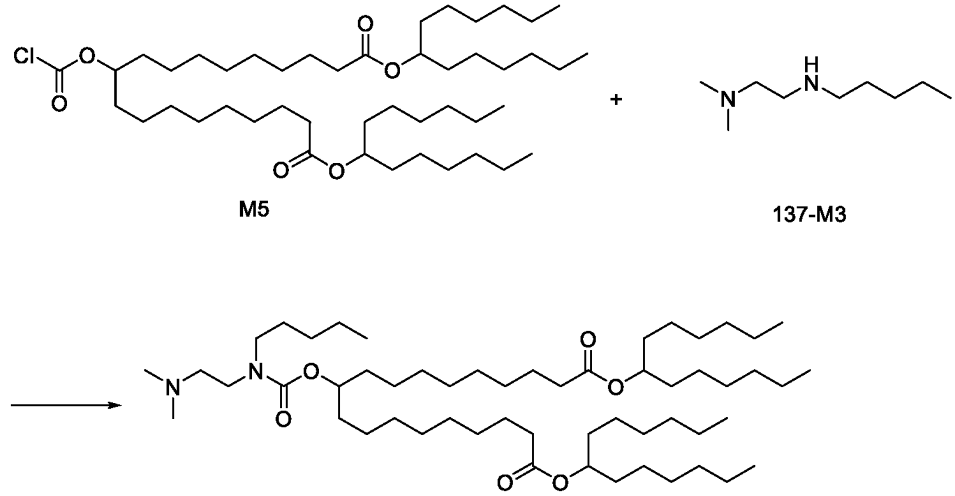

M5

137-M3

137

According to the general synthetic process, 200 mg amino lipid compound 137, as a colorless oil with a yield of 20% and a purity of 97.46%, was prepared from compound M5 (1.0 g, 1.29 mmol) and 137-M3 (1.55 mmol).

$^1$H NMR (600 MHz, $CDCl_3$) δ 5.00-4.83 (m, 2H), 4.77 (s, 1H), 3.38-3.33 (m, 2H), 3.29-3.17 (m, 2H), 2.60-2.44 (m, 2H), 2.32-2.29 (m, 10H), 1.69-1.60 (m, 4H), 1.56-1.54 (m, 14H), 1.34-1.30 (m, 56H), 0.94-0.90 (m, 15H).

LC-MS (ESI): (M + H) calculated 893.82, found 894.3.

**Example 11: Synthesis of Amino Lipid Compound 138**

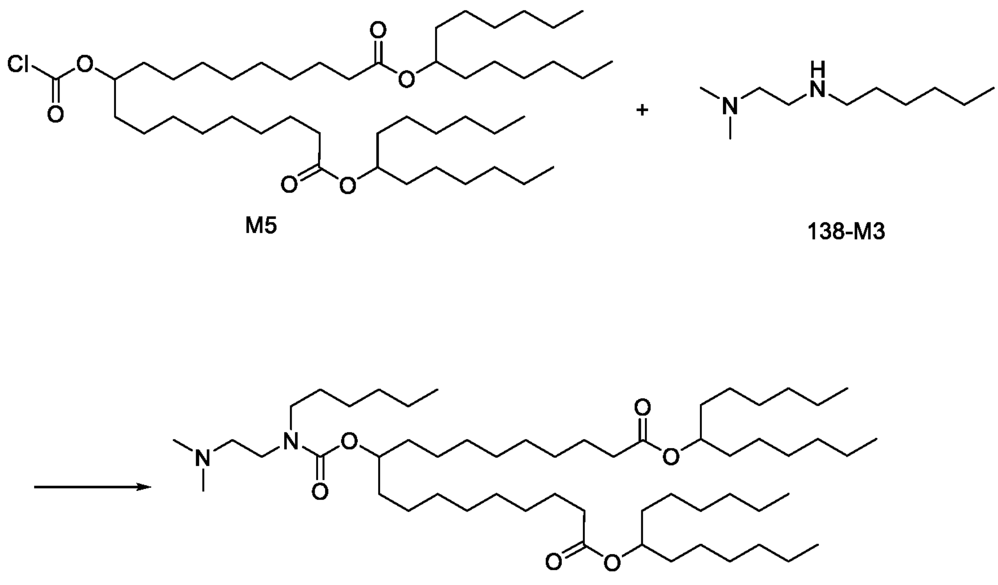

M5

138-M3

138

According to the general synthetic process, 490 mg amino lipid compound 138, as a colorless oil with a yield of 42% and a purity of 77.73%, was prepared from compound M5 (1.0 g, 1.29 mmol) and 138-M3 (1.55 mmol).

$^1$H NMR (600 MHz, $CDCl_3$) δ4.88-4.84 (m, 2H), 4.73 (m, 1H), 3.35-3.28 (m, 2H), 3.23-3.19 (m, 2H), 2.45-2.40 (m,

2H), 2.28-2.25 (m, 10H), 1.63-1.58 (m, 4H), 1.51-1.50 (m, 14H), 1.33-1.26 (m, 58H), 0.90-0.86 (m, 15H).

LC-MS (ESI): (M + H) calculated 907.84, found 908.3.

**Example 12: Synthesis of Amino Lipid Compound 139**

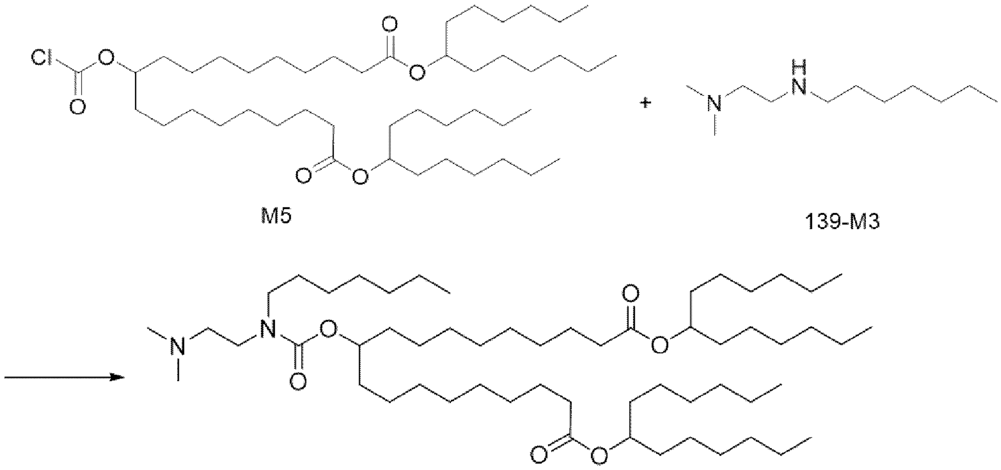

M5

139-M3

139

According to the general synthetic process, 470 mg amino lipid compound 139, as a colorless oil with a yield of 45% and a purity of 95.87%, was prepared from compound M5 (1.0 g, 1.29 mmol) and 139-M3 (1.55 mmol).

$^1H$ NMR (600 MHz, $CDCl_3$) δ4.88-4.84 (m, 2H), 4.73 (m, 1H), 3.35-3.28 (m, 2H), 3.23-3.19 (m, 2H), 2.46-2.40 (m, 2H), 2.28-2.26 (m, 10H), 1.63-1.58 (m, 4H), 1.51-1.50 (m, 14H), 1.33-1.26 (m, 60H), 0.88-0.86 (m, 15H).

LC-MS (ESI): (M + H) calculated 921.86, found 922.3.

**Example 13: Synthesis of Amino Lipid Compound 140**

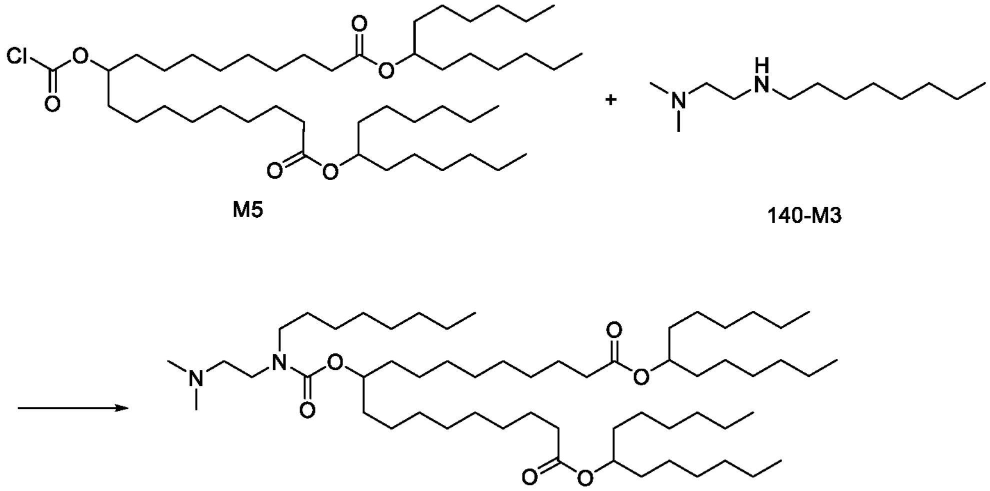

**M5**

**140-M3**

**140**

According to the general synthetic process, 470 mg amino lipid compound 140, as a colorless oil with a yield of 44% and a purity of 96.31%, was prepared from compound M5 (1.0 g, 1.29 mmol) and 140-M3 (1.55 mmol).

$^1H$ NMR (600 MHz, $CDCl_3$) δ4.87-4.84 (m, 2H), 4.73 (m, 1H), 3.35-3.28 (m, 2H), 3.23-3.19 (m, 2H), 2.46-2.40 (m, 2H), 2.28-2.26 (m, 10H), 1.63-1.58 (m, 4H), 1.51-1.50 (m, 14H), 1.33-1.26 (m, 62H), 0.88-0.86 (m, 15H).

LC-MS (ESI): (M + H) calculated 935.87, found 936.3.

**Example 14: Synthesis of Amino Lipid Compound 141**

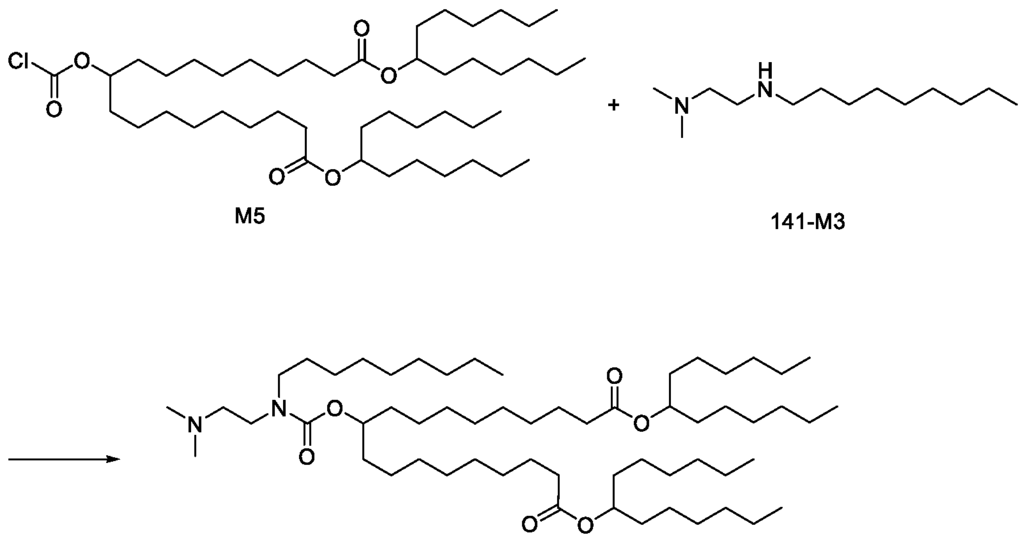

M5 141-M3

141

According to the general synthetic process, 330 mg amino lipid compound 141, as a colorless oil with a yield of 31% and a purity of 95.98%, was prepared from compound M5 (1.0 g, 1.29 mmol) and 141-M3 (1.55 mmol).

$^1H$ NMR (600 MHz, $CDCl_3$) δ4.89-4.84 (m, 2H), 4.73 (m, 1H), 3.35-3.28 (m, 2H), 3.23-3.19 (m, 2H), 2.46-2.40 (m, 2H), 2.28-2.26 (m, 10H), 1.63-1.58 (m, 4H), 1.51-1.50 (m, 14H), 1.33-1.26 (m, 62H), 0.88-0.86 (m, 15H).

LC-MS (ESI): (M + H) calculated 949.89, found 950.3.

**Example 15: Synthesis of Amino Lipid Compound 142**

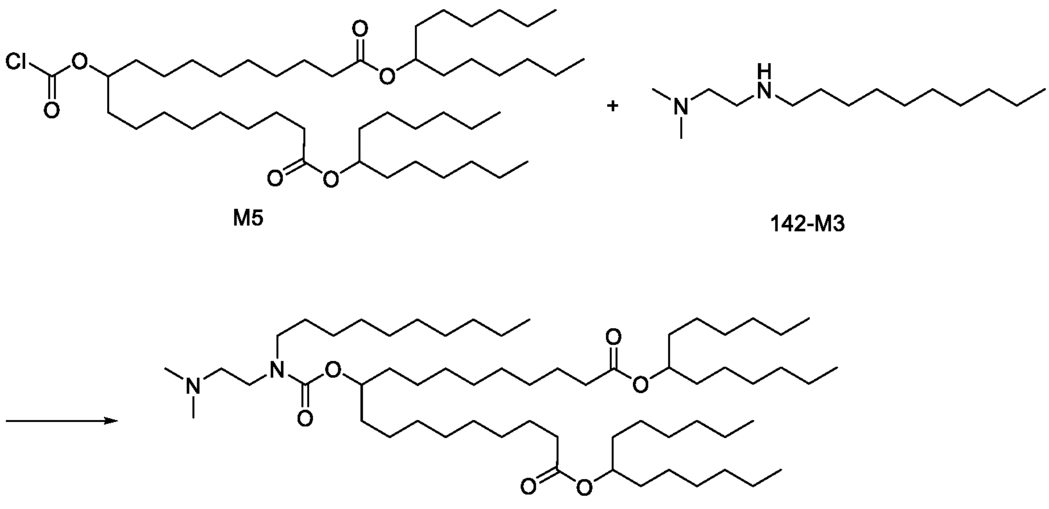

M5 142-M3

142

According to the general synthetic process, 580 mg amino lipid compound 142, as a colorless oil with a yield of 53% and a purity of 98.23%, was prepared from compound M5 (1.0 g, 1.29 mmol) and 142-M3 (1.55 mmol).

$^1H$ NMR (600 MHz, $CDCl_3$) δ4.89-4.84 (m, 2H), 4.73 (m, 1H), 3.35-3.28 (m, 2H), 3.23-3.19 (m, 2H), 2.46-2.40 (m, 2H), 2.28-2.26 (m, 10H), 1.63-1.58 (m, 4H), 1.51-1.50 (m, 14H), 1.28-1.26 (m, 66H), 0.89-0.86 (m, 15H).

LC-MS (ESI): (M + H) calculated 963.9, found 964.3

**Example 16: Synthesis of Amino Lipid Compound 255**

(1) Synthesis of Compound 255-B

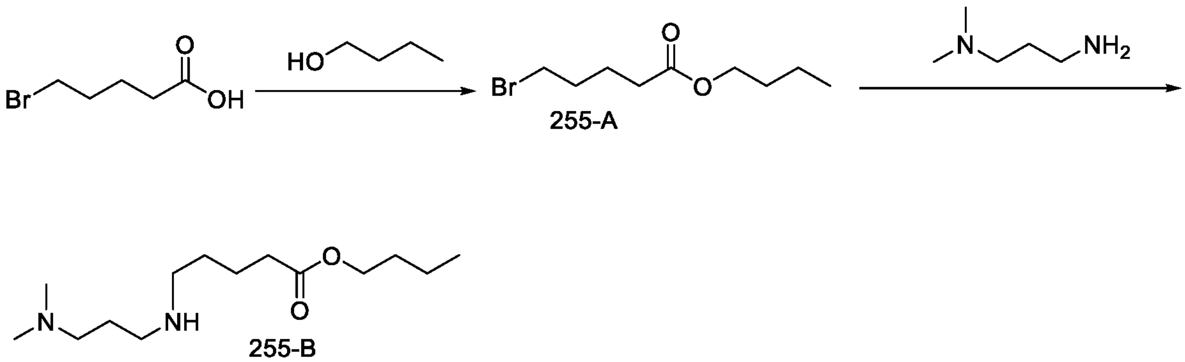

255-A

255-B

1) Synthesis of Compound 255-A

5-bromopentanoic acid (20.0 g, 110.5 mmol), DMF (269 mg, 3.7 mmol), and DCM (50 ml) were added to a 500 ml single-necked flask, cooled to 0°C while stirring, and thionyl chloride (16.29 g, 221 mmol) was slowly added dropwise at 0°C. After maintaining the temperature for 30 min, the mixture was transferred to room temperature to react overnight. The solvent and excess thionyl chloride were removed by evaporation under reduced pressure to obtain 24 g acyl chloride compound.

N-butanol (5.46 g, 73.7 mmol) was weighed and dissolved in DCM (50 ml), cooled to 0°C while stirring. The newly prepared acyl chloride was added dropwise at 0°C. After the completion of addition dropwise, the reaction solution was heated to room temperature, and added with 200 mL water after reacting for 5 h, and extracted twice with 200 mL DCM. The organic phases were combined, washed with 100 ml brine, dried over anhydrous sodium sulfate, and the solvent was removed by evaporation to obtain 28 g crude 255-A.

The crude product was purified by silica gel column chromatography, eluting with EA: n-heptane = 1:25 to obtain 15.2 g 255-A, as a light yellow oil with a yield of 83%.

2) Synthesis of Compound 255-B

3-dimethylaminopropylamine (9.8 g, 96.2 mmol) and acetonitrile (75ml) were added to a 500ml single-necked flask, dissolved by stirring, and added with potassium carbonate (6.64 g, 48.1 mmol) and cooled to -10°C. 255-A obtained in step 1) was dissolved in acetonitrile (75 ml), and slowly dropped into the reaction system. After the completion of dropwise addition, the mixture was reacted at -10°C for 5 h, and then transferred to room temperature to react overnight. The solvent was removed by evaporation. The residue was extracted with water (100 ml) and ethyl acetate (100 ml) for three times. The organic phases were combined, washed with 50 ml saturated aqueous sodium chloride solution, dried over anhydrous sodium sulfate, filtered, and the filtrate was concentrated to obtain 10.74 g crude 255-B, which was directly used in the next reaction.

(2) Synthesis of Amino Lipid Compound 255

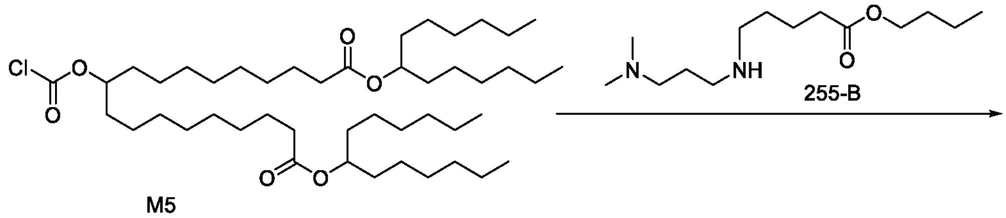

M5

255-B

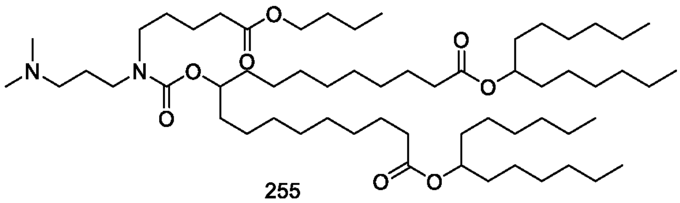

255

Crude 255-B (2.5 g, 9.72 mmol) and THF (50 ml) were added to a 250 mL flask, dissolved with stirring, and cooled to 0°C. M5 (5 g, 6.48 mmol) was weighed and dissolved in THF (50 ml), and slowly dropped into the reaction system. After the completion of dropwise addition, the mixture was reacted at 0 °C for 1 h, extracted with water (100 ml) and EA (100 ml) for three times. The organic phases were combined, washed sequentially with 50 ml water and 50 ml saturated aqueous sodium chloride solution, dried over anhydrous sodium sulfate, and filtered. The filtrate was concentrated to obtain 8 g crude 225-B, which was purified by silica gel column chromatography, eluted with EA: n-heptane = 1:25 to obtain 3.25 g amino lipid compound 225, as a light yellow oil with a purity of 94.68% and a yield of 50%.

$^1$H NMR (600 MHz, $CDCl_3$) δ 4.96-4.85 (m, 2H), 4.83-4.72 (m, 1H), 4.06 (t, J = 6.7 Hz, 2H), 3.28 (m, 4H), 2.49-2.18 (m, 14H), 1.88-1.48 (m, 26H), 1.37-1.17 (m, 52H), 0.97 (t, J = 7.4 Hz, 3H), 0.91 (t, J = 7.0 Hz, 12H).

LC-MS (ESI): (M + H) calculated 993.88, found 994.5.

### Example 17: Synthesis of Amino Lipid Compound 250

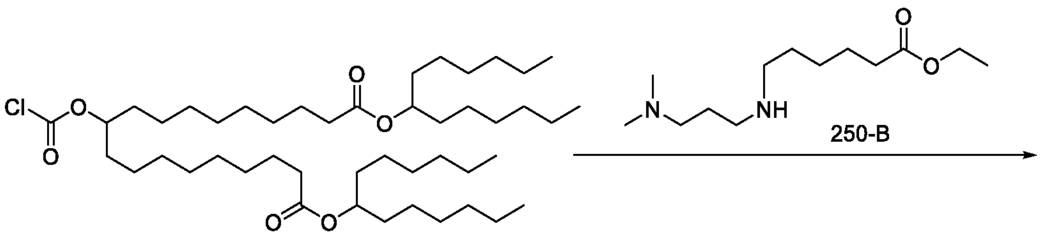

M5

250-B

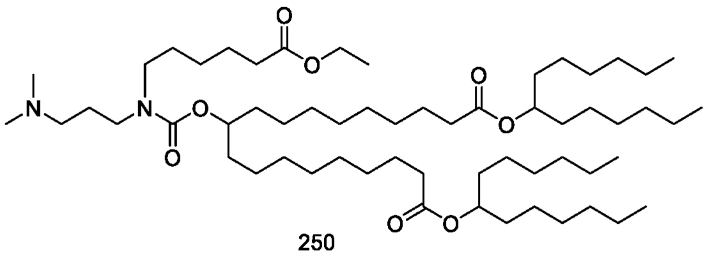

250

According to the general synthetic process, 600 mg amino lipid compound 250, as a pale yellow oil with a purity of 95.54% and a yield of 47%, was prepared from M5 (1.0 g, 1.29 mmol) and 250-B (0.47 g, 1.94 mmol).

$^1$H NMR (600 MHz, $CDCl_3$) δ 4.94-4.86 (m, 2H), 4.77 (m, 1H), 4.16 (q, J = 6.8 Hz, 2H), 3.34-3.14 (m, 4H), 2.32 (m, 8H), 2.25 (s, 4H), 1.54 (m, 25H), 1.38-1.24 (m, 56H), 0.91 (t, J = 7.0 Hz, 12H).

LC-MS (ESI): (M + H) calculated 979.86, found 980.4.

**Example 18: Synthesis of Amino Lipid Compound 251**

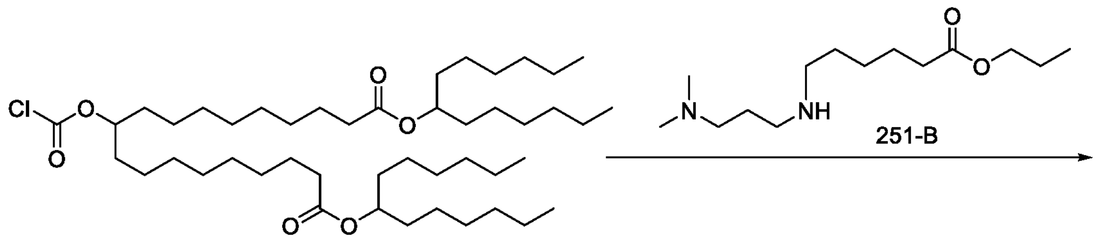


M5

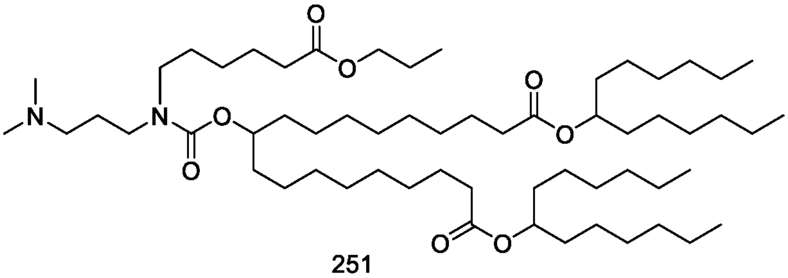

251

According to the general synthetic process, 1000 mg amino lipid compound 251, as a pale yellow oil with a purity of 95.54% and a yield of 52%, was prepared from M5 (1.5 g, 1.94 mmol) and 251-B (0.50 g, 1.94 mmol).
$^1H$ NMR (600 MHz, $CDCl_3$) δ 4.94-4.86 (m, 2H), 4.77 (m, 1H), 4.16 (q, J = 6.8 Hz, 2H), 3.34-3.14 (m, 4H), 2.32 (m, 8H), 2.25 (s, 4H), 1.54 (m, 27H), 1.38-1.24 (m, 53H), 0.97 (t, J = 7.4 Hz, 3H), 0.91 (t, J = 7.0 Hz, 12H).
LC-MS (ESI): (M + H) calculated 993.88, found 994.5.

**Example 19: Synthesis of Amino Lipid Compound 252**

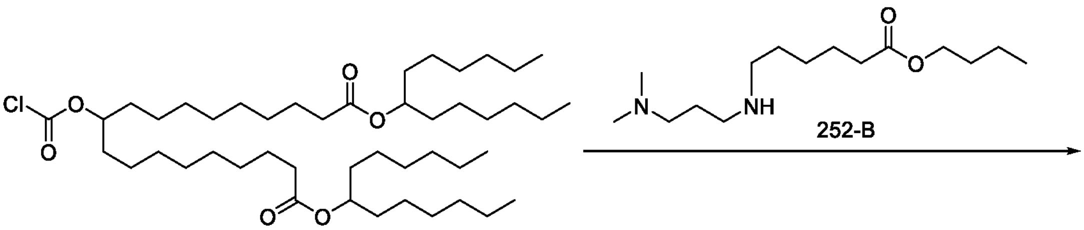


M5

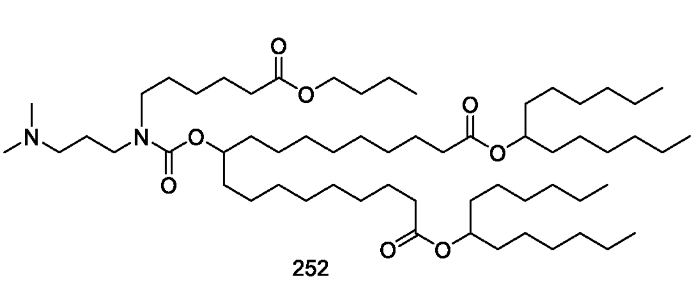

252

According to the general synthetic process, 650 mg amino lipid compound 252, as a pale yellow oil with a purity of 95.88% and a yield of 56%, was prepared from M5 (1.0 g, 1.29 mmol) and 252-B (0.53 g, 1.94 mmol).
$^1H$ NMR (600 MHz, $CDCl_3$) δ 4.94-4.86 (m, 2H), 4.77 (m, 1H), 4.16 (q, J = 6.8 Hz, 2H), 3.34-3.14 (m, 4H), 2.32 (m, 8H), 2.25 (s, 4H), 1.54 (m, 29H), 1.38-1.24 (m, 53H), 4.77 (t, J = 12.3, 6.1 Hz, 1H), 0.98 (t, J = 7.4 Hz, 3H), 0.92 (t, J = 7.0 Hz, 12H).

LC-MS (ESI): (M + H) calculated 1007.89, found 1008.5.

**Example 20: Synthesis of Amino Lipid Compound 253**

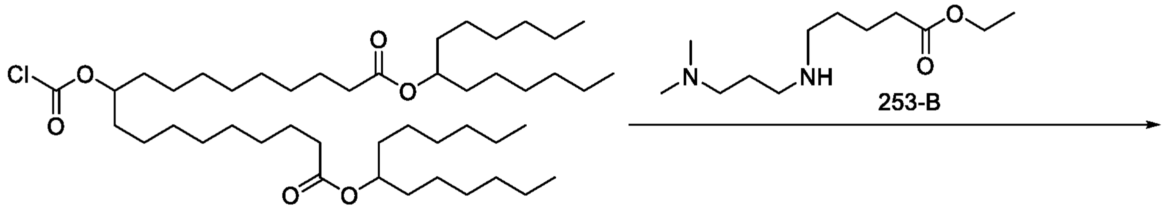

M5

253-B

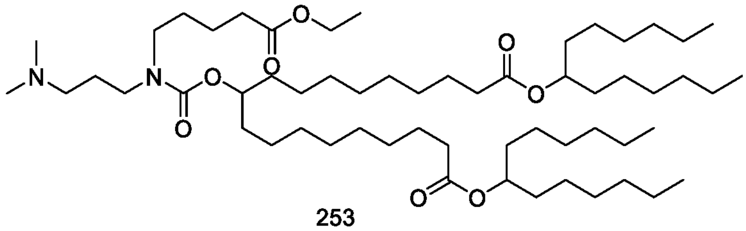

253

According to the general synthetic process, 300 mg amino lipid compound 253, as a pale yellow oil with a purity of 96.09% and a yield of 27%, was prepared from M5 (1.0 g, 1.29 mmol) and 253-B (0.45 g, 1.94 mmol).
$^1$H NMR (600 MHz, $CDCl_3$) δ 4.96-4.86 (m, 2H), 4.82-4.70 (m, 1H), 4.16 (q, J = 7.1 Hz, 2H), 3.36-3.18 (m, 4H), 2.42-2.15 (m, 14H), 1.86-1.46 (m, 22H), 1.43-1.20 (m, 55H), 0.92 (t, J = 7.0 Hz, 12H).
LC-MS (ESI): (M + H) calculated 965.85, found 966.5.

**Example 21: Synthesis of Amino Lipid Compound 254**

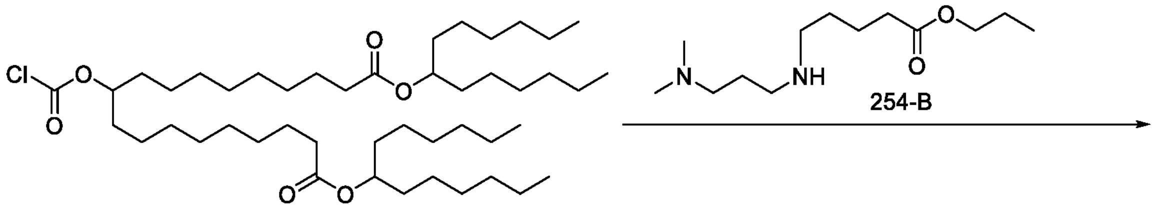

M5

254-B

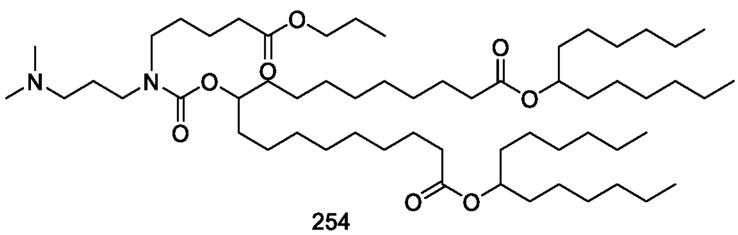

254

According to the general synthetic process, 100 mg amino lipid compound 254, as a pale yellow oil with a purity of 91.94% and a yield of 8.9%, was prepared from M5 (1.0 g, 1.29 mmol) and 254-B (0.47 g, 1.94 mmol).
$^1$H NMR (600 MHz, $CDCl_3$) δ 4.96-4.85 (m, 2H), 4.83-4.72 (m, 1H), 4.06 (t, J = 6.7 Hz, 2H), 3.28 (m, 4H), 2.49-2.18 (m, 14H), 1.88-1.48 (m, 24H), 1.37-1.17 (m, 52H), 0.97 (t, J = 7.4 Hz, 3H), 0.91 (t, J = 7.0 Hz, 12H).
LC-MS (ESI): (M + H) calculated 979.86, found 980.5.

**Example 22: Synthesis of Amino Lipid Compound 256**

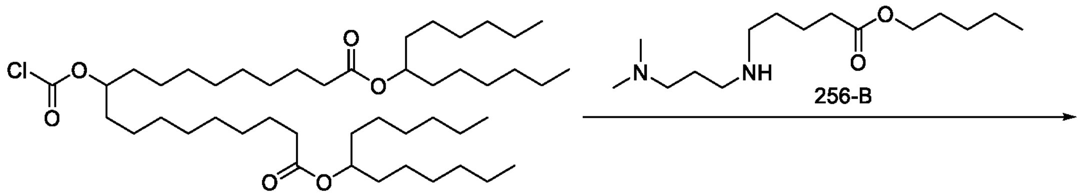


M5

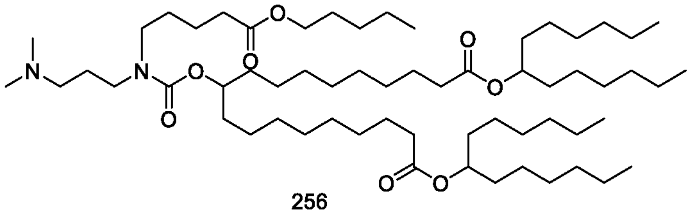

256

According to the general synthetic process, 480 mg amino lipid compound 256, as a pale yellow oil with a purity of 92.94% and a yield of 42%, was prepared from M5 (1.0 g, 1.29 mmol) and 256-B (0.53 g, 1.94 mmol).

$^1$H NMR (600 MHz, $CDCl_3$) δ 4.96-4.85 (m, 2H), 4.83-4.72 (m, 1H), 4.06 (t, J = 6.7 Hz, 2H), 3.28 (m, 4H), 2.49-2.18 (m, 14H), 1.88-1.48 (m, 26H), 1.37-1.17 (m, 54H), 0.91 (m, 15H).
LC-MS (ESI): (M + H) calculated 1007.89, found 1008.3

**Example 23: Synthesis of Amino Lipid Compound 257**

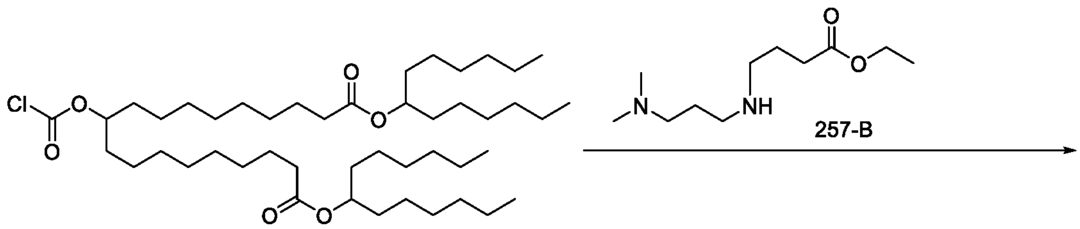


M5

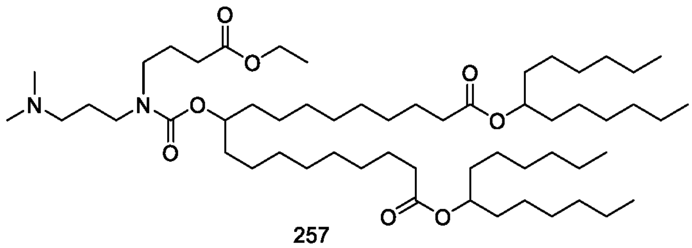

257

According to the general synthetic process, 290 mg amino lipid compound 257, as a pale yellow oil with a purity of 95.16% and a yield of 27%, was prepared from M5 (1.0 g, 1.29 mmol) and 257-B (0.42 g, 1.94 mmol).
$^1$H NMR (600 MHz, $CDCl_3$) δ 4.96-4.84 (m, 2H), 4.81-4.70 (m, 1H), 4.17 (q, 7.1 Hz, 2H), 3.37-3.18 (m, 4H), 2.31 (m, 8H), 2.25 (s, 6H), 1.94-1.82 (m, 2H), 1.72 (m, 2H), 1.68-1.60 (m, 4H), 1.54 (m, 12H), 1.40-1.22 (m, 55H), 0.91 (t, J = 7.0 Hz, 12H).

LC-MS (ESI): (M + H) calculated 951.83, found 952.4.

**Example 24: Synthesis of Amino Lipid Compound 258**

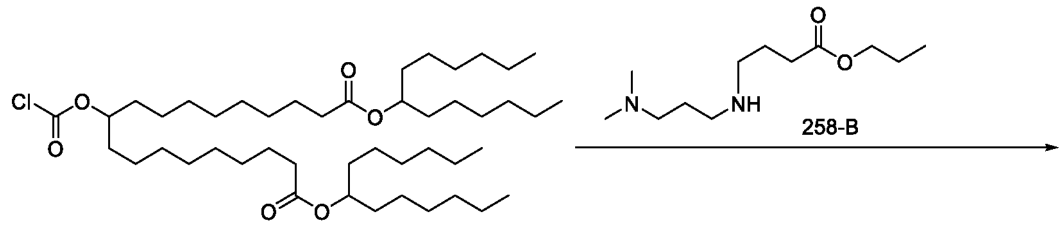
258-B

M5

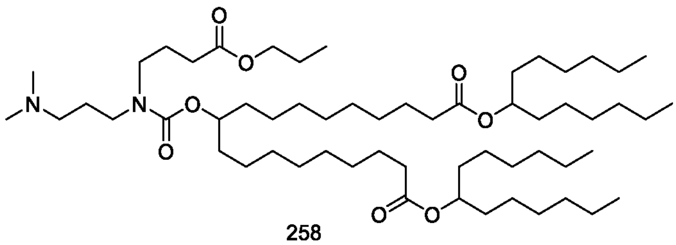

258

According to the general synthetic process, 340 mg amino lipid compound 258, as a pale yellow oil with a purity of 92.94% and a yield of 31%, was prepared from M5 (1.0 g, 1.29 mmol) and 258-B (0.45 g, 1.94 mmol).

$^1$H NMR (600 MHz, $CDCl_3$) δ 4.96-4.84 (m, 2H), 4.81-4.70 (m, 1H), 4.06 (t, J = 6.7 Hz, 2H), 3.37-3.18 (m, 4H), 2.31 (m, 8H), 2.25 (s, 6H), 1.94-1.82 (m, 2H), 1.72 (m, 2H), 1.68-1.60 (m, 6H), 1.54 (m, 12H), 1.40-1.22 (m, 52H), 0.97 (t, J = 7.4 Hz, 3H), 0.91 (t, J = 7.0 Hz, 12H).

LC-MS (ESI): (M + H) calculated 965.85, found 966.2.

**Example 25: Synthesis of Amino Lipid Compound 259**

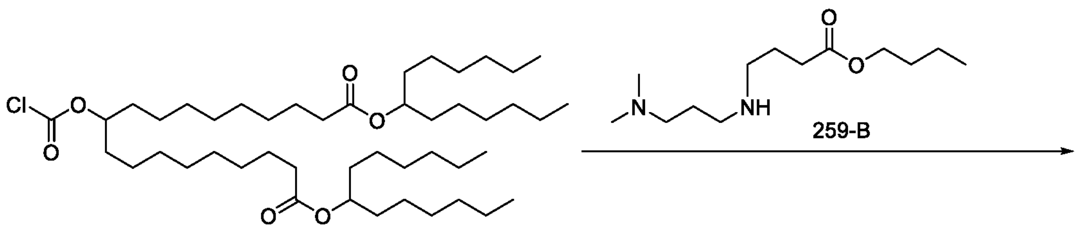
259-B

M5

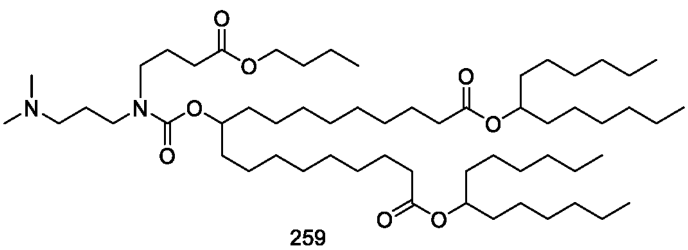

259

According to the general synthetic process, 320 mg amino lipid compound 259, as a pale yellow oil with a purity of 93.85% and a yield of 29%, was prepared from M5 (1.0 g, 1.29 mmol) and 259-B (0.47 g, 1.94 mmol).

$^1$H NMR (600 MHz, $CDCl_3$) δ 4.96-4.84 (m, 2H), 4.81-4.70 (m, 1H), 4.10 (t, J = 6.7 Hz, 2H), 3.37-3.18 (m, 4H), 2.31

(m, 8H), 2.25 (s, 6H), 1.94-1.82 (m, 2H), 1.72 (m, 2H), 1.68-1.60 (m, 6H), 1.54 (m, 12H), 1.40-1.22 (m, 54H), 0.97 (t, J = 7.4 Hz, 3H), 0.91 (t, J = 7.0 Hz, 12H).

LC-MS (ESI): (M + H) calculated 979.86, found 980.3.

**Example 26: Synthesis of Amino Lipid Compound 260**

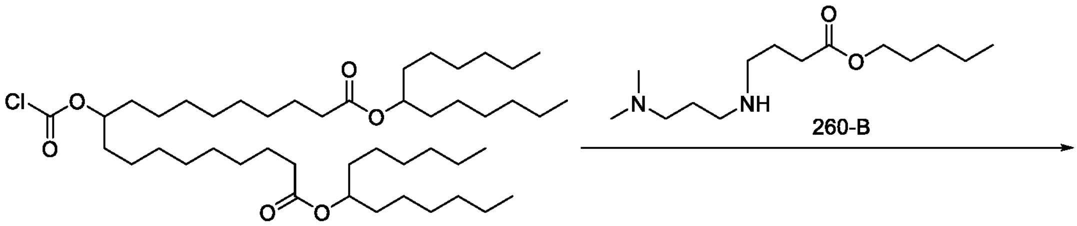
260-B

M5

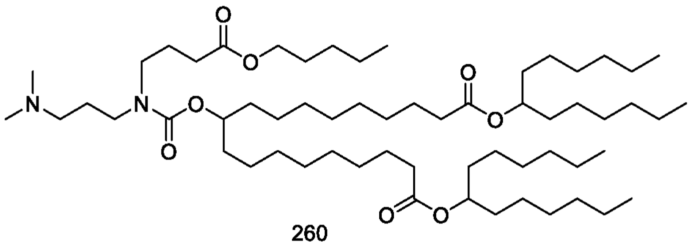

260

According to the general synthetic process, 440 mg amino lipid compound 260, as a pale yellow oil with a purity of 95.54% and a yield of 40%, was prepared from M5 (1.0 g, 1.29 mmol) and 260-B (0.50 g, 1.94 mmol).

$^1H$ NMR (600 MHz, $CDCl_3$) δ 4.95-4.86 (m, 2H), 4.82-4.71 (m, 1H), 4.09 (t, J = 6.8 Hz, 2H), 3.29 (m, 4H), 2.31 (m, 8H), 2.24 (s, 6H), 1.88 (m, 2H), 1.73 (m, 2H), 1.65 (m, 6H), 1.54 (m, 12H), 1.41-1.20 (m, 56H), 0.92 (m, 15H).

LC-MS (ESI): (M + H) calculated 993.88, found 994.2.

**Example 27: Synthesis of Amino Lipid Compound 261**

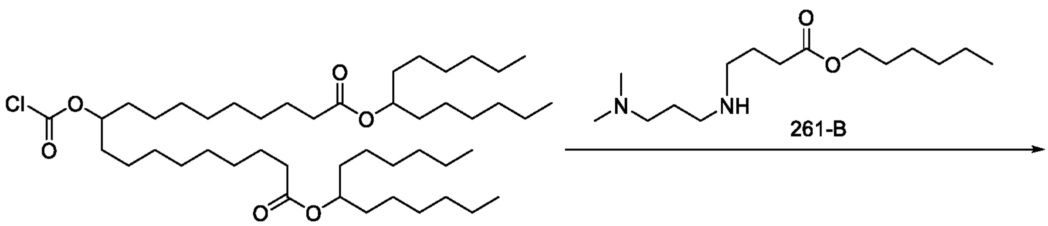
261-B

M5

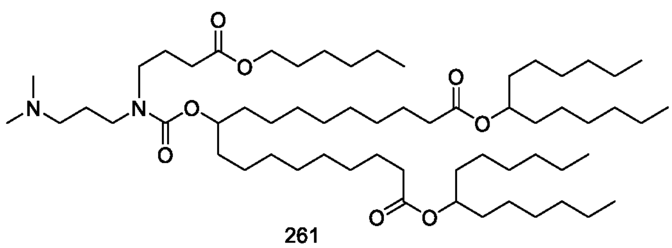

261

According to the general synthetic process, 140 mg amino lipid compound 261, as a pale yellow oil with a purity of 92.13% and a yield of 12%, was prepared from M5 (1.0 g, 1.29 mmol) and 261-B (0.53 g, 1.94 mmol).

$^{1}H$ NMR (600 MHz, $CDCl_3$) δ 4.94-4.85 (m, 2H), 4.80-4.73 (m, 1H), 4.09 (t, J = 6.8 Hz, 2H), 3.35-3.20 (m, 4H), 2.38-2.19 (m, 14H), 1.88 (m, 2H), 1.74 (m, 2H), 1.68-1.61 (m, 6H), 1.54 (m, 12H), 1.42-1.22 (m, 58H), 0.92 (m, 15H).

LC-MS (ESI): (M + H) calculated 1007.89, found 1008.3.

**Example 28: Synthesis of Amino Lipid Compound 262**

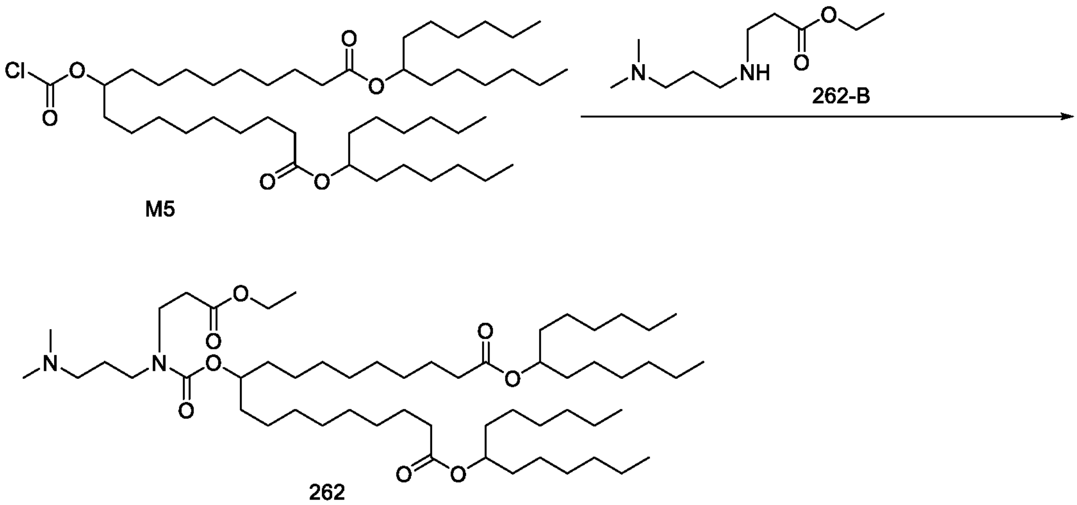

M5

262

According to the general synthetic process, 500 mg amino lipid compound 262, as a pale yellow oil with a purity of 88.60% and a yield of 47%, was prepared from M5 (1.0 g, 1.29 mmol) and 262-B (0.39 g, 1.94 mmol).

$^{1}H$ NMR (600 MHz, $CDCl_3$) δ 4.97-4.84 (m, 2H), 4.84-4.72 (m, 1H), 4.07 (t, J = 5.8 Hz, 2H), 3.54 (m, 2H), 3.37-3.23 (m, 2H), 2.60 (m, 2H), 2.30 (m, 6H), 2.25 (s, 6H), 1.73 (m, 2H), 1.68-1.60 (m, 4H), 1.54 (m, 12H), 1.39-1.21 (m, 55H), 0.91 (t, J = 7.0 Hz, 12H).

LC-MS (ESI): (M + H) calculated 937.81, found 938.2.

**Example 29: Synthesis of Amino Lipid Compound 263**

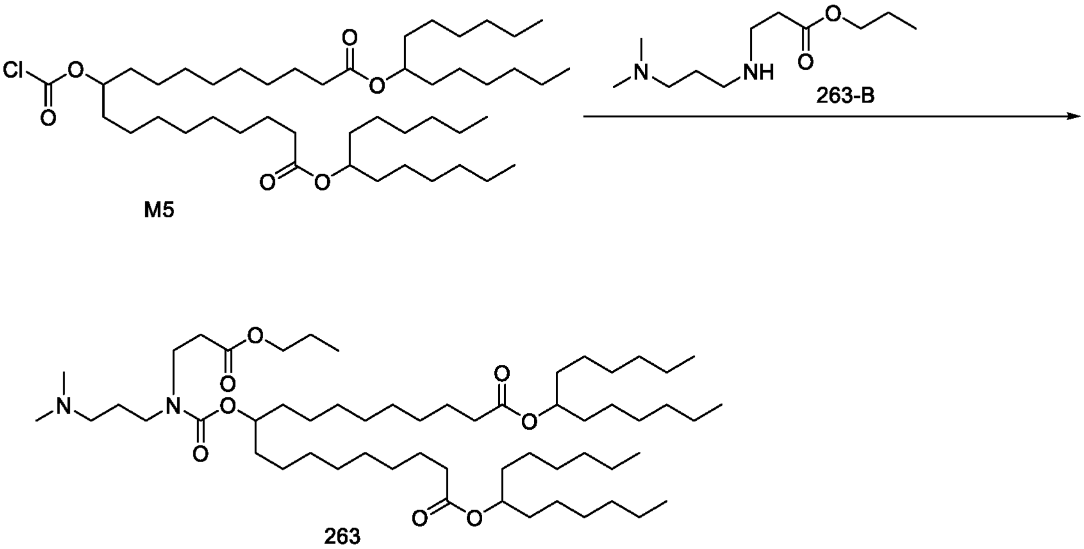

M5

263

According to the general synthetic process, 580 mg amino lipid compound 263, as a pale yellow oil with a purity of 95.37% and a yield of 55%, was prepared from M5 (1.0 g, 1.29 mmol) and 263-B (0.42 g, 1.94 mmol).

1HNMR (600 MHz, $CDCl_3$) δ 4.95-4.83 (m, 2H), 4.81-4.72 (m, 1H), 4.07 (t, J = 5.8 Hz, 2H), 3.55 (m, 2H), 3.29 (m,

2H), 2.61 (m, 2H), 2.30 (m, 6H), 2.25 (s, 6H), 1.80-1.62 (m, 8H), 1.54 (m, 12H), 1.30 (m, 52H), 0.96 (t, J = 7.2 Hz, 3H), 0.91 (t, J = 7.0 Hz, 12H).

LC-MS (ESI): (M + H) calculated 951.83, found 952.2.

**Example 30: Synthesis of Amino Lipid Compound 264**

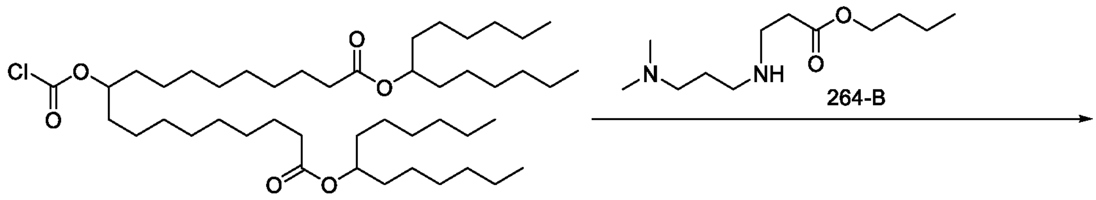


M5

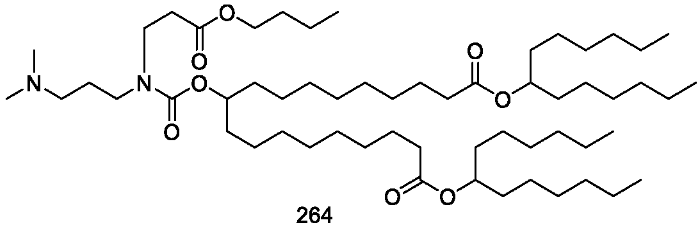

264

According to the general synthetic process, 220 mg amino lipid compound 264, as a pale yellow oil with a purity of 98.65% and a yield of 20%, was prepared from M5 (1.0 g, 1.29 mmol) and 264-B (0.45 g, 1.94 mmol).

$^1$H NMR (600 MHz, $CDCl_3$) δ 4.95-4.83 (m, 2H), 4.81-4.72 (m, 1H), 4.07 (t, J = 5.8 Hz, 2H), 3.55 (m, 2H), 3.29 (m, 2H), 2.61 (m, 2H), 2.30 (m, 6H), 2.25 (s, 6H), 1.80-1.62 (m, 8H), 1.54 (m, 12H), 1.30 (m, 54H), 0.96 (t, J = 7.2 Hz, 3H), 0.91 (t, J = 7.0 Hz, 12H).

LC-MS (ESI): (M + H) calculated 965.85, found 966.2.

**Example 31: Synthesis of Amino Lipid Compound 265**

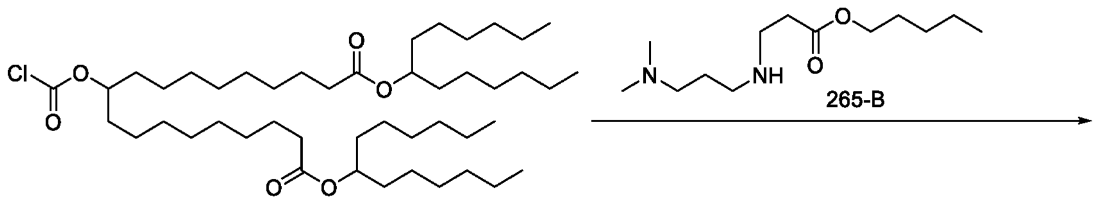


M5

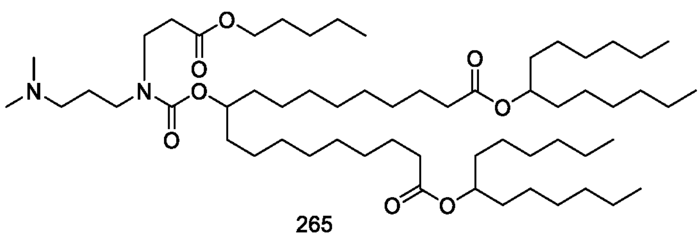

265

According to the general synthetic process, 450 mg amino lipid compound 265, as a pale yellow oil with a purity of 98.47% and a yield of 40%, was prepared from M5 (1.0 g, 1.29 mmol) and 265-B (0.47 g, 1.94 mmol).

$^1$H NMR (600 MHz, $CDCl_3$) δ 4.95-4.83 (m, 2H), 4.81-4.72 (m, 1H), 4.07 (t, J = 5.8 Hz, 2H), 3.55 (m, 2H), 3.29 (m, 2H), 2.61 (m, 2H), 2.30 (m, 6H), 2.25 (s, 6H), 1.80-1.62 (m, 8H), 1.54 (m, 12H), 1.42-1.16 (m, 56H), 0.98-0.86 (m, 15H).

LC-MS (ESI): (M + H) calculated 979.86, found 980.2.

**Example 32: Synthesis of Amino Lipid Compound 266**

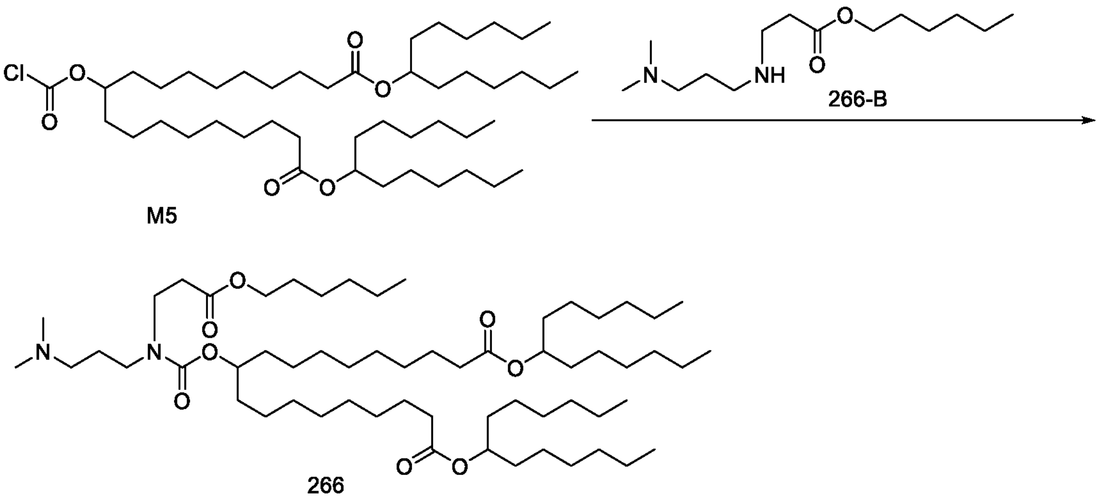

266-B

M5

266

According to the general synthetic process, 487 mg amino lipid compound 266, as a pale yellow oil with a purity of 94.01% and a yield of 42%, was prepared from M5 (1.0 g, 1.29 mmol) and 266-B (0.50 g, 1.94 mmol).
$^1H$ NMR (600 MHz, $CDCl_3$) δ 4.95-4.83 (m, 2H), 4.81-.72 (m, 1H), 4.07 (t, J = 5.8 Hz, 2H), 3.55 (m, 2H), 3.29 (m, 2H), 2.61 (m, 2H), 2.30 (m, 6H), 2.25 (s, 6H), 1.80-1.62 (m, 8H), 1.54 (m, 12H), 1.42-1.16 (m, 58H), 0.98-0.86 (m, 15H).
LC-MS (ESI): (M + H) calculated 993.88, found 994.3.

**Example 33: Synthesis of Amino Lipid Compound 267**

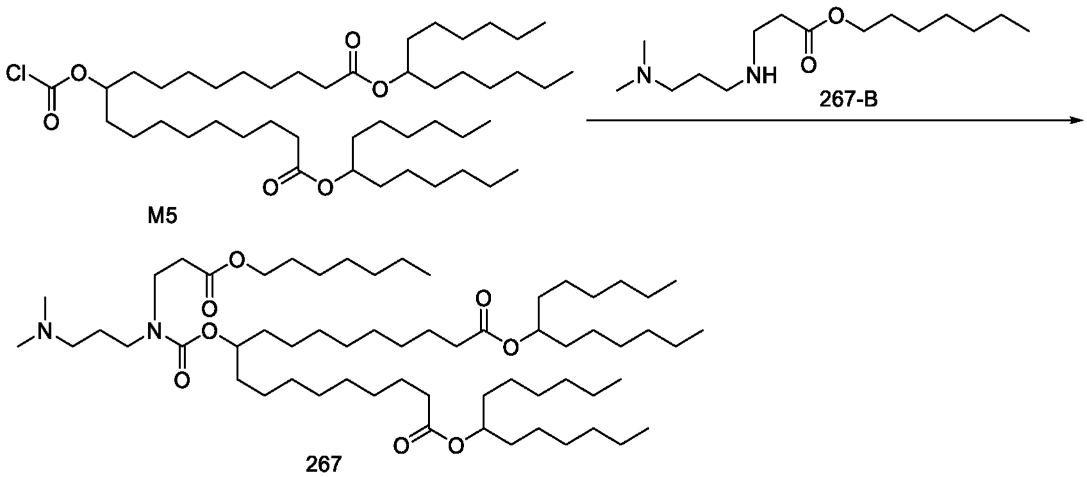

267-B

M5

267

According to the general synthetic process, 360 mg amino lipid compound 267, as a pale yellow oil with a purity of 98.16% and a yield of 31%, was prepared from M5 (1.0 g, 1.29 mmol) and 267-B (0.53 g, 1.94 mmol).
$^1H$ NMR (600 MHz, $CDCl_3$) δ 4.95-4.83 (m, 2H), 4.81-4.72 (m, 1H), 4.07 (t, J = 5.8 Hz, 2H), 3.55 (m, 2H), 3.29 (m, 2H), 2.61 (m, 2H), 2.30 (m, 6H), 2.25 (s, 6H), 1.80-1.62 (m, 8H), 1.54 (m, 12H), 1.42-1.16 (m, 60H), 0.98-0.86 (m, 15H).
LC-MS (ESI): (M + H) calculated 1007.89, found 1008.2.

**Example 34: Synthesis of Amino Lipid Compound 268**

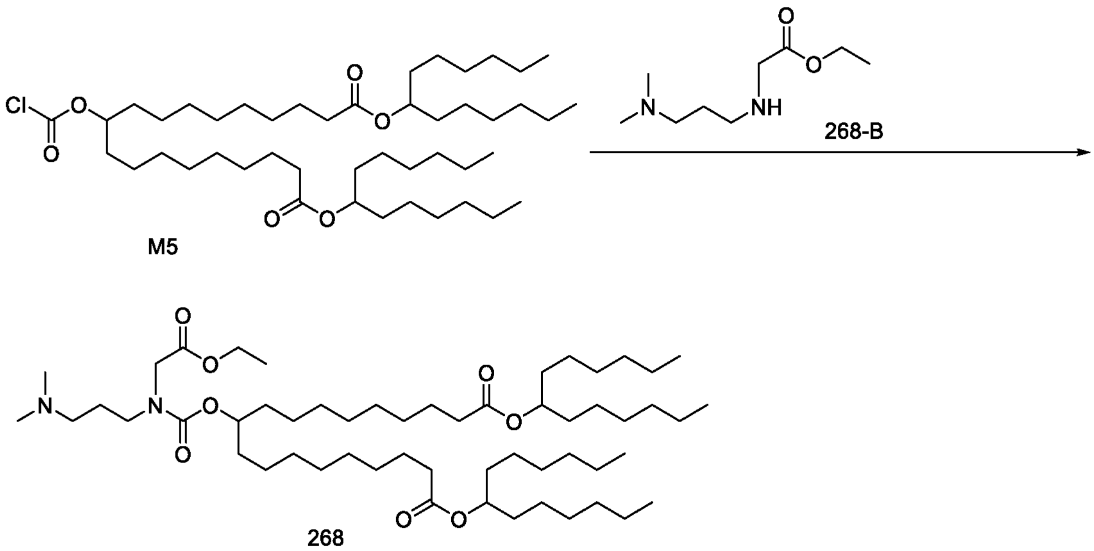

M5

268-B

268

According to the general synthetic process, 500 mg amino lipid compound 268, as a pale yellow oil with a purity of 90.61% and a yield of 47%, was prepared from M5 (1.0 g, 1.29 mmol) and 268-B (0.37 g, 1.94 mmol).

$^{1}$H NMR (600 MHz, $CDCl_3$) 4.97-4.80 (m,2H), 4.68-4.79 (m, 1H), 4.11-4.07 (m, 2H), 4.00 (s, 1H), 3.93 (s, 1H), 3.34 (dt, J = 31.0, 7.2 Hz, 2H), 2.50-2.10 (m, 12H), 1.66-1.09 (m, 70H), 0.96-0.81 (m, 15H).

LC-MS (ESI): (M + H) calculated 923.80, found 924.1.

**Example 35: Synthesis of Amino Lipid Compound 269**

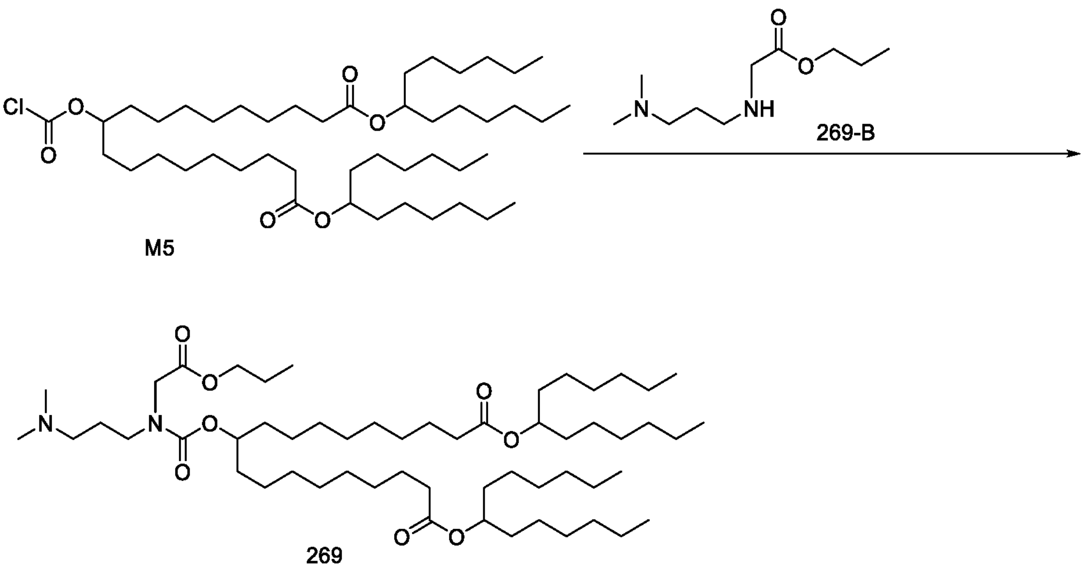

M5

269-B

269

According to the general synthetic process, 510 mg amino lipid compound 269, as a pale yellow oil with a purity of 96.02% and a yield of 48%, was prepared from M5 (1.0 g, 1.29 mmol) and 269-B (0.39 g, 1.94 mmol).

1H NMR (600 MHz, $CDCl_3$) 4.97-4.80 (m,2H), 4.68-4.79 (m, 1H), 4.11-4.07 (m, 2H), 4.00 (s, 1H), 3.93 (s, 1H), 3.34 (dt, J = 31.0, 7.2 Hz, 2H), 2.50-2.10 (m, 12H), 1.66-1.09 (m, 72H), 0.96-0.81 (m, 15H).

LC-MS (ESI): (M + H) calculated 937.81, found 938.1.

**Example 36: Synthesis of Amino Lipid Compound 270**

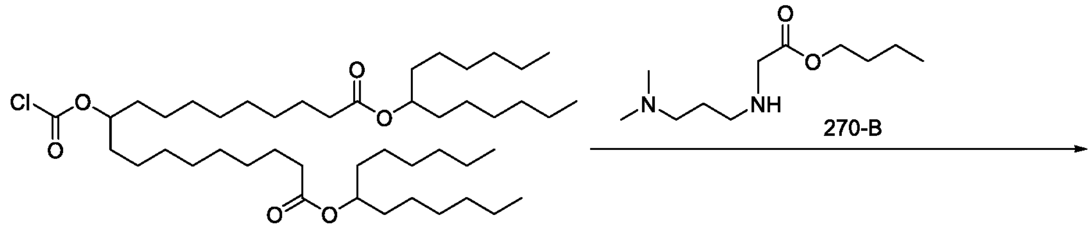
270-B

M5

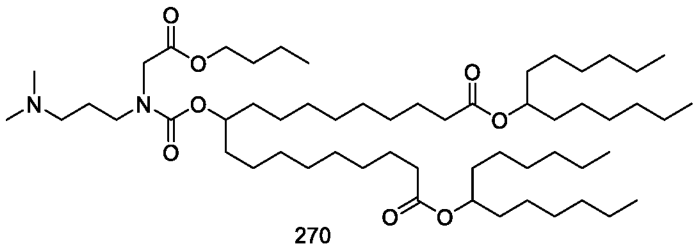

270

According to the general synthetic process, 550 mg amino lipid compound 270, as a pale yellow oil with a purity of 97.66% and a yield of 51%, was prepared from M5 (1.0 g, 1.29 mmol) and 270-B (0.42 g, 1.94 mmol).

$^1$H NMR (600 MHz, $CDCl_3$) 4.97-4.80 (m,2H), 4.68-4.79 (m, 1H), 4.11-4.07 (m, 2H), 4.00 (s, 1H), 3.93 (s, 1H), 3.34 (dt, J = 31.0, 7.2 Hz, 2H), 2.50-2.10 (m, 12H), 1.66-1.09 (m, 74H), 0.96-0.81 (m, 15H).

LC-MS (ESI): (M + H) calculated 951.83, found 952.2.

**Example 37: Synthesis of Amino Lipid Compound 271**

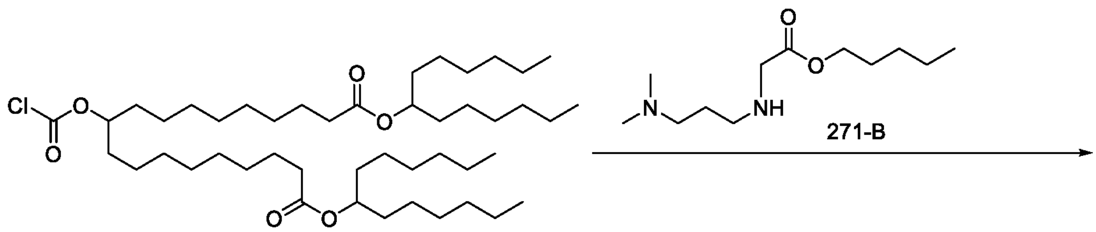
271-B

M5

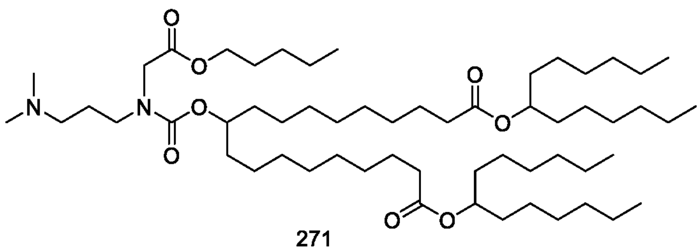

271

According to the general synthetic process, 230 mg amino lipid compound 271, as a pale yellow oil with a purity of 94.22% and a yield of 21%, was prepared from M5 (1.0 g, 1.29 mmol) and 271-B (0.45 g, 1.94 mmol).

$^1$H NMR (600 MHz, $CDCl_3$) 4.90-4.82 (m, 2H), 4.80-4.65 (m, 1H), 4.14-4.04 (m, 2H), 4.01 (s, 1H), 3.93 (s, 1H), 3.34 (dt, J = 31.0, 7.2 Hz, 2H), 1.56-1.44 (m, 12H), 1.39-1.18 (m, 76H), 0.95-0.82 (m, 15H).

LC-MS (ESI): (M + H) calculated 965.85, found 966.2.

**Example 38: Synthesis of Amino Lipid Compound 272**

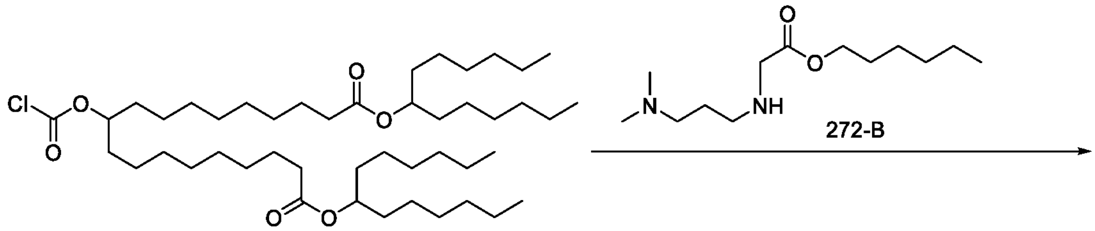


M5

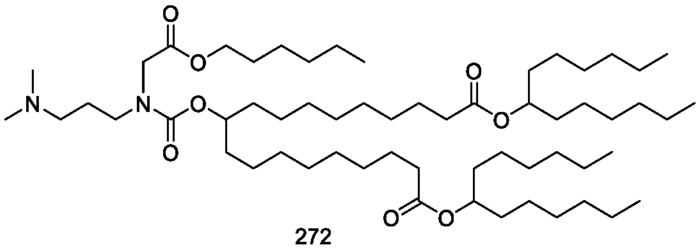

272

According to the general synthetic process, 450 mg amino lipid compound 272, as a pale yellow oil with a purity of 98.47% and a yield of 40%, was prepared from M5 (1.0 g, 1.29 mmol) and 272-B (0.47 g, 1.94 mmol).
$^1$H NMR (600 MHz, $CDCl_3$) 4.90-4.82 (m, 2H), 4.80-4.65 (m, 1H), 4.14-4.04 (m, 2H), 4.01 (s, 1H), 3.93 (s, 1H), 3.34 (dt, J = 31.0, 7.2 Hz, 2H), 1.56-1.44 (m, 12H), 1.39-1.18 (m, 78H), 0.95-0.82 (m, 15H).
LC-MS (ESI): (M + H) calculated 979.86, found 980.2.

**Example 39: Synthesis of Amino Lipid Compound 273**

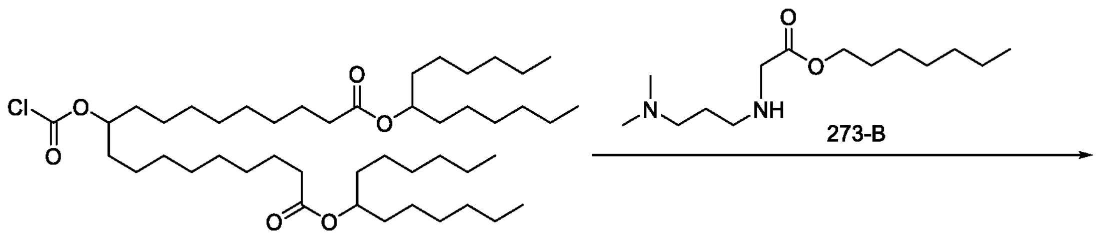


M5

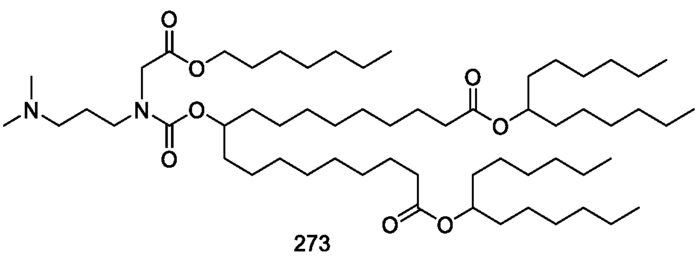

273

According to the general synthetic process, 460 mg amino lipid compound 273, as a pale yellow oil with a purity of 96.41% and a yield of 40%, was prepared from M5 (1.0 g, 1.29 mmol) and 273-B (0.50 g, 1.94 mmol).
$^1$H NMR (600 MHz, $CDCl_3$) 4.90-4.82 (m, 2H), 4.80-4.65 (m, 1H), 4.14-4.04 (m, 2H), 4.01 (s, 1H), 3.93 (s, 1H), 3.34 (dt, J = 31.0, 7.2 Hz, 2H), 1.56-1.44 (m, 12H), 1.39-1.18 (m, 80H), 0.95-0.82 (m, 15H).
LC-MS (ESI): (M + H) calculated 993.88, found 994.2.

**Example 40: Synthesis of Amino Lipid Compound 274**

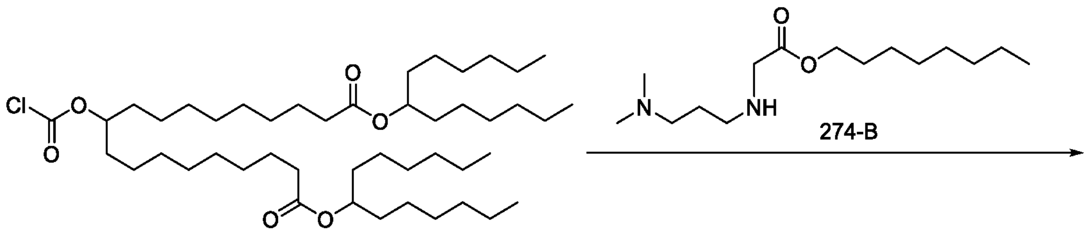


M5

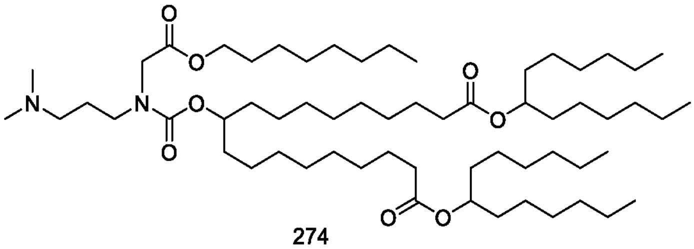

274

According to the general synthetic process, 420 mg amino lipid compound 274, as a pale yellow oil with a purity of 95.60% and a yield of 36%, was prepared from M5 (1.0 g, 1.29 mmol) and 274-B (0.53 g, 1.94 mmol).
$^1$H NMR (600 MHz, $CDCl_3$) 4.90-4.82 (m, 2H), 4.80-4.65 (m, 1H), 4.14-4.04 (m, 2H), 4.01 (s, 1H), 3.93 (s, 1H), 3.34 (dt, J = 31.0, 7.2 Hz, 2H), 1.56-1.44 (m, 12H), 1.39-1.18 (m, 82H), 0.95-0.82 (m, 15H).
LC-MS (ESI): (M + H) calculated 1007.89, found 1008.3.

**Example 41: Synthesis of Amino Lipid Compound 275**

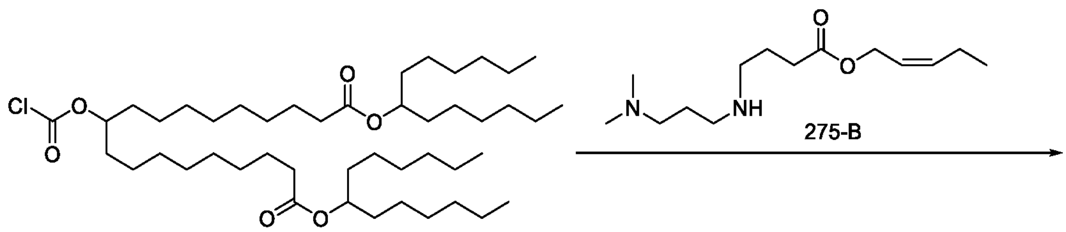


M5

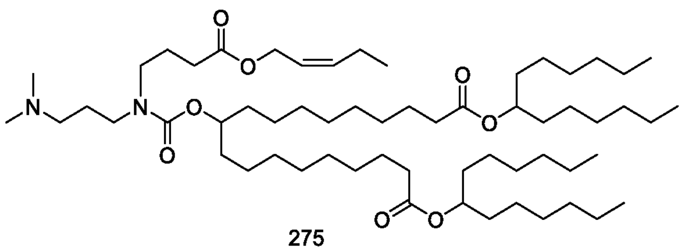

275

According to the general synthetic process, 280 mg amino lipid compound 275, as a pale yellow oil with a purity of 90.83% and a yield of 25%, was prepared from M5 (1.0 g, 1.29 mmol) and 275-B (0.50 g, 1.94 mmol).
$^1$H NMR (600 MHz, $CDCl_3$) δ 5.66 (t, J = 8.8 Hz, 1H), 5.56 (t, J = 8.8 Hz, 1H)4.90 (p, J = 6.2 Hz, 2H), 4.79-4.74 (m, 1H), 4.66 (d, J = 6.9 Hz, 2H), 3.34-3.23 (m, 4H), 2.31 (dd, J = 20.5, 13.1 Hz, 8H), 2.24 (s, 6H), 2.18-2.12 (m, 2H), 1.89 (s, 2H), 1.73 (s, 2H), 1.68-1.61 (m, 4H), 1.54 (d, J = 5.4 Hz, 12H), 1.38-1.23 (m, 54H), 1.05-0.99 (m, 3H), 0.91 (m, 12H).
LC-MS (ESI): (M + H) calculated 991.86, found 992.1.

**Example 42: Synthesis of Amino Lipid Compound 276**

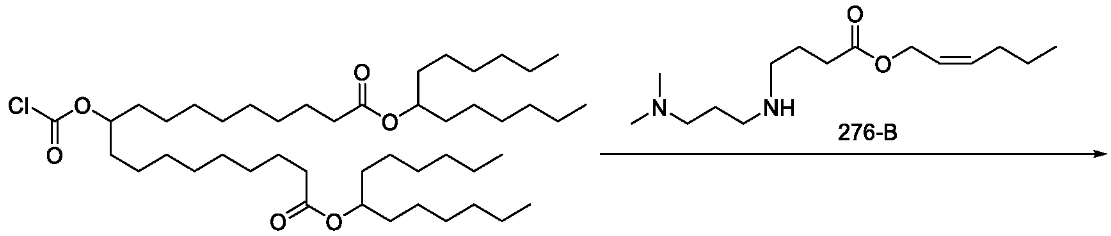

M5

276-B

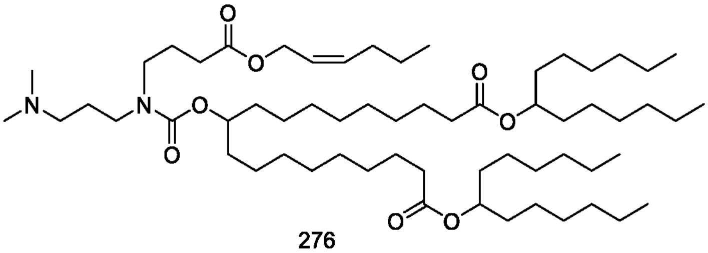

276

According to the general synthetic process, 110 mg amino lipid compound 276, as a pale yellow oil with a purity of 93.19% and a yield of 10%, was prepared from M5 (1.0 g, 1.29 mmol) and 276-B (0.52 g, 1.94 mmol).
$^1$H NMR (600 MHz, $CDCl_3$) 5.67 (m, 1H), 5.61-5.52 (m, 1H), 4.94-4.86 (m, 2H), 4.79-4.74 (m, 1H), 4.66 (d, J = 6.8 Hz, 2H), 3.29 (m, 4H), 2.38-2.08 (m, 16H), 1.93-1.23 (m, 74H), 0.93 (m, 15H).
LC-MS (ESI): (M + H) calculated 1005.88, found 1006.3.

**Example 43: Synthesis of Amino Lipid Compound 277**

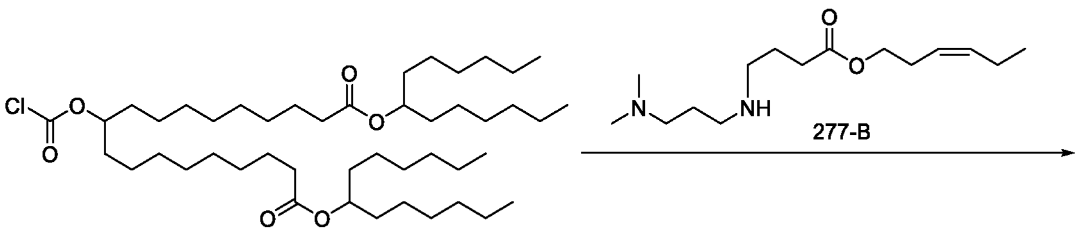

M5

277-B

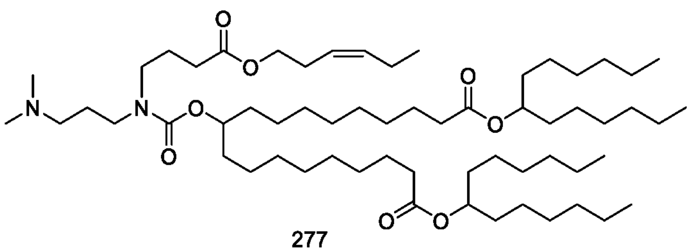

277

According to the general synthetic process, 350 mg amino lipid compound 277, as a pale yellow oil with a purity of 96.47% and a yield of 33%, was prepared from M5 (1.0 g, 1.29 mmol) and 277-B (0.52 g, 1.94 mmol).
1H NMR (600 MHz, $CDCl_3$) 5.54 (m, 1H), 5.37-5.30 (m, 1H), 4.94-4.85 (m, 2H), 4.80-4.74 (m, 1H), 4.10 (m, 2H), 3.34-3.21 (m, 4H), 2.44-2.04 (m, 18H), 1.93-1.22 (m, 72H), 1.00 (m, 3H), 0.91 (t, J = 7.0 Hz, 12H).
LC-MS (ESI): (M + H) calculated 1005.88, found 1006.3.

**Example 44: Synthesis of Amino Lipid Compound 279**

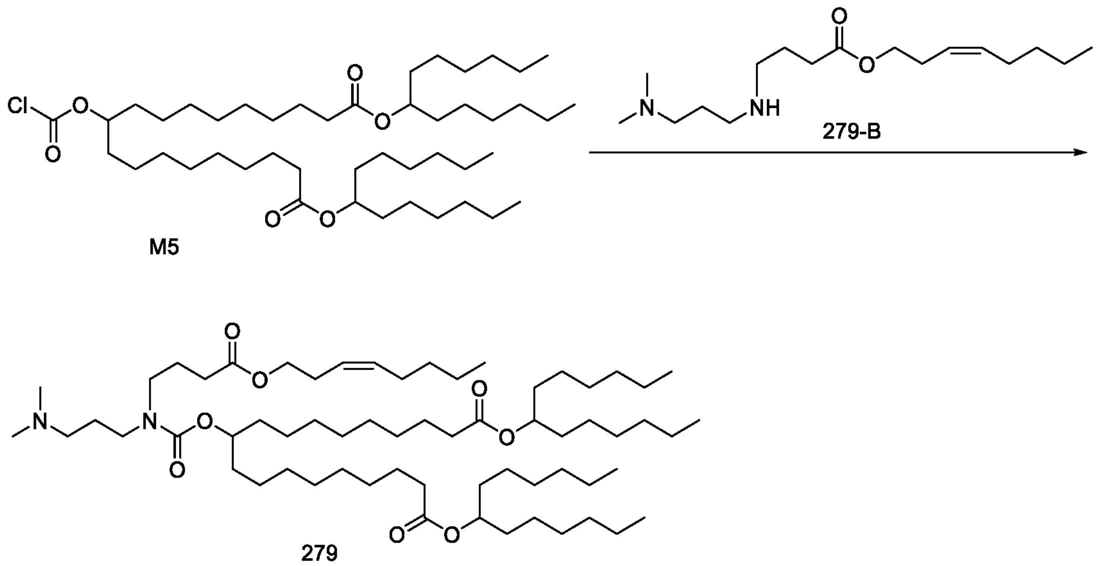

M5

279-B

279

According to the general synthetic process, 250 mg amino lipid compound 279, as a pale yellow oil with a purity of 98.62% and a yield of 21%, was prepared from M5 (1.0 g, 1.29 mmol) and 279-B (0.58 g, 1.94 mmol).

$^1$H NMR (600 MHz, $CDCl_3$) $\delta$ 5.50 (m, 1H), 5.36-5.30 (m, 1H), 4.89-4.84 (m, 2H), 4.73 (m, 1H), 4.06 (m, 2H), 3.31-3.18 (m, 4H), 2.30 (m, 16H), 2.08-1.21 (m, 78H), 0.94-0.82 (m, 15H).

LC-MS (ESI): (M + H) calculated 1033.91, found 1034.4.

**Example 45: Synthesis of Amino Lipid Compound 280**

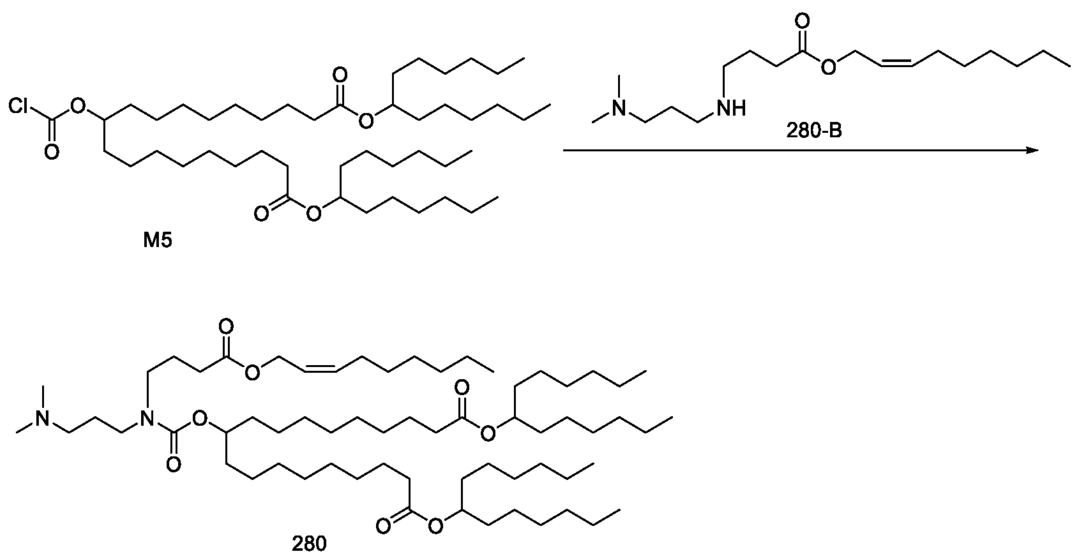

M5

280-B

280

According to the general synthetic process, 190 mg amino lipid compound 280, as a pale yellow oil with a purity of 91.43% and a yield of 16%, was prepared from M5 (1.0 g, 1.29 mmol) and 280-B (0.61 g, 1.94 mmol).

$^1$H NMR (600 MHz, $CDCl_3$) 5.66-5.60 (m, 1H), 5.54-5.48 (m, 1H), 4.89-4.83 (m, 2H), 4.76-4.71 (m, 1H), 4.62 (d, J = 6.8 Hz, 2H), 3.33-3.19 (m, 4H), 2.34-2.06 (m, 16H), 1.89-1.23 (m, 80H), 0.88 (td, J = 7.0, 4.8 Hz, 15H).

LC-MS (ESI): (M + H) calculated 1047.92, found 1048.4

**Example 46: Synthesis of Amino Lipid Compound 281**

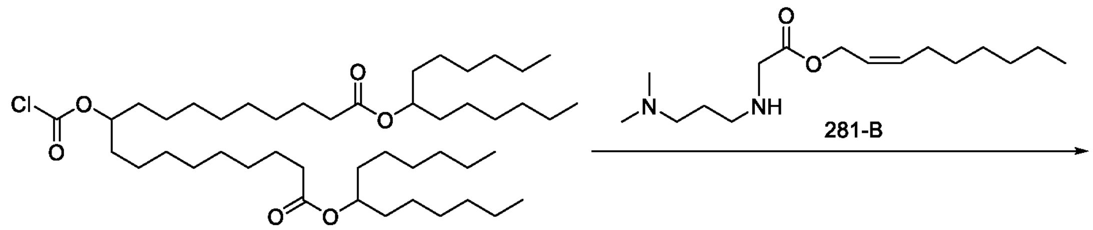

M5

281-B

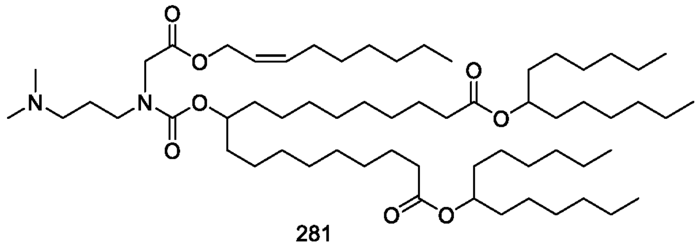

281

According to the general synthetic process, 480 mg amino lipid compound 281, as a pale yellow oil with a purity of 96.03% and a yield of 41%, was prepared from M5 (1.0 g, 1.29 mmol) and 281-B (0.55 g, 1.94 mmol).

$^1$H NMR (600 MHz, $CDCl_3$) 5.68-5.59 (m, 1H), 5.50 (d, J = 11.6, 6.7 Hz, 1H), 4.91-4.83 (m, 2H), 4.80-4.70 (m, 1H), 4.66 (m, 2H), 4.01(s, 1H), 3.94(s, 1H), 3.34 (m, 2H), 2.29-2.06 (m, 14H), 1.78-1.22 (m, 78H), 0.92-0.83 (m, 15H).

LC-MS (ESI): (M + H) calculated 1019.89, found 1020.3.

**Example 47: Synthesis of Amino Lipid Compound 282**

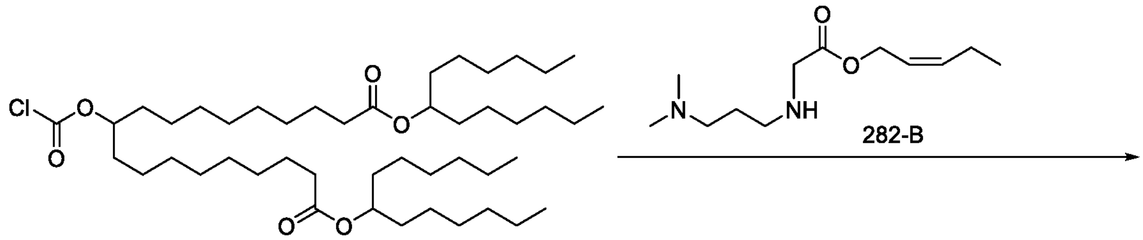

M5

282-B

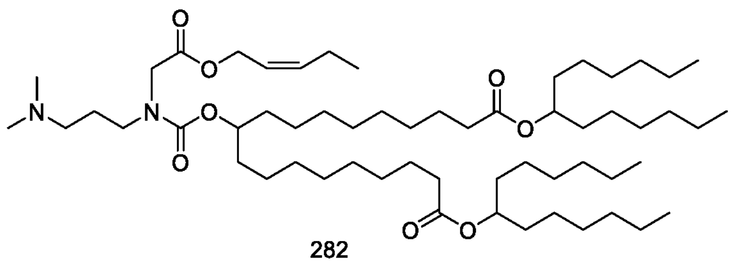

282

According to the general synthetic process, 300 mg amino lipid compound 282, as a pale yellow oil with a purity of 94.87% and a yield of 27%, was prepared from M5 (1.0 g, 1.29 mmol) and 282-B (0.44 g, 1.94 mmol).

$^1$H NMR (600 MHz, $CDCl_3$) $\delta$ 5.71-5.63 (m, 1H), 5.55-5.47 (m, 1H), 4.93-4.87 (m, 2H), 4.83-4.73 (m, 1H), 4.73-4.67 (m, 2H), 4.06 (s, 1H), 3.98 (s, 1H), 3.38 (dt, J = 29.1, 7.2 Hz, 2H), 2.36-2.10 (m, 14H), 1.80-1.23 (m, 70H), 1.03 (m, 3H), 0.91 m, 12H).

LC-MS (ESI): (M + H) calculated 963.83, found 964.2.

**Example 48: Synthesis of Amino Lipid Compound 284**

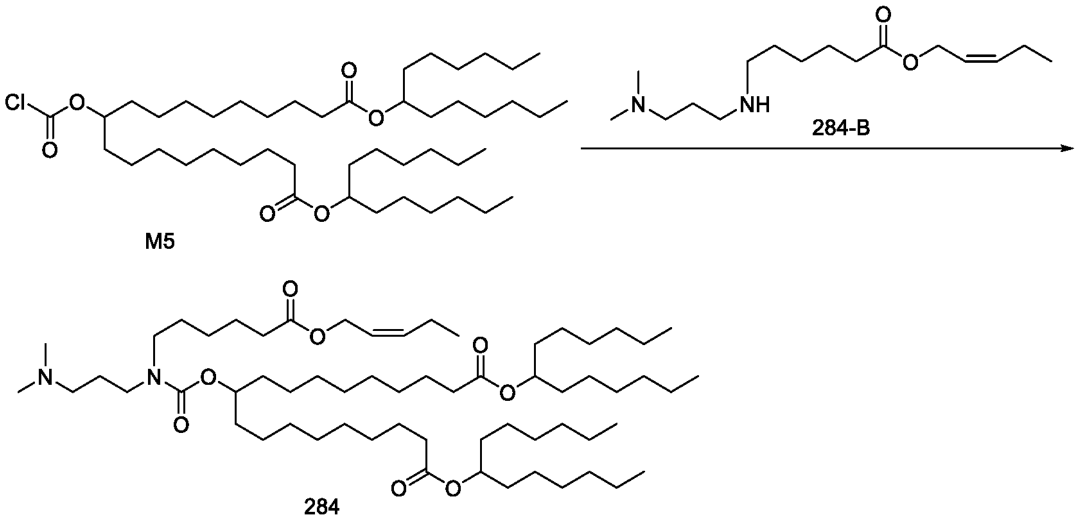

M5

284-B

284

According to the general synthetic process, 530 mg amino lipid compound 284, as a pale yellow oil with a purity of 94.29% and a yield of 45%, was prepared from M5 (1.0 g, 1.29 mmol) and 284-B (0.55 g, 1.94 mmol).

$^{1}H$ NMR (600 MHz, $CDCl_3$) 5.64 (m, 1H), 5.52-5.45 (m, 1H), 4.90-4.82 (m, 2H), 4.77-4.70 (m, 1H), 4.62 (d, J = 6.7 Hz, 2H), 3.29-3.13 (m, 4H), 2.32-2.09 (m, 16H), 1.75-1.20 (m, 78H), 1.02-0.97 (m, 3H), 0.87 (m, 12H).

LC-MS (ESI): (M + H) calculated 1019.89, found 1020.2.

**Example 49: Synthesis of Amino Lipid Compound 297**

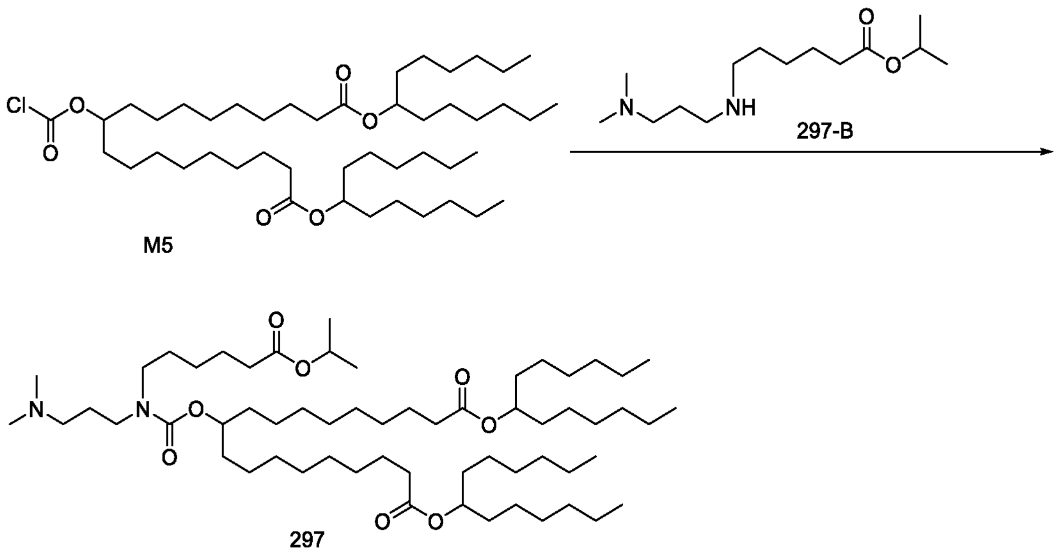

M5

297-B

297

According to the general synthetic process, 830 mg amino lipid compound 297, as a pale yellow oil with a purity of 97.59% and a yield of 73%, was prepared from M5 (1.0 g, 1.29 mmol) and 297-B (0.50 g, 1.94 mmol).

$^{1}H$ NMR (600 MHz, $CDCl_3$) $\delta$ 5.01-4.97 (m, 1H), 4.89-4.83 (m, 2H), 4.76-4.69 (m, 1H), 3.29-3.12 (m, 4H), 2.28-2.24 (m, 8H), 2.21 (s, 6H), 1.69 (s, 2H), 1.65-1.57 (m, 6H), 1.56-1.45 (m, 14H), 1.33-1.20 (m, 60H), 0.87 (t, J = 7.0 Hz, 12H).

LC-MS (ESI): (M + H) calculated 993.88, found 994.3.

**Example 50: Synthesis of Amino Lipid Compound 298**

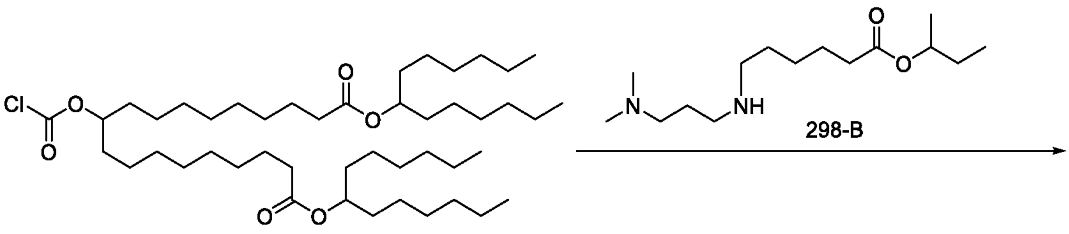

298-B

M5

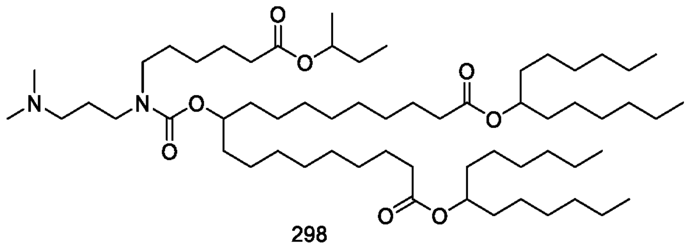

298

According to the general synthetic process, 410 mg amino lipid compound 298, as a pale yellow oil with a purity of 97.33% and a yield of 36%, was prepared from M5 (1.0 g, 1.29 mmol) and 298-B (0.53 g, 1.94 mmol).
$^1$H NMR (600 MHz, $CDCl_3$) δ 4.90-4.80 (m, 3H), 4.76-4.70 (m, 1H), 3.30-3.12 (m, 4H), 2.28-2.25 (m, 8H), 2.21 (s, 6H), 1.67 (s, 2H), 1.65-1.57 (m, 7H), 1.57-1.44 (m, 15H), 1.35-1.21 (m, 54H), 1.19 (d, J = 6.3 Hz, 3H), 0.91-0.84 (m, 15H).
LC-MS (ESI): (M + H) calculated 1007.89, found 1008.3.

**Example 51: Synthesis of Amino Lipid Compound 299**

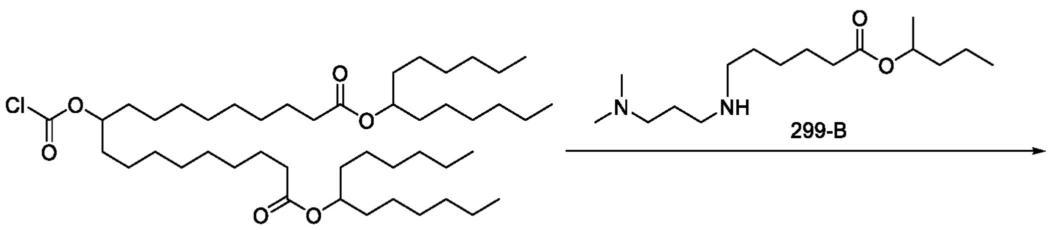

299-B

M5

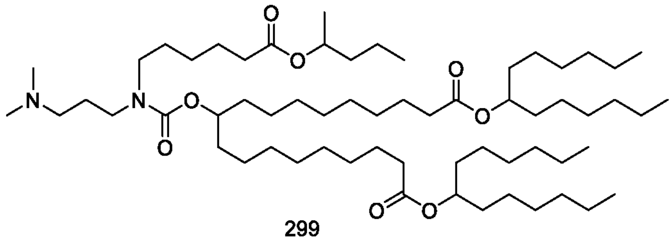

299

According to the general synthetic process, 640 mg amino lipid compound 299, as a pale yellow oil with a purity of 98.14% with a yield of 57%, was prepared from M5 (1.0 g, 1.29 mmol) and 299-B (0.56 g, 1.94 mmol).
$^1$H NMR (600 MHz, $CDCl_3$) δ 4.93-4.89 (d, J = 5.8 Hz, 1H), 4.89-4.83 (m, 2H), 4.76-4.70 (m, 1H), 3.26-3.15 (m, 4H), 2.28-2.26 (m, 8H), 2.21 (s, 6H), 1.69 (s, 2H), 1.64-1.58 (m, 7H), 1.65-1.55 (m, 15H), 1.33-1.21 (m, 56H), 1.19 (d, J = 6.2 Hz, 3H), 0.91 (t, J = 7.3 Hz, 3H), 0.87 (t, J = 7.0 Hz, 12H).
LC-MS (ESI): (M + H) calculated 1021.91, found 1022.3.

**Example 52: Synthesis of Amino Lipid Compound 300**

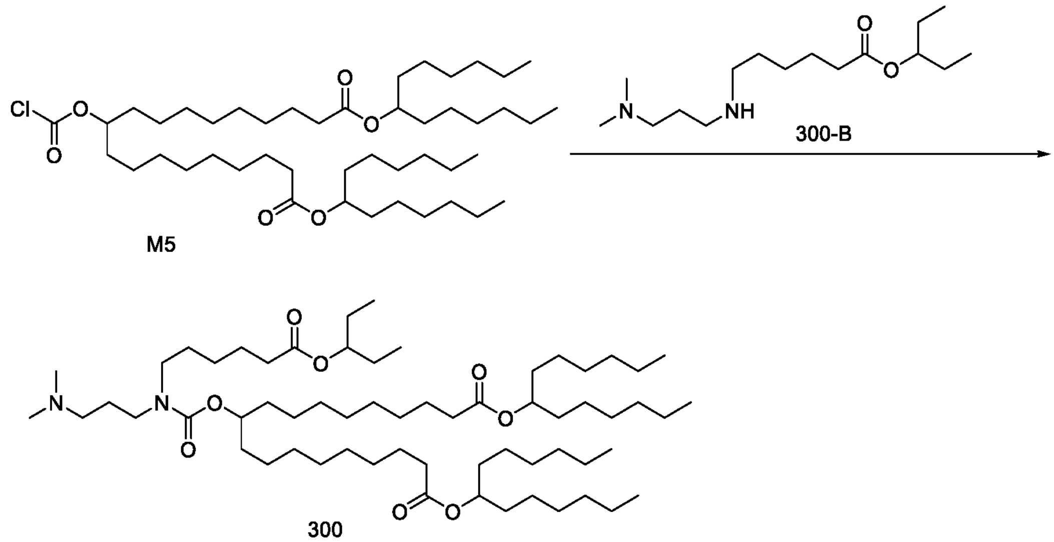

M5

300-B

300

According to the general synthetic process, 550 mg amino lipid compound 300, as a pale yellow oil with a purity of 98.00% and a yield of 47%, was prepared from M5 (1.0 g, 1.29 mmol) and 300-B (0.56 g, 1.94 mmol).

$^1$H NMR (600 MHz, $CDCl_3$) δ 4.89-4.83 (m, 2H), 4.76-4.71 (m, 2H), 3.26-3.15 (m, 4H), 2.31-2.24 (m, 8H), 2.21 (s, 6H), 1.68 (s, 2H), 1.67-1.57 (m, 8H), 1.57-1.44 (m, 16H), 1.35-1.20 (m, 54H), 0.87 (t, J = 7.1 Hz, 18H).

LC-MS (ESI): (M + H) calculated 1021.91, found 1022.1.

**Example 53: Synthesis of Amino Lipid Compound 301**

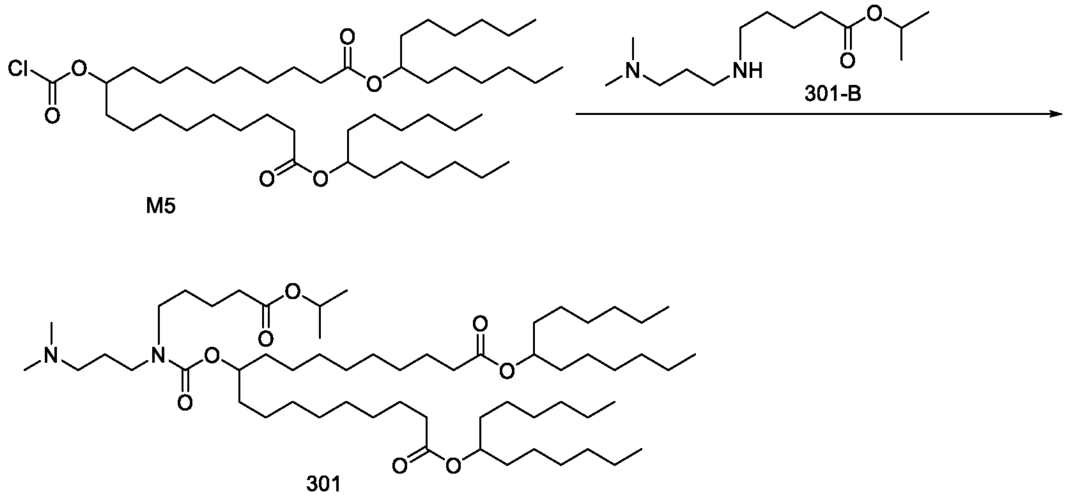

M5

301-B

301

According to the general synthetic process, 750 mg amino lipid compound 301, as a pale yellow oil with a purity of 98.18% and a yield of 67%, was prepared from M5 (1.0 g, 1.29 mmol) and 301-B (0.47 g, 1.94 mmol).

$^1$H NMR (600 MHz, $CDCl_3$) δ 5.03-4.95 (m, 1H), 4.89-4.83 (m, 2H), 4.76-4.70 (m, 1H), 3.28-3.18 (m, 4H), 2.26 (t, J = 7.5 Hz, 8H), 2.21 (s, 6H), 1.69 (s, 2H), 1.60 (dt, J = 14.5, 7.4 Hz, 6H), 1.51 (t, J = 14.9 Hz, 14H), 1.32-1.21 (m, 58H), 0.87 (t, J = 7.0 Hz, 12H).

LC-MS (ESI): (M + H) calculated 979.86, found 980.1.

**Example 54: Synthesis of Amino Lipid Compound 302**

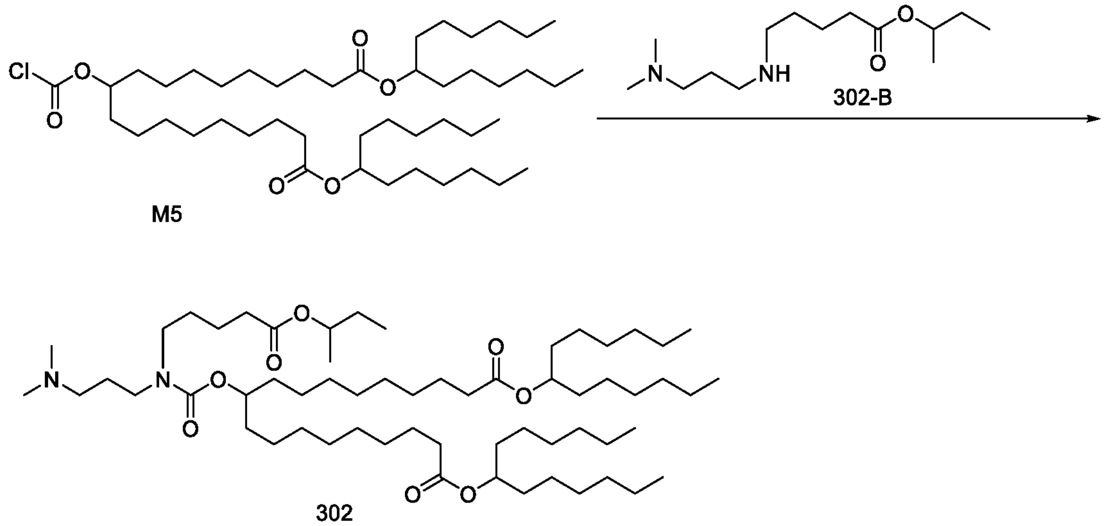


M5

302

According to the general synthetic process, 680 mg amino lipid compound 302, as a pale yellow oil with a purity of 97.32% and a yield of 60%, was prepared from M5 (1.0 g, 1.29 mmol) and 302-B (0.50 g, 1.94 mmol).

$^1$H NMR (600 MHz, $CDCl_3$) δ 4.89-4.79 (m, 3H), 4.75-4.69 (m, 1H), 3.30-3.13 (m, 4H), 2.32-2.23 (m, 8H), 2.21 (s, 6H), 1.74-1.65 (m, 2H), 1.63-1.55 (m, 8H), 1.55-1.47 (m, 14H), 1.32-1.21 (m, 52H), 1.19 (d, J = 6.2 Hz, 3H), 0.90-0.85 (m, 15H).

LC-MS (ESI): (M + H) calculated 993.88, found 994.1.

**Example 55: Synthesis of Amino Lipid Compound 303**

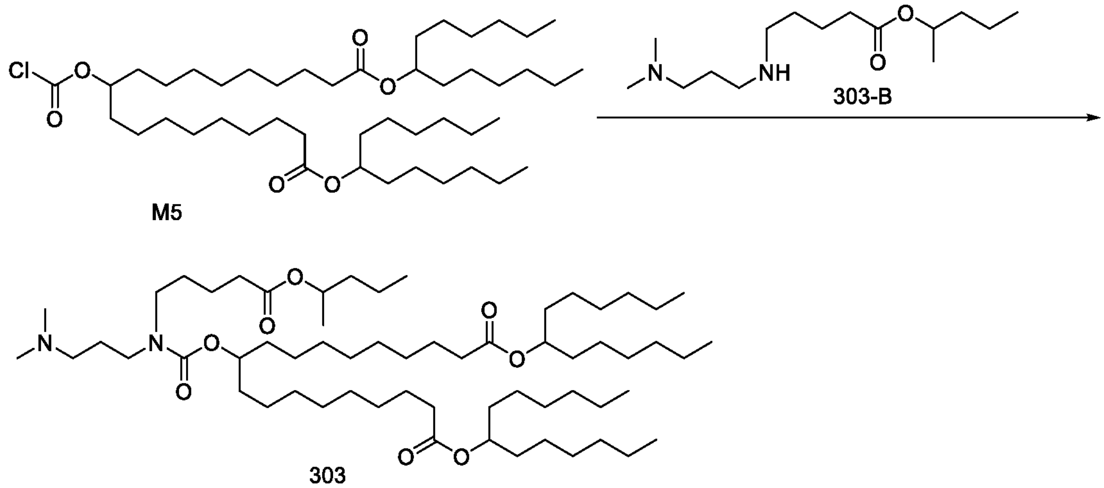


M5

303

According to the general synthetic process, 390 mg amino lipid compound 303, as a pale yellow oil with a purity of 95.21% and a yield of 34%, was prepared from M5 (1.0 g, 1.29 mmol) and 303-B (0.53 g, 1.94 mmol).

$^1$H NMR (600 MHz, $CDCl_3$) δ 4.93-4.90 (m, 1H), 4.88-4.83 (m, 2H), 4.76-4.69 (m, 1H), 3.27-3.19 (m, 4H), 2.31-2.22 (t, J = 7.5 Hz, 8H), 2.20 (s, 6H), 1.69 (s, 2H), 1.61-1.57 (m, 8H), 1.53-1.47 (m, 14H), 1.33-1.21 (m, 54H), 1.19 (d, J = 6.2 Hz, 3H), 0.91-0.86 (m, 15H).

LC-MS (ESI): (M + H) calculated 1007.89, found 1008.1.

**Example 56: Synthesis of Amino Lipid Compound 304**

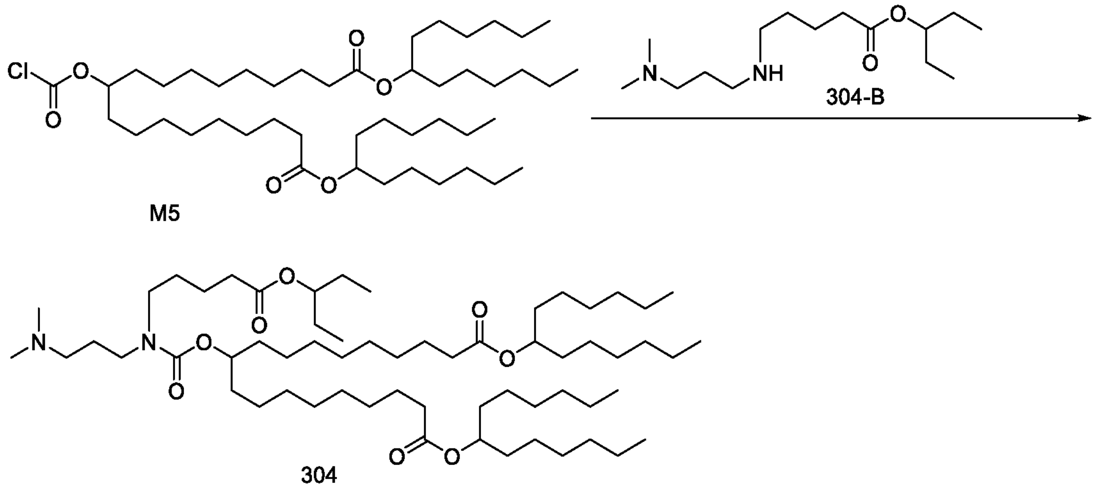

M5

304-B

304

According to the general synthetic process, 660 mg amino lipid compound 304, as a pale yellow oil with a purity of 98.51% and a yield of 57%, was prepared from M5 (1.0 g, 1.29 mmol) and 304-B (0.53 g, 1.94 mmol).
$^{1}$H NMR (600 MHz, $CDCl_3$) δ 4.89-4.83 (m, 2H), 4.77-4.70 (m, 2H), 3.27-3.17 (m, 4H), 2.31 (s, 2H), 2.26 (t, J = 7.5 Hz, 6H), 2.21 (s, 6H), 1.69 (s, 2H), 1.63-1.44 (m, 24H), 1.35-1.18 (m, 52H), 0.87 (t, J = 7.0 Hz, 18H).
LC-MS (ESI): (M + H) calculated 1007.89, found 1008.1.

**Example 57: Synthesis of Amino Lipid Compound 305**

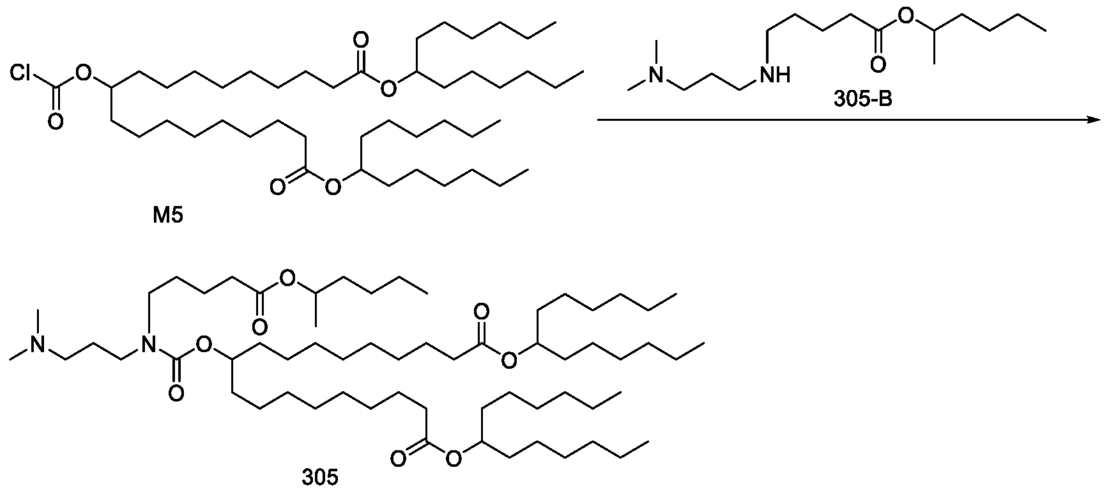

M5

305-B

305

According to the general synthetic process, 490 mg amino lipid compound 305, as a pale yellow oil with a purity of 97.96% and a yield of 42%, was prepared from M5 (1.0 g, 1.29 mmol) and 305-B (0.56 g, 1.94 mmol).
$^{1}$H NMR (600 MHz, $CDCl_3$) δ 4.94-4.83 (m, 3H), 4.76-4.69 (m, 1H), 3.28-3.18 (m, 4H), 2.31-2.25 (m, 8H), 2.21 (s, 6H), 1.69 (s, 2H), 1.63-1.56 (m, 8H), 1.54-1.46 (m, 14H), 1.35-1.21 (m, 56H), 1.19 (d, J = 6.2 Hz, 3H), 0.90-0.86 (m, 15H).
LC-MS (ESI): (M + H) calculated 1021.91, found 1022.1.

**Example 58: Synthesis of Amino Lipid Compound 306**

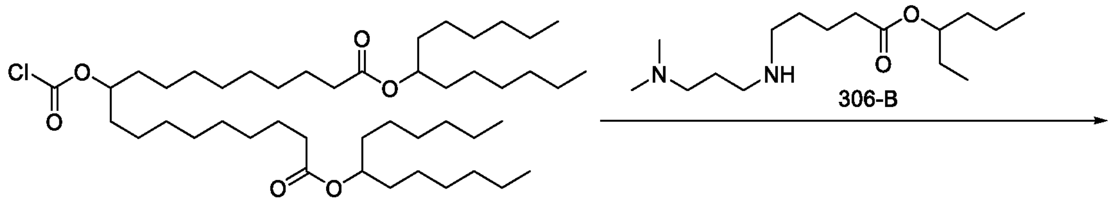

M5

306-B

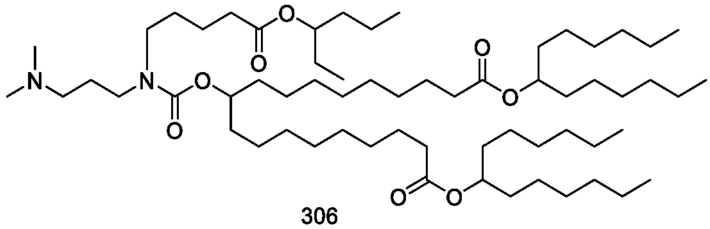

306

According to the general synthetic process, 410 mg amino lipid compound 306, as a pale yellow oil with a purity of 96.94% and a yield of 35%, was prepared from M5 (1.0 g, 1.29 mmol) and 306-B (0.56 g, 1.94 mmol).

$^1$H NMR (600 MHz, $CDCl_3$) δ 4.89-4.79 (m, 3H), 4.75-4.69 (m, 1H), 3.29-3.15 (m, 4H), 2.31 (s, 2H), 2.27-2.23 (m, 6H), 2.20 (s, 6H), 1.69 (s, 2H), 1.64-1.44 (m, 24H), 1.36-1.19 (m, 54H), 0.88 (dt, J = 14.0, 7.3 Hz, 18H).

LC-MS (ESI): (M + H) calculated 1021.91, found 1022.1.

**Example 59: Synthesis of Amino Lipid Compound 307**

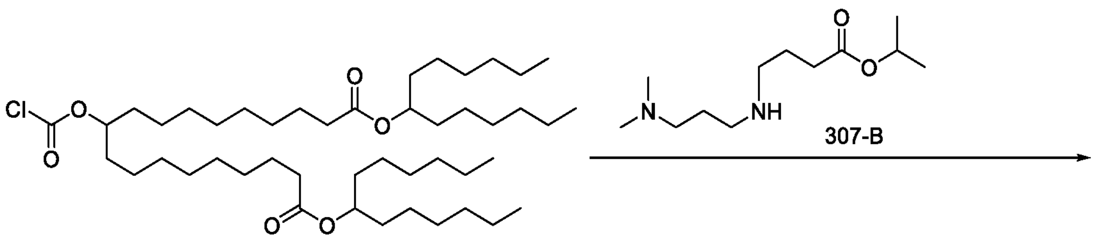

M5

307-B

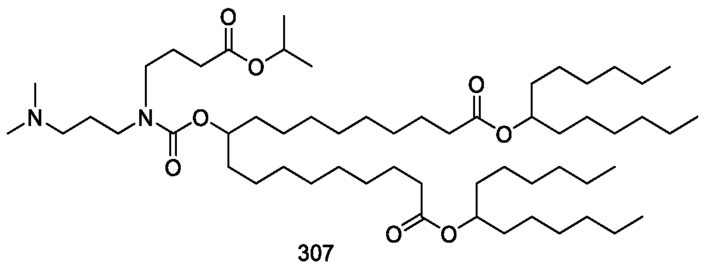

307

According to the general synthetic process, 160 mg amino lipid compound 307, as a pale yellow oil with a purity of 92.29% and a yield of 16%, was prepared from M5 (1.0 g, 1.29 mmol) and 307-B (0.45 g, 1.94 mmol).

$^1$H NMR (600 MHz, $CDCl_3$) δ5.02-4.98 (m, 1H), 4.88-4.84(m, 2H), 4.75-4.71(m, 1H), 3.27-3.23 (m, 4H), 2.28-2.25 (m, 8H), 2.21(m, 6H), 1.83 (m, 2H), 1.73-1.69 (m, 2H), 1.61-1.59 (m, 4H), 1.50-1.49 (m, 12H), 1.28-1.22 (m, 58H), 0.87 (t, J=6.9Hz, 12H).

LC-MS (ESI): (M + H) calculated 965.85, found 966.1.

**Example 60: Synthesis of Amino Lipid Compound 308**

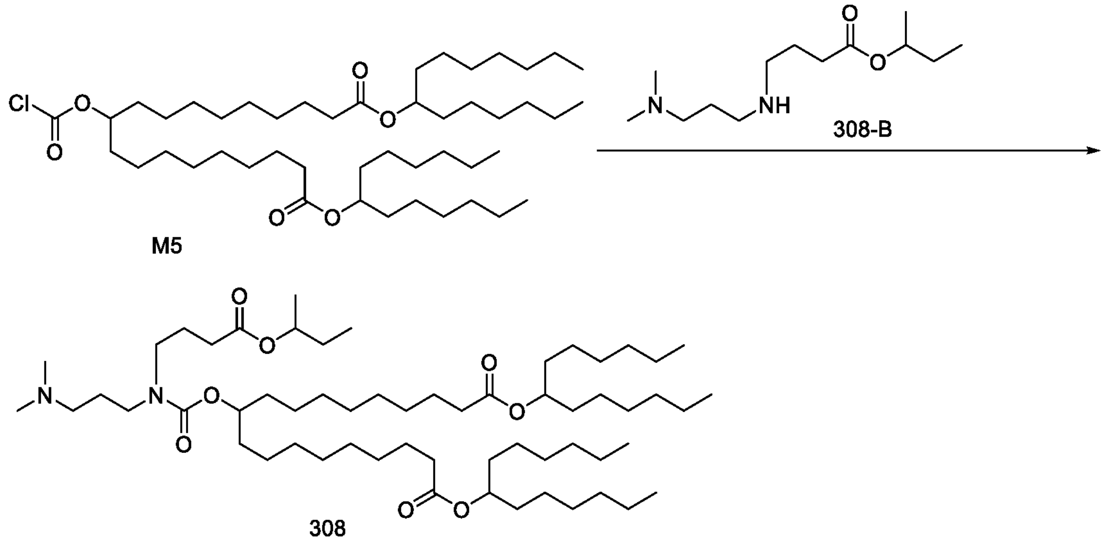

M5

308-B

308

According to the general synthetic process, 660 mg amino lipid compound 308, as a pale yellow oil with a purity of 98.69% and a yield of 59%, was prepared from M5 (1.0 g, 1.29 mmol) and 308-B (0.47 g, 1.94 mmol).

$^1$H NMR (600 MHz, $CDCl_3$) δ4.88-4.82 (m, 3H), 4.75-4.71 (m, 1H), 3.26-3.22 (m, 4H), 2.27-2.25 (m, 8H), 2.20 (s, 6H), 1.84 (m, 2H), 1.69 (m, 2H), 1.61-1.49 (m, 18H), 1.29-1.25 (m, 52H), 1.19 (d, J=6.2Hz, 3H), 0.89-0.85 (m, 15H).

LC-MS (ESI): (M + H) calculated 979.86, found 980.1.

## Example 61: Synthesis of Amino Lipid Compound 309

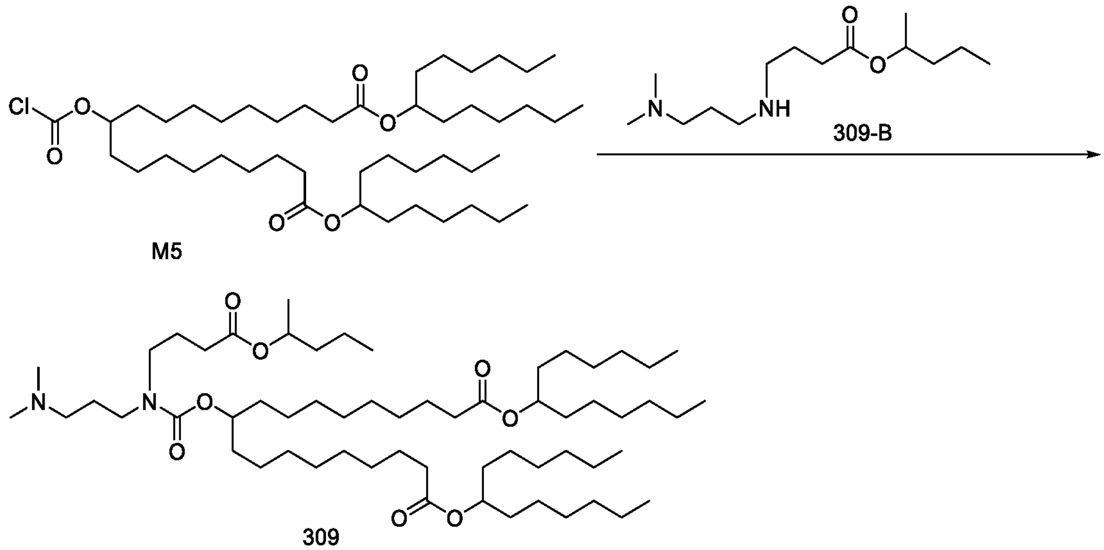

M5

309-B

309

According to the general synthetic process, 740 mg amino lipid compound 309, as a pale yellow oil with a purity of 97.45% and a yield of 65%, was prepared from M5 (1.0 g, 1.29 mmol) and 309-B (0.50 g, 1.94 mmol).

$^1$H NMR (600 MHz, $CDCl_3$) δ4.94-4.84 (m, 3H), 4.75-4.71 (m, 1H), 3.27-3.23 (m, 4H), 2.28-2.25 (m, 8H), 2.21 (s, 6H), 1.84 (m, 2H), 1.70 (m, 2H), 1.63-1.48 (m, 18H), 1.31-1.26 (m, 54H), 1.19 (d, J=6.2Hz, 3H), 0.92-0.86 (m, 15H).

LC-MS (ESI): (M + H) calculated 993.88, found 994.1.

## Example 62: Synthesis of Amino Lipid Compound 310

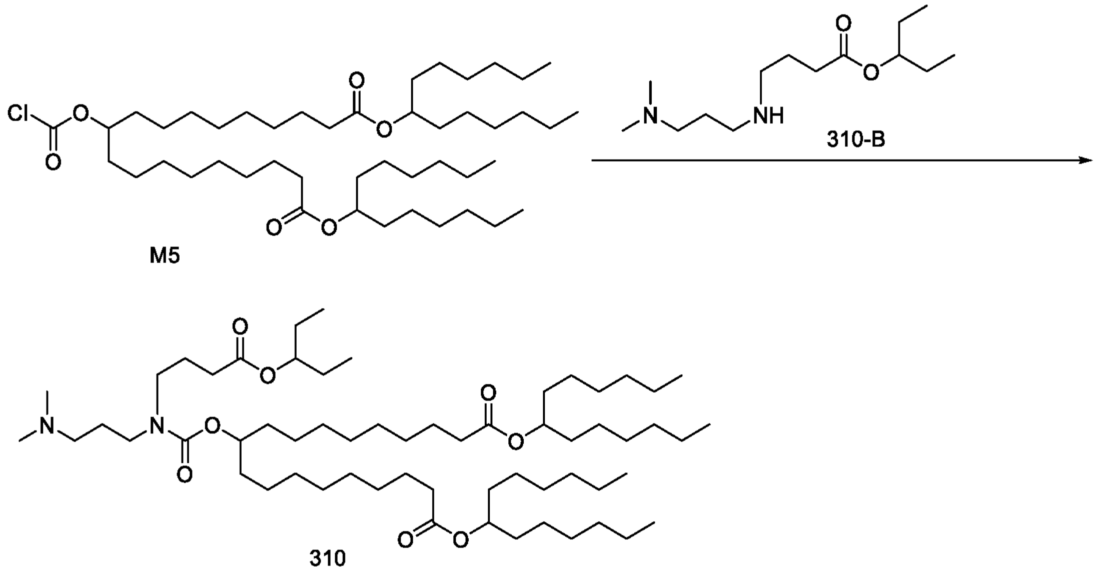

M5

310-B

310

According to the general synthetic process, 650 mg amino lipid compound 310, as a pale yellow oil with a purity of 99.28% and a yield of 57%, was prepared from M5 (1.0 g, 1.29 mmol) and 310-B (0.50 g, 1.94 mmol).

$^1H$ NMR (600 MHz, $CDCl_3$) δ4.88-4.84 (m, 2H), 4.78-4.72 (m, 2H), 3.27-3.23 (m, 4H), 2.30-2.26 (m, 8H), 2.21 (s, 6H), 1.86 (m, 2H), 1.70 (m, 2H), 1.62-1.50 (m, 20H), 1.30-1.26 (m, 52H), 0.87 (t, J=7.0Hz, 18H).

LC-MS (ESI): (M + H) calculated 993.88, found 994.3.

**Example 63: Synthesis of Amino Lipid Compound 311**

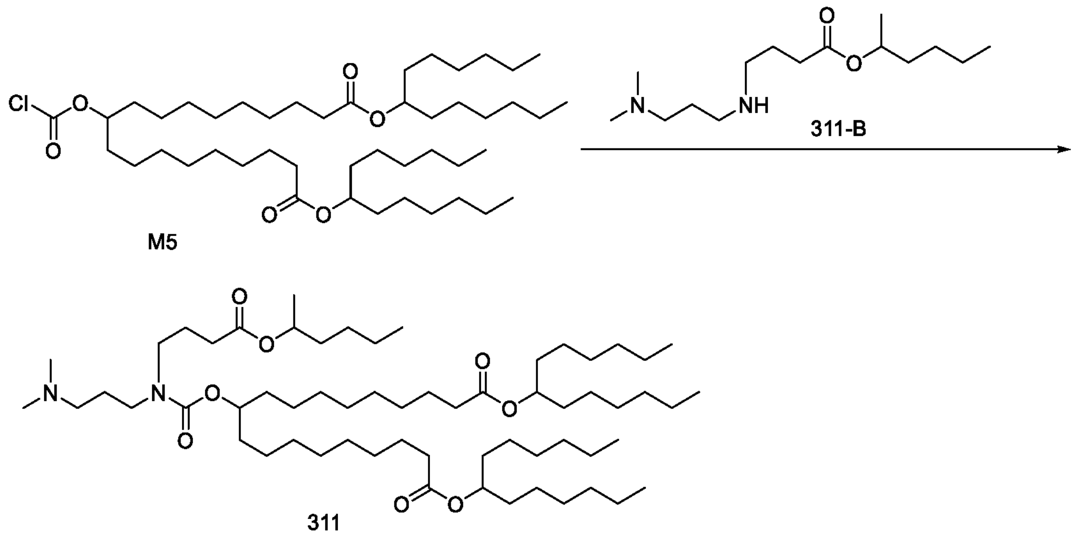

M5

311-B

311

According to the general synthetic process, 420 mg amino lipid compound 311, as a pale yellow oil with a purity of 97.21% and a yield of 36%, was prepared from M5 (1.0 g, 1.29 mmol) and 311-B (0.53 g, 1.94 mmol).

$^1H$ NMR (600 MHz, $CDCl_3$) δ4.92-4.84 (m, 3H), 4.75-4.71 (m, 1H), 3.27-3.23 (m, 4H), 2.28-2.25 (m, 8H), 2.21 (m, 6H), 1.84 (m, 2H), 1.70 (m, 2H), 1.62-1.48 (m, 18H), 1.28-1.25 (m, 56H), 1.19 (d, J=6.3Hz, 3H), 0.90-0.86 (m, 15H).

LC-MS (ESI): (M + H) calculated 1007.89, found 1008.1.

**Example 64: Synthesis of Amino Lipid Compound 312**

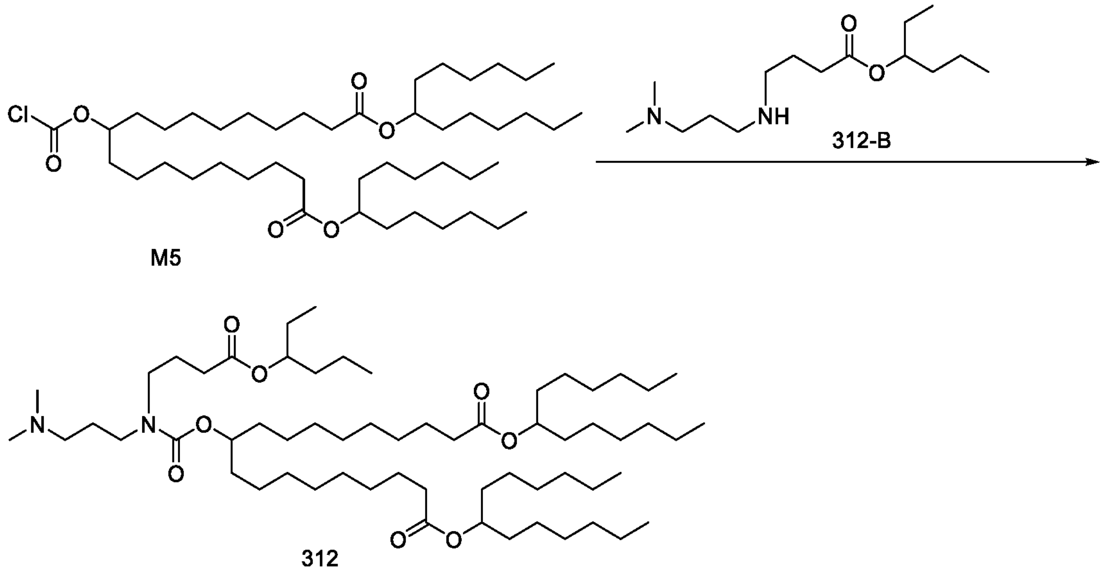

312-B

M5

312

According to the general synthetic process, 650 mg amino lipid compound 312, as a pale yellow oil with a purity of 97.79% and a yield of 56%, was prepared from M5 (1.0 g, 1.29 mmol) and 312-B (0.53 g, 1.94 mmol).
$^1$H NMR (600 MHz, $CDCl_3$) δ4.88-4.81 (m, 3H), 4.76-4.71 (m, 1H), 3.27-3.23 (m, 4H), 2.28-2.25 (m, 8H), 2.20 (s, 6H), 1.85 (m, 2H), 1.70 (m, 2H), 1.62-1.49 (m, 20H), 1.27-1.25 (m, 54H), 0.91-0.86 (m, 18H).
LC-MS (ESI): (M + H) calculated 1007.89, found 1008.1.

**Example 65: Synthesis of Amino Lipid Compound 313**

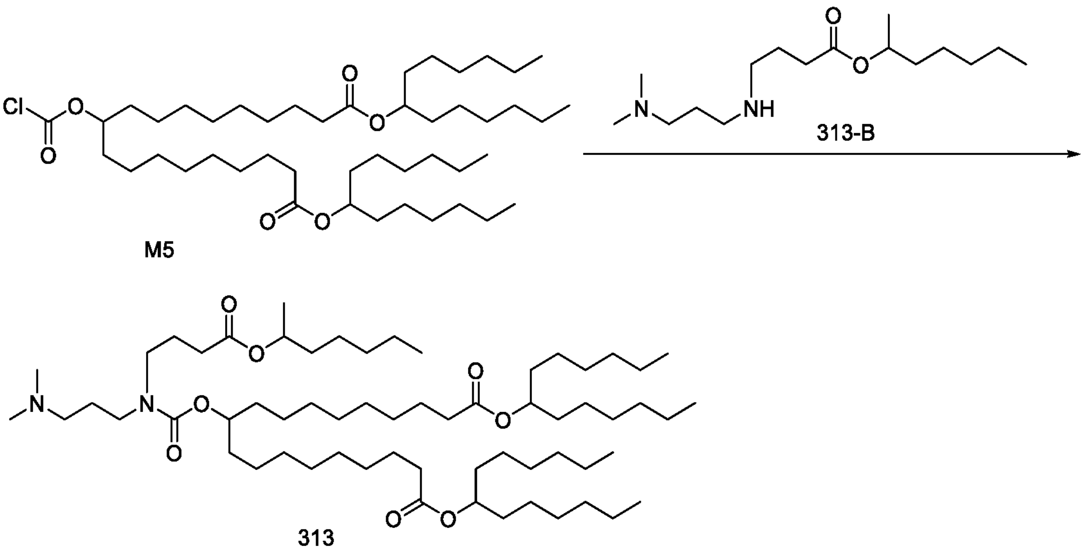

313-B

M5

313

According to the general synthetic process, 330 mg amino lipid compound 313, as a pale yellow oil with a purity of 96.97% and a yield of 28%, was prepared from M5 (1.0 g, 1.29 mmol) and 313-B (0.56 g, 1.94 mmol).
$^1$H NMR (600 MHz, $CDCl_3$) δ4.92-4.84 (m, 3H), 4.75-4.71 (m, 1H), 3.27-3.23 (m, 4H), 2.28-2.25 (m, 8H), 2.20 (s, 6H), 1.84 (s, 2H), 1.70 (s, 2H), 1.61-1.50 (m, 18H), 1.30-1.26 (m, 58H), 1.19 (d, J=6.3Hz, 3H), 0.89-0.86 (m, 15H).
LC-MS (ESI): (M + H) calculated 1021.91, found 1022.2.

**Example 66: Synthesis of Amino Lipid Compound 314**

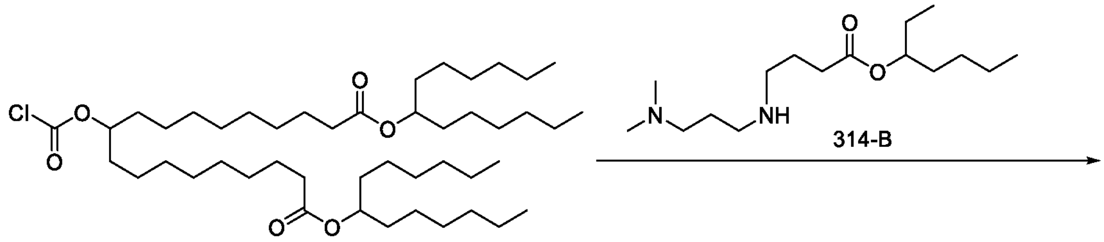

314-B

M5

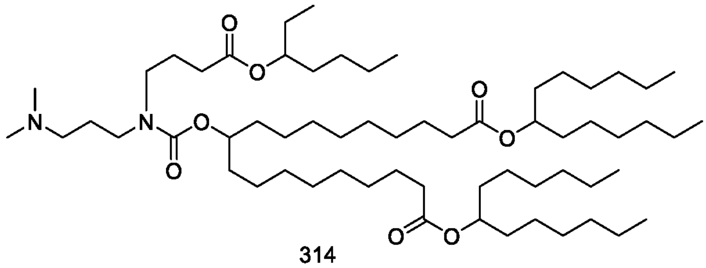

314

According to the general synthetic process, 122 mg amino lipid compound 314, as a pale yellow oil with a purity of 91.54% and a yield of 10%, was prepared from M5 (1.0 g, 1.29 mmol) and 314-B (0.56 g, 1.94 mmol).

$^1H$ NMR (600 MHz, $CDCl_3$) δ4.88-4.84 (m, 2H), 4.83-4.79 (m, 1H), 4.76-4.71 (m, 1H), 3.27-3.23 (m, 4H), 2.29-2.25 (m, 8H), 2.21 (s, 6H), 1.85 (m, 2H), 1.70 (m, 2H), 1.61-1.49 (m, 20H), 1.28-1.25 (m, 56H), 0.89-0.86 (m, 18H).

LC-MS (ESI): (M + H) calculated 1021.91, found 1022.3.

**Example 67: Synthesis of Amino Lipid Compound 315**

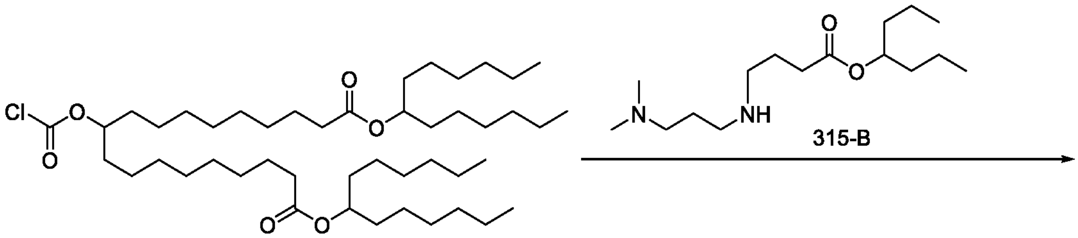

315-B

M5

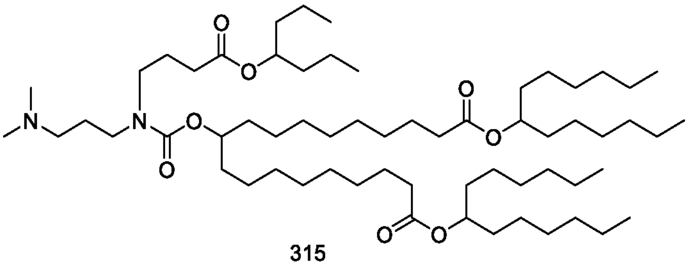

315

According to the general synthetic process, 480 mg amino lipid compound 315, as a pale yellow oil with a purity of 98.27% and a yield of 41%, was prepared from M5 (1.0 g, 1.29 mmol) and 315-B (0.56 g, 1.94 mmol).

$^1H$ NMR (600 MHz, $CDCl_3$) δ4.92-4.85 (m, 3H), 4.76-4.72 (m, 1H), 3.27-3.23 (m, 4H), 2.28-2.25 (m, 8H), 2.21 (s, 6H), 1.84 (m, 2H), 1.70 (m, 2H), 1.63-1.58 (m, 4H), 1.53-1.46 (m, 16H), 1.28-1.26 (m, 56H), 0.91-0.86 (m, 18H).

LC-MS (ESI): (M + H) calculated 1021.91, found 1022.1.

Example 68: Synthesis of Amino Lipid Compound 316

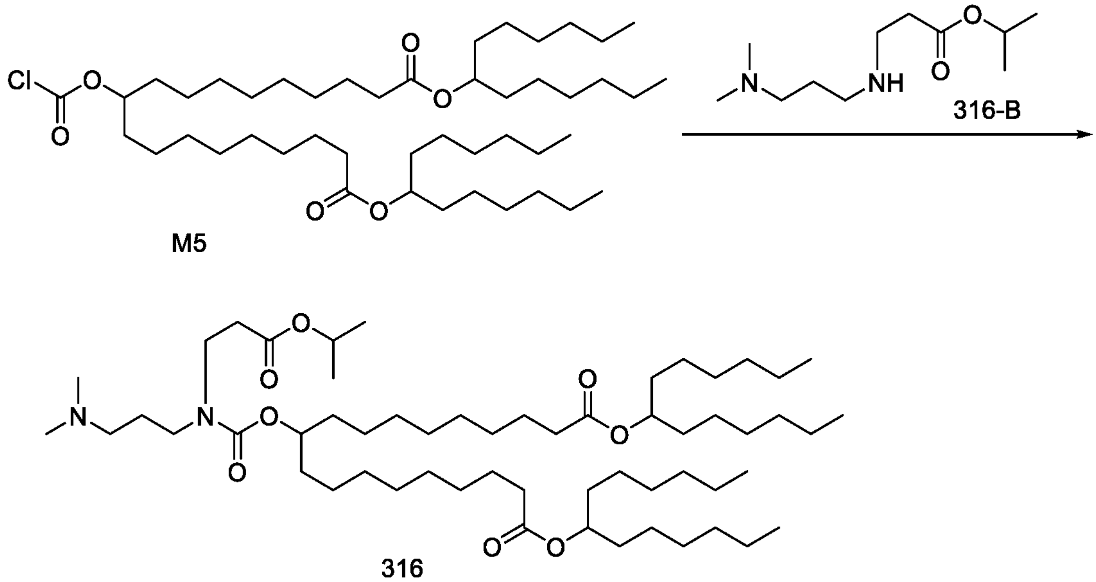

M5

316-B

316

According to the general synthetic process, 300 mg amino lipid compound 316, as a pale yellow oil with a purity of 94.90% and a yield of 28%, was prepared from M5 (1.0 g, 1.29 mmol) and 316-B (0.42 g, 1.94 mmol).

$^1H$ NMR (600 MHz, $CDCl_3$) δ5.02-4.98 (m, 1H), 4.88-4.84 (m, 2H), 4.75-4.73 (m, 1H), 3.52-3.48 (m, 2H), 3.29-3.25 (m, 2H), 2.56-2.51 (m, 2H), 2.27 (t, J=7.5Hz, 6H), 2.21 (m, 6H), 1.70 (m, 2H), 1.63-1.58 (m, 4H), 1.51-1.50 (m, 12H), 1.30-1.23 (m, 58H), 0.87 (t, J=6.9Hz, 12H).

LC-MS (ESI): (M + H) calculated 951.83, found 952.0.

**Example 69: Synthesis of Amino Lipid Compound 317**

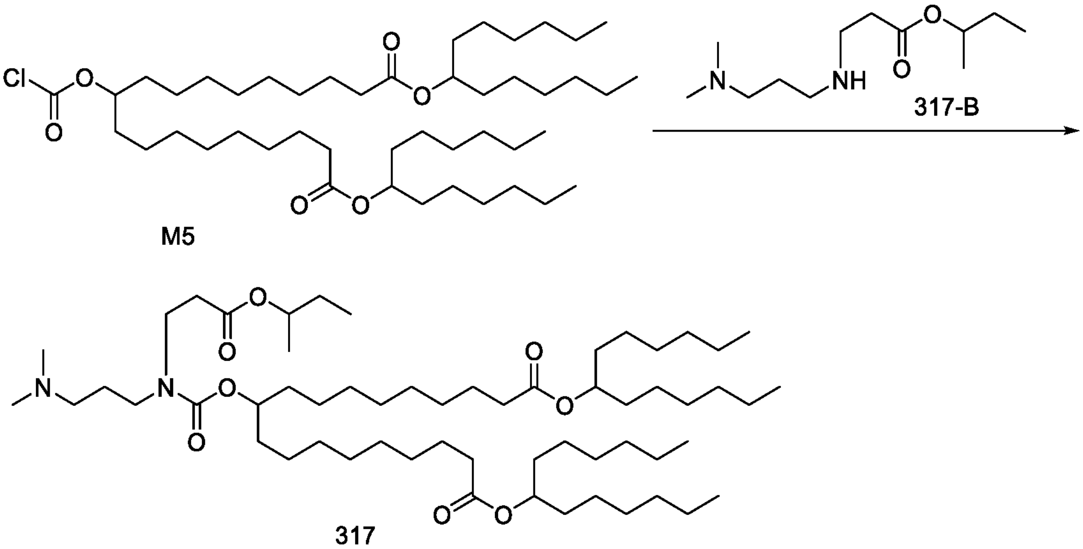

M5

317-B

317

According to the general synthetic process, 300 mg amino lipid compound 317, as a pale yellow oil with a purity of 92.50% and a yield of 27%, was prepared from M5 (1.0 g, 1.29 mmol) and 317-B (0.45 g, 1.94 mmol).

$^1H$ NMR (600 MHz, $CDCl_3$) δ4.88-4.82 (m, 3H), 4.74 (m, 1H), 3.52-3.48 (m, 2H), 3.29-3.25 (m, 2H), 2.58-2.53 (m, 2H), 2.27 (t, J=7.6Hz, 6H), 2.21 (s, 6H), 1.69 (m, 2H), 1.61-1.49 (m, 18H), 1.28-1.19 (m, 55H), 0.87(t, J=7.0Hz, 15H).

LC-MS (ESI): (M + H) calculated 965.85, found 966.0.

**Example 70: Synthesis of Amino Lipid Compound 318**

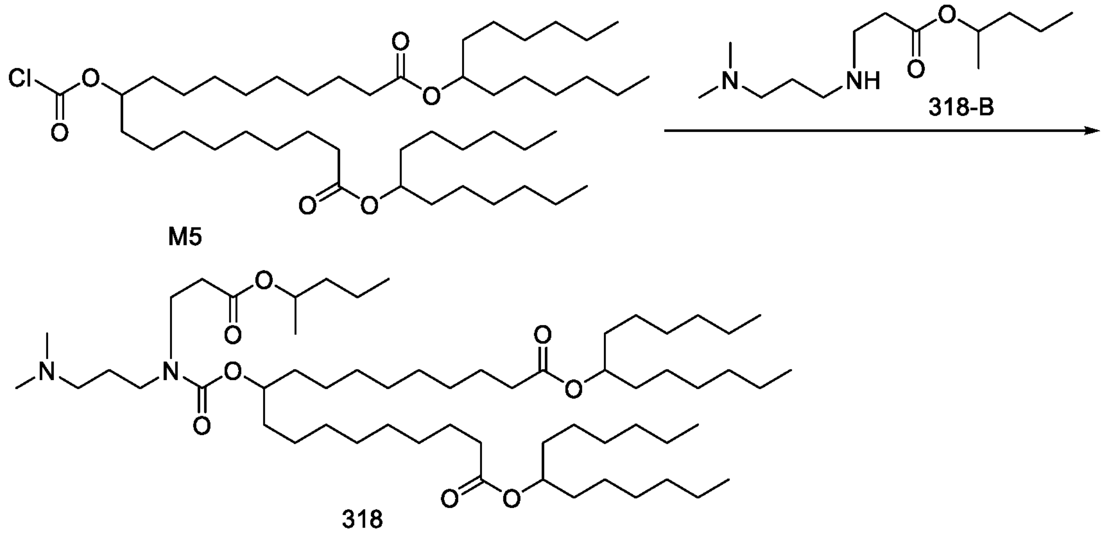

M5

318-B

318

According to the general synthetic process, 283 mg amino lipid compound 318, as a pale yellow oil with a purity of 94.86% and a yield of 25%, was prepared from M5 (1.0 g, 1.29 mmol) and 318-B (0.47 g, 1.94 mmol).

$^1H$ NMR (600 MHz, $CDCl_3$) δ4.93-4.90 (m, 1H), 4.88-4.84 (m, 2H), 4.74 (m, 1H), 3.51-3.47 (m, 2H), 3.28-3.25 (m, 2H), 2.57-2.52 (m, 2H), 2.28-2.25 (m, 6H), 2.21 (m, 6H), 1.69 (m, 2H), 1.61-1.49 (m, 18H), 1.27-1.19 (m, 57H), 0.90-0.86 (m, 15H).

LC-MS (ESI): (M + H) calculated 979.86, found 980.1.

**Example 71: Synthesis of Amino Lipid Compound 319**

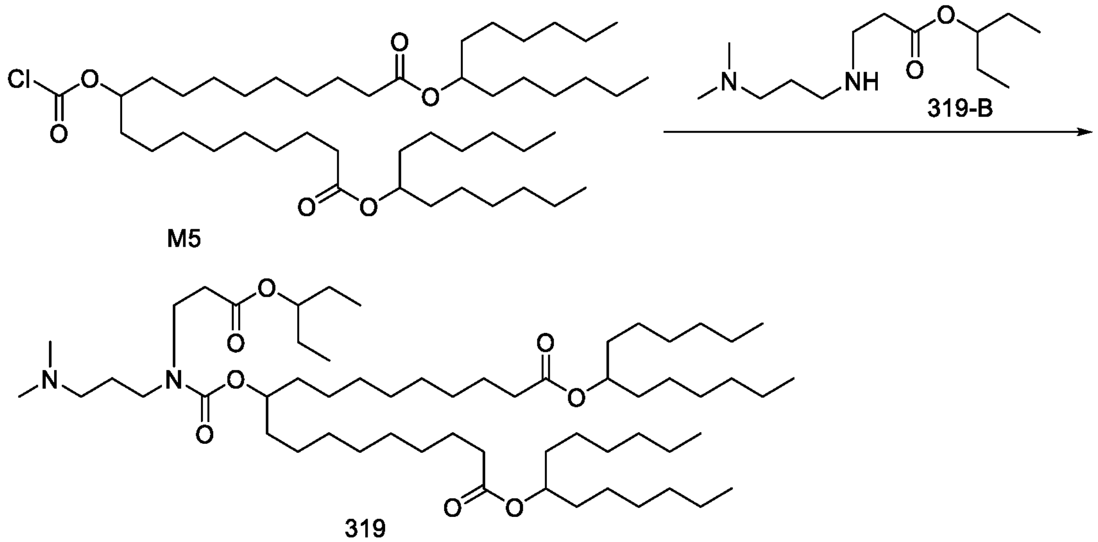

M5

319-B

319

According to the general synthetic process, 350 mg amino lipid compound 319, as a pale yellow oil with a purity of 97.46% and a yield of 31%, was prepared from M5 (1.0 g, 1.29 mmol) and 319-B (0.47 g, 1.94 mmol).

$^1H$ NMR (600 MHz, $CDCl_3$) δ4.88-4.84 (m, 2H), 4.76-4.73 (m, 2H), 3.52-3.48 (m, 2H), 3.29-3.25 (m, 2H), 2.61-2.55 (m, 2H), 2.28-2.25 (m, 6H), 2.20 (s, 6H), 1.69 (m, 2H), 1.61-1.49 (m, 20H), 1.27-1.25 (m, 52H), 0.88-0.86 (m, 18H).

LC-MS (ESI): (M + H) calculated 979.86, found 980.0.

**Example 72: Synthesis of Amino Lipid Compound 320**

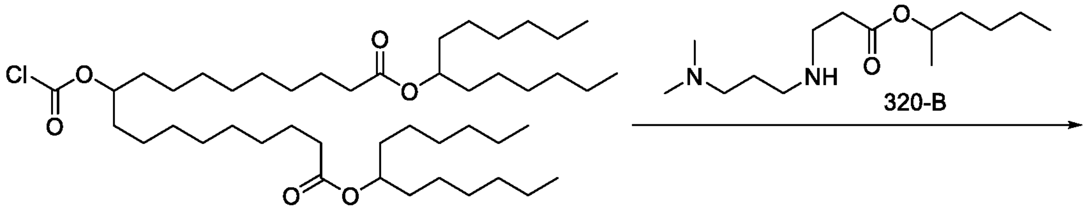

320-B

M5

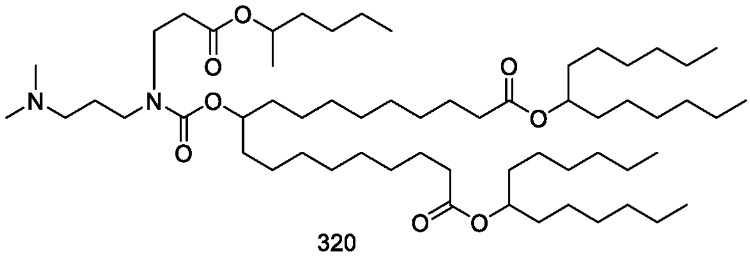

320

According to the general synthetic process, 350 mg amino lipid compound 320, as a pale yellow oil with a purity of 96.53% and a yield of 31%, was prepared from M5 (1.0 g, 1.29 mmol) and 320-B (0.50 g, 1.94 mmol).
$^1$H NMR (600 MHz, $CDCl_3$) δ4.90-4.83 (m, 3H), 4.73 (m, 1H), 3.51-3.47 (m, 2H), 3.28-3.24 (m, 2H), 2.57-2.51 (m, 2H), 2.26 (t, J=7.5Hz, 6H), 2.20 (s, 6H), 1.69 (m, 2H), 1.61-1.49 (m, 18H), 1.27-1.19 (m, 59H), 0.89-0.85 (m, 15H).
LC-MS (ESI): (M + H) calculated 993.88, found 994.1.

**Example 73: Synthesis of Amino Lipid Compound 321**

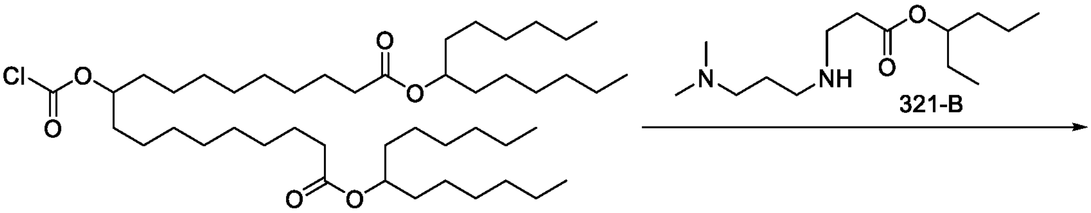

321-B

M5

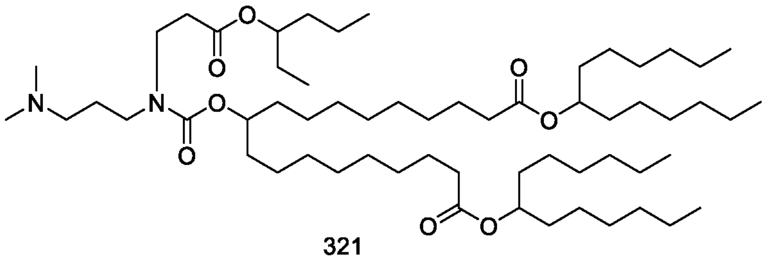

321

According to the general synthetic process, 450 mg amino lipid compound 321, as a pale yellow oil with a purity of 98.04% and a yield of 40%, was prepared from M5 (1.0 g, 1.29 mmol) and 321-B (0.50 g, 1.94 mmol).
$^1$H NMR (600 MHz, $CDCl_3$) δ4.88-4.81 (m, 3H), 4.73 (m, 1H), 3.51-3.48 (m, 2H), 3.28-3.25 (m, 2H), 2.59-2.54 (m, 2H), 2.27-2.25 (m, 6H), 2.20 (s, 6H), 1.69 (m, 2H), 1.61-1.49 (m, 20H), 1.27-1.25 (m, 54H), 0.89-0.85 (m, 18H).
LC-MS (ESI): (M + H) calculated 993.88, found 994.1.

**Example 74: Synthesis of Amino Lipid Compound 322**

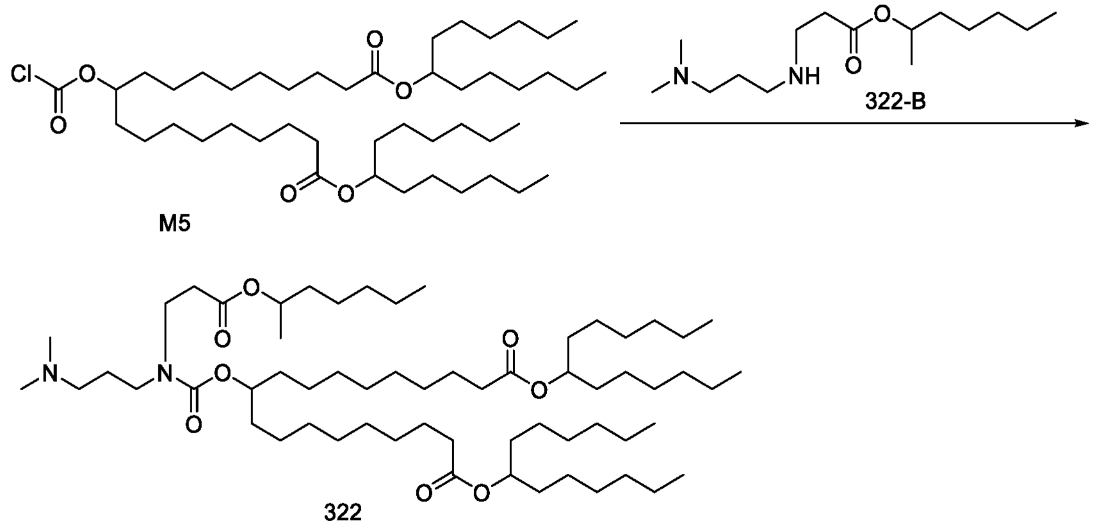

M5

322-B

322

According to the general synthetic process, 350 mg amino lipid compound 322, as a pale yellow oil with a purity of 97.74% and a yield of 30%, was prepared from M5 (1.0 g, 1.29 mmol) and 322-B (0.53 g, 1.94 mmol).

$^1$H NMR (600 MHz, $CDCl_3$) δ4.91-4.84 (m, 3H), 4.73 (m, 1H), 3.51-3.47 (m, 2H), 3.28-3.24 (m, 2H), 2.57-2.52 (m, 2H), 2.28-2.25 (m, 6H), 2.20 (m, 6H), 1.69 (m, 2H), 1.61-1.49 (m, 18H), 1.27-1.19 (m, 61H), 0.89-0.86 (m, 15H).

LC-MS (ESI): (M + H) calculated 1007.89, found 1008.1.

**Example 75: Synthesis of Amino Lipid Compound 323**

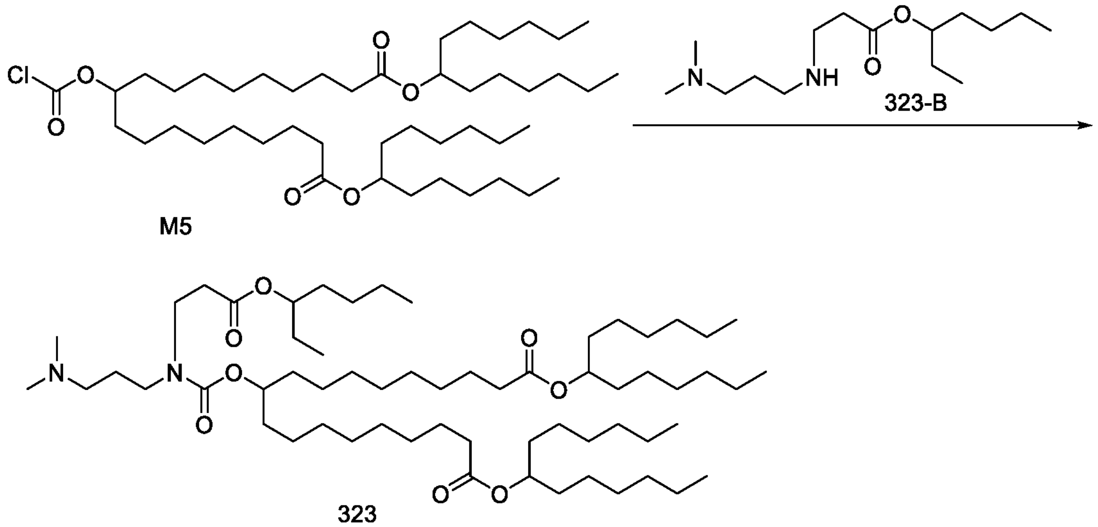

M5

323-B

323

According to the general synthetic process, 520 mg amino lipid compound 323, as a pale yellow oil with a purity of 99.64% and a yield of 45%, was prepared from M5 (1.0 g, 1.29 mmol) and 323-B (0.53 g, 1.94 mmol).

$^1$H NMR (600 MHz, $CDCl_3$) δ4.88-4.84 (m, 2H), 4.83-4.80 (m, 1H), 4.74 (m, 1H), 3.52-3.48 (m, 2H), 3.29-3.25 (m, 2H), 2.60-2.55 (m, 2H), 2.28-2.25 (m, 6H), 2.20 (s, 6H), 1.69- 1.50 (m, 22H), 1.28-1.26 (m, 56H), 0.88-0.86 (m, 18H).

LC-MS (ESI): (M + H) calculated 1007.89, found 1008.0.

**Example 76: Synthesis of Amino Lipid Compound 325**

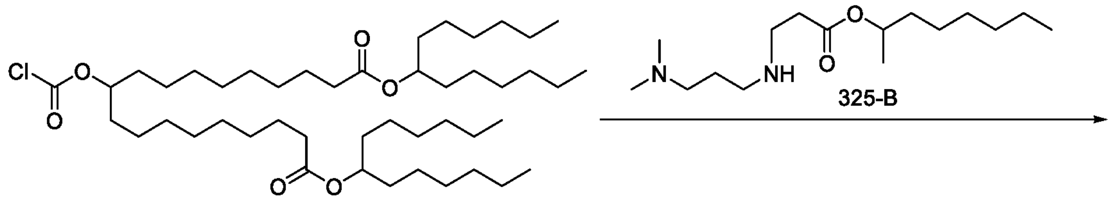

M5 325-B

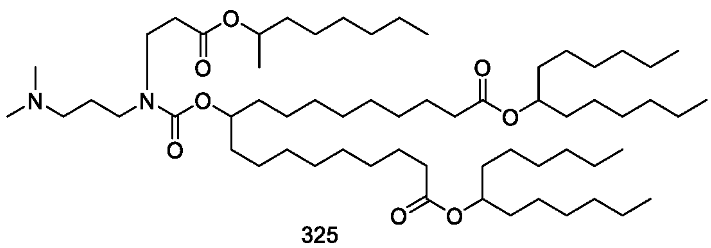

325

According to the general synthetic process, 620 mg amino lipid compound 325, as a pale yellow oil with a purity of 93.75% and a yield of 54%, was prepared from M5 (1.0 g, 1.29 mmol) and 325-B (0.56 g, 1.94 mmol).

$^1$H NMR (600 MHz, $CDCl_3$) δ4.91-4.84 (m, 3H), 4.74 (m, 1H), 3.51-3.47 (m, 2H), 3.28-3.23 (m, 2H), 2.57-2.52 (m, 2H), 2.26 (t, J=7.5Hz, 6H), 2.21 (m, 6H), 1.69 (m, 2H), 1.61-1.59 (m, 18H), 1.27-1.19 (m, 63H), 0.89-0.86 (m, 15H).

LC-MS (ESI): (M + H) calculated 1021.91, found 1022.2.

**Example 77: Synthesis of Amino Lipid Compound 326**

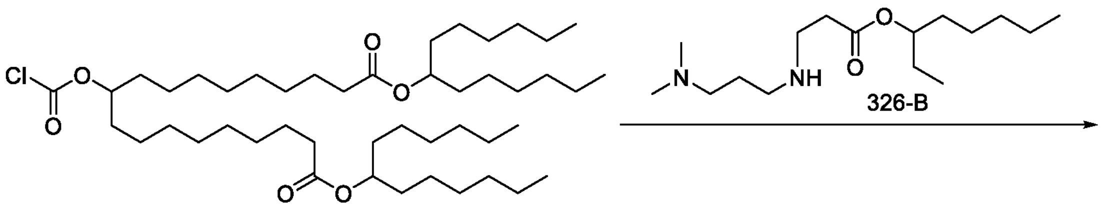

M5 326-B

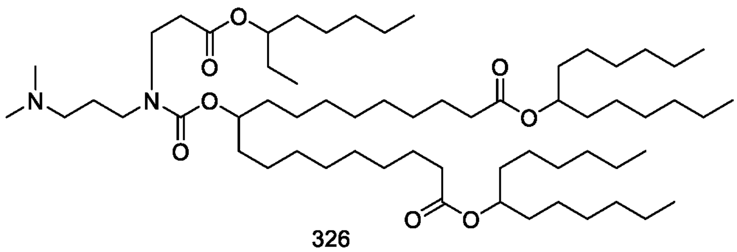

326

According to the general synthetic process, 700 mg amino lipid compound 326, as a pale yellow oil with a purity of 98.56% and a yield of 60%, was prepared from M5 (1.0 g, 1.29 mmol) and 326-B (0.56 g, 1.94 mmol).

$^1$H NMR (600 MHz, $CDCl_3$) δ4.88-4.84 (m, 2H), 4.83-4.81 (m, 1H), 4.74 (m, 1H), 3.52-3.49 (m, 2H), 3.29-3.26 (m, 2H), 2.60-2.55 (m, 2H), 2.60-2.55 (m, 2H), 2.28-2.26 (m, 6H), 2.21 (s, 6H), 1.70-1.50 (m, 22H), 1.28-1.26 (m, 58H), 0.88-0.86 (m, 18H).

LC-MS (ESI): (M + H) calculated 1021.91, found 1022.3.

**Example 78: Synthesis of Amino Lipid Compound 327**

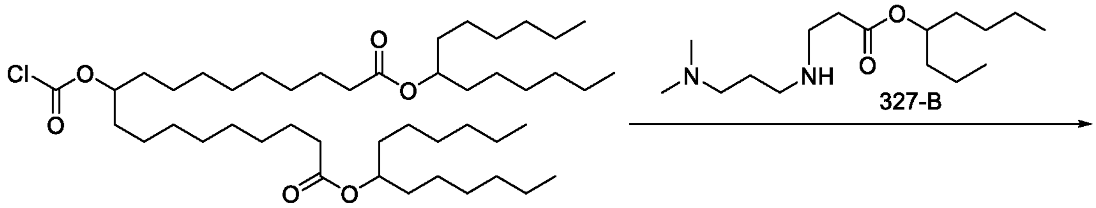
327-B

M5

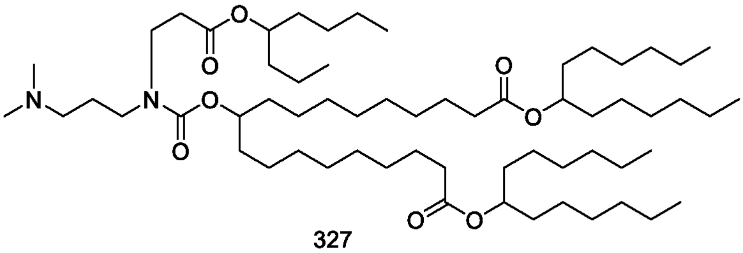
327

According to the general synthetic process, 550 mg amino lipid compound 327, as a pale yellow oil with a purity of 98.53% and a yield of 46%, was prepared from M5 (1.0 g, 1.29 mmol) and 327-B (0.56 g, 1.94 mmol).

$^1H$ NMR (600 MHz, $CDCl_3$) δ4.91-4.84 (m, 3H), 4.74 (m, 1H), 3.52-3.48 (m, 2H), 3.29-3.25 (m, 2H), 2.59-2.54 (m, 2H), 2.28-2.25 (m, 6H), 2.21 (s, 6H), 1.70 (m, 2H), 1.62-1.59 (m, 4H), 1.51-1.50 (m, 16H), 1.28-1.26 (m, 58H), 0.90-0.86 (m, 18H).

LC-MS (ESI): (M + H) calculated 1021.91, found 1022.2.

**Example 79: Synthesis of Amino Lipid Compound 328**

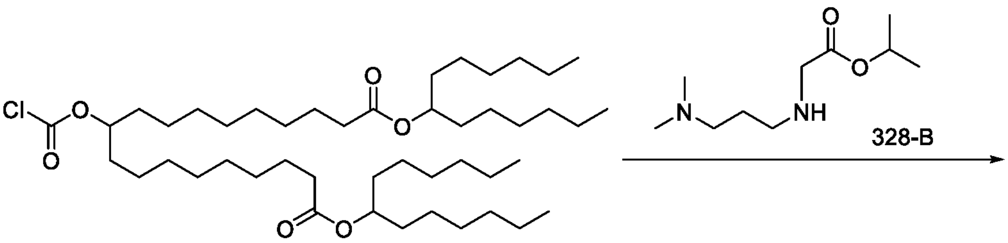
328-B

M5

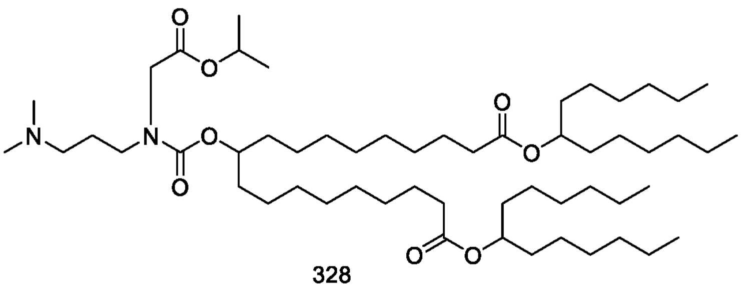
328

According to the general synthetic process, 220 mg amino lipid compound 328, as a pale yellow oil with a purity of 97.73% and a yield of 21%, was prepared from M5 (1.0 g, 1.29 mmol) and 328-B (0.39 g, 1.94 mmol).

$^1H$ NMR (600 MHz, $CDCl_3$) δ5.06-5.00 (m, 1H), 4.88-4.84 (m, 2H), 4.78-1.70 (m, 1H), 3.96 (m, 1H), 3.88 (s, 1H), 3.37-3.29 (m, 2H), 2.28-2.25 (m, 6H), 2.21-2.20 (m, 6H), 1.71 (m, 2H), 1.62-1.58 (m, 4H), 1.50-1.49 (m, 12H), 1.27-1.23 (m, 58H), 0.87 (m, 12H).

LC-MS (ESI): (M + H) calculated 937.81, found 938.3.

**Example 80: Synthesis of Amino Lipid Compound 329**

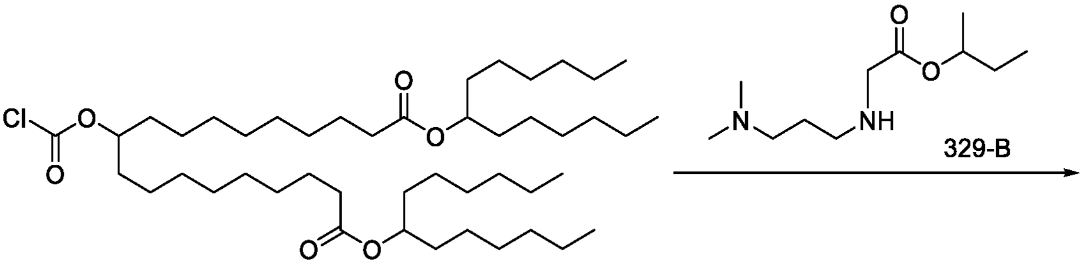

M5

329-B

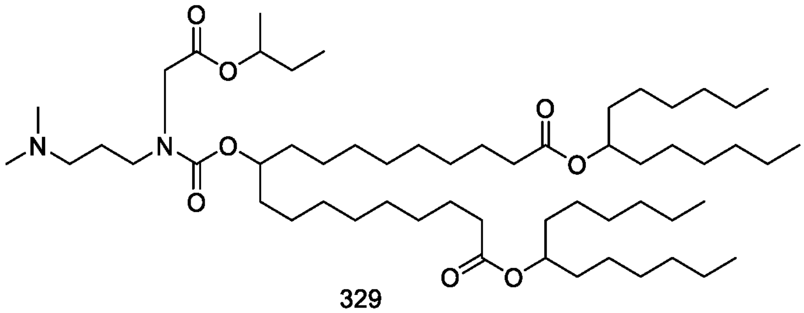

329

According to the general synthetic process, 650 mg amino lipid compound 329, as a pale yellow oil with a purity of 98.08% and a yield of 60%, was prepared from M5 (1.0 g, 1.29 mmol) and 329-B (0.42 g, 1.94 mmol).

$^1$H NMR (600 MHz, $CDCl_3$) δ4.90-4.84 (m, 3H), 4.78-4.70 (m, 1H), 3.98 (s, 1H), 3.94-3.87 (m, 1H), 3.40-3.29 (m, 2H), 2.28-2.25 (m, 6H), 2.20-2.19 (m, 6H), 1.74-1.67 (m, 2H), 1.61-1.49 (m, 18H), 1.27-1.20 (m, 55H), 0.90-0.86 (m, 15H).

LC-MS (ESI): (M + H) calculated 951.83, found 952.3.

**Example 81: Synthesis of Amino Lipid Compound 330**

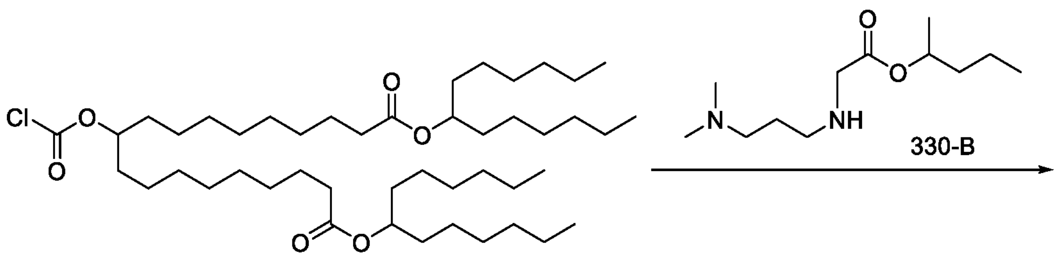

M5

330-B

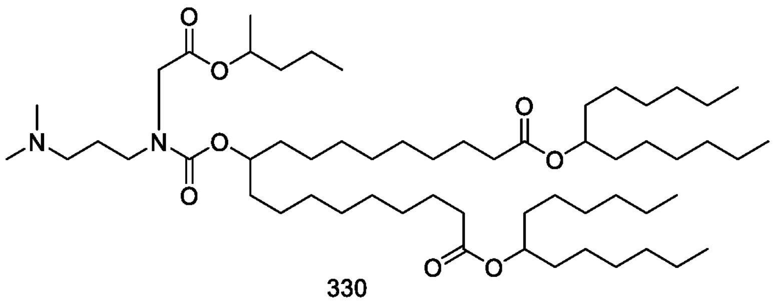

330

According to the general synthetic process, 740 mg amino lipid compound 330, as a pale yellow oil with a purity of 92.28% and a yield of 67%, was prepared from M5 (1.0 g, 1.29 mmol) and 330-B (0.45 g, 1.94 mmol).

$^1H$ NMR (600 MHz, $CDCl_3$) δ4.98-4.92 (m, 1H), 4.88-4.85 (m, 2H), 4.78-4.71 (m, 1H), 3.97 (dd, J1=19.6Hz, J2=17.6Hz, 1H), 3.90 (dd, J1=25Hz, J2=18Hz, 1H), 3.40-3.30 (m, 2H), 2.27 (t, J=7.5Hz, 6H), 2.22-2.21 (m, 6H), 1.74-1.45 (m, 20H), 1.36-1.21 (m, 57H), 0.92-0.86 (m, 15H).

LC-MS (ESI): (M + H) calculated 965.85, found 966.6.

**Example 82: Synthesis of Amino Lipid Compound 331**

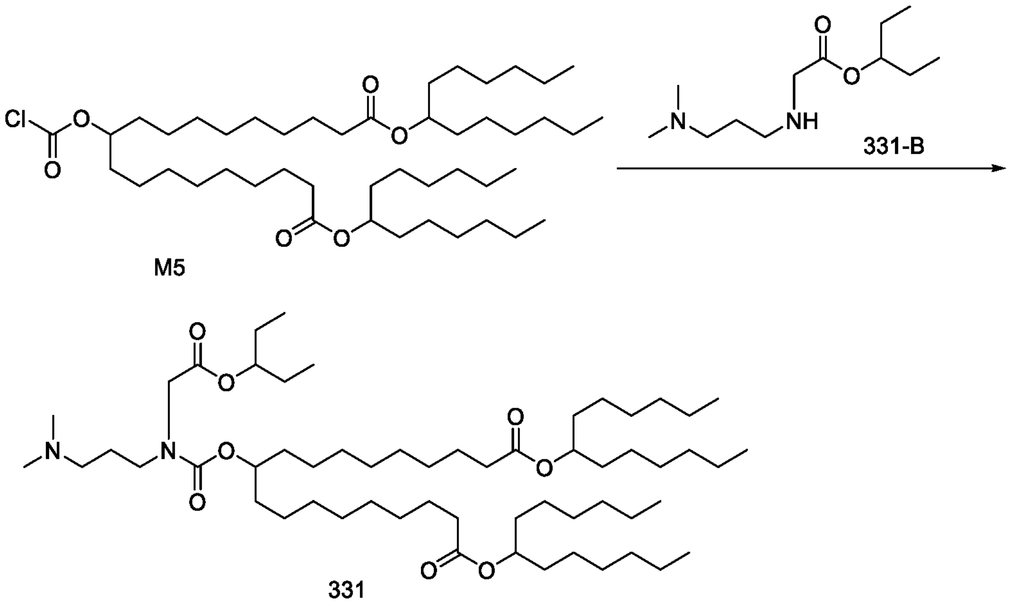

M5

331-B

331

According to the general synthetic process, 338 mg amino lipid compound 331, as a pale yellow oil with a purity of 93.50% and a yield of 31%, was prepared from M5 (1.0 g, 1.29 mmol) and 331-B (0.45 g, 1.94 mmol).

$^1H$ NMR (600 MHz, $CDCl_3$) δ4.89-4.84 (m, 2H), 4.82-4.71 (m, 2H), 4.01 (s, 1H), 3.94 (s, 1H), 3.38-3.30 (m, 2H), 2.27 (t, J=7.5Hz, 6H), 2.21-2.20 (m, 6H), 1.74-1.46 (m, 22H), 1.28-1.26 (m, 52H), 0.89-0.86 (m, 18H).

LC-MS (ESI): (M + H) calculated 965.85, found 966.6.

**Example 83: Synthesis of Amino Lipid Compound 332**

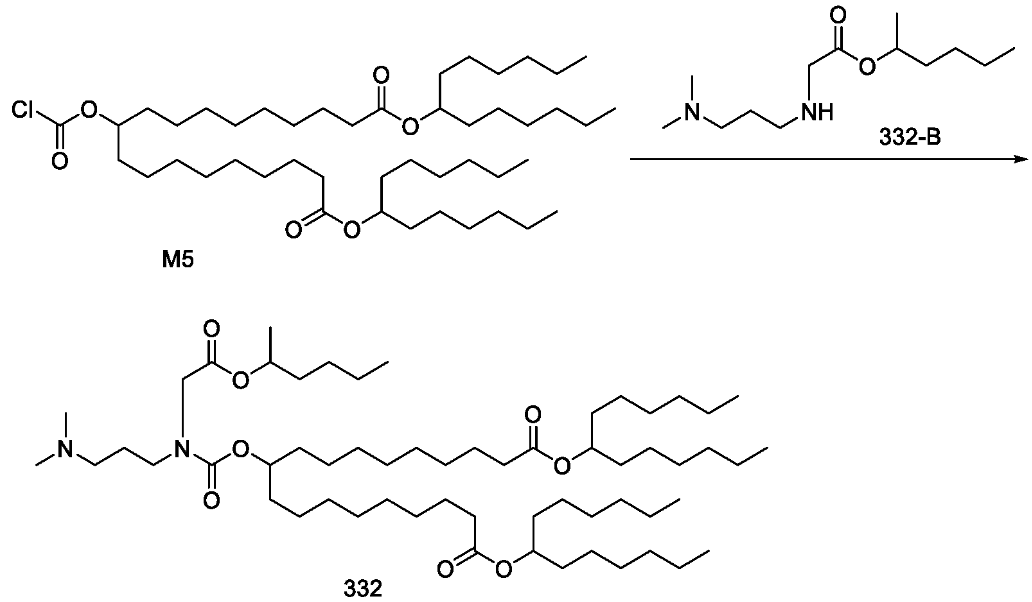


332

According to the general synthetic process, 517 mg amino lipid compound 332, as a pale yellow oil with a purity of 96.71% and a yield of 46%, was prepared from M5 (1.0 g, 1.29 mmol) and 332-B (0.47 g, 1.94 mmol).

$^1H$ NMR (600 MHz, $CDCl_3$) δ4.95-4.90 (m, 1H), 4.89-4.84 (m, 2H), 4.78-4.71 (m, 1H), 3.98 (s, 1H), 3.94-3.87 (m, 1H), 3.39-3.30 (m, 2H), 2.28-2.26 (m, 6H), 2.21-2.20 (m, 6H), 1.73-1.47 (m, 20H), 1.33-1.21 (m, 59H) , 0.91-0.86 (m, 15H).

LC-MS (ESI): (M + H) calculated 979.86, found 980.3.

**Example 84: Synthesis of Amino Lipid Compound 333**

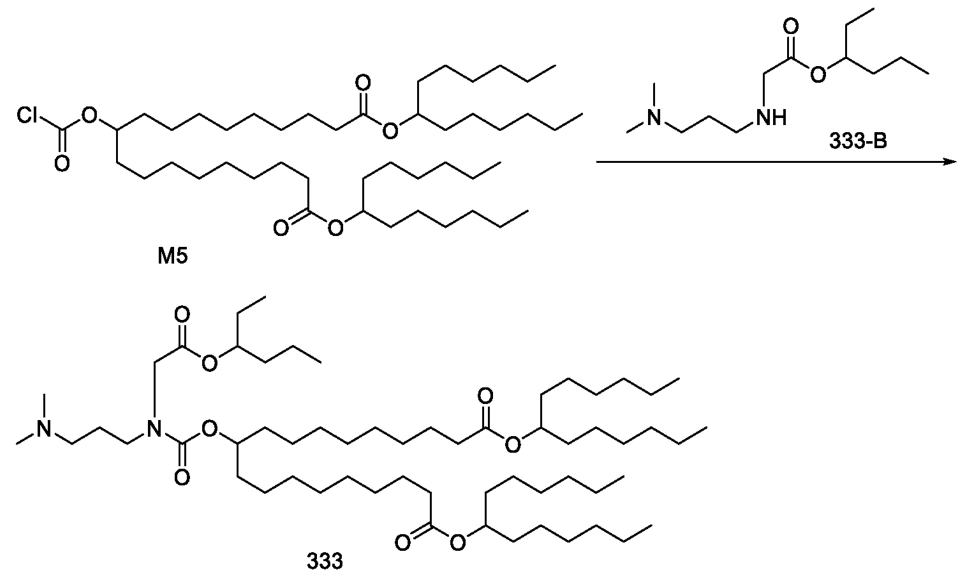


333

According to the general synthetic process, 519 mg amino lipid compound 333, as a pale yellow oil with a purity of 96.56% and a yield of 46%, was prepared from M5 (1.0 g, 1.29 mmol) and 333-B (0.47 g, 1.94 mmol).

$^1H$ NMR (600 MHz, $CDCl_3$) δ4.89-4.84 (m, 3H), 4.78-4.71 (m, 1H), 4.00 (s, 1H), 3.93 (s, 1H), 3.37-3.30 (m, 2H), 2.28-2.26 (m, 6H), 2.21-2.20 (m, 6H), 1.71-1.49 (m, 22H), 1.30-1.26 (m, 54H), 0.91-0.86 (m, 18H).

LC-MS (ESI): (M + H) calculated 979.86, found 980.4.

**Example 85: Synthesis of Amino Lipid Compound 334**

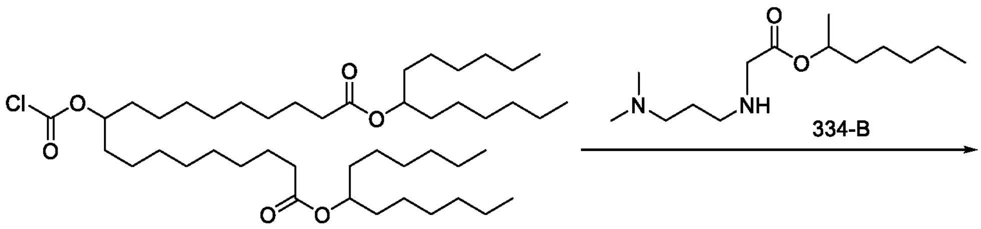


M5

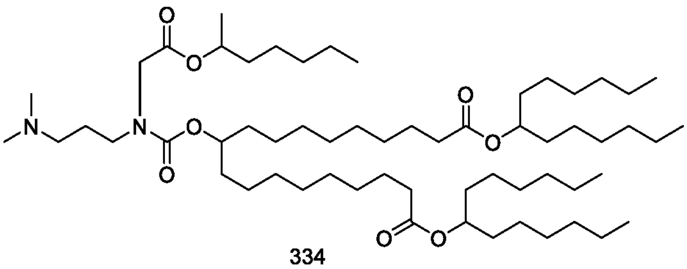

334

According to the general synthetic process, 935 mg amino lipid compound 334, as a pale yellow oil with a purity of 93.38% and a yield of 82%, was prepared from M5 (1.0 g, 1.29 mmol) and 334-B (0.50 g, 1.94 mmol).

$^{1}$H NMR (600 MHz, $CDCl_3$) δ4.95-4.90 (m, 1H), 4.89-4.84 (m, 2H), 4.78-4.71 (m, 1H), 3.98 (s, 1H), 3.93-3.86 (m, 1H), 3.39-3.30 (m, 2H), 2.28-2.26 (m, 6H), 2.22-2.20 (m, 6H), 1.74-1.69 (m, 2H), 1.63-1.49 (m, 18H), 1.29-1.21 (m, 61H), 0.88 (t, J=6.8Hz, 15H).

LC-MS (ESI): (M + H) calculated 993.88, found 994.3.

**Example 86: Synthesis of Amino Lipid Compound 335**

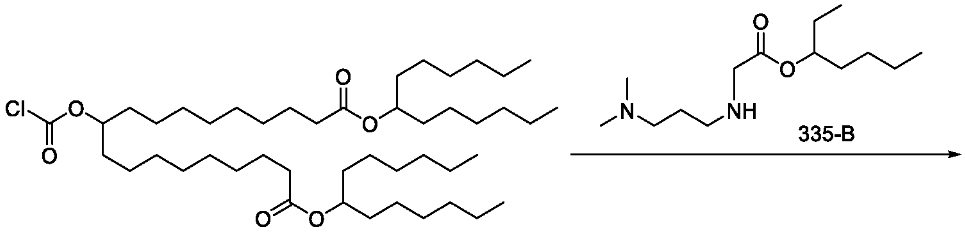


M5

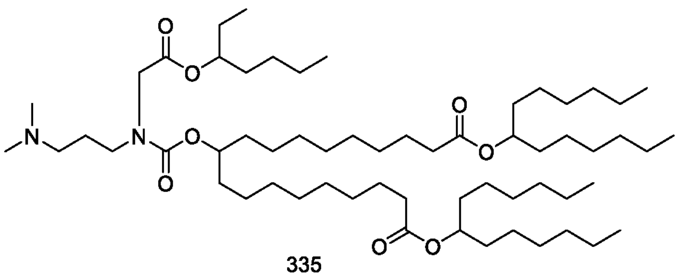

335

According to the general synthetic process, 432 mg amino lipid compound 335, as a pale yellow oil with a purity of 94.97% and a yield of 46%, was prepared from M5 (1.0 g, 1.29 mmol) and 335-B (0.50 g, 1.94 mmol).

$^{1}$H NMR (600 MHz, $CDCl_3$) δ4.88-4.83 (m, 3H), 4.78-4.71 (m, 1H), 4.00 (s, 1H), 3.93 (s, 1H), 3.37-3.30 (m, 2H), 2.28-2.26 (m, 6H), 2.21-2.20 (m ,6H), 1.74-1.68 (m, 2H), 1.62-1.50 (20H), 1.28-1.26 (m, 56H), 0.89-0.86 (m, 18H).

LC-MS (ESI): (M + H) calculated 993.88, found 994.3.

**Example 87: Synthesis of Amino Lipid Compound 336**

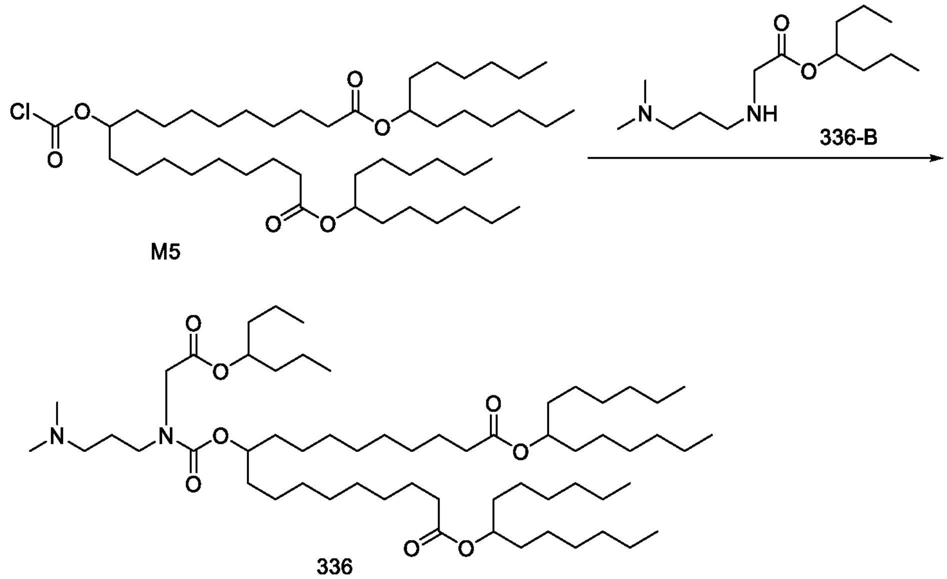


M5

336

According to the general synthetic process, 375 mg amino lipid compound 336, as a pale yellow oil with a purity of 95.91% and a yield of 33%, was prepared from M5 (1.0 g, 1.29 mmol) and 336-B (0.50 g, 1.94 mmol).

$^1H$ NMR (600 MHz, $CDCl_3$) δ4.96-4.91 (m, 1H), 4.88-4.84 (m, 2H), 4.78-4.71 (m, 1H), 3.99 (s, 1H), 3.92 (s, 1H), 3.35-3.30 (m, 2H), 2.28-2.26 (m, 6H), 2.21-2.20 (m, 6H), 1.73-1.69 (m, 2H), 1.63-1.58 (m, 4H), 1.54-1.46 (m, 16H), 1.28-1.26 (m, 56H), 0.92-0.86 (m, 18H).

LC-MS (ESI): (M + H) calculated 993.88, found 994.3.

**Example 88: Synthesis of Amino Lipid Compound 338**

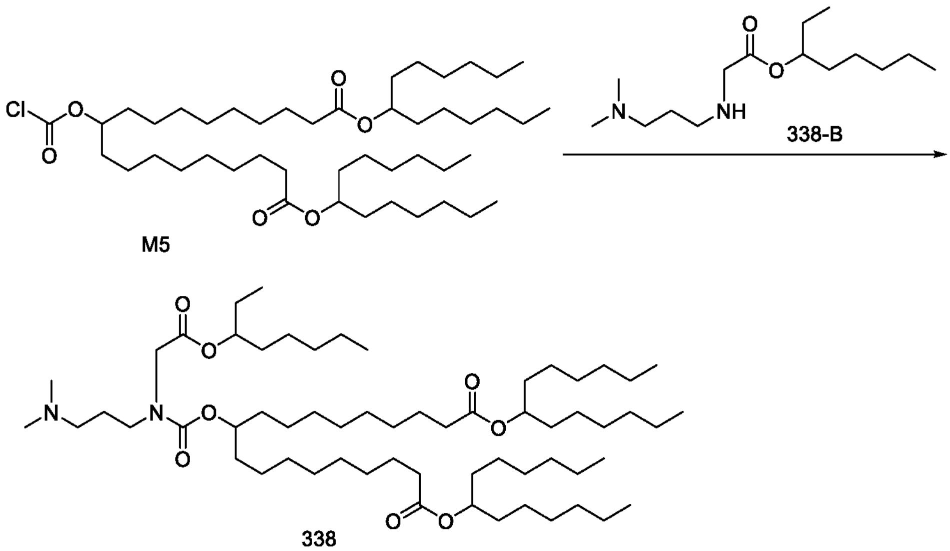


M5

338

According to the general synthetic process, 460 mg amino lipid compound 338, as a pale yellow oil with a purity of 96.25% and a yield of 40%, was prepared from M5 (1.0 g, 1.29 mmol) and 338-B (0.53 g, 1.94 mmol).

$^1H$ NMR (600 MHz, $CDCl_3$) δ4.88-4.83 (m, 3H), 4.77-4.72 (m, 1H), 4.00 (s, 1H), 3.92 (s, 1H), 3.36-3.31 (m, 2H), 2.28-2.25 (m, 6H), 2.21-2.20 (m, 6H), 1.82-1.46 (m, 22H), 1.28-1.26 (m, 58H), 0.87 (t, J=6.7Hz, 18H).

LC-MS (ESI): (M + H) calculated 1007.90, found 1008.4.

**Example 89: Synthesis of Amino Lipid Compound 339**

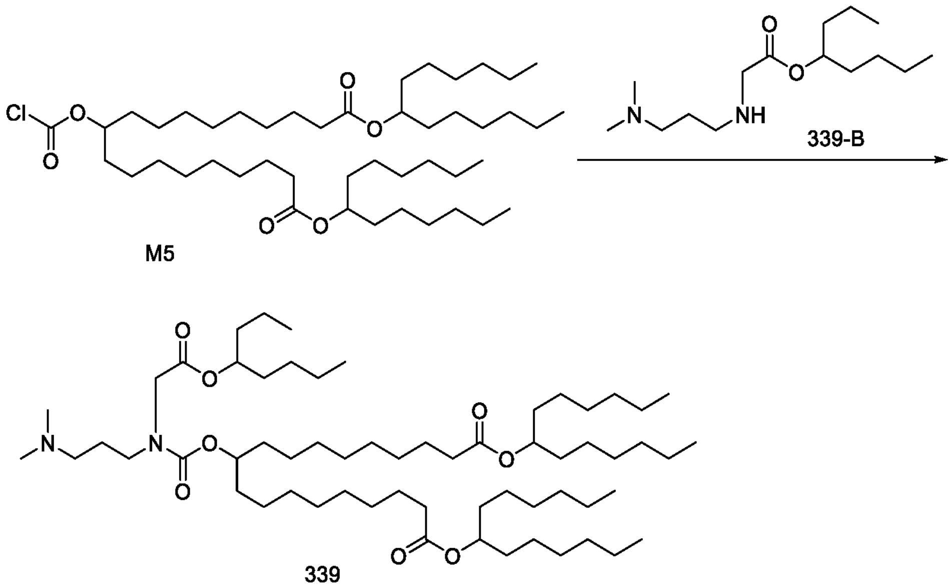


M5

339

According to the general synthetic process, 207 mg amino lipid compound 339, as a pale yellow oil with a purity of 92.98% and a yield of 18%, was prepared from M5 (1.0 g, 1.29 mmol) and 339-B (0.53 g, 1.94 mmol).

$^{1}H$ NMR (600 MHz, $CDCl_3$) δ4.93-4.90 (m, 1H), 4.88-4.84 (m, 2H), 4.78-4.71 (m, 1H), 3.99 (s, 1H), 3.91 (s, 1H), 3.37-3.29 (m, 2H), 2.28-2.21 (m, 12H), 1.78-1.50 (m, 22H), 1.28-1.26 (m, 58H), 0.90-0.86 (m, 18H) .

LC-MS (ESI): (M + H) calculated 1007.89, found 1008.3.

**Example 90: Synthesis of Amino Lipid Compound 365**

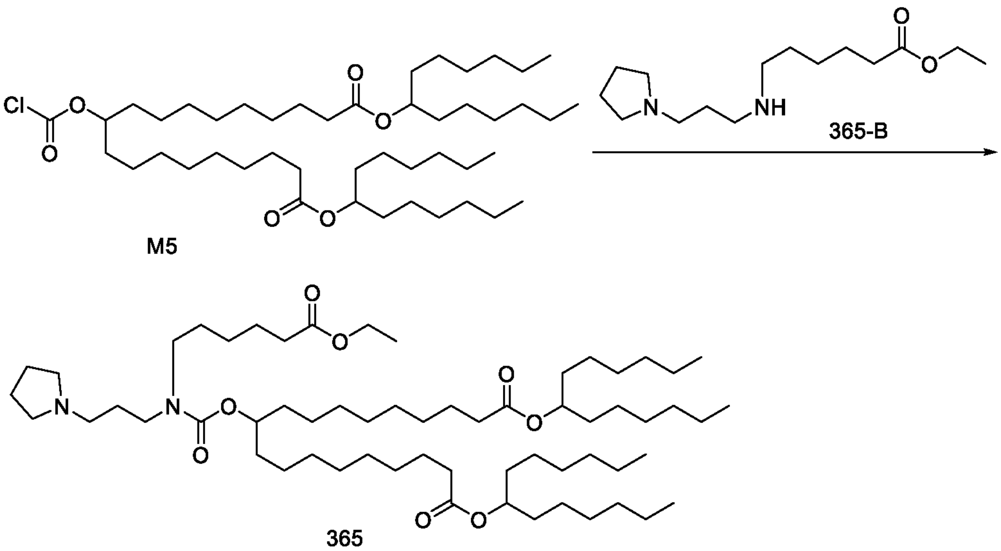


M5

365

According to the general synthetic process, 750 mg amino lipid compound 365, as a pale yellow oil with a purity of 96.10% and a yield of 65%, was prepared from M5 (1.0 g, 1.29 mmol) and 365-B (0.52 g, 1.94 mmol).

$^{1}H$ NMR (600 MHz, $CDCl_3$) δ4.88-4.84 (m, 2H), 4.75-4.71 (m, 1H), 4.14-4.10 (m, 2H), 3.28-3.18 (m, 4H), 2.49-2.44 (m, 6H), 2.30-2.26 (m, 6H), 1.79-1.50 (m, 26H), 1.30-1.24 (m, 57H), 0.87 (t, J=6.8Hz, 12H).

LC-MS (ESI): (M + H) calculated 1005.88, found 1006.4.

**Example 91: Synthesis of Amino Lipid Compound 366**

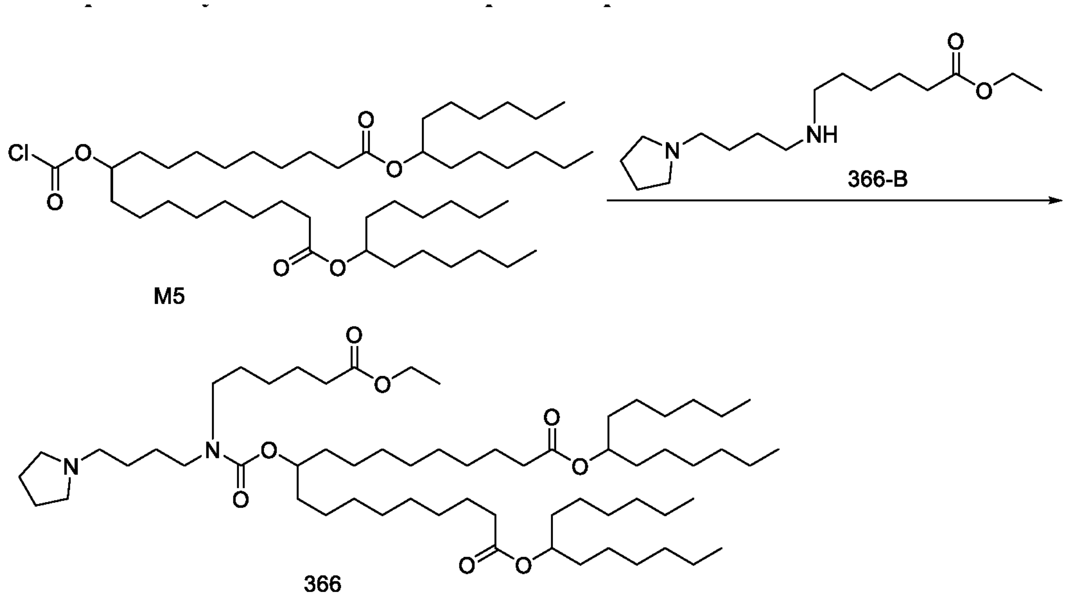


M5

366

According to the general synthetic process, 750 mg amino lipid compound 366, as a pale yellow oil with a purity of 93.80% and a yield of 64%, was prepared from M5 (1.0 g, 1.29 mmol) and 366-B (0.55 g, 1.94 mmol).

$^{1}$H NMR (600 MHz, $CDCl_3$) δ4.88-4.84 (m, 2H), 4.73-4.69 (m, 1H), 4.13-4.10 (m, 2H), 3.21-3.17 (m, 4H), 2.48 (m, 6H), 2.29-2.25 (m, 6H), 1.77 (m, 4H), 1.62-1.59 (m, 6H), 1.50-1.49 (m, 18H), 1.27-1.22 (m, 57H), 0.87 (t, J=6.8Hz, 12H).

LC-MS (ESI): (M + H) calculated 1019.89, found 1020.4.

**Example 92: Synthesis of Amino Lipid Compound 367**

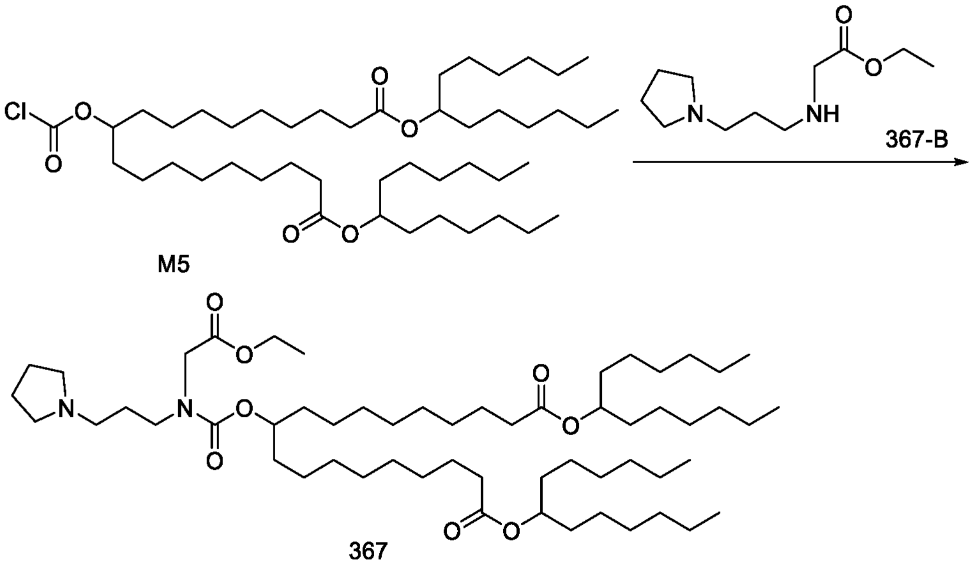


M5

367

According to the general synthetic process, 650 mg amino lipid compound 367, as a pale yellow oil with a purity of 96.49% and a yield of 60%, was prepared from M5 (1.0 g, 1.29 mmol) and 367-B (0.42 g, 1.94 mmol).

$^{1}$H NMR (600 MHz, $CDCl_3$) δ4.88-4.84 (m, 2H), 4.78-4.70 (m, 1H), 4.19-4.14 (m, 2H), 3.99 (s, 1H), 3.92 (s, 1H), 3.40-3.32 (m, 2H), 2.51-2.44 (m, 6H), 2.26 (t, J=7.5Hz, 4H), 1.77 (m, 6H), 1.61-1.59 (m, 4H), 1.50-1.48 (m, 12H), 1.30-1.22 (m, 55H), 0.87 (t, J=7.0Hz, 12H).

LC-MS (ESI): (M + H) calculated 949.81, found 950.2.

**Example 93: Synthesis of Amino Lipid Compound 368**

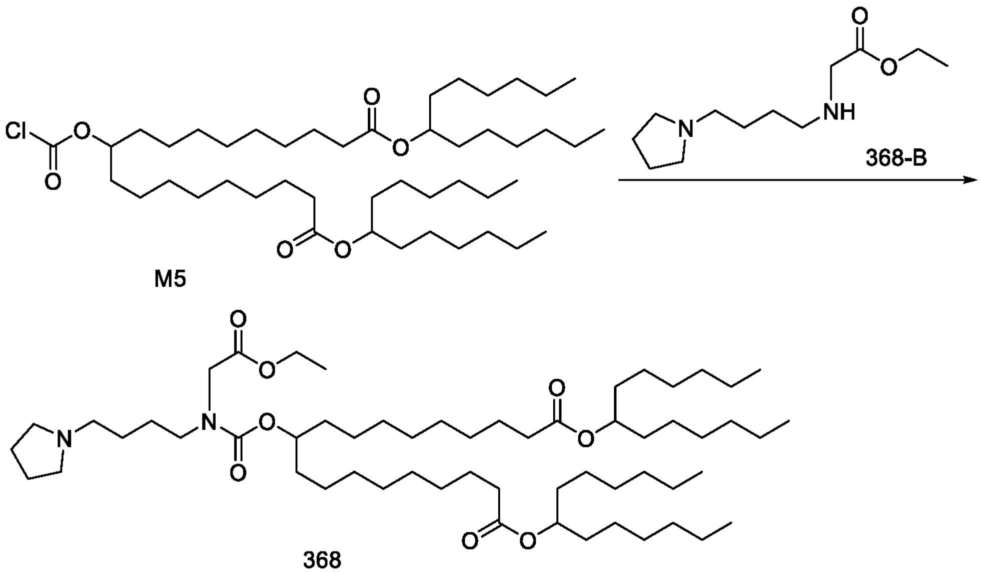


According to the general synthetic process, 550 mg amino lipid compound 368, as a pale yellow oil with a purity of 93.24% and a yield of 50%, was prepared from M5 (1.0 g, 1.29 mmol) and 368-B (0.44 g, 1.94 mmol).

$^1$H NMR (600 MHz, $CDCl_3$) δ4.88-4.84 (m, 2H), 4.77-4.70 (m, 1H), 4.19-4.14 (m, 2H), 3.97 (s, 1H), 3.89 (s, 1H), 3.35-3.27 (m, 2H), 2.49-2.44 (m, 6H), 2.27 (t, J=7.5Hz, 4H), 1.78 (m, 4H), 1.61-1.47 (m, 20H), 1.30-1.22 (m, 55H), 0.87 (t, J=7.0Hz, 12H).

LC-MS (ESI): (M + H) calculated 963.83, found 964.3.

**Example 94: Synthesis of Amino Lipid Compound 369**

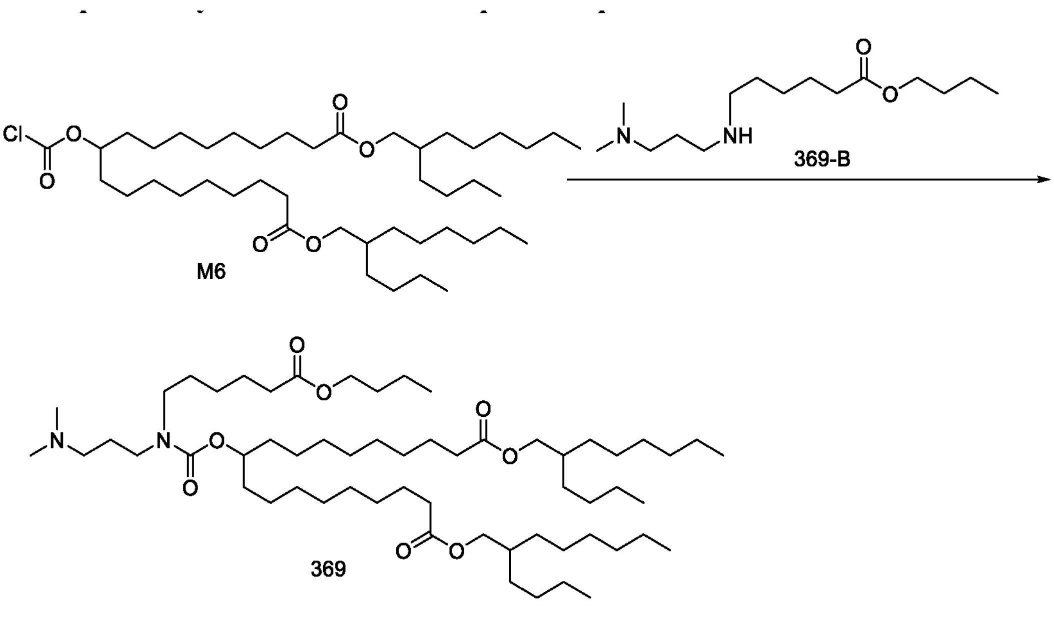


According to the general synthetic process, 206 mg amino lipid compound 369, as a pale yellow oil with a purity of 92.16% and a yield of 15%, was prepared from M6 (1.0 g, 1.34 mmol) and 369-B (0.53 g, 1.94 mmol).

$^1H$ NMR (600 MHz, $CDCl_3$) δ4.75-4.71 (m, 1H), 4.06 (t, J= 6.3 Hz, 2H), 3.96 (d, J= 5.9Hz, 4H), 3.20 (m, 4H), 2.29-2.27 (m, 8H), 2.23-2.22 (m, 6H), 1.69-1.27 (m, 74H), 0.93 (t, J= 7.5Hz, 3H) 0.89-0.86 (m, 12H).

LC-MS (ESI): (M + H) calculated 979.86, found 980.3.

**Example 95: Synthesis of Amino Lipid Compound 370**

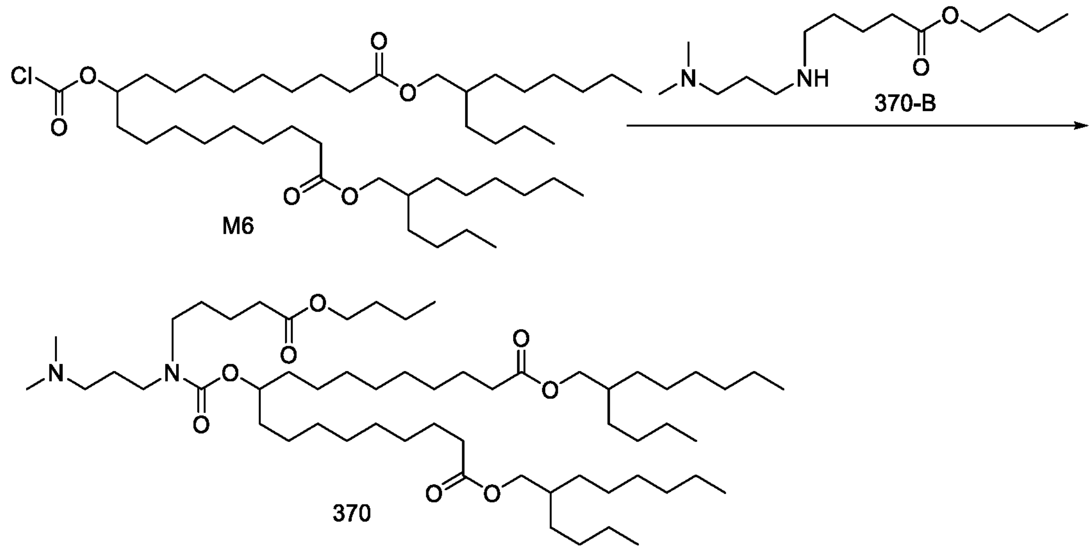
M6
370-B
370

According to the general synthetic process, 155 mg amino lipid compound 370, as a pale yellow oil with a purity of 92.69% and a yield of 11%, was prepared from M6 (1.0 g, 1.34 mmol) and 370-B (0.50 g, 1.94 mmol).

$^1H$ NMR (600 MHz, $CDCl_3$) δ4.75-4.71 (m, 1H), 4.06 (t, J= 6.7 Hz, 2H), 3.97 (d, J= 5.6Hz, 4H), 3.25-3.21 (m, 4H), 2.32-2.26 (m, 8H), 2.21 (s, 6H), 1.88 (bs, 2H), 1.71-1.69 (m, 2H), 1.61-1.60 (m, 10H), 1.51-1.50 (m, 4H), 1.39-1.36 (m, 2H), 1.28-1.27 (m, 52H), 0.93 (t, J= 7.5Hz, 3H) 0.90-0.87 (m, 12H).

LC-MS (ESI): (M + H) calculated 965.85, found 966.3.

**Example 96: Synthesis of Amino Lipid Compound 371**

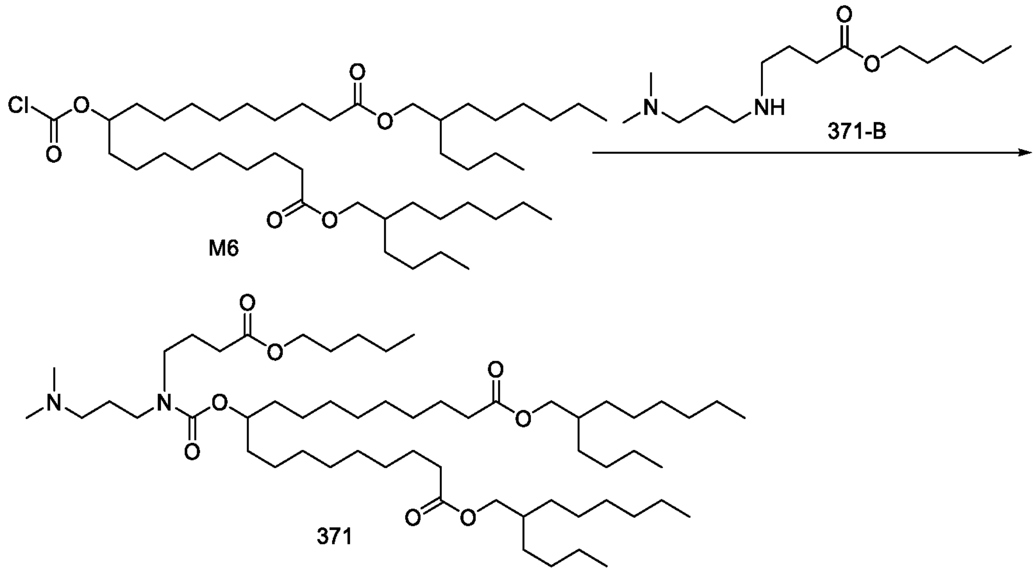
M6
371-B
371

According to the general synthetic process, 300 mg amino lipid compound 371, as a pale yellow oil with a purity of

93.23% and a yield of 22%, was prepared from M6 (1.0 g, 1.34 mmol) and 371-B (0.50 g, 1.94 mmol).

$^1H$ NMR (600 MHz, $CDCl_3$) δ4.75-4.71 (m, 1H), 4.05 (t, J=6.8Hz, 2H), 3.95 (d, J= 5.9Hz, 4H), 3.26-3.23 (m, 4H), 2.28 (t, J=7.5Hz, 8H), 2.21 (s, 6H), 1.84 (m, 2H), 1.70 (m, 2H), 1.62-1.58 (m, 8H), 1.50 (m, 4H), 1.32-1.26 (m, 56H), 0.91-0.86 (m, 15H).

LC-MS (ESI): (M + H) calculated 965.85, found 966.2.

**Example 97: Synthesis of Amino Lipid Compound 372**

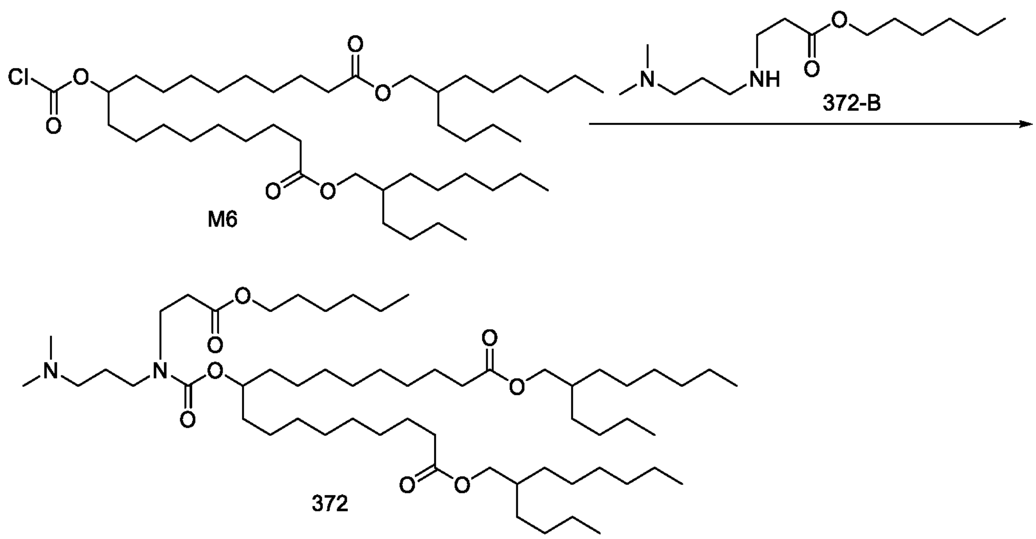
M6
372-B
372

According to the general synthetic process, 150 mg amino lipid compound 372, as a pale yellow oil with a purity of 94.31% and a yield of 10%, was prepared from M6 (1.0 g, 1.34 mmol) and 372-B (0.50 g, 1.94 mmol).

$^1H$ NMR (600 MHz, $CDCl_3$) δ4.74 (m, 1H), 4.06 (m, 2H), 3.97 (d, J=5.6Hz, 4H), 3.52-3.49 (m, 2H), 3.29-3.25 (m, 2H), 2.60-2.55 (m, 2H), 2.30-2.26 (m, 6H), 2.21 (m, 6H), 1.73-1.67 (m, 2H), 1.62-1.58 (m, 8H), 1.51 (m, 4H), 1.28-1.23 (m, 58H), 0.90-0.87 (m, 15H).

LC-MS (ESI): (M + H) calculated 965.85, found 966.3.

**Example 98: Synthesis of Amino Lipid Compound 373**

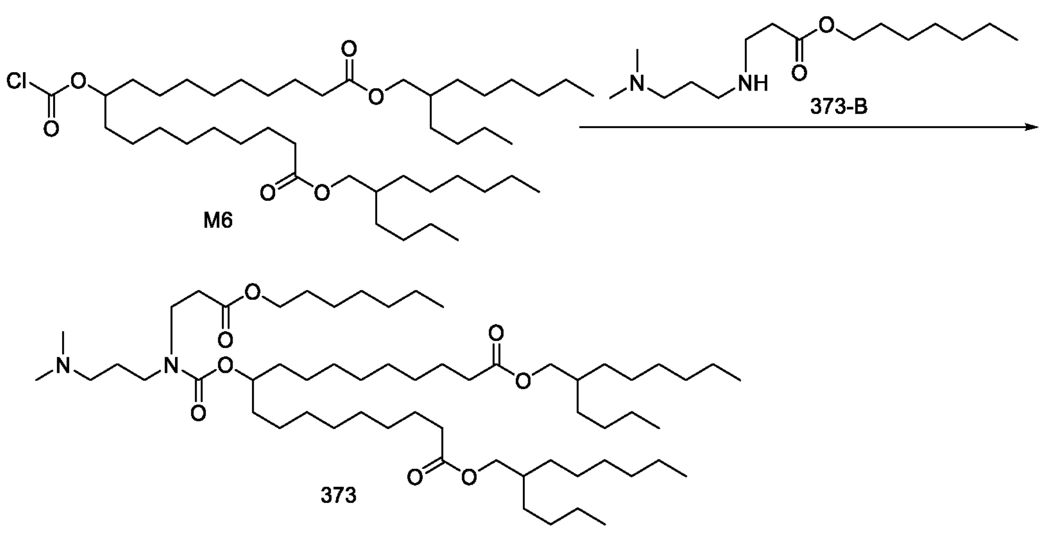
M6
373-B
373

According to the general synthetic process, 296 mg amino lipid compound 373, as a pale yellow oil with a purity of

91.57% and a yield of 22%, was prepared from M6 (1.0 g, 1.34 mmol) and 373-B (0.53 g, 1.94 mmol).

$^1H$ NMR (600 MHz, $CDCl_3$) δ4.74 (m, 1H), 4.06 (m, 2H), 3.96 (d, J=5.6Hz, 4H), 3.52-3.49 (m, 2H), 3.28-3.24 (m, 2H), 2.59-2.54 (m, 2H), 2.30-2.25 (m, 6H), 2.21 (s, 6H), 1.69 (m, 2H), 1.61-1.59 (m, 8H), 1.51 (m, 4H), 1.28 (m, 58H), 0.89-0.86 (m, 15H).

LC-MS (ESI): (M + H) calculated 979.86, found 980.3.

**Example 99: Synthesis of Amino Lipid Compound 374**

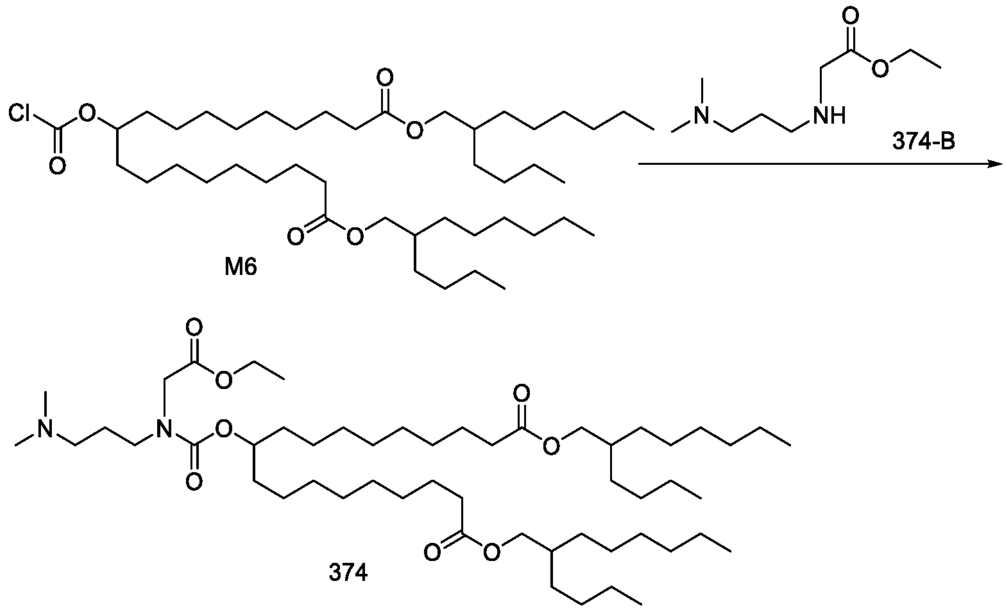
M6
374-B
374

According to the general synthetic process, 405 mg amino lipid compound 374, as a pale yellow oil with a purity of 93.54% and a yield of 31%, was prepared from M6 (1.0 g, 1.34 mmol) and 374-B (0.37 g, 1.94 mmol).

$^1H$ NMR (600 MHz, $CDCl_3$) δ 4.78-4.70 (m, 1H), 4.19-4.14 (m, 2H), 4.00-3.92 (m, 6H), 3.38-3.25 (m, 2H), 2.29 (t, J-7.5 Hz, 6H), 2.22 (d, J = 16.7 Hz, 6H), 1.75-1.70 (m, 2H), 1.61-1.26 (m, 65H), 0.90-0.87 (m, 12H).

LC-MS (ESI): (M + H) calculated 895.77, found 896.2.

**Example 100: Synthesis of Amino Lipid Compound 375**

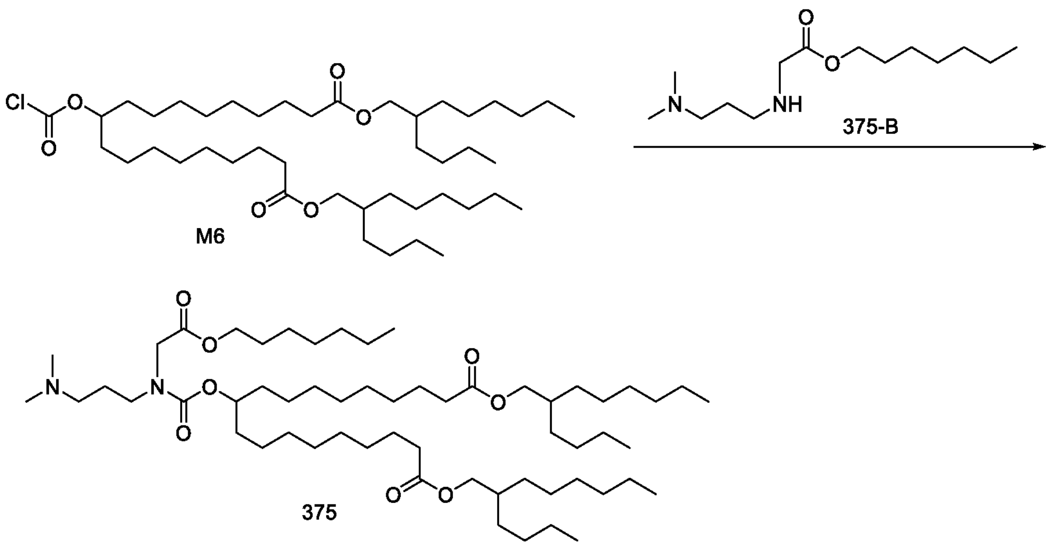
M6
375-B
375

According to the general synthetic process, 977 mg amino lipid compound 375, as a pale yellow oil with a purity of 92.77% and a yield of 70%, was prepared from M6 (1.0 g, 1.34 mmol) and 375-B (0.50 g, 1.94 mmol).

$^{1}$H NMR (600 MHz, $CDCl_3$) δ 4.79-4.70 (m, 1H), 4.12-4.08 (m, 2H), 4.00-3.92 (m, 6H), 3.37-3.25 (m, 2H), 2.29 (t, J-7.5 Hz, 6H), 2.22 (d, J = 10.2 Hz, 6H), 1.74-1.27 (m, 74H), 0.90-0.87 (m, 15H).

LC-MS (ESI): (M + H) calculated 965.85, found 966.6.

**Example 101: Synthesis of Amino Lipid Compound 376**

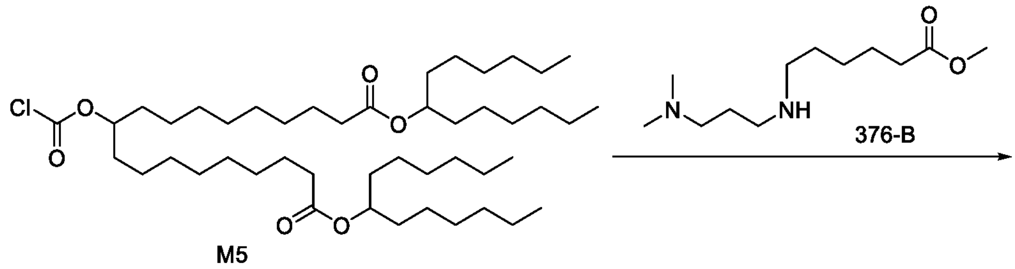

M5

376-B

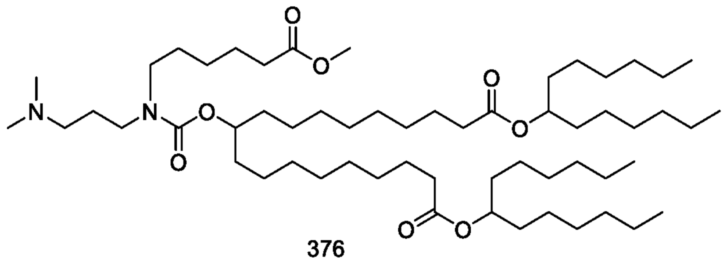

376

According to the general synthetic process, 380 mg amino lipid compound 376, as a pale yellow oil with a purity of 93.62% and a yield of 27%, was prepared from M5 (1.0 g, 1.29 mmol) and 376-B (0.45 g, 1.94 mmol).

$^{1}$H NMR (600 MHz, $CDCl_3$) δ4.85-4.82 (m, 2H), 4.72-4.71 (m, 1H), 3.65 (s, 3H), 3.20(m, 4H), 2.30-2.24 (m, 8H), 2.21 (bs, 6H), 1.69 (m, 2H), 1.62-1.59 (m, 6H), 1.49 (m, 14H), 1.26-1.25 (m, 54H), 0.87-0.84 (m, 12H).

LC-MS (ESI): (M + H) calculated 965.85, found 966.3.

**Example 102: Synthesis of Amino Lipid Compound 377**

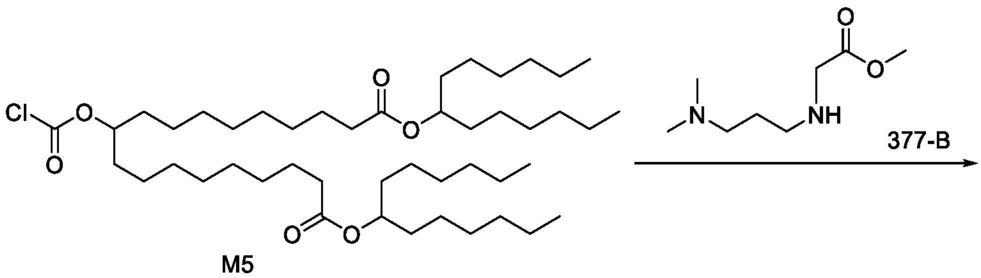

M5

377-B

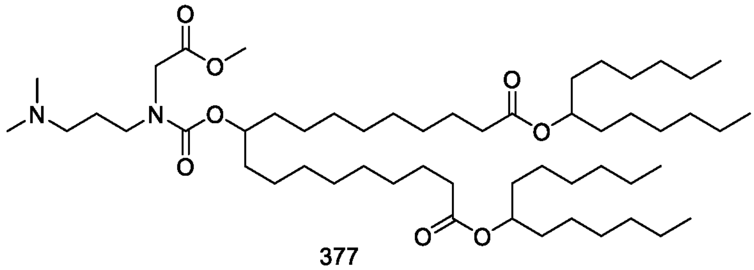

377

According to the general synthetic process, 468 mg amino lipid compound 377, as a pale yellow oil with a purity of 91.13% and a yield of 41%, was prepared from M5 (1.0 g, 1.29 mmol) and 377-B (0.34 g, 1.94 mmol).

$^1H$ NMR (600 MHz, $CDCl_3$) δ 4.88-4.84 (m, 2H), 4.78-4.70 (m, 1H), 4.02, (s, 1H), 3.94 (s, 1H), 3.71 (d, J= 7.6 Hz, 3H), 3.36 (t, J= 7.1 Hz, 1H), 3.32 (t, J= 7.1 Hz, 1H), 2.33-2.25 (m, 6H), 2.22 (d, J= 14.2 Hz, 6H), 1.75-1.67 (m, 2H), 1.62-1.58 (m, 4H), 1.53-1.46 (m, 12H), 1.33-1.25 (m, 52H), 0.87 (t, J= 7.0 Hz, 12H).

LC-MS (ESI): (M + H) calculated 909.78, found 910.2.

**Example 103: Synthesis of Amino Lipid Compound 181**

(1) Synthesis of Compound 181-B

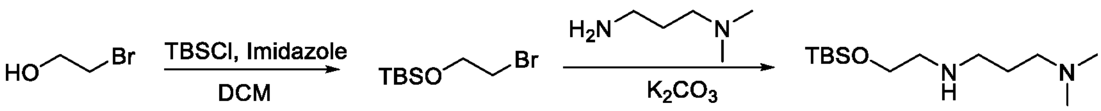


181-A 181-B

1) Synthesis of (2-bromoethoxy)-tert-butyldimethylsilane (181-A)

2-bromoethanol (5 g, 40 mmol), DCM (100 ml), and imidazole (4.08 g, 60 mmol) were added to a 250 mL round-bottom flask, cooled to 0°C while stirring, added with TBSCl (7.23 g, 48 mmol), and heated to room temperature, and reacted at room temperature for 14 h. The reaction mixture was quenched by addition of saturated aqueous sodium bicarbonate, extracted twice with DCM (200 ml). The organic phases were combined, washed with 50 ml saturated aqueous sodium chloride, dried over anhydrous sodium sulfate, and filtered. The filtrate was concentrated to obtain 10.78 g crude product, which was used directly in the next reaction.

2) Synthesis of N1-(2-(tert-butyldimethylsilyloxy)ethyl)-N3,N3-dimethylpropane-1,3-diamine (181-B)

Potassium carbonate (3.87 g, 28 mmol), acetonitrile (80 ml), and 3-dimethylaminopropylamine (7.55 ml, 60 mmol) were added to a 250 mL round-bottom flask, stirred at room temperature for 1 h, and then cooled to -10°C. 181-A (9.57 g, 40 mmol, dissolved in 20 ml acetonitrile) was slowly added through a dropping funnel. The mixture was reacted overnight at -10°C, and filtered. The filtrate was concentrated, and extracted with water (60 ml) and ethyl acetate (60 ml). The organic phase was separated, washed with saturated aqueous sodium chloride solution, dried over anhydrous sodium sulfate, and filtered. The filtrate was concentrated to obtain 7.8 g crude 181-B, which was purified by silica gel column chromatography, eluted with EA: MeOH = 5:1 to obtain 3.98 g 181-B, as a pale yellow oil.

(2) Synthesis of Amino Lipid Compound 181

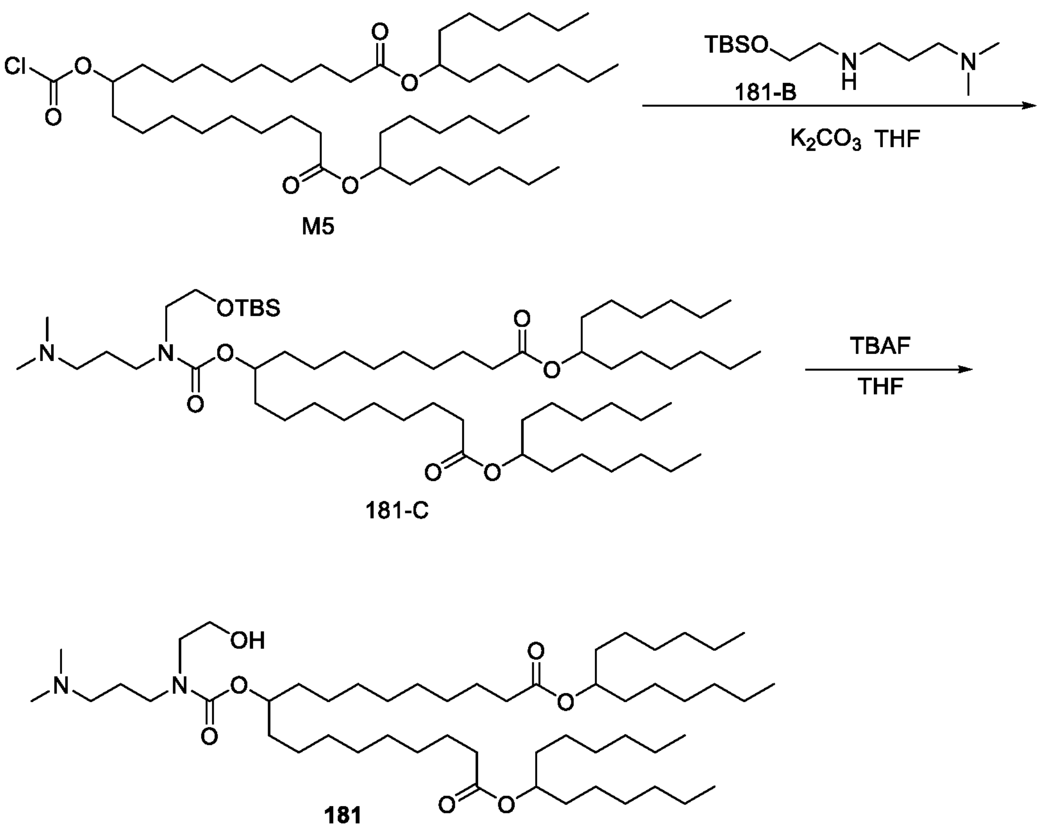


M5

181-C

181

1) Synthesis of Compound 181-C

181-B (0.50 g, 1.92 mmol) and THF (20 ml) were added to a 25ml single-necked flask, stirred at 0°C for 10min, and potassium carbonate (0.19 g, 1.41 mmol) was added, and M5 (0.99 g, 1.28 mmol, dissolved in 5 ml THF) was added dropwise. After the completion of addition dropwise, the mixture was reacted for 30 min. Water (50 ml) and DCM (50 ml) were added to the reaction solution for extraction. The organic phase was separated, and the aqueous phase was extracted twice with DCM (100 ml). The organic phases were combined, washed with saturated sodium chloride aqueous solution (50 ml), dried over anhydrous sodium sulfate, and filtered. The filtrate was concentrated, and the concentrate was purified by silica gel column chromatography, eluted with EA to obtain 597 mg 181-C, as a pale yellow oil with a yield of 47%.

2) Synthesis of Compound 181

181-C (0.60 g, 0.6 mmol) and THF (5 ml) were added to a 50 ml single-necked flask, TEA·3HF (863 mg, 5.36 mmol) was added dropwise while stirring at room temperature, and the mixture was reacted with stirring for 1 h. The reaction solution was quenched by addition of 10 ml saturated aqueous ammonium chloride solution, and extracted with ethyl acetate (50 ml) and water (50 ml). The organic phase was separated, and the aqueous phase was extracted twice with ethyl acetate (100 ml). The organic phases were combined, washed with saturated aqueous sodium chloride solution, dried over anhydrous sodium sulfate, and filtered. The filtrate was concentrated, and the concentrate was purified by silica gel column chromatography, eluted with DCM: MeOH = 20:1 to obtain 236 mg amino lipid compound 181, as a light yellow oil with a purity of 93.86% and a yield of 44.67%.

$^1$H NMR (600 MHz, $CDCl_3$) δ 5.34 (s, 1H), 4.94-4.85 (m, 2H), 4.78 (m, 1H), 3.75 (t, J = 4.8 Hz, 2H), 3.56-3.33 (m, 4H), 2.78 (s, 1H), 2.55 (s, 4H), 2.42 (s, 3H), 2.31 (t, J = 7.5 Hz, 4H), 2.08 (m, 1H), 1.91 (m, 1H), 1.68-1.59 (m, 4H), 1.52 (m, 12H), 1.38-1.20 (m, 52H), 0.91 (t, J = 6.5 Hz, 12H).

LC-MS (ESI): (M + H) calculated 881.42, found 882.2.

**Example 104: Synthesis of Amino Lipid Compound 187**

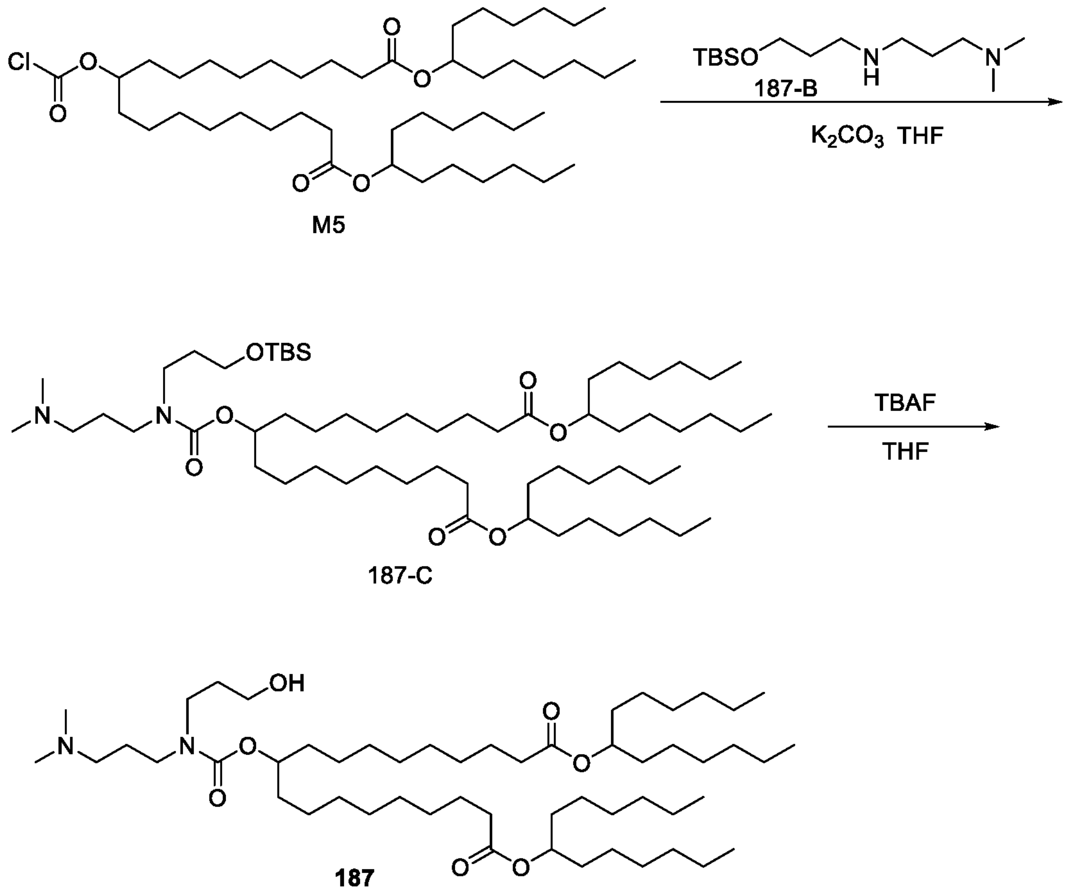


M5

187-C

187

According to the general synthetic process, amino lipid compound 187-C was prepared from compound M5 (1.0 g, 1.29 mmol) and 187-B (0.53 g, 1.9 mmol), and then 382 mg compound 187, as a colorless oil with a yield of 33.1% and a purity of 93.90%, was prepared from 187-C.

$^1H$ NMR (600 MHz, $CDCl_3$) δ 5.34 (s, 1H), 4.95-4.86 (m, 2H), 4.81 (m, 1H), 3.62-3.55 (t, J = 4.8 Hz, 2H), 3.53-3.41 (t, J = 6.6 Hz, 2H), 3.31-3.19 (t, J = 6.9 Hz, 2H), 2.42-2.25 (m, 12H), 1.76 (m, 4H), 1.69-1.61 (m, 4H), 1.53 (m, 12H), 1.38-1.21 (m, 52H), 0.96-0.85 (m, 12H).

LC-MS (ESI): (M + H) calculated 895.45, found 896.2.

**Biological tests**

The amino lipid compounds 118, SM102, and ALC-0315 used in the biological tests of the present disclosure have the following structures:

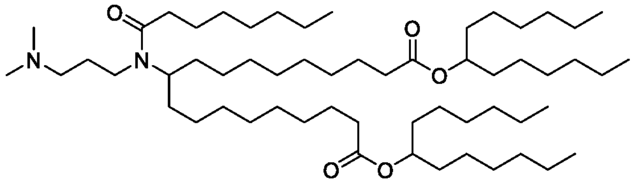

118,

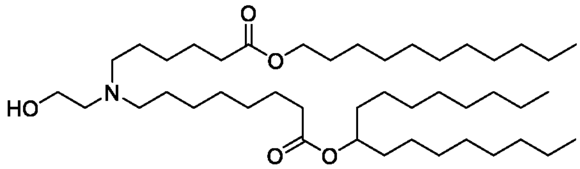

SM102,

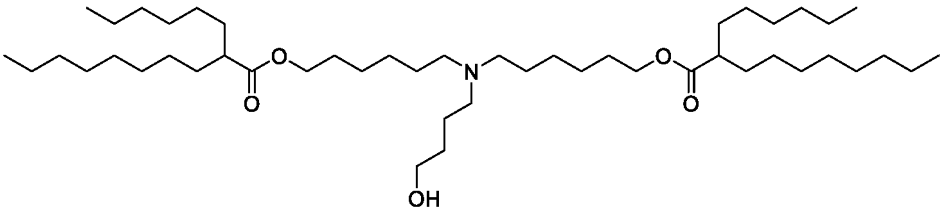

ALC-0315.

Amino lipid compound 118 can be prepared according to the synthesis method of compound 10 (Example 9) described in Chinese patent application CN107922364A; SM102 and ALC-0315 are commercially available or can be prepared according to the well-known techniques in the art.

**Experimental Example 1: Preparation of Lipid Nanoparticle Encapsulating Luciferase mRNA (Flue mRNA)**

(1) Formulating:

A specified amount of Flue mRNA stock solution, 0.2 M sodium acetate buffer, and DEPC water were added to a container, and mixed well to obtain a water phase;

The amino lipid compound of the present disclosure, a helper lipid, a structural lipid, and a PEG-lipid were separately dissolved in absolute ethanol to prepare respective solutions at concentrations of 20 mg/mL, 10 mg/mL, 20 mg/mL, and 25 mg/mL, respectively. The above four solutions were pipetted at a molar ratio of Lipid: DSPC: CHO-HP: M-DMG-2000 of 48:10:40.5:1.5, and mixed well to prepare an alcohol phase.

(2) Encapsulation: The water phase and the alcohol phase were injected into a microfluidic chip at a flow rate of water phase: alcohol phase = 12 mL/min: 4 mL/min by using a microfluidic preparation instrument (MPE-L2), and encapsulation was carried out at a flow rate of water phase: alcohol phase = 9 mL/min: 3 mL/min to obtain mRNA-encapsulating lipid nanoparticle (mRNA-LNP).

(3) Dialysis: The product of step (2) was loaded in a dialysis bag and placed in a Tris Buffer-8% sucrose solution for replacement to remove ingredients such as residual ethanol, unassembled lipids. Dialysis was conducted for 2 h with magnetic stirring at room temperature and under protection from light (dialysate was replaced every 1 hour).

(4) The product of step (3) was sterilized by passing through a 0.22 μm microporous membrane, and then packaged.

Lipid nanoparticle formulations encapsulating Flue mRNA were prepared, with a concentration of Flue mRNA of 0.2 μg/μL, a mass ratio of Flue mRNA to Lipid of 1:10, a particle size of 80-130 nm, and a encapsulation efficiency of 85% or higher.

**Experimental Example 2: Performance Evaluation of *in vivo* Delivery of Lipid Nanoparticle**

Animal preparation: Female BALB/c mice of 6-8 weeks old were selected and raised in an SPF grade breeding room. Animal testing was conducted in strict accordance with the guidelines of national health institutions and animal ethics requirements.

*In vivo* Delivery: prior to injection of the test LNP formulations, the LNP formulations were gently and repeatedly inverted to thoroughly mix the formulation samples. A corresponding amount of the formulation samples were aspirated with a 1 ml insulin syringe, and the LNP formulations were injected by tail vein injection (IV), with 3 mice in duplicate per formulation. Each mouse was injected with 75 μL of the luciferase mRNA (Flue mRNA)-encapsulating lipid nanoparticle formulation prepared in Experimental Example 1.

6 hours after injection of LNP formulations, mice were injected with 200 μL D-Luciferin luciferase developing substrate (Catalog No. 122799; Manufacturer: Perkin Elmer). After the substrate was injected, the mice were anesthetized with isoflurane inhalation, and the injection time of luciferase developing substrate was recorded. 10 minutes after the substrate injection, the animals were placed in supine position, and the signal distribution and expression intensity of luciferase in the body and various organs of the animals were observed with In Vivo Imaging System (IVIS).

The encapsulation efficiency of the lipid nanoparticle encapsulating luciferase mRNA (Flue mRNA) with a representative amino lipid compound and the fluorescence expression intensity induced by the same are shown in Table 4, with the amino lipid compound 118 as a control.

Table 4

| Amino lipid compound | Encapsulation efficiency | Total flux | Liver | Spleen | (Spleen/Total Flux) * 100% |
|---|---|---|---|---|---|
| 108 | 91.78% | 3.40E+08 | 6.84E+07 | 1.02E+06 | 0.30 |
| 109 | 92.87% | 2.80E+08 | 5.22E+07 | 8.53E+05 | 0.30 |
| 110 | 90.82% | 2.44E+08 | 5.83E+07 | 8.71E+05 | 0.36 |
| 259 | 94.08% | 6.40E+08 | 1.37E+08 | 1.29E+07 | 2.02 |
| 260 | 95.36% | 4.11E+08 | 9.60E+07 | 3.27E+06 | 0.80 |
| 264 | 94.12% | 4.95E+08 | 1.61E+08 | 8.05E+06 | 1.63 |
| 265 | 94.59% | 3.93E+08 | 7.57E+07 | 1.91E+06 | 0.49 |
| 266 | 94.26% | 2.67E+08 | 8.09E+07 | 1.60E+06 | 0.60 |
| 267 | 93.13% | 4.01E+08 | 9.91E+07 | 1.54E+06 | 0.38 |
| 268 | 92.22% | 1.23E+08 | 2.11E+07 | 8.01E+06 | 6.51 |
| 270 | 93.46% | 4.40E+08 | 1.34E+08 | 7.36E+06 | 1.67 |
| 271 | 93.59% | 2.68E+08 | 7.57E+07 | 4.76E+06 | 1.78 |
| 272 | 93.96% | 4.04E+08 | 7.42E+07 | 3.03E+06 | 0.75 |
| 273 | 87.42% | 1.78E+08 | 4.33E+07 | 2.37E+06 | 1.33 |
| 274 | 93.06% | 3.25E+08 | 7.08E+07 | 2.60E+06 | 0.80 |
| 275 | 90.61% | 8.16E+08 | 1.23E+08 | 8.84E+06 | 1.08 |
| 276 | 88.92% | 7.70E+08 | 1.43E+08 | 4.67E+06 | 0.61 |
| 277 | 91.00% | 3.14E+08 | 7.98E+07 | 8.42E+06 | 2.68 |
| 279 | 92.43% | 6.29E+08 | 9.55E+07 | 4.57E+06 | 0.73 |
| 284 | 91.63% | 3.42E+08 | 3.29E+07 | 5.20E+06 | 1.52 |
| 298 | 92.85% | 2.61E+08 | 3.32E+07 | 2.13E+06 | 0.82 |
| 303 | 93.23% | 6.41E+08 | 8.58E+07 | 5.56E+06 | 0.87 |
| 304 | 92.03% | 5.18E+08 | 1.06E+08 | 1.48E+07 | 2.86 |
| 305 | 90.70% | 6.71E+08 | 9.19E+07 | 7.03E+06 | 1.05 |
| 306 | 91.92% | 2.45E+08 | 4.13E+07 | 5.44E+06 | 2.22 |
| 308 | 93.71% | 6.29E+08 | 9.58E+07 | 4.93E+06 | 0.78 |
| 309 | 91.62% | 3.69E+08 | 4.90E+07 | 3.44E+06 | 0.93 |
| 316 | 90.67% | 8.16E+08 | 1.26E+08 | 2.03E+07 | 2.49 |
| 320 | 92.13% | 7.04E+08 | 1.13E+08 | 4.97E+06 | 0.71 |
| 321 | 93.85% | 7.21E+08 | 1.27E+08 | 1.47E+07 | 2.04 |
| 322 | 90.32% | 7.90E+08 | 9.73E+07 | 6.56E+06 | 0.83 |
| 337 | 92.38% | 3.58E+08 | 6.49E+07 | 3.30E+06 | 0.92 |
| 338 | 91.13% | 6.32E+08 | 1.07E+08 | 3.20E+06 | 0.51 |

(continued)

| Amino lipid compound | Encapsulation efficiency | Total flux | Liver | Spleen | (Spleen/Total Flux) * 100% |
|---|---|---|---|---|---|
| 365 | 92.45% | 6.73E+08 | 9.03E+07 | 4.45E+06 | 0.66 |
| 366 | 93.63% | 5.82E+08 | 8.92E+07 | 4.11E+06 | 0.71 |
| 367 | 91.21% | 9.45E+07 | 1.06E+07 | 4.33E+05 | 0.46 |
| 368 | 87.51% | 3.97E+08 | 6.14E+07 | 1.25E+07 | 3.15 |
| 371 | 93.17% | 1.12E+09 | 2.92E+08 | 2.36E+07 | 2.11 |
| 372 | 92.17% | 5.43E+08 | 1.67E+08 | 8.59E+06 | 1.58 |
| 373 | 92.01% | 4.77E+08 | 9.15E+07 | 6.93E+06 | 1.45 |
| 375 | 94.18% | 4.06E+08 | 1.55E+08 | 7.63E+06 | 1.88 |
| 118 | 90.53% | 6.46E+08 | 1.88E+08 | 2.19E+06 | 0.34 |

The multiple of spleen delivery/total delivery of fluorescence expression intensity induced by lipid nanoparticle encapsulating luciferase mRNA (Flue mRNA) with a representative amino lipid compound to amino lipid compound 118 is shown in Table 5.

Table 5

| Amino lipid compound | Multiple |
|---|---|
| 108 | 0.88 |
| 109 | 0.90 |
| 110 | 1.05 |
| 259 | 5.93 |
| 260 | 2.34 |
| 264 | 4.78 |
| 265 | 1.43 |
| 266 | 1.76 |
| 267 | 1.13 |
| 268 | 19.15 |
| 270 | 4.92 |
| 271 | 5.22 |
| 272 | 2.21 |
| 273 | 3.92 |
| 274 | 2.35 |
| 275 | 3.19 |
| 276 | 1.78 |
| 277 | 7.89 |
| 279 | 2.14 |
| 284 | 4.47 |
| 298 | 2.40 |
| 303 | 2.55 |
| 304 | 8.40 |
| 305 | 3.08 |
| 306 | 6.53 |

(continued)

| Amino lipid compound | Multiple |
|---|---|
| 308 | 2.31 |
| 309 | 2.74 |
| 316 | 7.32 |
| 320 | 2.08 |
| 321 | 6.00 |
| 322 | 2.44 |
| 337 | 2.71 |
| 338 | 1.49 |
| 365 | 1.94 |
| 366 | 2.08 |
| 367 | 1.35 |
| 368 | 9.26 |
| 371 | 6.20 |
| 372 | 4.65 |
| 373 | 4.27 |
| 375 | 5.53 |
| 118 | 1.00 |

As can be seen from Table 5, all amino lipid compounds, except amino lipid compounds 108 and 109, showed a stronger delivery preference to spleen, as compared to amino lipid compound 118.

**Experimental Example 3: Performance Evaluation of Delivery to Lymph Nodes of Lipid Nanoparticle**

Animal preparation: Female BALB/c mice of 6-8 weeks old were selected and raised in an SPF grade breeding room. Animal testing was conducted in strict accordance with the guidelines of national health institutions and animal ethics requirements.

*In vivo* Delivery: prior to injection of the test LNP formulations, the LNP formulations were gently and repeatedly inverted to thoroughly mix the formulation samples. A corresponding amount of the formulation samples were aspirated with a 1 ml insulin syringe, and the LNP formulations were injected by tail vein injection (IV), with 3 mice per formulation. Each mouse was injected with 75 μL the luciferase mRNA (Flue mRNA)-encapsulating lipid nanoparticle formulation prepared in Experimental Example 1.

6 hours after injection of LNP formulations, mice were injected with 200 μL D-Luciferin luciferase developing substrate (Catalog No.122799; Manufacturer: Perkin Elmer). After the substrate was injected, the mice were anesthetized with isoflurane inhalation, and the injection time of luciferase developing substrate was recorded. 10 minutes after the substrate injection, the animals were placed in supine position, and the signal distribution and expression intensity of luciferase in lymph nodes were observed with In Vivo Imaging System (IVIS).

The intensity of fluorescence expression induced by the lipid nanoparticle encapsulating luciferase mRNA (Flue mRNA) with a representative amino lipid compound is shown in Table 6, with SM-102 as a control.

Table 6

| Amino lipid compound | Expression intensity |
|---|---|
| 250 | 1.77E+06 |
| 268 | 1.78E+06 |
| SM-102 | 4.94 E+05 |

As can be seen from Table 6, the amino lipid compounds 250 and 268 have a better delivery effect on lymph nodes

than SM102, showing a stronger preference to lymph nodes.

**Experimental Example 4: Evaluation *of in vivo* Safety of Lipid Nanoparticle**

A human erythropoietin mRNA (hEPO mRNA)-encapsulating lipid nanoparticle formulation was prepared according to the method as described in Experimental Example 1, with replacing the luciferase mRNA (Flue mRNA) with human erythropoietin mRNA (hEPO mRNA), with a concentration of hEPO mRNA of 0.2 μg/μL, a mass ratio of hEPO mRNA to Lipid of 1:10, a particle size of 90-130 nm, and an encapsulation efficiency of above 90% or higher.

Animal preparation: Female BALB/c mice of 6-8 weeks old were selected and raised in an SPF grade breeding room. Animal testing was conducted in strict accordance with the guidelines of national health institutions and animal ethics requirements.

*In vivo* Delivery: Prior to injection of the test LNP formulations, the LNP formulations were gently and repeatedly inverted to thoroughly mix the formulation samples. A corresponding amount of the formulation samples were aspirated with a 1 ml insulin syringe, and the LNP formulations were injected by tail vein injection (IV), with 5 mice per formulation. Each mouse was injected with 300 μL of the human erythropoietin mRNA (hEPO mRNA)-encapsulating lipid nanoparticle formulation.

Serum acquisition: Blood samples of mice were collected 12 h after injection, placed in tubes without anticoagulants, and naturally coagulated at room temperature for 30-60 min, and then centrifuged at a speed of 3500 rpm for 10 min to obtain the supernatant, which was the serum.

The detection of alanine transaminase was carried out according to instructions of the kit (Nanjing Jiancheng Bioengineering Institute, Catalog. No. C009-2-1), and a standard curve was made using the standard provided in the kit. D-PBS was used in the experiment, which was purchased from Sangon Biotech (Shanghai) Co., Ltd., Catalog No. E607009-0600.

The method of detection of enzyme activity of alanine aminotransferase (ALT) in serum of the mice is as follows:

Preparation of ALT standard curve:

(1) Enzymatic reaction: 0, 2, 4, 6, 8 and 10 μL of 2 μmol/mL sodium pyruvate standard solution were sequentially added to 5 μL of 0.1 mol/L phosphate buffer, and the volume was supplemented to 25 μL with a matrix solution, and repeatedly aspirating and spitting with a pipette for mixing well;
(2) Addition reaction: 20 μL 2,4-dinitrophenylhydrazine solution was added to all reaction wells in (1), mixed by aspirating and spitting, and then placed in an incubator at 37°C to react for 20 min;
(3) Developing: 200 μL of 0.4 mol/L NaOH solution was added to all reaction wells in (2) to stop the reaction, mixed by aspirating and spitting, and incubated at room temperature for 15 min. The OD value of each well was measured at 510 nm in a microplate reader; and
(4) Data processing of standard curve: The corresponding absolute OD value for each well was obtained by subtracting the OD value for 0 μL well from the measured OD value for each well, the corresponding ALT Karmen units being 0, 28, 57, 97, 150 and 200 U/L, respectively. The standard curve was obtain by taking the absolute OD value as the abscissa and the corresponding Karmen unit as the ordinate.

Detection of ALT enzyme activity in serum samples:

(1) Reagent preparation: an ALT matrix solution was placed in an incubator at 37°C for preheating;
(2) Enzymatic reaction: 5 μL diluted serum was aspirated and added to a 96-well plate, then 20 μL matrix solution was added to the corresponding sample well and mixed by repeatedly aspirating and spitting to avoid bubbling, and then placed in an incubator at 37°C for 30 min;
(3) Addition reaction: 20 μL 2,4-dinitrophenylhydrazine was added to all reaction wells in (2), mixed by aspirating and spitting, and then reacted in an incubator at 37°C for 20 min;
(4) Developing: 200 μL of 0.4 mol/L NaOH solution was added to all reaction wells in (3) to stop the reaction, mixed by aspirating and spitting, and then incubated at room temperature for 15 min. The OD value for each well was measured at a wavelength of 510 nm in a microplate reader; and
(5) Calculation of ALT enzyme activity in serum: The absolute OD value of the corresponding sample well was obtained by subtracting the OD value for the control well from the obtained OD value of the sample well, and was substituted into the standard curve formula to calculate the ALT enzyme activity (Karmen unit) for the corresponding

serum sample.

The *in vivo* safety evaluation results of the lipid nanoparticle encapsulating human erythropoietin (hEPO) mRNA with the representative amino lipid compounds are shown in FIG. 1, with ALC00315 and SM-102 as controls.

As can be seen from FIG. 1, as compared to the commercially available ALC0315 and SM-102, the representative amino lipid compounds exhibit comparable or lower ALT enzyme activity (Karmen unit), with 252, 255, 259, 260, 263, 264, 266, 267, 270, 272, and 273 having significantly lower ALT enzyme activity (Karmen unit) and having better safety.

**Experimental Example 5: IFN-γ Elispot Cellular Immunity Test**

An IN002.5.1 mRNA-encapsulating lipid nanoparticle formulation was prepared according to the method as described in Experimental Example 1, with replacing the luciferase mRNA (Flue mRNA) with IN002.5.1 mRNA, with a concentration of IN002.5.1 mRNA of 0.2 μg/μL, a mass ratio of IN002.5.1 mRNA to Lipid of 1:10, a particle size of 80-130 nm, and an encapsulation efficiency of 90% or higher.

The sequence of IN002.5.1 mRNA is one obtained by replacing all uracil (u) in SEQ ID NO.1 with N1-methylpseudouridine. Of note is that the t (thymine) in the RNA sequence, SEQ ID NO.1, in the Sequence Listing is actually u (uracil), according to the WIPO Standard ST.26 for nucleotide or amino acid sequence listing.

Animal preparation: Female BALB/c mice of 6-8 weeks old were selected and raised in an SPF grade breeding room. Animal testing was conducted in strict accordance with the guidelines of national health institutions and animal ethics requirements.

Immunization of mice: Prior to injection of the test LNP formulations, the LNP formulations were gently and repeatedly inverted to thoroughly mix the formulation samples. A corresponding amount of the formulation samples were aspirated with a 1 ml insulin syringe, and the LNP formulations were injected by intramuscular injection (IM) in the tails, with 8 mice per formulation. Each mouse was injected with 50 μL of the IN002.5.1 mRNA encapsulating lipid nanoparticle formulation.

Spleen acquisition: On the 7th day after immunization, 3 to 4 mice from each LNP immunization group were selected and euthanized, and their spleens were acquired in a super clean bench.

Serum collection: On the 14th day after immunization, 150 μL orbital blood was collected from 5 mice for each LNP immunization group, and the blood was placed in a tube without anticoagulant, and naturally coagulated at room temperature for 30-60 min, and then centrifuged at a speed of 3500 rpm for 10 min to obtain the supernatant, which was the serum.

Elispot test of Mouse lymphocytes:

Isolation of lymphocytes: The spleens of mice were taken out in a super clean bench. 7 mL of mouse lymphocyte separation solution was added to a 6-well cell culture plate. Mouse spleen cells were ground with a syringe piston, and the suspension of the spleen cells was filtered through a cell screen and immediately transferred to a 15 mL centrifuge tube. 1000 μL RPMI 1640 medium was slowly added with keeping the liquid interface distinct. After centrifugation at 800g with a horizontal rotor at room temperature for 30 minutes, a clear stratification can be visible. The lymphocyte layer was aspirated, then added with 10 mL RPMI 1640 medium, and inverted for washing. The cells were collected by centrifugation at 250 g for 10 min at room temperature, and the red blood cells were lysed. After completing lysis of red blood cells, the supernatant was poured, and the cells were resuspended in culture medium and counted.

Addition of stimulant and culture of lymphocyte: RPMI-1640 medium containing 10% fetal bovine serum was used to activate the pre-coated plate. After standing at room temperature for at least 30 min, the medium was removed, and a cell suspension at adjusted concentration was added (100 μL/well). The medium used to resuspend the cells was used as a background negative control. 10 μL positive stimulant working solution (PMA + Ionomycin (dissolved in DPBS) was added to a positive control well; 10 μL medium used to resuspend the cells was added to a negative control well; 10 μL/well of a peptide library diluted with RPMI 1640 was added to experimental wells. After all samples and the stimulant were added, the plate was covered, incubated in an incubator with 5% $CO_2$ at 37°C for 16-24 h.

Elispot detection after culture: The cells and the culture medium in the wells were dumped, and the wells were lysed with 200 μL/well of ice-cold deionized water at 4°C for 10 min. The liquid in the wells were shaken out, and the wells were washed five times with PBS buffer. The wells were incubated with 100 μL/well of 1 μg/ml detection antibody for 2 h at room temperature; and after washing the plate 5 times with PBS, the wells were incubated with 100 μL/well of 1000-fold diluted enzyme labeled avidin (Streptavidin-HRP) for 1 h at room temperature. After washing the plate with PBS for 5 times, the wells were incubated with TMB Substrate developing solution which had been equilibrated to room temperature, at room temperature in the dark for 15 min. The developing solution was dumped, and the front side and the back side of the plate and the base were washed for 3 to 5 times with deionize water to stop developing.

Spot counting of ELISPOT plate: The plate was placed at room temperature in a cool and dark place, and the base

was closed after the plate was naturally dried. Various parameters of spots were recorded by reading the plate in an enzyme-linked immunospot analyzer.

The results of the IFN-γ Elispot cellular immunity test with lipid nanoparticle encapsulating IN002.5.1 mRNA with representative amino lipid compounds are shown in FIG. 2, FIG. 3 and FIG. 4, with ALC0315 as a control.

The test results are shown in FIG. 2, FIG. 3 and FIG. 4. It can be seen from FIG. 2 that the amino lipid compounds 270, 271, 272, 273 and 274 show better cellular immunity effect compared to the commercially available ALC0315. It can be seen from FIG. 3 that the amino lipid compounds 263, 264, 265, 267 and 268 show better cellular immunity effect compared to the commercially available ALC0315. It can be seen from FIG. 4 that the amino lipid compounds 302 and 307 show better cellular immunity effect compared to the commercially available ALC0315.

**Experimental Example 6: Humoral Immunity Test for total binding IgG antibody**

The serum obtained in Experimental Example 5 was used to perform a humoral immunity test for the antigen-specific IgG against the expression of IN002.5.1 mRNA.

**Test reagent preparation:**

Washing solution: taking a suitable amount of a coating solution, adding Tween 20 to reach a final concentration of Tween 20 of 0.05%, and mixing thoroughly for later use.

Blocking solution: accurately weighing BSA, adding it into the washing solution to 3% w/v and then mixing thoroughly for later use (prepare and use immediately).

Sample diluent: accurately weighing BSA, adding it to the washing solution to 1% w/v, and mixing thoroughly for later use (prepare and use immediately).

**Operation steps:**

Coating: diluting the coat protein with SARS-COV-2 antigen protein (Aero, # SPN-C52He) in PBS buffer to a concentration required for the test, and then mixing thoroughly for later use; 100 μL/well, sealing with a sealing film, and then placing at 2 to 8°C overnight (16 to 20 h) or incubating at 37°C for 2 h.

Plate washing: after incubation, washing by machine three times with the washing solution at 300 μl/well, and patting for drying on clean paper.

Blocking: adding the blocking solution to the ELISA plate at 250 μl/well, sealing the plate with a sealing film, and incubating at 37°C for 40-60 min.

Plate washing: after blocking, washing the plate with machine 3 times with the washing solution at 300μl/well, and patting for drying on clean paper.

Sample preparation: taking the serum separated in advance, mixing through vortex for IgG titer detection (if stored in a refrigerator at -80°C, dissolving it at 4°C in advance).

Serum sample dilution: taking the separated serum, determining the first dilution multiple (generally 300-3000 times) according to different immunization time, and taking this dilution multiple as the first dilution multiple for gradient dilution with 8 gradients in total; adding the diluted serum to the ELISA plate at 100 μl/well, and incubating at 37°C. (Incubation time depends on different test requirement.)

Plate washing: after the incubation, washing the plate by machine three times with the washing solution at 300 μl/well, and patting for drying on clean paper.

Enzyme-labeled secondary antibody: diluting the enzyme-labeled secondary antibody with the sample diluent at a certain dilution multiple, 100 μl/well, and incubating at 37°C. (Incubation time depends on different test requirement.)

Plate washing: after the incubation, washing the plate by machine three times with the washing solution at 300 μl/well, and patting for drying on clean paper

Developing: equilibrating TMB single-component development solution to room temperature in advance, adding it to the plate at 100 μl/well, and incubating at room temperature in the dark.

Stopping: after developing, adding a stop solution at 50 μl/well.

Reading: selecting a detection wavelength of 450 nm and a reference wavelength of 630 nm, and reading and analyzing in a microplate reader.

**Result calculation**

Data analysis was performed based on the $OD_{450}$ values obtained in the microplate reader SoftMax Pro. The formula for calculating the cut-off value was as following:

$$\text{Cut-off value} = \text{mean } OD_{450} \text{ of negative serum solution} \times 2.1$$

Note: when the mean value of $OD_{450}$ of a negative serum solution is ≤ 0.05, it shall be calculated as 0.05; when the mean value of $OD_{450}$ is > 0.05, it shall be calculated as the actual $OD_{450}$ value, and the Cut-off value shall be kept to three decimal places.

The antibody titer was the maximum dilution corresponding to the mean value of $OD_{450}$ of control and test serum > the Cut-off value.

The results of the humoral immunity test for binding total anti-IgG using lipid nanoparticle encapsulating IN002.5.1 mRNA with representative amino lipid compounds are shown in FIG. 5 and FIG. 6, with ALC00315 as a control.

The test results are shown in FIG. 5 and FIG. 6. It can be seen from FIG. 5 that the amino lipid compounds 269, 270, 271, 273 and 274 show better humoral immunity effect compared to the commercially available ALC0315. It can be seen from FIG. 6 that the amino lipid compounds 263, 264, 265 and 266 show better humoral immunity effect than the commercially available ALC0315.

**DNA sequence corresponding to IN002.5.1 mRNA sequence (SEQ ID NO.1):**

GGGGAAAGCTTTAATACGACTCACTATAGGACAGATCGCCTGGAGACGCCATC
CACGCTGTTTTGACCTCCATAGAAGACACCGGGACCGATCCAGCCTCCGCGGCCG
GGAACGGTGCATTGGAACGCGGATTCCCCGTGCCAAGAGTGACGTAAGTACCGCA
GTCACCGTCCTTGACACGGGATCCGCCACCATGTTCGTGTTCCTGGTGCTGCTGCC
TCTGGTGTCCAGCCAGTGTGTGAACCTGAGGACCAGAACACAGCTGCCTCCAGCC
TACACCAACAGCTTTACCAGAGGCGTGTACTACCCCGACAAGGTGTTCAGATCCA
GCGTGCTGCACTCTACCCAGGACCTGTTCCTGCCTTTCTTCAGCAACGTGACCTGG

TTCCACGCCATCCACGTGTCCGGCACCAATGGCACCAAGAGATTCGACAACCCCG
TGCTGCCCTTCAACGACGGGGTGTACTTTGCCAGCATCGAGAAGTCCAACATCATC
AGAGGCTGGATCTTCGGCACCACACTGGACAGCAAGACCCAGAGCCTGCTGATCG
TGAACAACGCCACCAACGTGGTCATCAAAGTGTGCGAGTTCCAGTTCTGCAACGA
CCCCTTCCTGGACGTCTACTACCACAAGAACAACAAGAGCTGGATGGAAAGCGGC
GTGTACAGCAGCGCCAACAACTGCACCTTCGAGTACGTGTCCCAGCCTTTCCTGAT
GGACCTGGAAGGCAAGCAGGGCAACTTCAAGAACCTGCGCGAGTTCGTGTTTAA
GAACATCGACGGCTACTTCAAGATCTACAGCAAGCACACCCCTATCAACCTCGTGC
GGGATCTGCCTCAGGGCTTCTCTGCTCTGGAACCCCTGGTGGATCTGCCCATCGGC
ATCAACATCACCCGGTTTCAGACACTGCTGGCCCTGCACAGAAGCTACCTGACAC
CTGGCGATAGCAGCAGCGGACTGACAGCTGGTGCCGCCGCTTACTATGTGGGCTAC
CTGCAGCCTAGAACCTTCCTGCTGAAGTACAACGAGAACGGCACCATCACCGACG
CCGTGGATTGTGCTCTGGACCCTCTGAGCGAGACAAAGTGCACCCTGAAGTCCTT
CACCGTGGAAAAGGGCATCTACCAGACCAGCAACTTCCGGGTGCAGCCCACCGAA
TCCATCGTGCGGTTCCCCAATATCACCAATCTGTGCCCCTTCGGCGAGGTGTTCAAT
GCCACCAGATTCGCCTCTGTGTACGCCTGGAACCGGAAGCGGATCAGCAATTGCG
TGGCCGACTACTCCGTGCTGTACAACTCCGCCAGCTTCAGCACCTTCAAGTGCTAC
GGCGTGTCCCCTACCAAGCTGAACGACCTGTGCTTCACAAACGTGTACGCCGACA
GCTTCGTGATCCGGGGAGATGAAGTGCGGCAGATTGCCCCTGGACAGACAGGCAA
CATCGCCGACTACAACTACAAGCTGCCCGACGACTTCACCGGCTGTGTGATTGCCT
GGAACAGCAACAACCTGGACTCCAAAGTCGGCGGCAACTACAATTACCGGTACAG
GCTGTTCCGGAAGTCCAATCTGAAGCCCTTCGAGCGGGACATCTCCACCGAGATCT
ATCAGGCCGGCAGCAAGCCTTGTAACGGCGTGGAAGGCTTCAACTGCTACTTCCC
ACTGCAGTCCTACGGCTTTCAGCCCACAAATGGCGTGGGCTATCAGCCCTACAGAG
TGGTGGTGCTGAGCTTCGAACTGCTGCATGCCCCTGCCACAGTGTGCGGCCCTAA
GAAAAGCACCAATCTCGTGAAGAACAAATGCGTGAACTTCAACTTCAACGGCCTG
ACCGGCACCGGCGTGCTGACAGAGAGCAACAAGAAGTTCCTGCCATTCCAGCAGT
TTGGCCGGGATATCGCCGATACCACAGACGCCGTTAGAGATCCCCAGACACTGGA
AATCCTGGACATCACCCCTTGCAGCTTCGGCGGAGTGTCTGTGATCACCCCTGGCA
CCAACACCAGCAATCAGGTGGCAGTGCTGTACCAGGGCGTGAACTGTACCGAAGT
GCCCGTGGCCATTCACGCCGATCAGCTGACACCTACATGGCGGGTGTACTCCACCG
GCAGCAATGTGTTTCAGACCAGAGCCGGCTGTCTGATCGGAGCCGAGCACGTGAA
CAATAGCTACGAGTGCGACATCCCCATCGGCGCTGGAATCTGCGCCAGCTACCAGA
CACAGACAAACAGCCGGCGGAGAGCCAGAAGCGTGGCCAGCCAGAGCATCATTG

CCTACACAATGTCTCTGGGCGCCGAGAACAGCGTGGCCTACTCCAACAACTCTATC GCTATCCCCACCAACTTCACCATCAGCGTGACCACAGAGATCCTGCCTGTGTCCAT GACCAAGACCAGCGTGGACTGCACCATGTACATCTGCGGCGATTCCACCGAGTGC TCCAACCTGCTGCTGCAGTACGGCAGCTTCTGCACCCAGCTGAATAGAGCCCTGA CAGGGATCGCCGTGGAACAGGACAAGAACACCCAAGAGGTGTTCGCCCAAGTGA AGCAGATCTACAAGACCCCTCCTATCAAGGACTTCGGCGGCTTCAATTTCAGCCAG ATTCTGCCCGATCCTAGCAAGCCCAGCAAGCGGAGCTTCATCGAGGACCTGCTGTT CAACAAAGTGACACTGGCCGACGCCGGCTTCATCAAGCAGTATGGCGATTGTCTG GGCGACATTGCCGCCAGGGATCTGATTTGCGCCCAGAAGTTTAACGGACTGACAG TGCTGCCTCCTCTGCTGACCGATGAGATGATCGCCCAGTACACATCTGCCCTGCTG GCCGGCACAATCACAAGCGGCTGGACATTTGGAGCAGGCGCCGCTCTGCAGATCC CCTTTGCTATGCAGATGGCCTACCGGTTCAACGGCATCGGAGTGACCCAGAATGTG CTGTACGAGAACCAGAAGCTGATCGCCAACCAGTTCAACAGCGCCATCGGCAAGA TCCAGGACAGCCTGAGCAGCACAGCAAGCGCCCTGGGAAAGCTGCAGAACGTGG TCAACCAGAATGCCCAGGCACTGAACACCCTGGTCAAGCAGCTGTCCTCCAACTT CGGCGCCATCAGCTCTGTGCTGAACGATATCCTGAGCAGACTGGACCCTCCTGAG GCCGAGGTGCAGATCGACAGACTGATCACAGGCAGACTGCAGAGCCTCCAGACAT ACGTGACCCAGCAGCTGATCAGAGCCGCCGAGATTAGAGCCTCTGCCAATCTGGC CGCCACCAAGATGTCTGAGTGTGTGCTGGGCCAGAGCAAGAGAGTGGACTTTTGC GGCAAGGGCTACCACCTGATGAGCTTCCCTCAGTCTGCCCCTCACGGCGTGGTGTT TCTGCACGTGACATATGTGCCCGCTCAAGAGAAGAATTTCACCACCGCTCCAGCCA TCTGCCACGACGGCAAAGCCCACTTTCCTAGAGAAGGCGTGTTCGTGTCCAACGG CACCCATTGGTTCGTGACACAGCGGAACTTCTACGAGCCCCAGATCATCACCACCG ACAACACCTTCGTGTCTGGCAACTGCGACGTCGTGATCGGCATTGTGAACAATACC GTGTACGACCCTCTGCAGCCCGAGCTGGACAGCTTCAAAGAGGAACTGGACAAGT ACTTTAAGAACCACACAAGCCCCGACGTGGACCTGGGCGATATCAGCGGAATCAA TGCCAGCGTCGTGAACATCCAGAAAGAGATCGACCGGCTGAACGAGGTGGCCAA GAATCTGAACGAGAGCCTGATCGACCTGCAAGAACTGGGGAAGTACGAGCAGTAC ATCAAGTGGCCCTGGTACATCTGGCTGGGCTTTATCGCCGGACTGATTGCCATCGT GATGGTCACAATCATGCTGTGTTGCATGACCAGCTGCTGTAGCTGCCTGAAGGGCT GTTGTAGCTGTGGCAGCTGCTGCAAGTTCGACGAGGACGATTCTGAGCCCGTGCT GAAGGGCGTGAAACTGCACTACACATGATGAGGTACCCGGGTGGCATCCCTGTGA CCCCTCCCCAGTGCCTCTCCTGGCCCTGGAAGTTGCCACTCCAGTGCCCACCAGCC TTGTCCTAATAAAATTAAGTTGCATCGGGCCCAAAAAAAAAAAAAAAAAAAAAAA

AAAAAAAGCATATGACTAAAAAAAAAAAAAAAAAAAAAAAAAAAAAAAAAAAA

AAAAAAAAAAAAAAAAAAAAAAAAAAAAAAAAAA

In addition to those described in this disclosure, various modifications to this disclosure will be apparent to those skilled in the art from the foregoing description. Such modifications are also intended to fall within the scope of the appended claims.

## Claims

**1.** An amino lipid compound having a structure of formula (I):

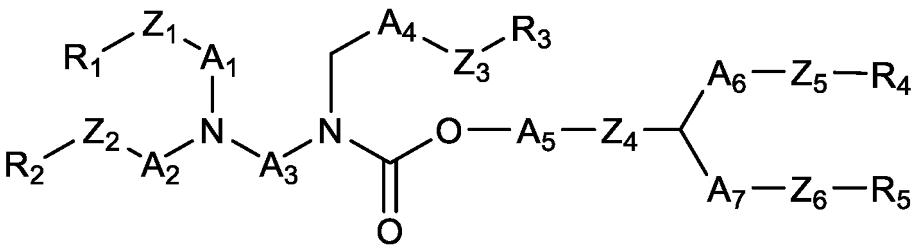

(I)

or a pharmaceutically acceptable salt or stereoisomer thereof, wherein:

$Z_1$, $Z_2$, $Z_3$, $Z_4$, $Z_5$, and $Z_6$ are each independently -CH($OR_7$)-, -C=C-, -C=C-, -O-, -C(=O)O-, -OC(=O)-, -N($R_6$)C(=O)-, -C(=O)N($R_6$)-, -N($R_6$)C(=O)N($R_6$)-, -OC(=O)N($R_6$)-, -N($R_6$)C(=O)O-, -C(=O)-, -C(=O)S-, -SC(=O)-, -S-S- or a bond;
$A_1$, $A_2$, $A_3$, $A_4$, $A_5$, $A_6$, and $A_7$ are each independently $C_1$-$C_{12}$ hydrocarbylene, $C_3$-$C_7$ cyclohydrocarbyl, phenyl, benzyl, 4- to 7-membered heterocycle, or a bond;
$R_1$ and $R_2$ are each independently H or $C_1$-$C_{18}$ hydrocarbyl, or $C_3$-$C_7$ cyclohydrocarbyl, phenyl, benzyl, or 4- to 7-membered heterocycle; or $R_1$ and $R_2$ together with the nitrogen atom to which they are attached form a 4- to 7-membered heterocycle having said nitrogen atom and 0, 1, 2 or 3 additional heteroatoms independently selected from N, O and S in the ring, the heterocycle being optionally substituted with 1, 2, 3 or more substituents independently selected from $C_1$-$C_8$ alkyl, $C_1$-$C_8$ haloalkyl, -O-$C_1$-$C_8$ alkyl, -O-$C_1$-$C_8$ haloalkyl, halogen, OH, CN, nitro, $NH_2$, -NH($C_1$-$C_6$ alkyl), and -N($C_1$-$C_6$ alkyl)$_2$;
$R_3$ is H or $C_1$-$C_{18}$ hydrocarbyl, or $C_3$-$C_7$ cyclohydrocarbyl, phenyl, benzyl, 4- to 7-membered heterocycle;
$R_4$ and $R_5$ are each independently $C_1$-$C_{18}$ hydrocarbyl, or $C_3$-$C_7$ cyclohydrocarbyl, phenyl, benzyl, or 4- to 7-membered heterocycle;
$R_6$ is H or $C_1$-$C_{18}$ hydrocarbyl, or -OH, -O-optionally substituted $C_2$-$C_{18}$ hydrocarbyl, or -C=C-, or -C=C-optionally substituted $C_4$-$C_{18}$ hydrocarbyl, or $C_1$-$C_{18}$ heterohydrocarbyl; and $R_7$ is H or $C_1$-$C_{12}$ hydrocarbyl.

**2.** The amino lipid compound according to claim 1, wherein $A_1$, $A_2$, $A_3$, $A_4$, $A_5$, $A_6$ and $A_7$ are each independently $C_1$-$C_{12}$ alkylene, $C_2$-$C_{12}$ alkenylene having 1, 2, 3, 4 or more double bonds, $C_3$-$C_6$ cycloalkyl, phenyl, benzyl, 5- to 6-membered heterocycle or a bond, preferably $C_1$-$C_{12}$ alkylene, $C_2$-$C_{12}$ alkenylene having 1, 2, 3, 4, or more double bonds, or a bond;

preferably, $A_1$ and $A_2$ are each independently $C_1$-$C_6$ alkylene or a bond, more preferably $C_1$, $C_2$, $C_3$, $C_4$ or $C_5$ alkylene or a bond, further preferably $C_1$ or $C_2$ alkylene or a bond;
preferably, $A_3$ is $C_1$-$C_6$ alkylene or a bond, more preferably $C_2$, $C_3$, $C_4$ or $C_5$ alkylene, further preferably $C_2$, $C_3$ or $C_4$ alkylene;
preferably, $A_4$ is $C_1$-$C_6$ alkylene or a bond, more preferably $C_1$, $C_2$, $C_3$, $C_4$ or $C_5$ alkylene or a bond, further preferably $C_1$, $C_2$, $C_3$ or $C_4$ alkylene or a bond;
preferably, $A_5$ is $C_1$-$C_8$ alkylene or a bond, more preferably $C_1$, $C_2$, $C_3$, $C_4$ or $C_5$ alkylene or a bond, further preferably $C_1$, $C_2$, $C_3$ or $C_4$ alkylene or a bond;
preferably, $A_6$ and $A_7$ are each independently $C_5$-$C_{12}$ alkylene, more preferably $C_6$-$C_{11}$ alkylene, further

preferably $C_7$, $C_8$, $C_9$ or $C_{10}$ alkylene.

**3.** The amino lipid compound according to claim 1 or 2, wherein $R_1$ and $R_2$ are each independently H or $C_1$-$C_{18}$ alkyl, $C_2$-$C_{18}$ alkenyl having 1, 2, 3, 4 or more double bonds, or $C_3$-$C_6$ cycloalkyl, phenyl, benzyl, or 5- to 6- membered heterocycle, preferably $C_1$-$C_{18}$ alkyl, or $C_2$-$C_{18}$ alkenyl having 1, 2, 3, 4 or more double bonds; or

$R_1$ and $R_2$ together with the nitrogen atom to which they are attached form a 4- to 7-membered heterocycle having said nitrogen atom and 0, 1, 2 or 3 additional heteroatoms independently selected from N, O and S in the ring, the heterocycle being optionally substituted with 1, 2, 3 or more substituents independently selected from $C_1$-$C_8$ alkyl, $C_1$-$C_8$ haloalkyl, -O-$C_1$-$C_8$ alkyl, -O-$C_1$-$C_8$ haloalkyl, halogen, OH, CN, nitro, $NH_2$ , -NH($C_1$-$C_6$ alkyl), and -N($C_1$-$C_6$ alkyl)$_2$;
preferably, $R_1$ and $R_2$ are each independently $C_1$-$C_6$ alkyl or a bond, preferably $C_1$, $C_2$, $C_3$, $C_4$ or $C_5$ alkyl or a bond, more preferably methyl, ethyl, n-propyl, or isopropyl; or,
preferably, $R_1$ and $R_2$ together with the nitrogen atom to which they are attached form a 4- to 7-membered heterocycle, preferably 5- to 6-membered heterocycle, having said nitrogen atom in the ring.

**4.** The amino lipid compound according to any one of claims 1 to 3, wherein $R_3$ is H, $C_1$-$C_{18}$ alkyl, $C_2$ -$C_{18}$ alkenyl having 1, 2, 3, 4 or more double bonds, or $C_3$-$C_6$ cycloalkyl, phenyl, benzyl, or 5- to 6-membered heterocycle, preferably H, $C_1$-$C_{18}$ alkyl, or $C_2$-$C_{18}$ alkenyl having 1, 2, 3, 4 or more double bonds;
preferably, $R_3$ is $C_1$-$C_{16}$ alkyl, more preferably $C_1$-$C_{14}$ alkyl, further preferably $C_1$, $C_2$, $C_3$, $C_4$, $C_5$, $C_6$, $C_7$, $C_8$, $C_9$ or $C_{10}$ alkyl.

**5.** The amino lipid compound according to any one of claims 1 to 4, wherein $R_4$ and $R_5$ are each independently $C_1$-$C_{18}$ alkyl, $C_2$-$C_{18}$ alkenyl having 1, 2, 3, 4 or more double bonds, or $C_3$-$C_6$ cycloalkyl, phenyl, benzyl, or 5- to 6-membered heterocycle, preferably $C_1$-$C_{18}$ alkyl, or $C_2$-$C_{18}$ alkenyl having 1, 2, 3, 4 or more double bonds;
preferably, $R_4$ and $R_5$ are each independently branched or straight $C_1$-$C_{18}$ alkyl, more preferably branched or straight $C_8$, $C_9$, $C_{10}$, $C_{11}$, $C_{12}$, $C_{13}$ or $C_{14}$ alkyl, further preferably branched $C_{12}$ or $C_{13}$ alkyl.

**6.** The amino lipid compound according to any one of claims 1 to 5, wherein $R_6$ is H, $C_1$-$C_{18}$ alkyl, $C_2$-$C_{18}$ alkenyl having 1, 2, 3, 4 or more double bonds, -OH, -O-optionally substituted $C_2$-$C_{18}$ alkyl, -O-optionally substituted $C_2$-$C_{18}$ alkenyl having 1, 2, 3, 4 or more double bonds, -C=C-, -C = C-optionally substituted $C_4$-$C_{18}$ alkyl, or -C = C-optionally substituted $C_4$-$C_{18}$ alkyl having 1, 2, 3, 4 or more double bonds, or $C_1$-$C_{18}$ heteroalkyl containing O, N or S, or $C_2$-$C_{18}$ heteroalkenyl containing O, N or S, or $C_2$-$C_{18}$ heteroalkynyl containing O, N, or S; or
$R_6$ is H, Ci-Cs alkyl, $C_2$-$C_{12}$ alkenyl having 1, 2 or 3 double bonds, -OH, -O-optionally substituted $C_2$-$C_{12}$ alkyl, -O-optionally substituted $C_2$-$C_{12}$ alkenyl having 1, 2 or 3 double bonds, -C=C-, -C=C-optionally substituted $C_4$-$C_{12}$ alkyl, or -C=C-optionally substituted $C_4$-$C_{12}$ alkyl having 1, 2 or 3 double bonds, or Ci-Cs heteroalkyl containing O, N, or S, or $C_2$-$C_{12}$ heteroalkenyl containing O, N, or S, or $C_2$-$C_{12}$ heteroalkynyl containing O, N, or S.

**7.** The amino lipid compound according to any one of claims 1 to 6, wherein $R_7$ is H, $C_1$-$C_{12}$ alkyl, or $C_2$-$C_{12}$ alkenyl having 1, 2, or 3 double bonds, preferably H, or $C_1$-$C_{12}$ alkyl.

**8.** The amino lipid compound according to any one of claims 1 to 7, wherein $Z_5$ and $Z_6$ are each independently -OC(=O)-, -C(=O)O-, or a bond.

**9.** The amino lipid compound according to any one of claims 1 to 8, wherein at least one of $Z_1$ and $Z_2$ is a bond, preferably both $Z_1$ and $Z_2$ are bonds.

**10.** The amino lipid compound according to any one of claims 1 to 9, wherein:

at least one of $A_1$ and $A_2$ is a bond, preferably both $A_1$ and $A_2$ are bonds; and/or
$A_5$ is a bond; and/or
at least one of $A_6$ and $A_7$ is a bond, preferably both $A_6$ and $A_7$ are bonds; and/or
Z4 is a bond.

**11.** The amino lipid compound of any one of claims 1 to 10, wherein $Z_3$ is -CH($OR_7$)-, -(C=O)O-, -O(C=O)-, or a bond.

**12.** The amino lipid compound according to any one of claims 1 to 11, wherein:

$Z_1$, $Z_2$, and $Z_4$ are each independently a bond;
$Z_3$ is -CH($OR_7$)-, -C(=O)O-, -OC(=O)-, or a bond;
$Z_5$ and $Z_6$ are each independently -C(=O)O- or -OC(=O)-;
$A_1$, $A_2$, and $A_5$ are each independently a bond;
$A_3$, $A_6$ and $A_7$ are each independently $C_1$-$C_{12}$ alkylene or $C_2$-$C_{12}$ alkenylene having 1, 2, 3, 4 or more double bonds;
$A_4$ is $C_1$-$C_{12}$ alkylene, $C_2$-$C_{12}$ alkenylene having 1, 2, 3, 4 or more double bonds, or a bond;
$R_1$ and $R_2$ are each independently $C_1$-$C_{18}$ alkyl, or $C_2$-$C_{18}$ alkenyl having 1, 2, 3, 4 or more double bonds; or $R_1$ and $R_2$ together with the nitrogen atom to which they are attached form a 4- to 7-membered heterocycle having said nitrogen atom and 0, 1, 2 or 3 additional heteroatoms independently selected from N, O and S in the ring, the heterocycle being optionally substituted with 1, 2, 3 or more substituents independently selected from $C_1$-$C_8$ alkyl, $C_1$-$C_8$ haloalkyl, -O-$C_1$-$C_8$ alkyl, -O-$C_1$-$C_8$ haloalkyl, halogen, OH, CN, nitro, $NH_2$ , -NH($C_1$-$C_6$ alkyl), and -N($C_1$-$C_6$ alkyl)$_2$;
$R_3$ is H, $C_1$-$C_{18}$ alkyl, or $C_2$-$C_{18}$ alkenyl having 1, 2, 3, 4 or more double bonds;
$R_4$ and $R_5$ are each independently $C_1$-$C_{18}$ alkyl, or $C_2$-$C_{18}$ alkenyl having 1, 2, 3, 4 or more double bonds; and
$R_7$ is H, $C_1$-$C_{12}$ alkyl, or $C_2$-$C_{12}$ alkenyl having 1, 2 or 3 double bonds, preferably H, or $C_1$-$C_{12}$ alkyl.

**13.** The amino lipid compound according to any one of claims 1 to 12, wherein:

$Z_3$ is a bond;
$A_4$ is a bond;
$R_1$ and $R_2$ are each independently $C_1$-$C_{18}$ alkyl, or $C_2$-$C_{18}$ alkenyl having 1, 2, 3, 4 or more double bonds; or $R_1$ and $R_2$ together with the nitrogen atom to which they are attached form a 4- to 7-membered heterocycle having said nitrogen atom and 0, 1 or 2 additional heteroatoms independently selected from N, O and S in the ring, the heterocycle being optionally substituted with 1, 2, or 3 substituents independently selected from $C_1$-$C_8$ alkyl, $C_1$-$C_8$ haloalkyl, -O-$C_1$-$C_8$ alkyl, -O-$C_1$-$C_8$ haloalkyl, halogen, OH, CN, nitro, $NH_2$, -NH($C_1$-$C_6$ alkyl) and -N($C_1$-$C_6$ alkyl)$_2$; and
$R_3$ is $C_1$-$C_{18}$ alkyl, or $C_2$-$C_{18}$ alkenyl having 1, 2, 3, 4 or more double bonds, preferably $C_1$-$C_{18}$ alkyl.

**14.** The amino lipid compound according to any one of claims 1 to 12, wherein:

$Z_3$ is -CH($OR_7$)-; and
$R_1$ and $R_2$ are each independently $C_1$-$C_{18}$ alkyl, or $C_2$-$C_{18}$ alkenyl having 1, 2, 3, 4 or more double bonds, preferably $C_1$-$C_{18}$ alkyl.

**15.** The amino lipid compound according to claim 14, wherein $R_7$ is H.

**16.** The amino lipid compound according to claim 15, wherein $A_4$ is a bond.

**17.** The amino lipid compound according to claim 15, wherein $A_4$ is $C_1$-$C_{12}$ alkylene, $C_2$-$C_{12}$ alkenylene having 1, 2, 3, 4 or more double bonds, preferably $C_1$-$C_{10}$ alkylene, more preferably $C_1$-$C_8$ alkylene, more preferably -$(CH_2)_{n4}$-, wherein n4 = 1, 2, 3, 4, 5, 6, 7 or 8.

**18.** The amino lipid compound according to any one of claims 15 to 17, wherein $R_3$ is $C_1$-$C_{18}$ alkyl, or $C_2$-$C_{18}$ alkenyl having 1, 2, 3, 4 or more double bonds, preferably $C_1$-$C_{18}$ alkyl.

**19.** The amino lipid compound according to claim 10, wherein $R_7$ is $C_1$-$C_{12}$ alkyl, or $C_2$-$C_{12}$ alkenyl having 1, 2, or 3 double bonds, preferably $C_1$-$C_{12}$ alkyl.

**20.** The amino lipid compound according to claim 19, wherein $R_3$ is H.

**21.** The amino lipid compound according to claim 19, wherein $R_3$ is $C_1$-$C_{18}$ alkyl, or $C_2$-$C_{18}$ alkenyl having 1, 2, 3, 4 or more double bonds, preferably $C_1$-$C_{18}$ alkyl.

**22.** The amino lipid compound according to claim 20 or 21, wherein $A_4$ is a bond.

**23.** The amino lipid compound according to claim 20 or 21, wherein $A_4$ is $C_1$-$C_{12}$ alkylene, or $C_2$-$C_{12}$ alkenylene having 1, 2, 3, 4 or more double bonds, preferably $C_1$-$C_{10}$ alkylene, more preferably $C_1$-$C_8$ alkylene, more preferably -$(CH_2)_{n4}$-,

wherein n4 = 1, 2, 3, 4, 5, 6, 7, or 8.

**24.** The amino lipid compound according to any one of claims 1 to 8, wherein:
$Z_3$ is -C(=O)O- or -OC(=O)-.

**25.** The amino lipid compound according to claim 24, wherein:

$Z_3$ is -C(=O)O-, wherein the C(=O) moiety is bonded to $A_4$; and
$R_3$ is $C_1$-$C_{18}$ alkyl, or $C_2$-$C_{18}$ alkenyl having 1, 2, 3, 4 or more double bonds.

**26.** The amino lipid compound according to claim 24 or 25, wherein $A_4$ is a bond.

**27.** The amino lipid compound according to claim 24 or 25, wherein $A_4$ is $C_1$-$C_{12}$ alkylene, or $C_2$-$C_{12}$ alkenylene having 1, 2, 3, 4 or more double bonds, preferably $C_1$-$C_{10}$ alkylene, more preferably $C_1$-$C_8$ alkylene, more preferably $-(CH_2)_{n4}-$, wherein n4 = 1, 2, 3, 4, 5, 6, 7, or 8.

**28.** The amino lipid compound according to any one of claims 1 to 27, wherein: the hydrocarbylene or alkylene as defined for $A_1$, $A_2$, $A_3$, $A_4$, $A_5$, $A_6$ or $A_7$ is each independently $C_1$-$C_{10}$ alkylene.

**29.** The amino lipid compound according to any one of claims 1 to 27, wherein: the hydrocarbylene or alkenylene as defined for $A_1$ , $A_2$ , $A_3$ , $A_4$ , $A_5$ , $A_6$, or $A_7$ is each independently $C_2$-$C_{10}$ alkenylene having 1, 2, 3, 4, or more double bonds.

**30.** The amino lipid compound according to any one of claims 1 to 29, wherein $A_3$ is $C_1$-$C_8$ alkylene or $C_2$-$C_8$ alkenylene having 1 or 2 double bonds, preferably $C_1$-$C_6$ alkylene or $C_2$-$C_6$ alkenylene having 1 double bond, more preferably $C_2$-$C_4$ alkylene or $C_2$-$C_4$ alkenylene having 1 double bond, and more preferably $-(CH_2)_2-$, $-(CH_2)_3-$ or $-(CH_2)_4-$.

**31.** The amino lipid compound according to any one of claims 1 to 30, wherein $A_6$ and $A_7$ are each independently $C_1$-$C_{10}$ alkylene, or $C_2$-$C_{10}$ alkenylene having 1, 2, 3, 4 or more double bonds; preferably, $A_6$ and $A_7$ are each independently $C_5$-$C_{10}$ alkylene, or $C_5$-$C_{10}$ alkenylene having 1, 2, 3, 4 or more double bonds, more preferably $C_6$-$C_9$ alkylene, or $C_6$-$C_9$ alkenylene having 1, 2, 3, 4 or more double bonds, and more preferably $C_7$-$C_8$ alkylene, or $C_7$-$C_8$ alkenylene having 1, 2 or more double bonds.

**32.** The amino lipid compound according to any one of claims 1 to 31, wherein the hydrocarbyl or alkyl as defined for $R_1$ or $R_2$ is $C_1$-$C_8$ alkyl, preferably $C_1$-$C_6$ alkyl, more preferably $C_1$-$C_4$ alkyl, and more preferably $C_1$-$C_2$ alkyl.

**33.** The amino lipid compound according to any one of claims 1 to 31, wherein the heterocycle formed by $R_1$ and $R_2$ together with the nitrogen atom to which they are attached is a 5- to 7-membered heterocycle having said nitrogen atom and 0, 1 or 2 additional heteroatoms independently selected from N, O and S in the ring, the heterocycle being optionally substituted with 1, 2, or 3 substituents independently selected from $C_1$-$C_6$ alkyl, $C_1$-$C_6$ haloalkyl, -O-$C_1$-$C_6$ alkyl, -O-$C_1$-$C_6$ haloalkyl, halogen, OH, CN, nitro, $NH_2$, -NH($C_1$-$C_6$ alkyl) and -N($C_1$-$C_6$ alkyl)$_2$;
preferably, the heterocycle is a 5- to 6-membered heterocycle having said nitrogen atom and no additional heteroatom in the ring, the heterocycle being optionally substituted with 1, 2 or 3 substituents independently selected from $C_1$-$C_4$ alkyl, preferably $C_1$-$C_3$ alkyl, preferably

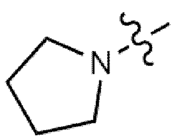 , 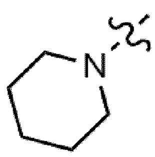 , or 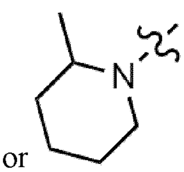 .

**34.** The amino lipid compound according to any one of claims 1 to 33, wherein the hydrocarbyl or alkyl as defined for $R_3$ is $C_1$-$C_{12}$ alkyl, preferably $C_1$-$C_{10}$ alkyl, and more preferably $-(CH_2)_{n31}-CH_3$, or $-CH((CH_2)_{n32}-CH_3)-(CH_2)_{n33}-CH_3$, wherein n31 is 0, 1, 2, 3, 4, 5, 6, 7, or 8; n32 is 0, 1, 2, 3, 4, 5 or 6, preferably 0, 1, 2 or 3; and n33 is 0, 1, 2, 3, 4, 5, 6, 7 or 8, preferably 0, 1, 2, 3, 4, 5 or 6.

**35.** The amino lipid compound according to any one of claims 1 to 33, wherein the hydrocarbyl or alkenyl as defined for $R_3$

is $C_2$-$C_{12}$ alkenyl having 1, 2, or 3 double bonds, preferably $C_2$-$C_{10}$ alkenyl having 1 double bond.

**36.** The amino lipid compound according to any one of claims 1 to 35, wherein the hydrocarbyl or alkyl as defined for $R_7$ is $C_1$-$C_{10}$ alkyl, preferably $C_1$-$C_8$ alkyl, more preferably $-(CH_2)_{n7}-CH_3$, wherein n7 is 0, 1, 2, 3, 4, 5, 6, or 7.

**37.** The amino lipid compound according to any one of claims 1 to 35, wherein the hydrocarbyl or alkenyl as defined for $R_7$ is $C_2$-$C_{10}$ alkenyl having 1, 2, or 3 double bonds, preferably $C_2$-$C_8$ alkenyl having 1 or 2 double bonds.

**38.** The amino lipid compound according to any one of claims 1 to 37, wherein at least one of $Z_5$ and $Z_6$ is -C(=O)O-, preferably both $Z_5$ and $Z_6$ are -C(=O)O-, more preferably the C(=O) moiety is bonded to $A_6$ or $A_7$.

**39.** The amino lipid compound according to any one of claims 1 to 38, wherein the hydrocarbyl or alkyl as defined for $R_4$ or $R_5$ is branched, preferably branched $C_3$-$C_{18}$ alkyl, more preferably branched $C_8$-$C_{18}$ alkyl, such as branched $C_{11}$-$C_{18}$ alkyl or branched $C_{13}$-$C_{15}$ alkyl, and preferably

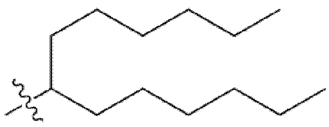
, or
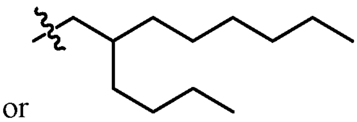
.

**40.** The amino lipid compound according to any one of claims 1 to 39, wherein the hydrocarbyl or alkenyl as defined for $R_4$ or $R_5$ is branched, preferably branched $C_3$-$C_{18}$ alkenyl, more preferably branched $C_8$-$C_{18}$ alkenyl, such as branched $C_{11}$-$C_{18}$ alkenyl or branched $C_{13}$-$C_{15}$ alkenyl, with the alkenyl having 1, 2, or 3 double bonds.

**41.** The amino lipid compound according to claim 1, or a pharmaceutically acceptable salt or stereoisomer thereof, wherein the amino lipid compound has a structure represented by

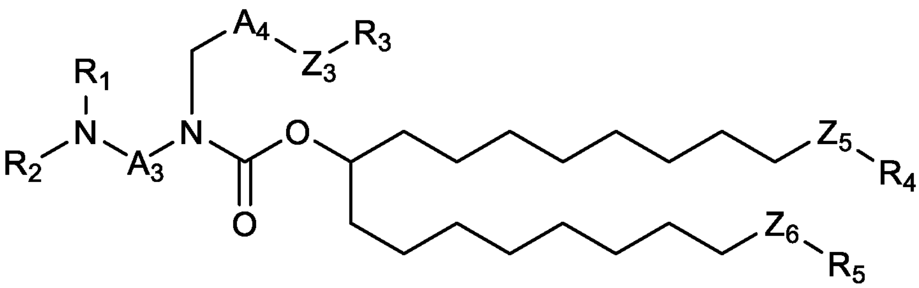

formula (II): (II) wherein,

- $Z_3$, $Z_5$ and $Z_6$ are each independently -C(=O)O- or -OC(=O)-;
- $A_3$ is $C_2$-$C_5$ alkylene;
- $A_4$ is $C_1$-$C_5$ alkylene or a bond;
- $R_1$ and $R_2$ are each independently H or $C_1$-$C_3$ alkyl; or $R_1$ and $R_2$ together with the nitrogen atom to which they are attached form a 4- to 7-membered heterocycle having said nitrogen atom in the ring, the heterocycle being optionally substituted with 1, 2, 3 or more substituents independently selected from $C_1$-$C_8$ alkyl, $C_1$-$C_8$ haloalkyl, -O-$C_1$-$C_8$ alkyl, -O-$C_1$-$C_8$ haloalkyl, halogen, OH, CN, nitro, $NH_2$, -NH($C_1$-$C_6$ alkyl), and -N($C_1$-$C_6$ alkyl)$_2$;
- $R_3$ is $C_1$-$C_{10}$ alkyl; and
- $R_4$ and $R_5$ are each independently branched $C_1$-$C_{18}$ alkyl.

**42.** The amino lipid compound according to claim 41, wherein $Z_3$ is -C(=O)O-.

**43.** The amino lipid compound according to any one of claim 41 or 42, wherein $Z_5$ is -C(=O)O-.

**44.** The amino lipid compound according to any one of claims 41 to 43, wherein $Z_6$ is -C(=O)O-.

**45.** The amino lipid compound according to any one of claims 41 to 44, wherein $A_3$ is $C_3$-$C_4$ alkylene.

**46.** The amino lipid compound of any one of claims 41 to 45, wherein $A_4$ is $C_1$-$C_2$ alkylene, $C_3$-$C_4$ alkylene, or a bond.

**47.** The amino lipid compound of any one of claims 41 to 46, wherein $R_1$ is methyl, ethyl, n-propyl, or isopropyl.

**48.** The amino lipid compound according to any one of claims 41 to 47, wherein $R_2$ is methyl, ethyl, n-propyl, or isopropyl.

**49.** The amino lipid compound according to any one of claims 41 to 48, wherein $R_1$ and $R_2$ together with the nitrogen atom to which they are attached form a 5- to 6-membered heterocycle having said nitrogen atom in the ring, the heterocycle being optionally substituted with 1, 2, 3 or more substituents independently selected from $C_1$-$C_8$ alkyl, $C_1$-$C_8$ haloalkyl, -O-$C_1$-$C_8$ alkyl, -O-$C_1$-$C_8$ haloalkyl, halogen, OH, CN, nitro, $NH_2$, -NH($C_1$-$C_6$ alkyl) and -N($C_1$-$C_6$ alkyl)$_2$, for example, substituted by methyl.

**50.** The amino lipid compound according to claim 49, wherein the heterocycle is

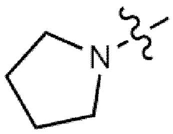
,
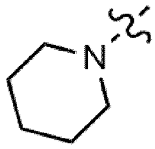
, or
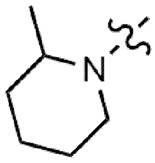
.

**51.** The amino lipid compound according to any one of claims 41 to 50, wherein $R_3$ is $C_1$, $C_2$, $C_3$, $C_4$, $C_5$, $C_6$, $C_7$ or $C_8$ alkyl, the alkyl being straight or branched.

**52.** The amino lipid compound according to any one of claims 41 to 51, wherein $R_4$ is branched $C_{13}$-$C_{15}$ alkyl.

**53.** The amino lipid compound according to claim 52, wherein $R_4$ is

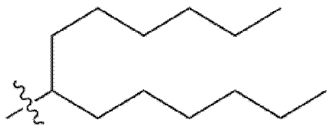
or
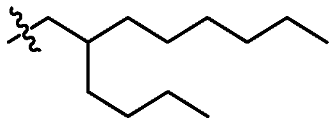
.

**54.** The amino lipid compound according to any one of claims 41 to 53, wherein $R_5$ is branched $C_{13}$-$C_{15}$ alkyl.

**55.** The amino lipid compound according to claim 54, wherein $R_5$ is

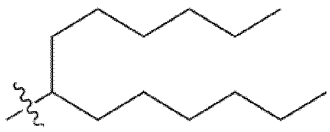
or
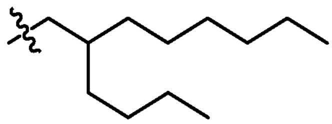
.

**56.** The amino lipid compound according to any one of claims 1 to 55, having one of the structures shown below:

| No. | Structural formula |
|---|---|
| 108 | 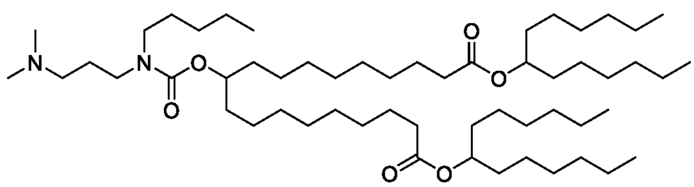 |

(continued)

| No. | Structural formula |
| --- | --- |
| 109 | 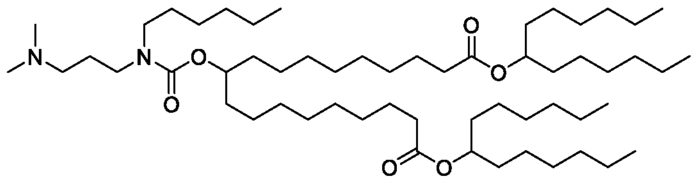 |
| 110 | 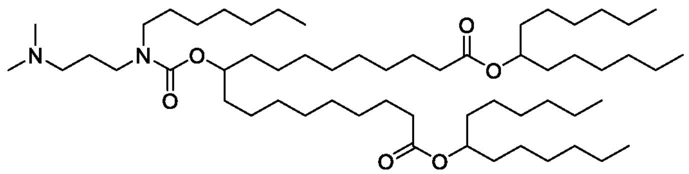 |
| 111 | 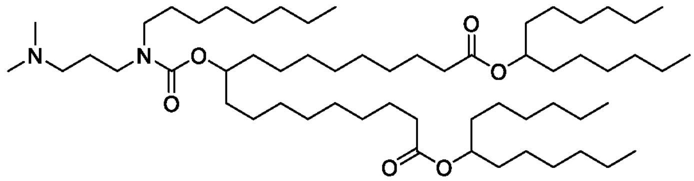 |
| 112 | 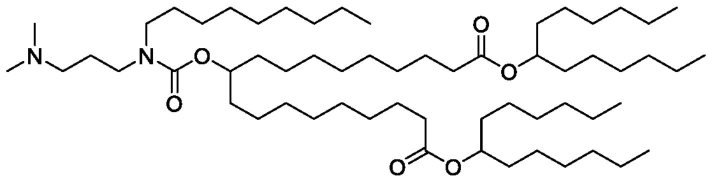 |
| 113 | 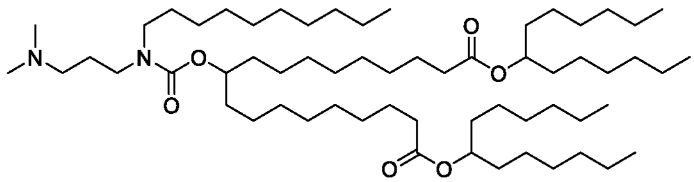 |
| 114 | 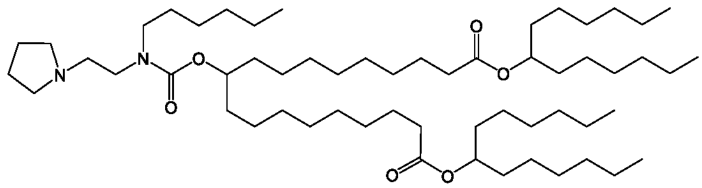 |
| 115 | 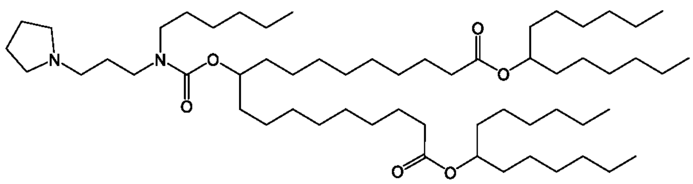 |
| 116 | 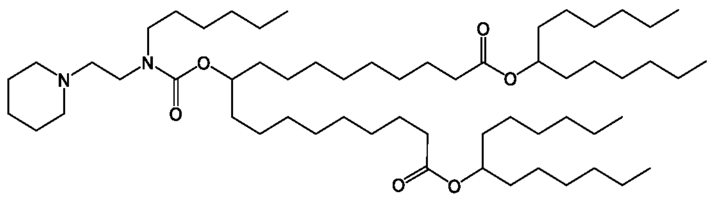 |

(continued)

| No. | Structural formula |
|---|---|
| 117 | 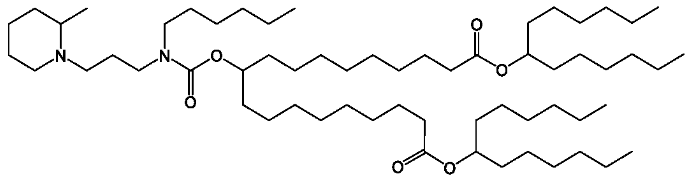 |
| 137 | 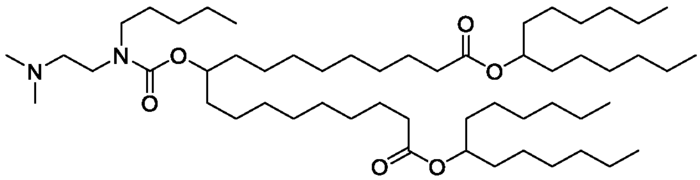 |
| 138 | 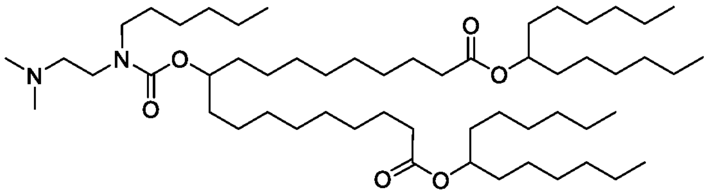 |
| 139 | 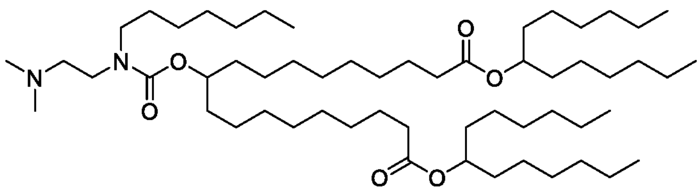 |
| 140 | 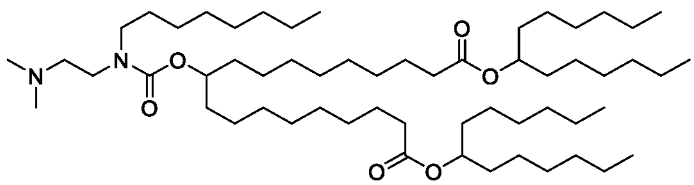 |
| 141 | 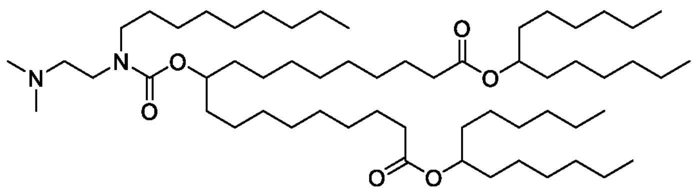 |
| 142 | 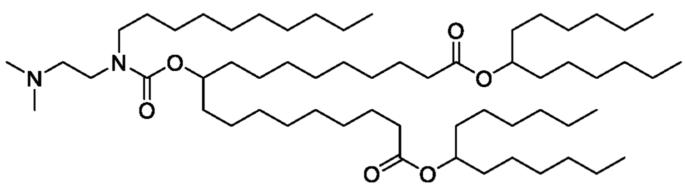 |
| 159 | 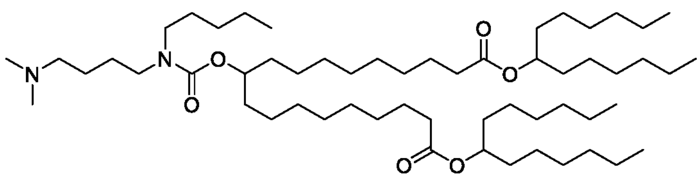 |

(continued)

| No. | Structural formula |
|---|---|
| 160 | 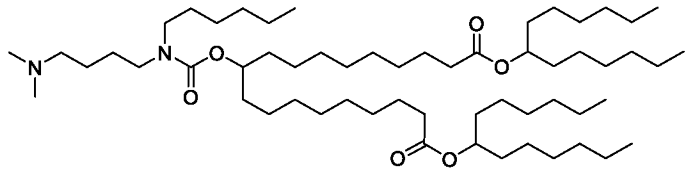 |
| 161 | 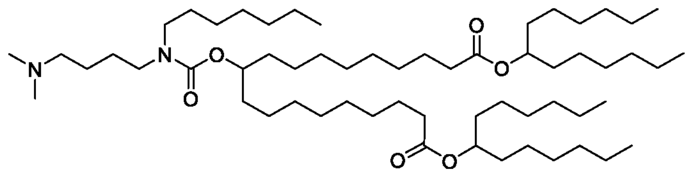 |
| 162 | 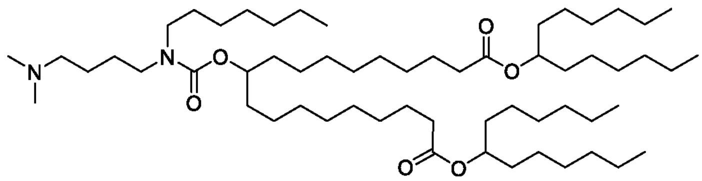 |
| 163 | 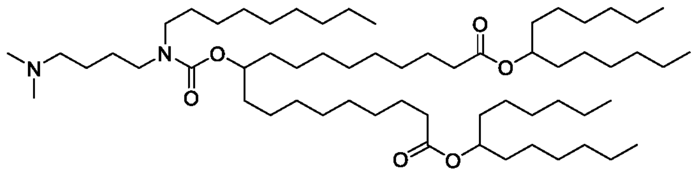 |
| 164 | 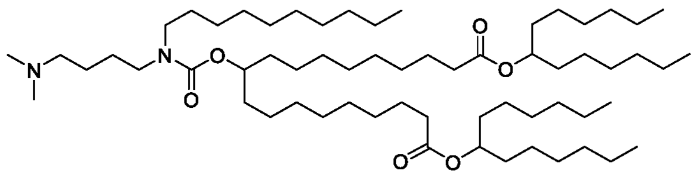 |
| 181 | 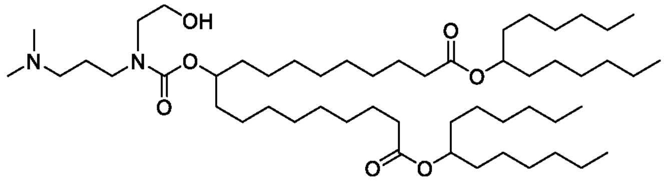 |
| 182 | 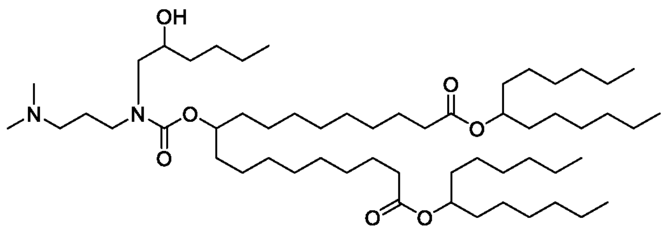 |
| 183 | 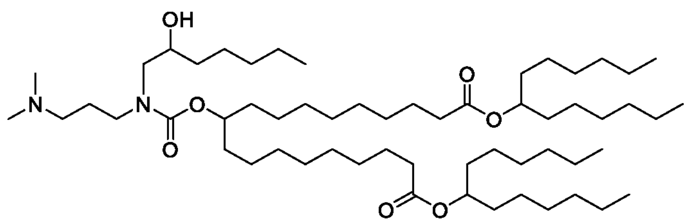 |

(continued)

| No. | Structural formula |
|---|---|
| 184 | 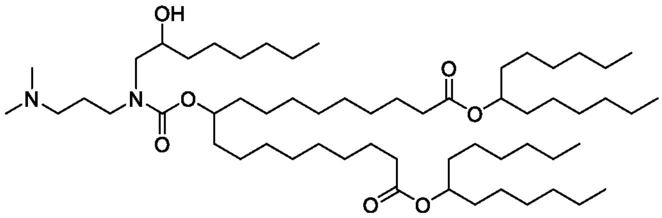 |
| 185 | 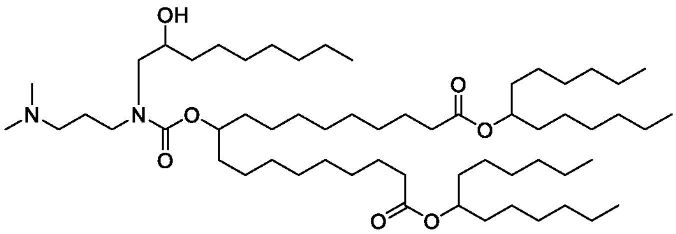 |
| 186 | 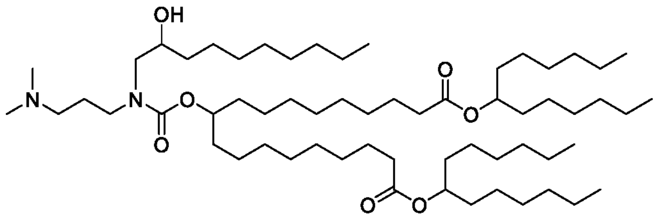 |
| 187 | 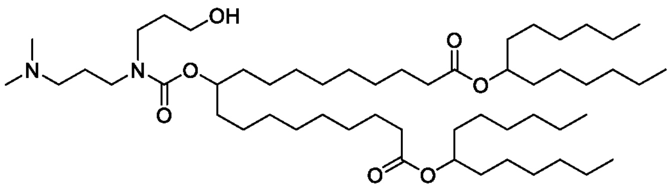 |
| 188 | 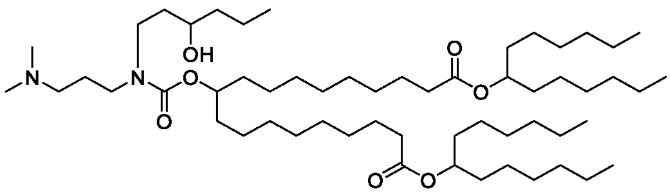 |
| 189 | 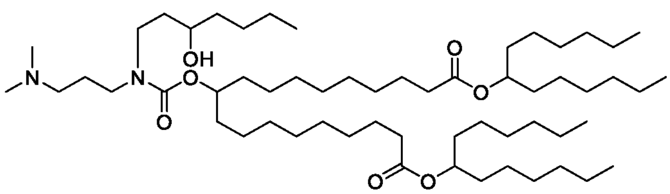 |
| 190 | 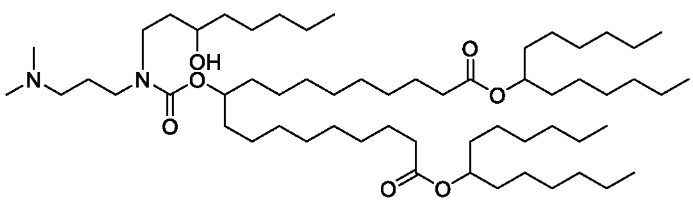 |

(continued)

| No. | Structural formula |
|---|---|
| 191 | 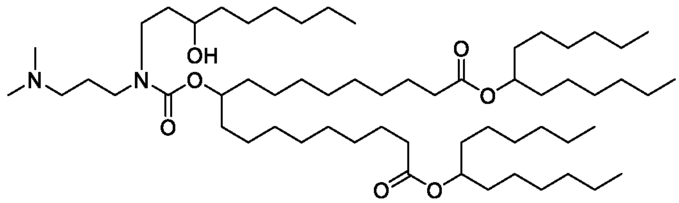 |
| 192 | 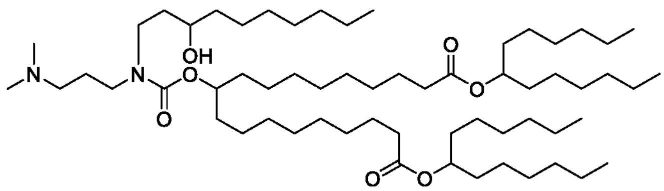 |
| 193 | 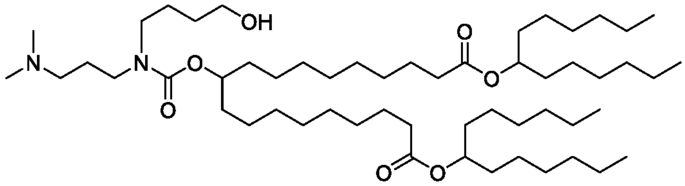 |
| 194 | 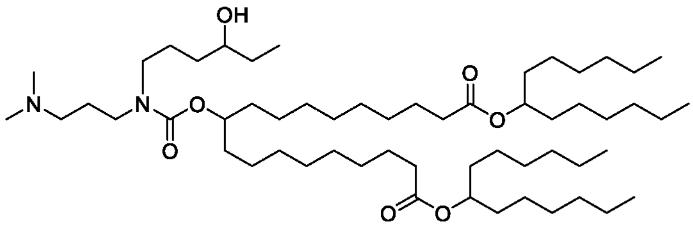 |
| 195 | 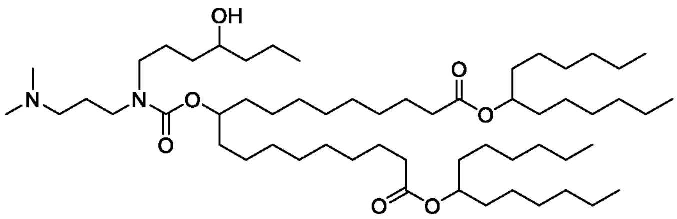 |
| 196 | 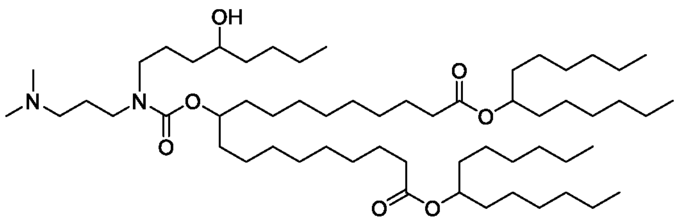 |
| 197 | 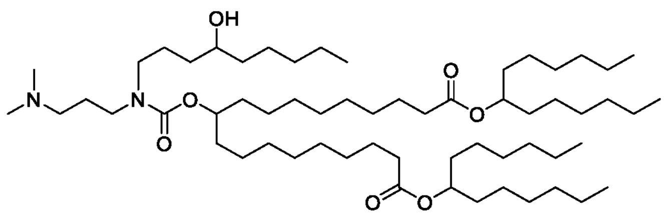 |

(continued)

| No. | Structural formula |
|---|---|
| 198 | 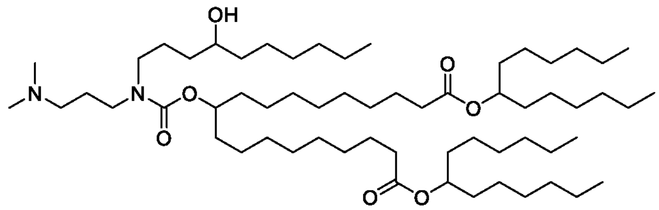 |
| 199 | 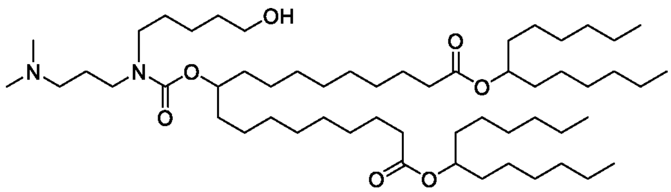 |
| 200 | 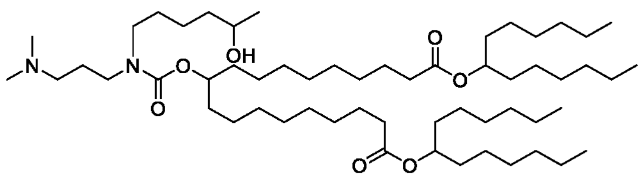 |
| 201 | 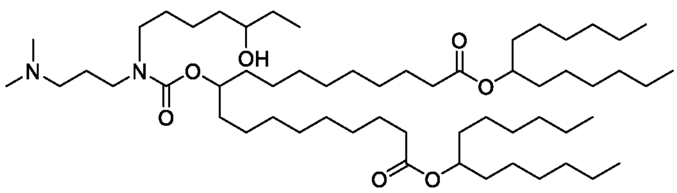 |
| 202 | 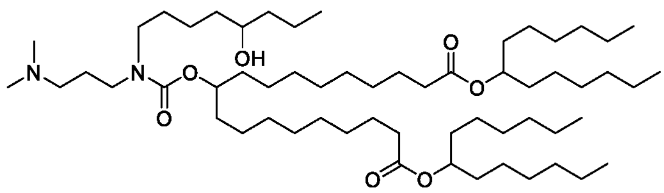 |
| 203 | 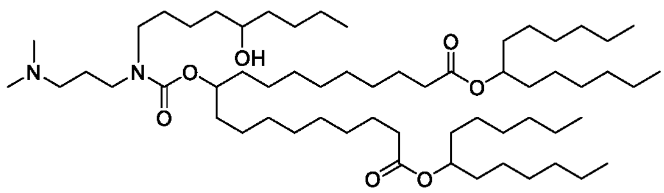 |
| 204 | 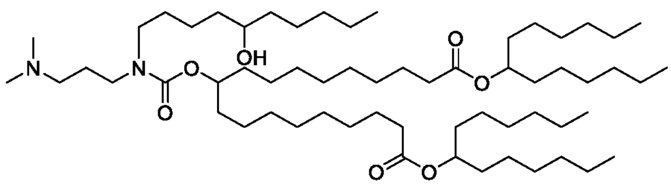 |
| 205 | 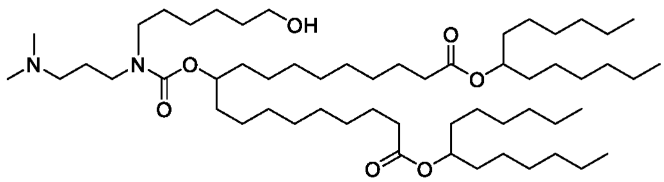 |

(continued)

| No. | Structural formula |
|---|---|
| 206 | 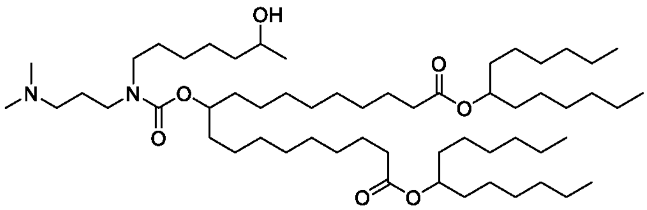 |
| 207 | 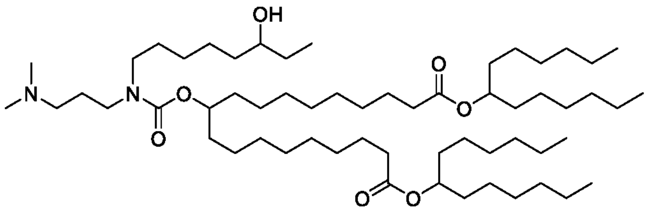 |
| 208 | 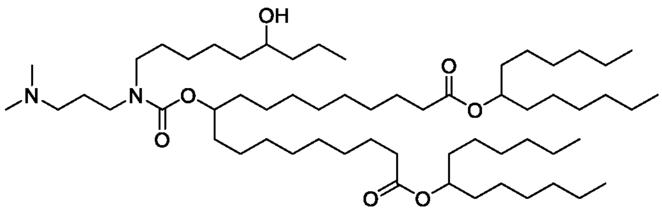 |
| 209 | 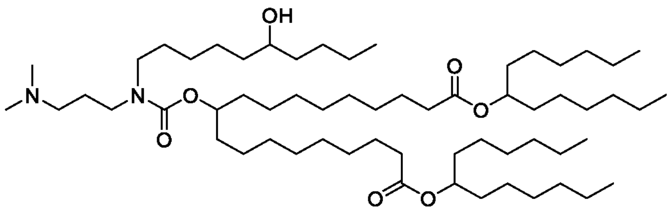 |
| 210 | 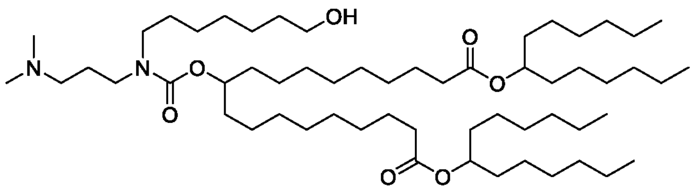 |
| 211 | 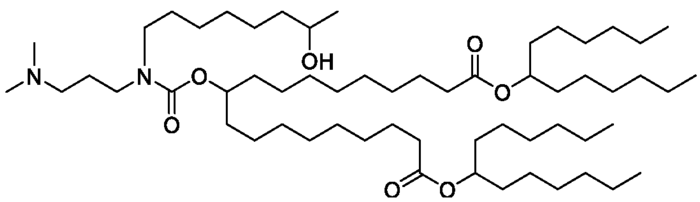 |
| 212 | 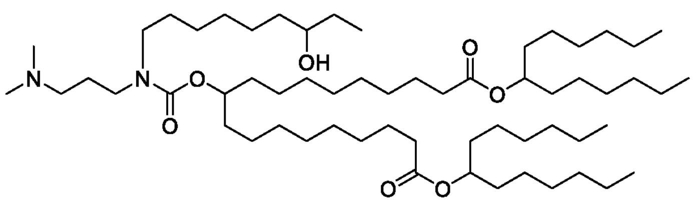 |

(continued)

| No. | Structural formula |
| --- | --- |
| 213 | 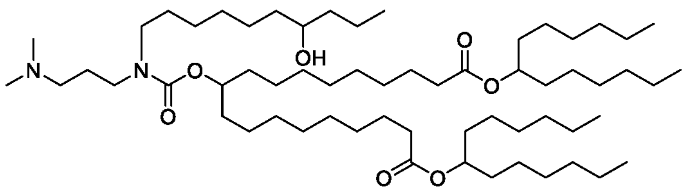 |
| 214 | 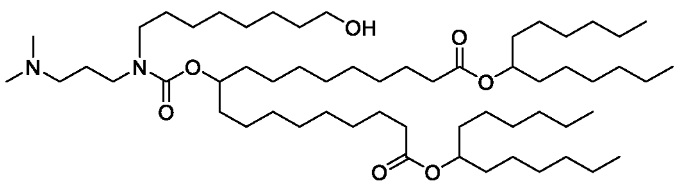 |
| 215 | 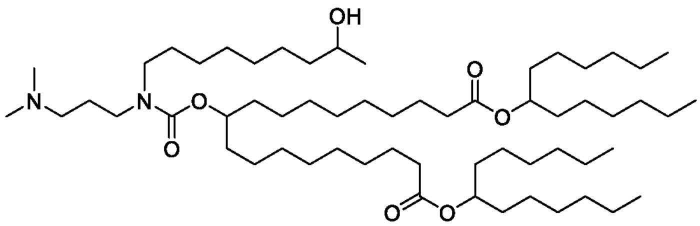 |
| 216 | 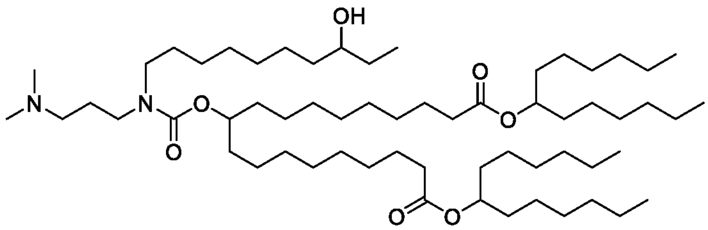 |
| 217 | 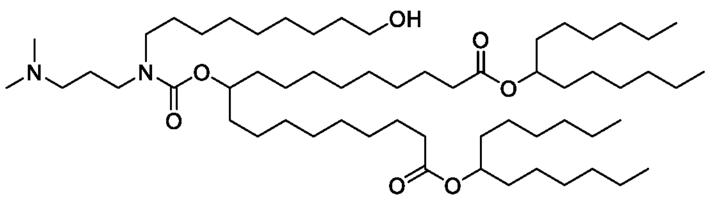 |
| 218 | 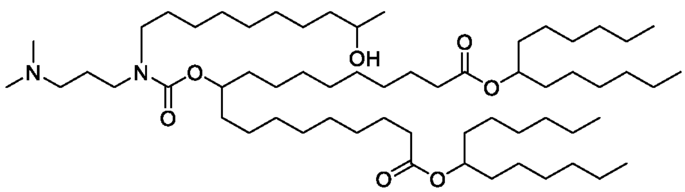 |
| 219 | 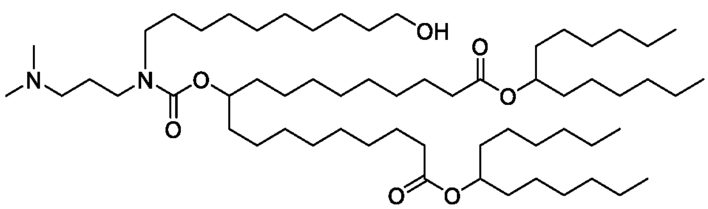 |

(continued)

| No. | Structural formula |
|---|---|
| 220 | 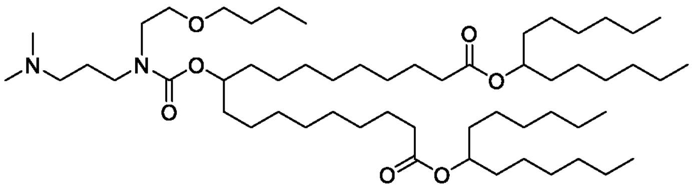 |
| 221 | 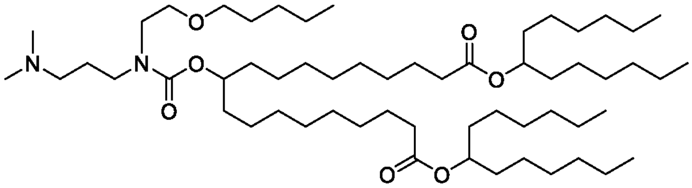 |
| 222 | 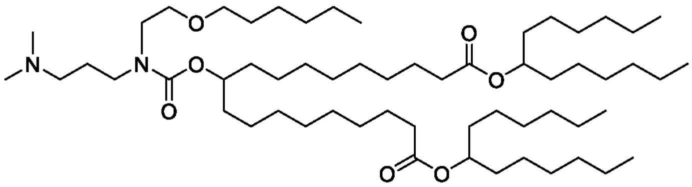 |
| 223 | 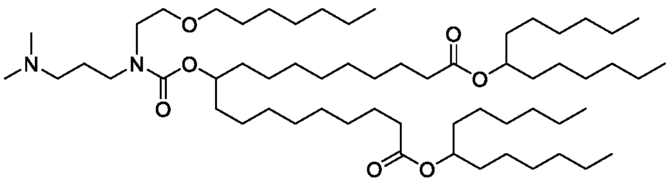 |
| 224 | 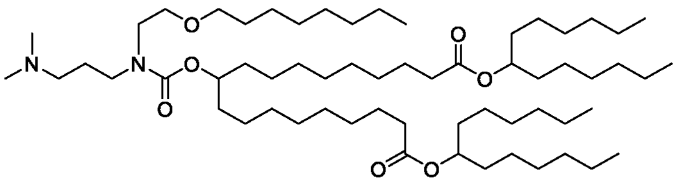 |
| 225 | 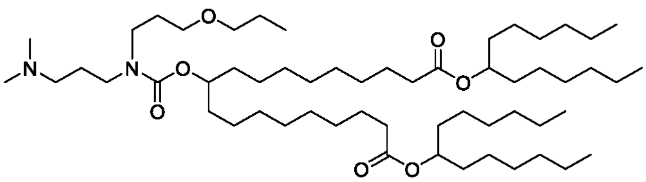 |
| 226 | 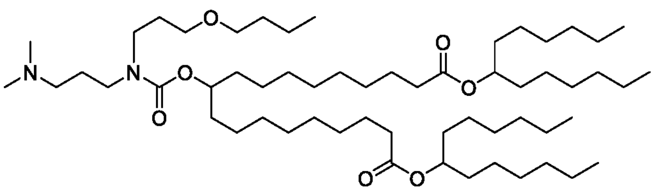 |
| 227 | 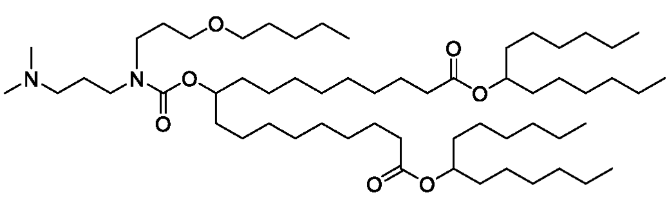 |

(continued)

| No. | Structural formula |
|---|---|
| 228 | 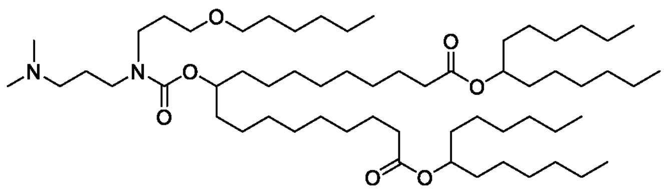 |
| 229 | 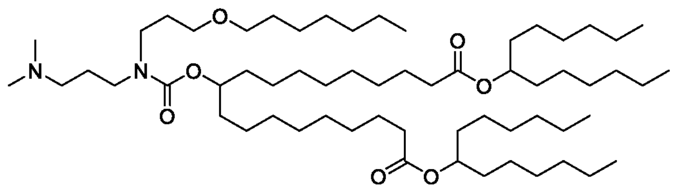 |
| 230 | 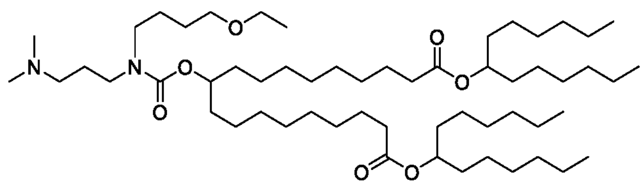 |
| 231 | 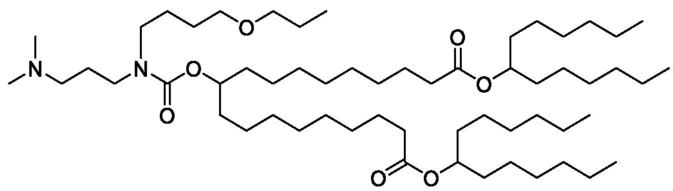 |
| 232 | 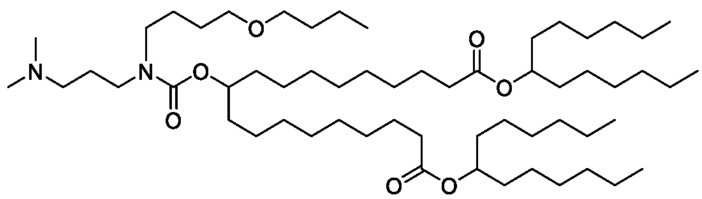 |
| 233 | 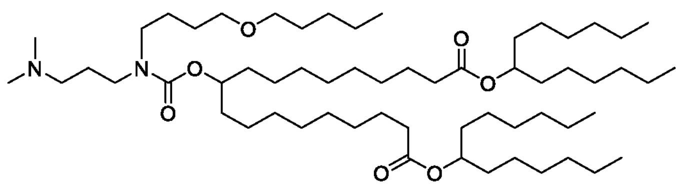 |
| 234 | 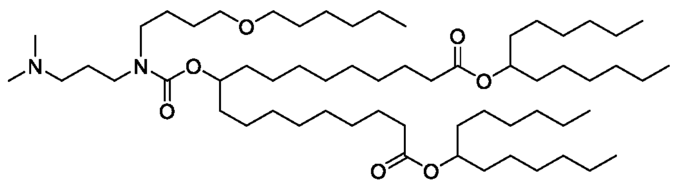 |
| 235 | 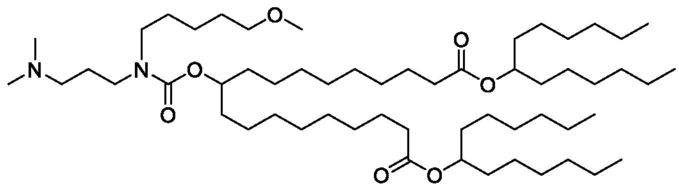 |

(continued)

| No. | Structural formula |
|---|---|
| 236 | 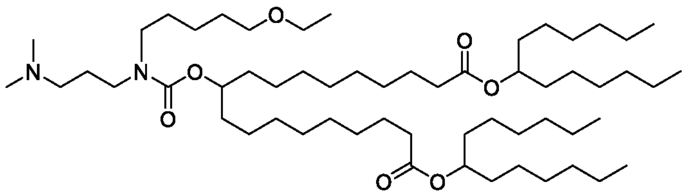 |
| 237 | 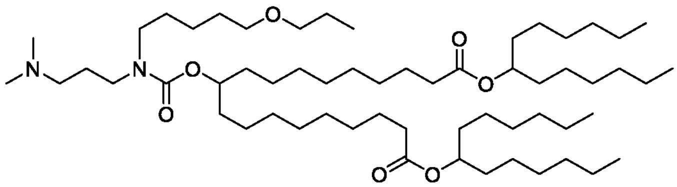 |
| 238 | 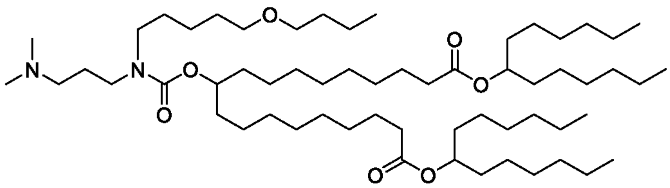 |
| 239 | 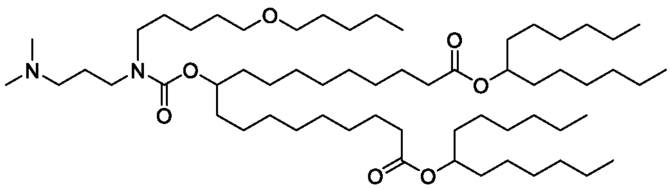 |
| 240 | 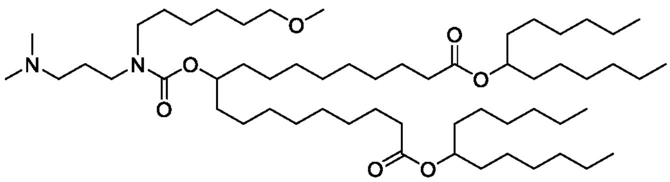 |
| 241 | 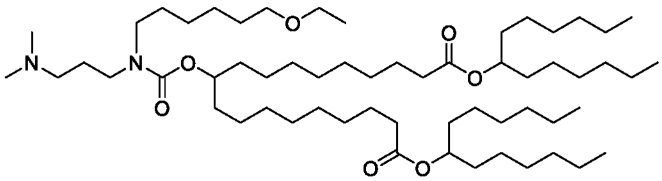 |
| 242 | 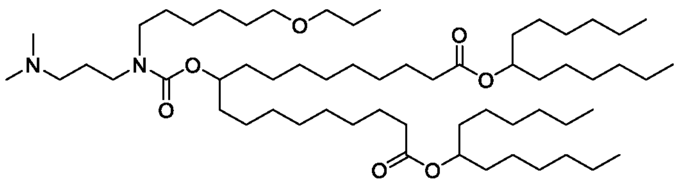 |
| 243 | 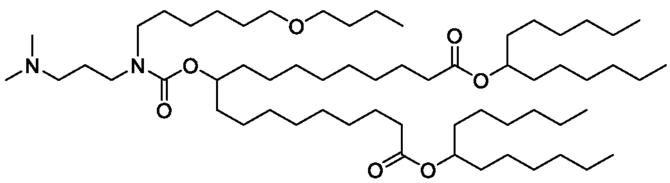 |

(continued)

| No. | Structural formula |
| --- | --- |
| 244 | 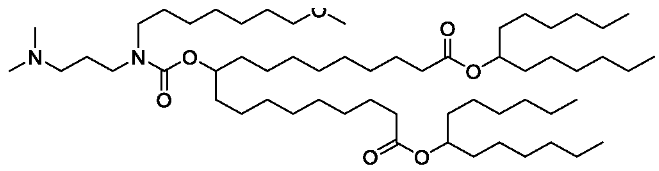 |
| 245 | 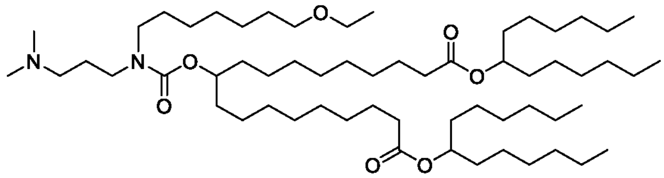 |
| 246 | 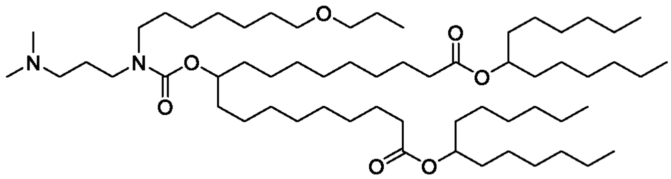 |
| 247 | 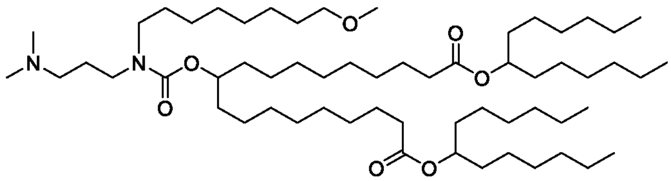 |
| 248 | 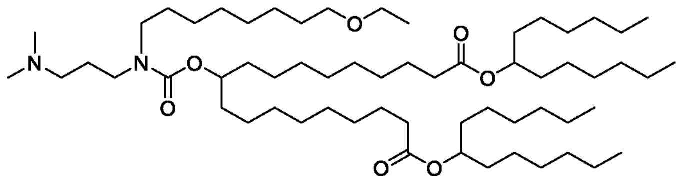 |
| 249 | 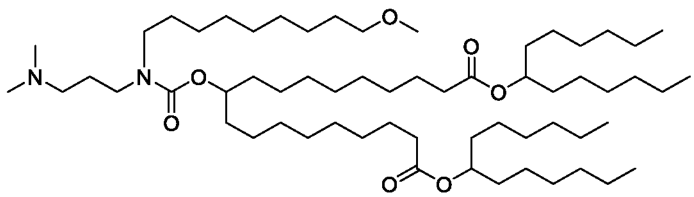 |
| 250 | 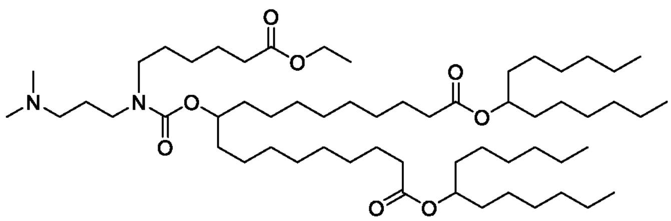 |
| 251 | 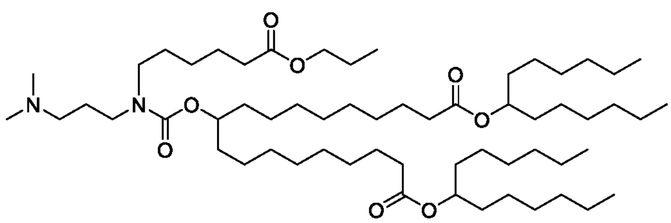 |

(continued)

| No. | Structural formula |
|---|---|
| 252 | 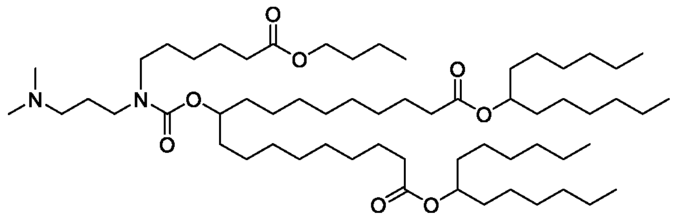 |
| 253 | 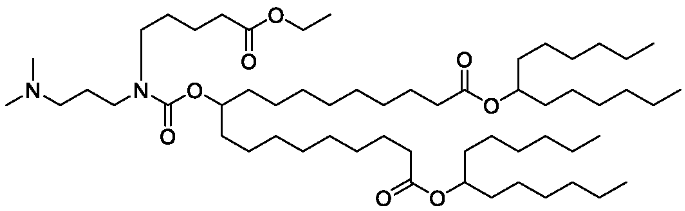 |
| 254 | 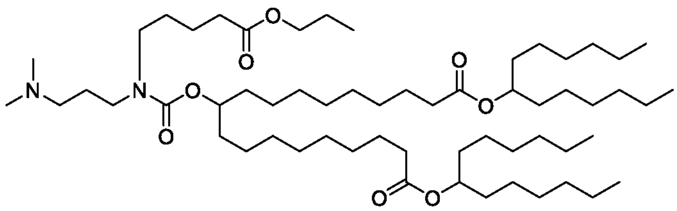 |
| 255 | 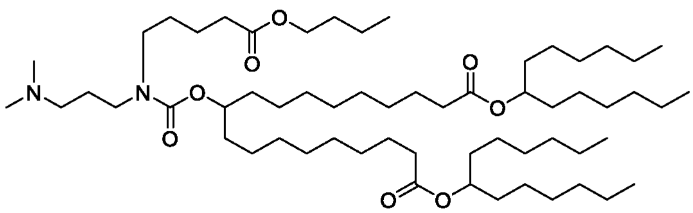 |
| 256 | 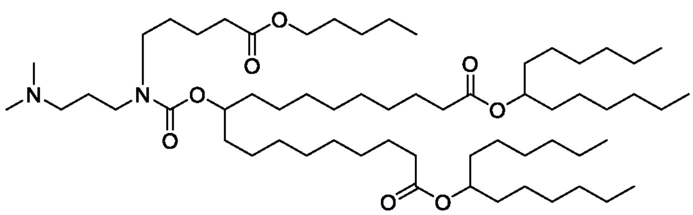 |
| 257 | 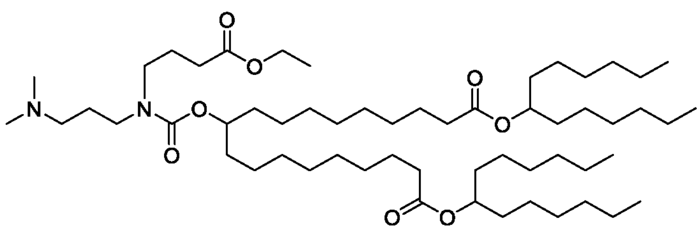 |
| 258 | 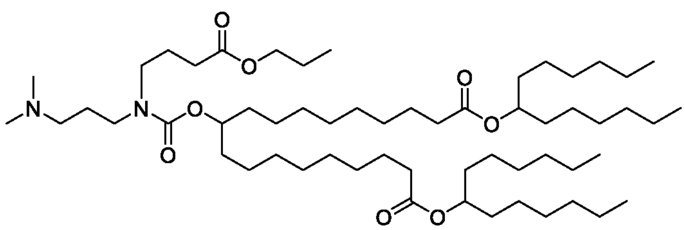 |

(continued)

| No. | Structural formula |
|---|---|
| 259 | 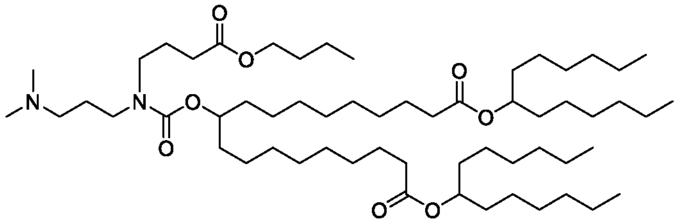 |
| 260 | 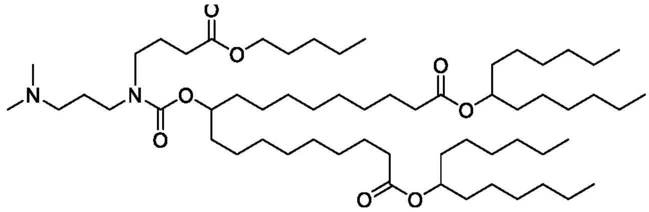 |
| 261 | 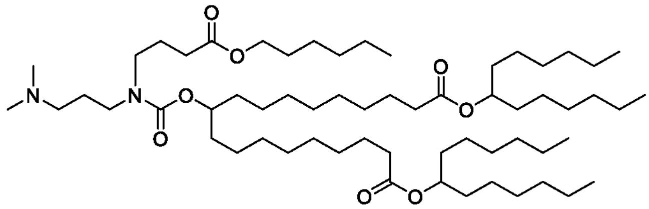 |
| 262 | 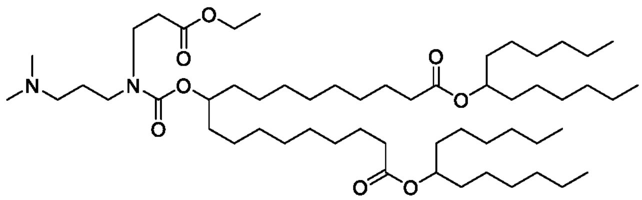 |
| 263 | 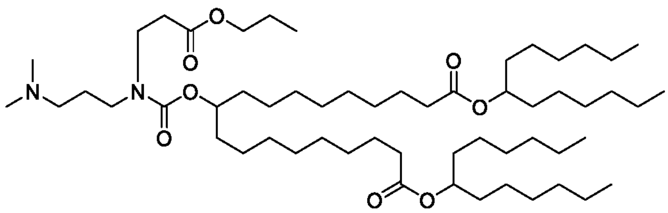 |
| 264 | 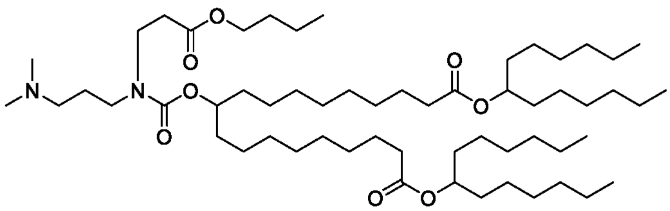 |
| 265 | 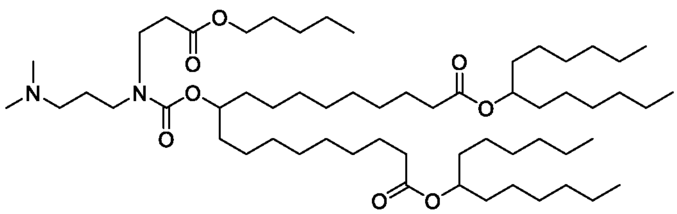 |

(continued)

| No. | Structural formula |
|---|---|
| 266 | 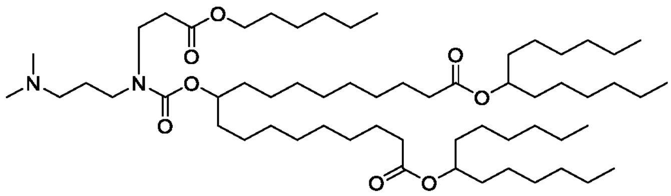 |
| 267 | 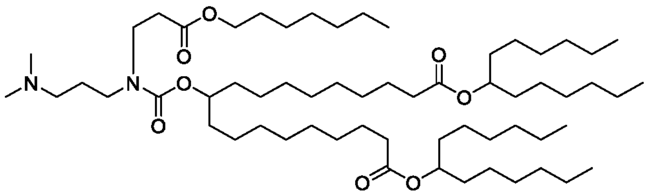 |
| 268 | 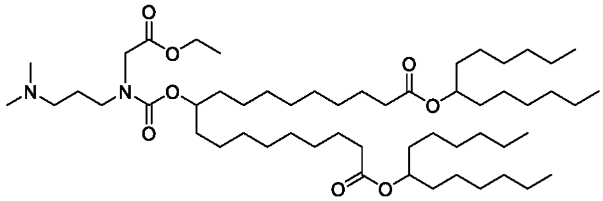 |
| 269 | 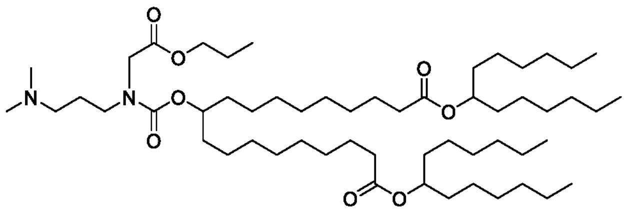 |
| 270 | 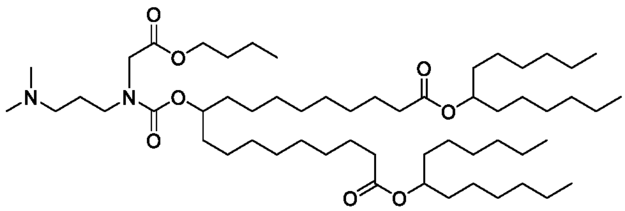 |
| 271 | 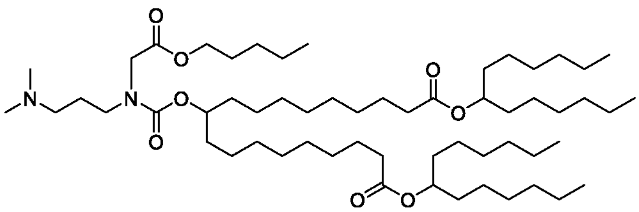 |
| 272 | 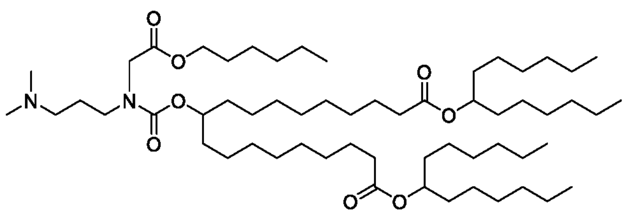 |

(continued)

| No. | Structural formula |
|---|---|
| 273 | 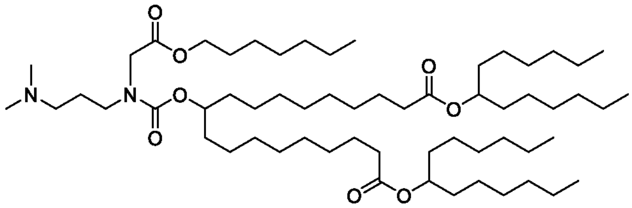 |
| 274 | 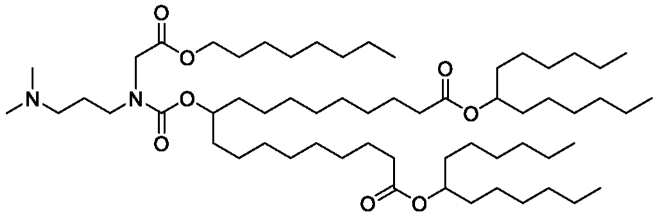 |
| 275 | 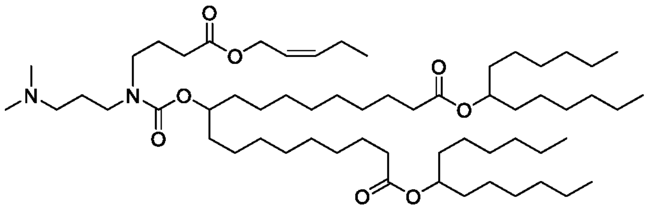 |
| 276 | 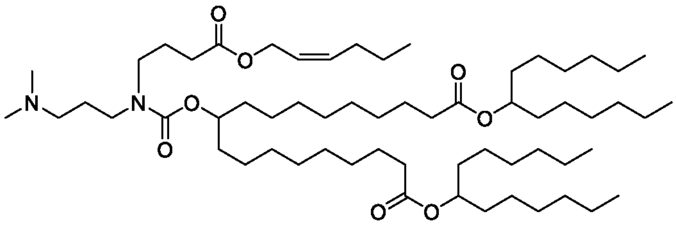 |
| 277 | 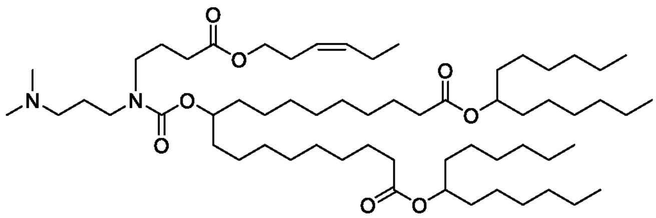 |
| 278 | 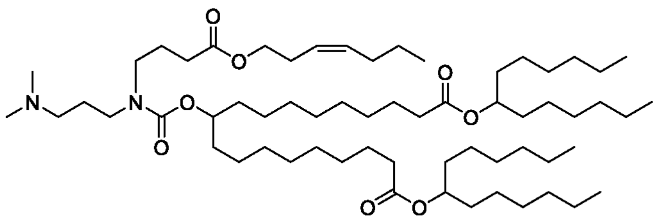 |
| 279 | 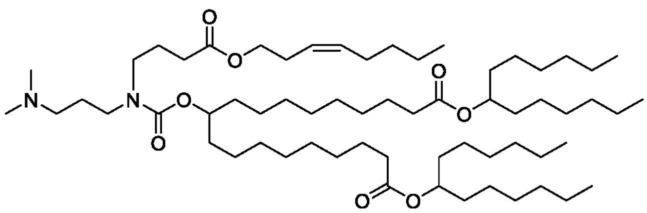 |

(continued)

| No. | Structural formula |
|---|---|
| 280 | 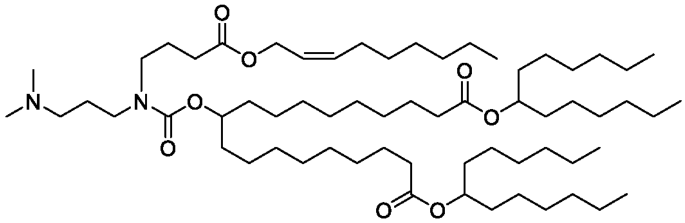 |
| 281 | 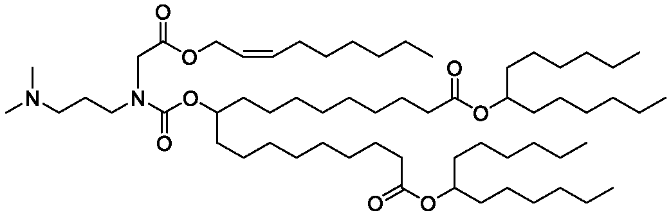 |
| 282 | 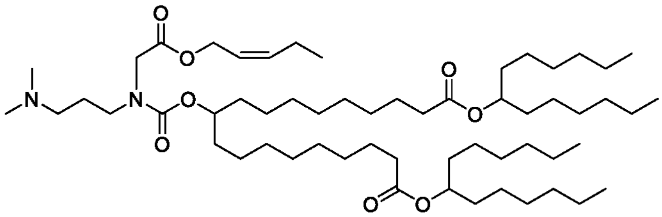 |
| 283 | 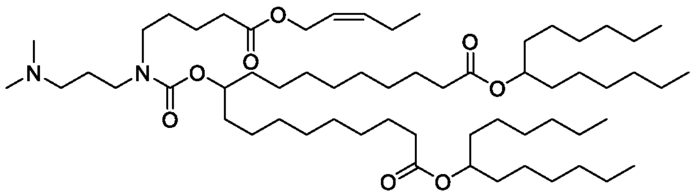 |
| 284 | 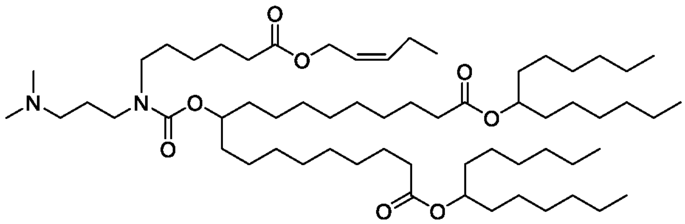 |
| 285 | 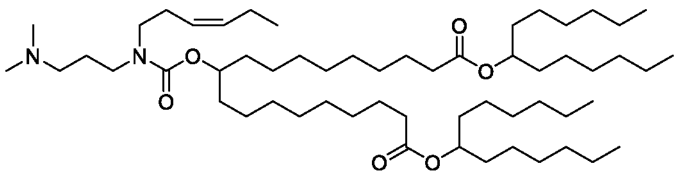 |
| 286 | 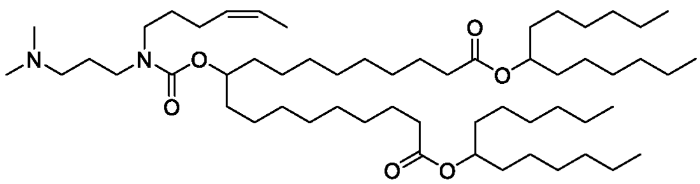 |

(continued)

| No. | Structural formula |
|---|---|
| 287 | 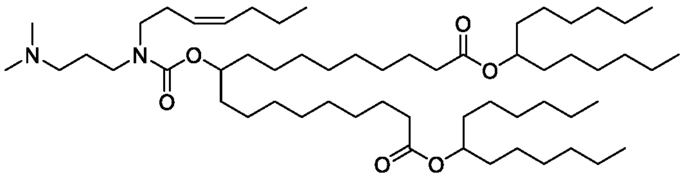 |
| 288 | 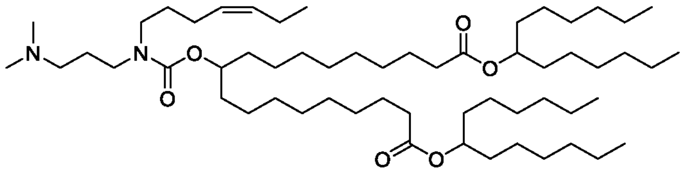 |
| 289 | 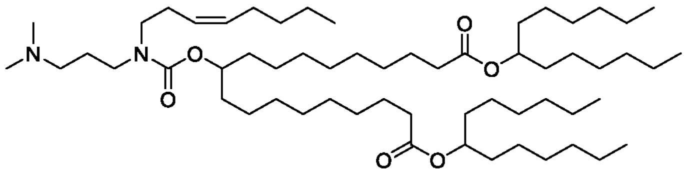 |
| 290 | 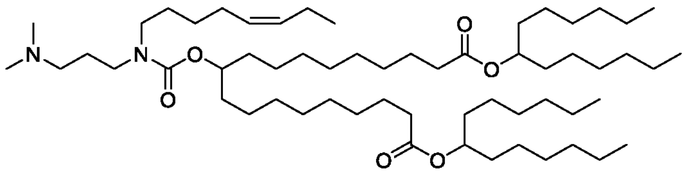 |
| 291 | 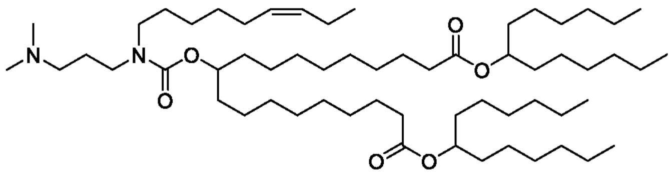 |
| 292 | 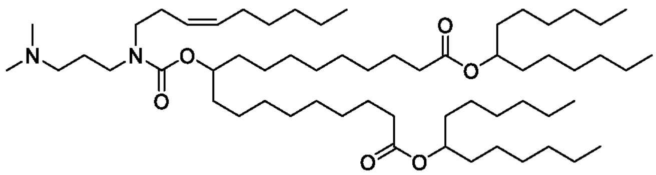 |
| 293 | 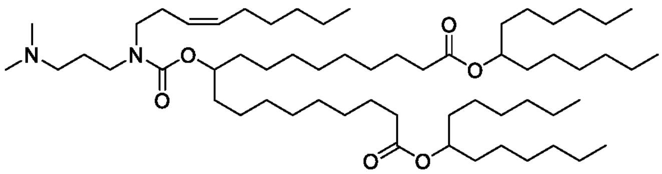 |
| 297 | 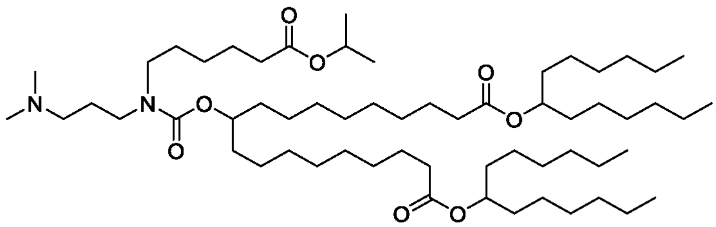 |

(continued)

| No. | Structural formula |
|---|---|
| 298 | 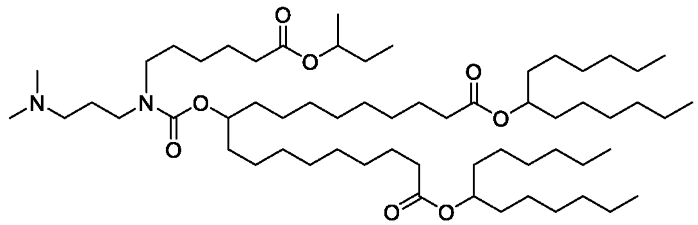 |
| 299 | 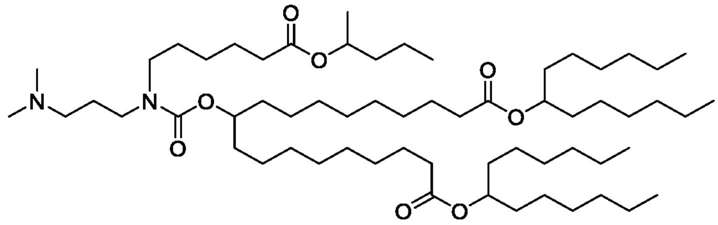 |
| 300 | 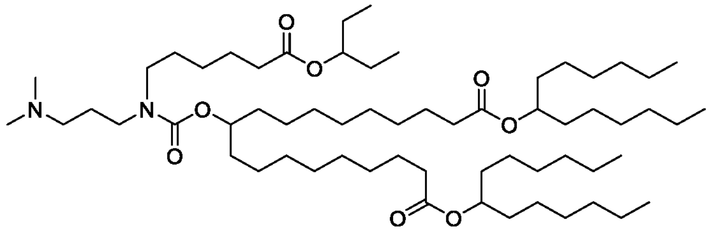 |
| 301 | 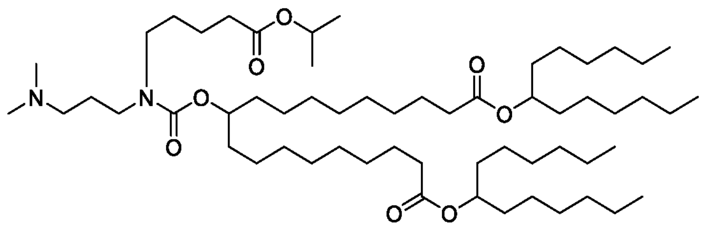 |
| 302 | 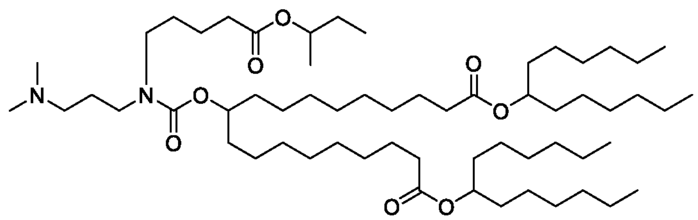 |
| 303 | 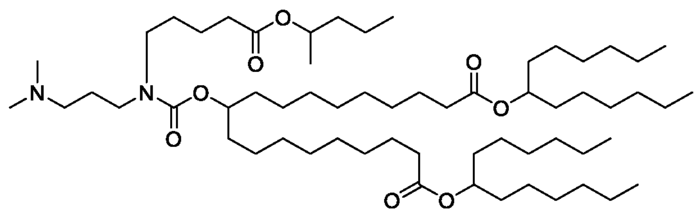 |
| 304 | 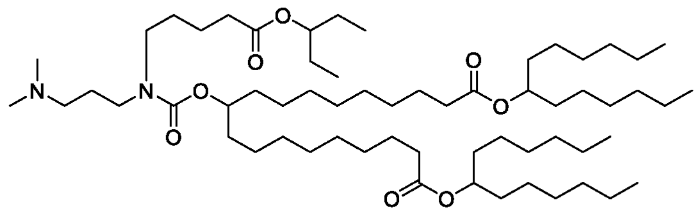 |

(continued)

| No. | Structural formula |
|---|---|
| 305 | 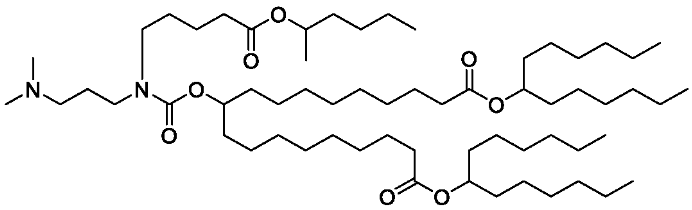 |
| 306 | 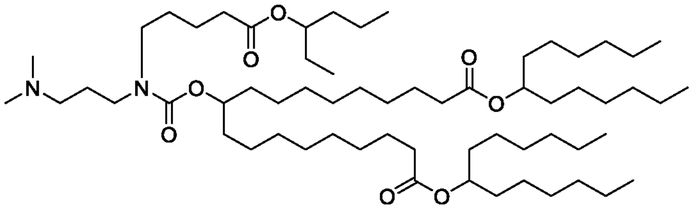 |
| 307 | 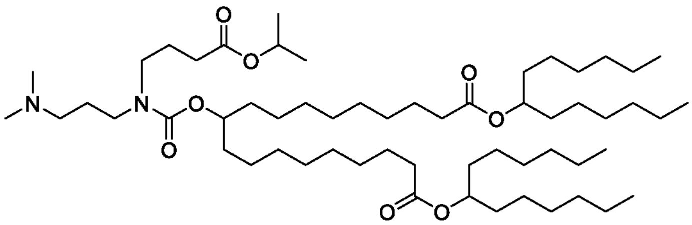 |
| 308 | 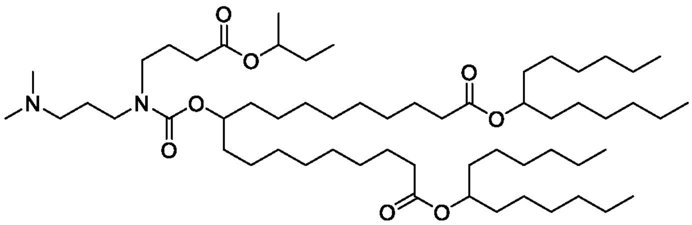 |
| 309 | 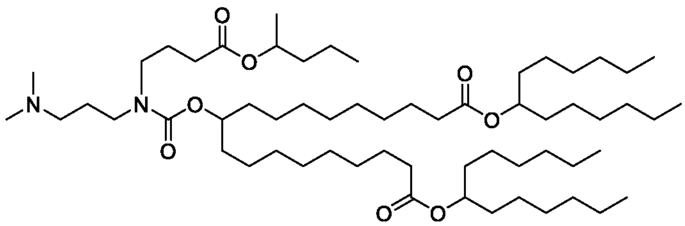 |
| 310 | 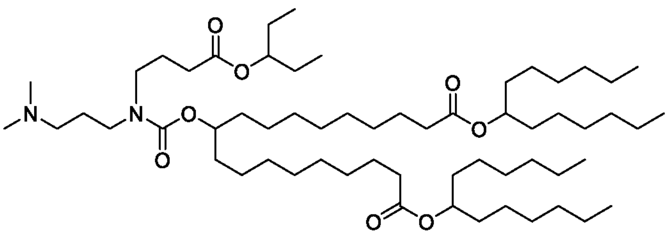 |
| 311 | 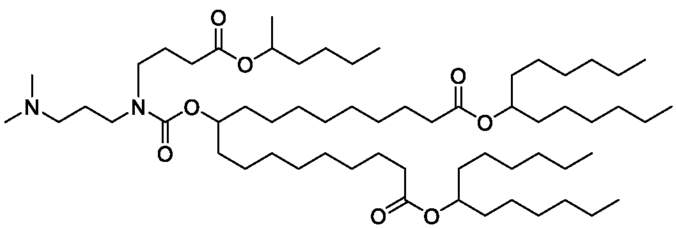 |

(continued)

| No. | Structural formula |
|---|---|
| 312 | 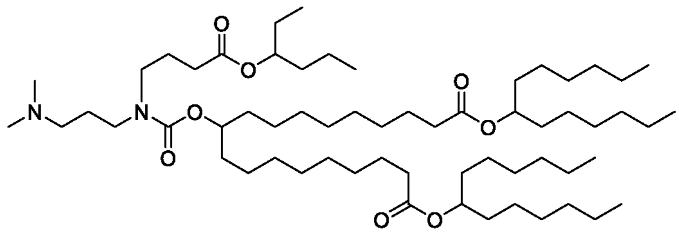 |
| 313 | 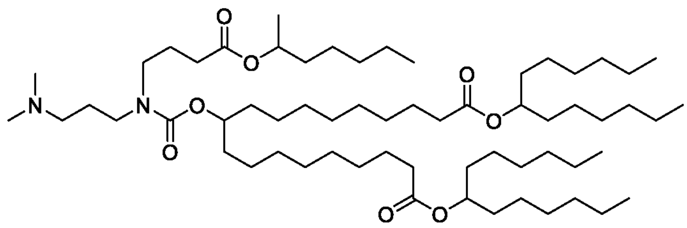 |
| 314 | 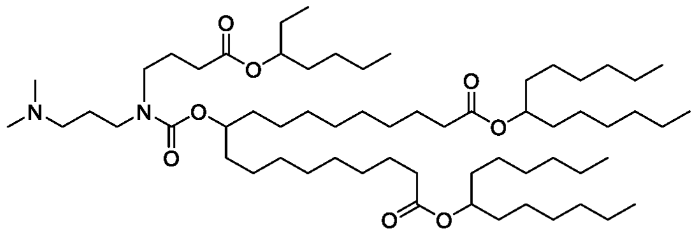 |
| 315 | 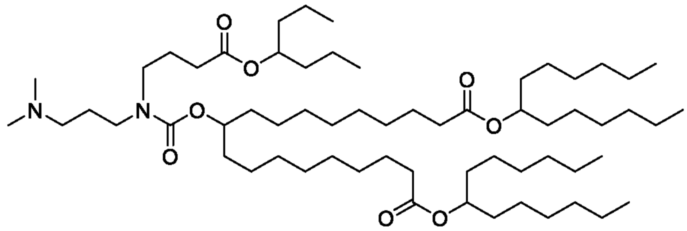 |
| 316 | 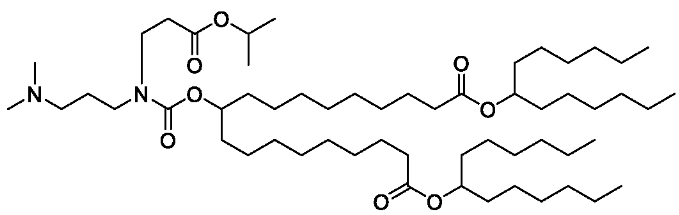 |
| 317 | 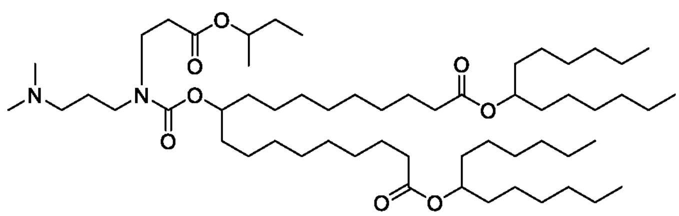 |
| 318 | 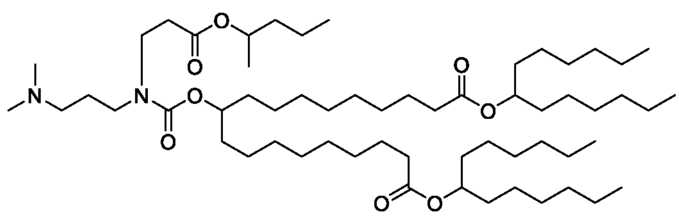 |

(continued)

| No. | Structural formula |
|---|---|
| 319 | 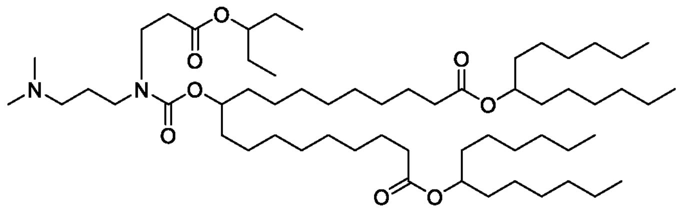 |
| 320 | 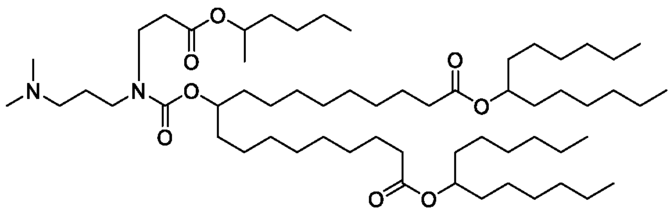 |
| 321 | 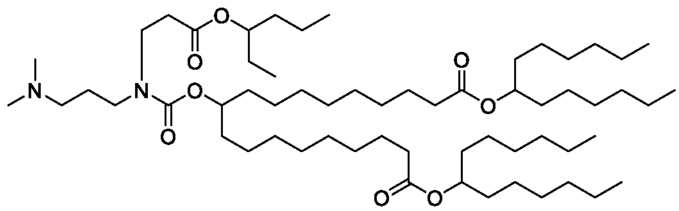 |
| 322 | 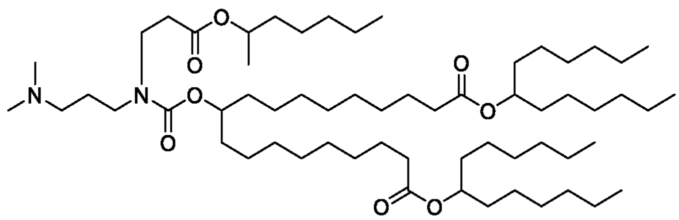 |
| 323 | 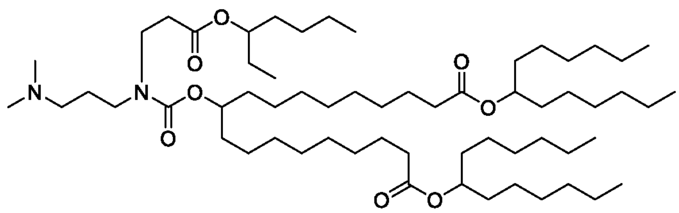 |
| 324 | 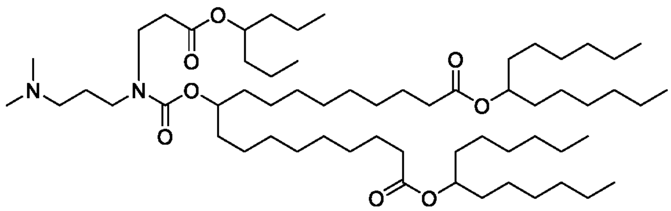 |
| 325 | 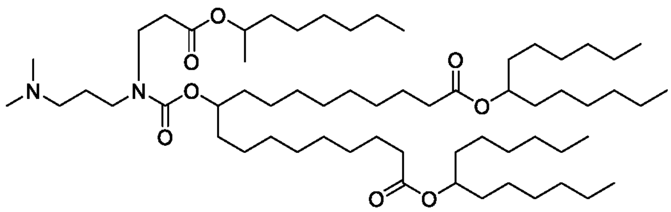 |

(continued)

| No. | Structural formula |
|---|---|
| 326 | 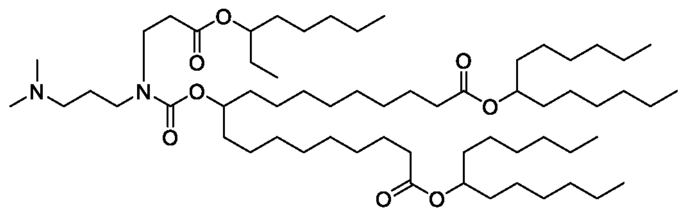 |
| 327 | 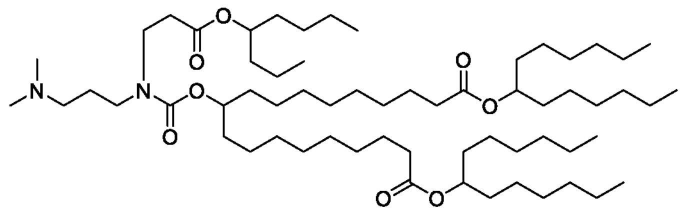 |
| 328 | 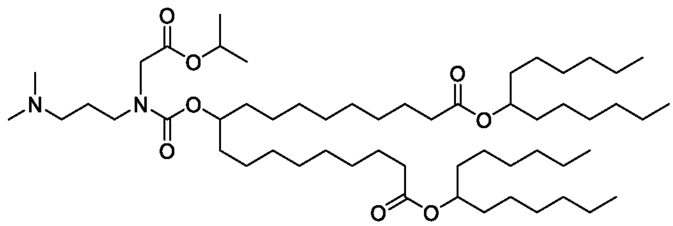 |
| 329 | 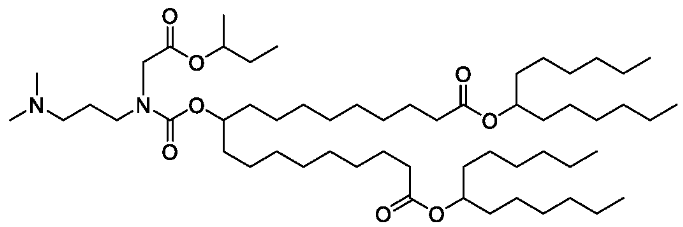 |
| 330 | 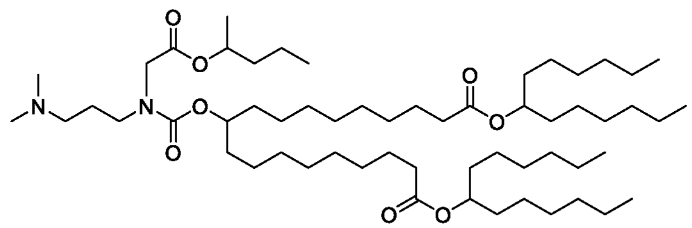 |
| 331 | 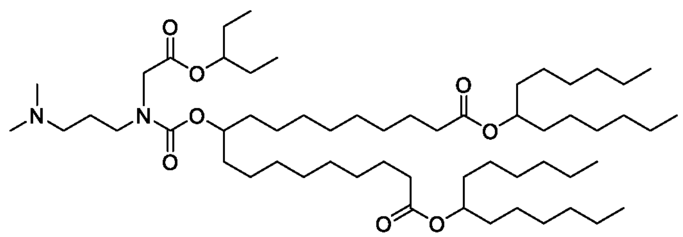 |
| 332 | 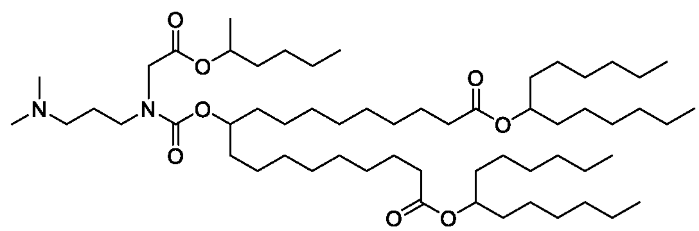 |

(continued)

| No. | Structural formula |
|---|---|
| 333 | 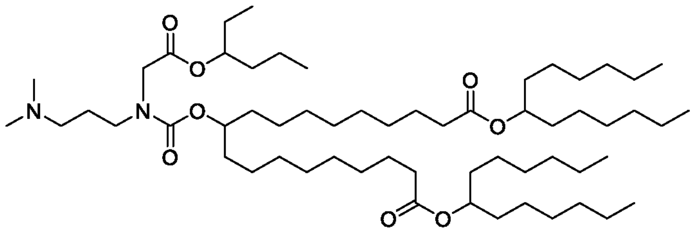 |
| 334 | 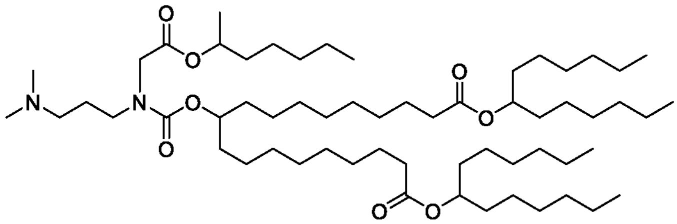 |
| 335 | 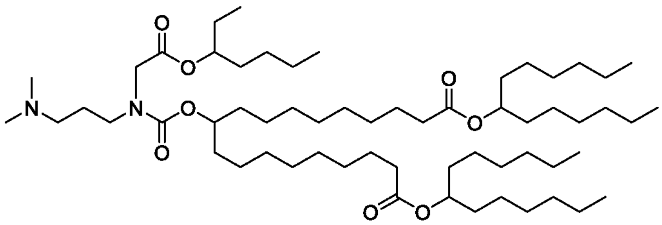 |
| 336 | 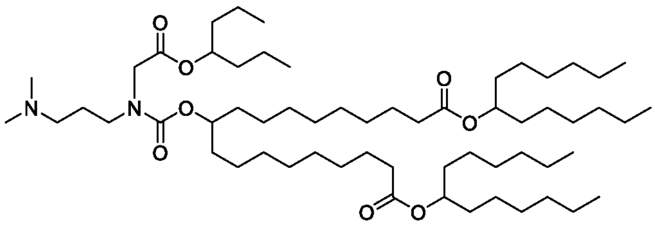 |
| 337 | 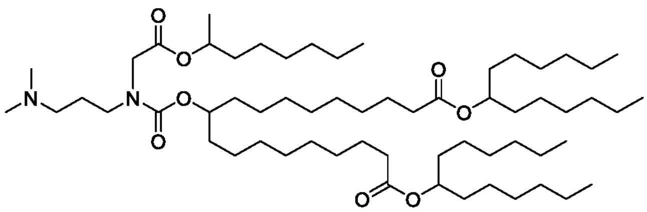 |
| 338 | 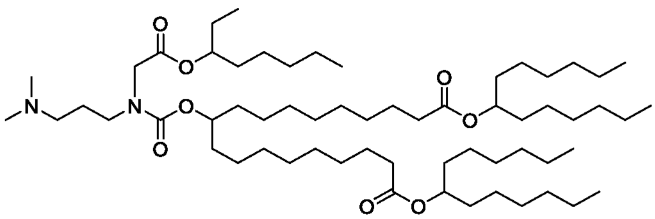 |
| 339 | 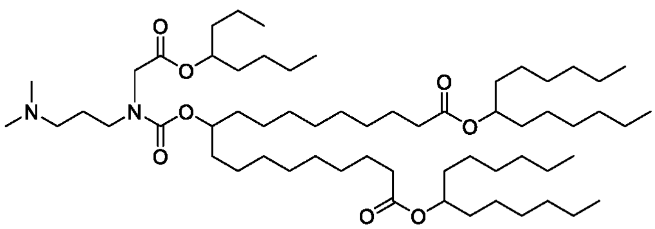 |

(continued)

| No. | Structural formula |
|---|---|
| 340 | 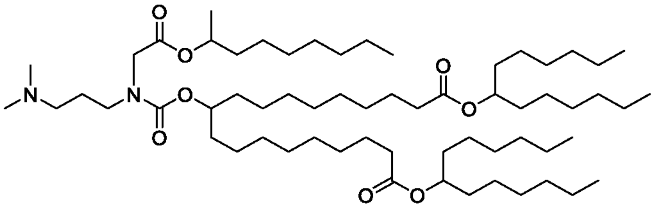 |
| 341 | 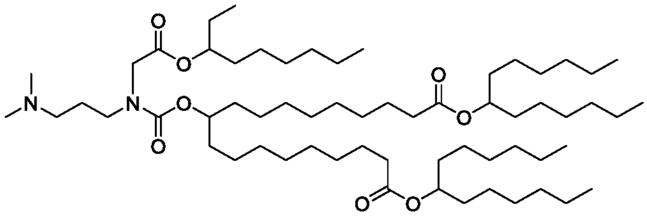 |
| 342 | 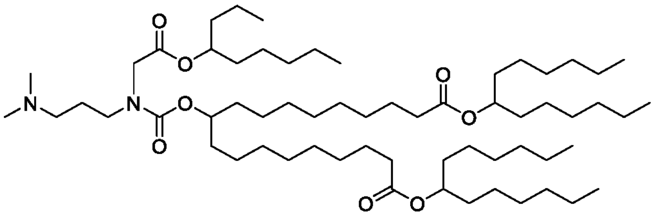 |
| 343 | 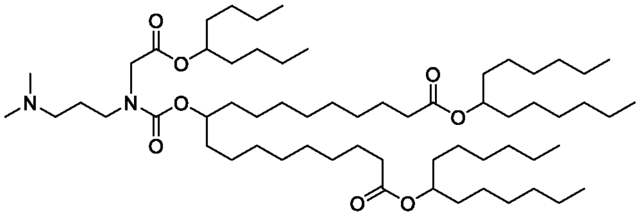 |
| 358 | 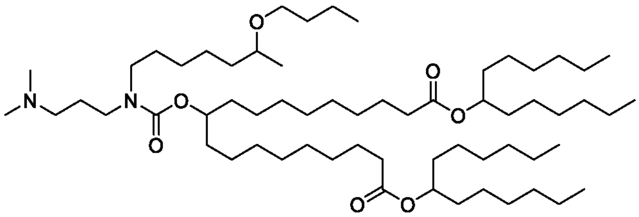 |
| 359 | 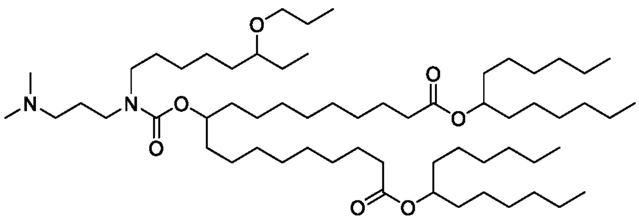 |
| 360 | 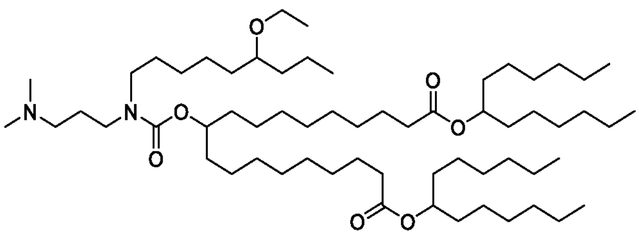 |

(continued)

| No. | Structural formula |
|---|---|
| 361 | 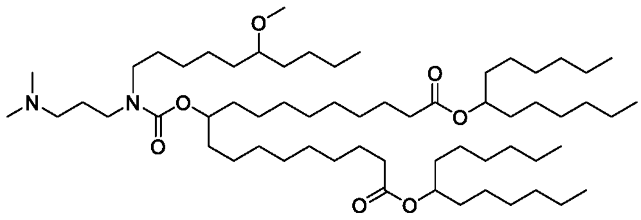 |
| 362 | 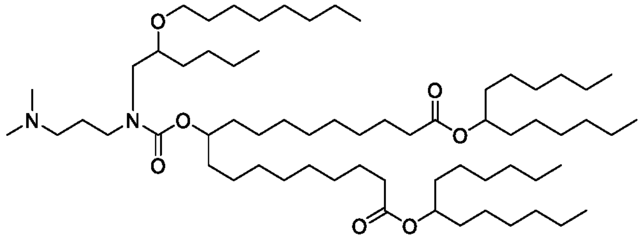 |
| 363 | 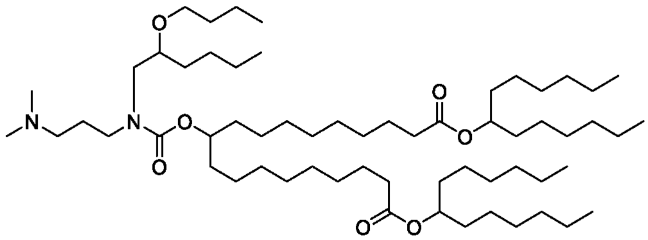 |
| 364 | 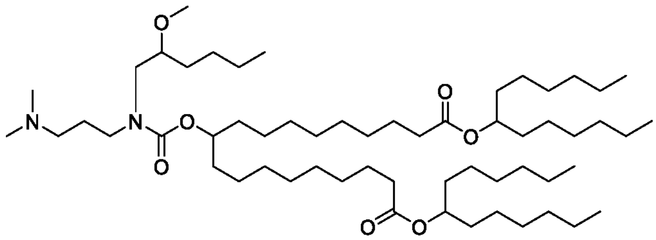 |
| 365 | 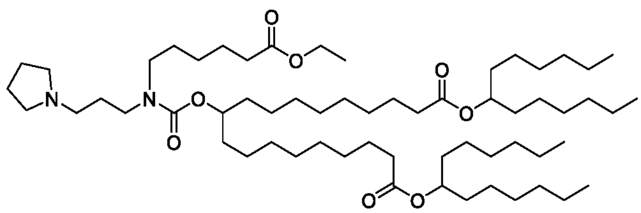 |
| 366 | 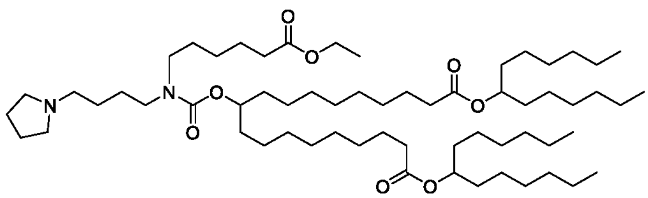 |
| 367 | 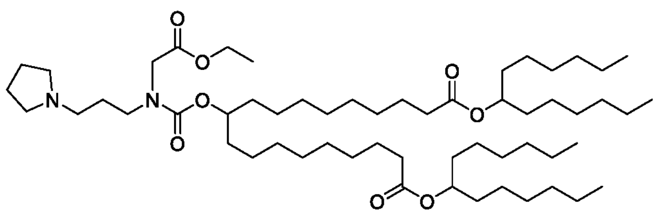 |

(continued)

| No. | Structural formula |
|---|---|
| 368 | 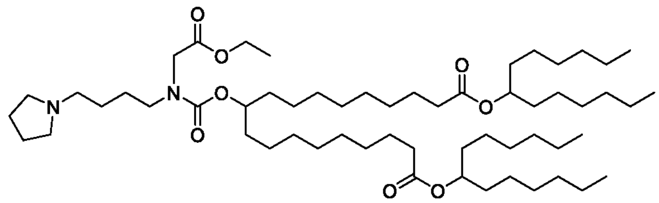 |
| 369 | 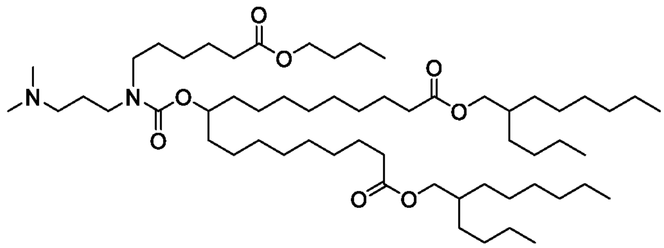 |
| 370 | 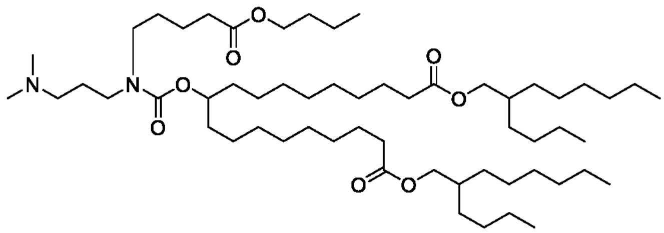 |
| 371 | 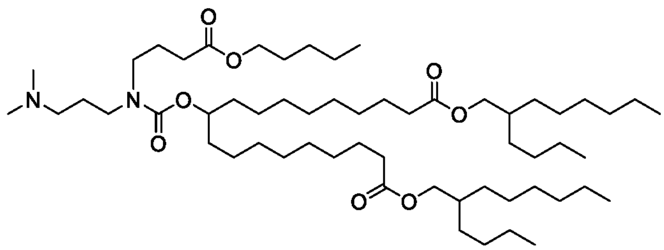 |
| 372 | 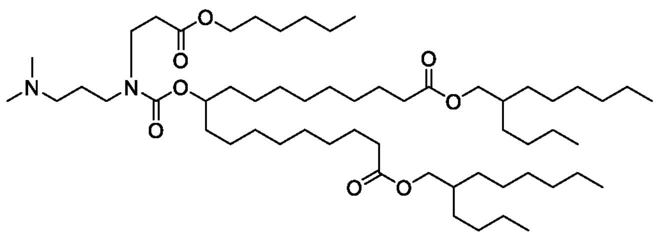 |
| 373 | 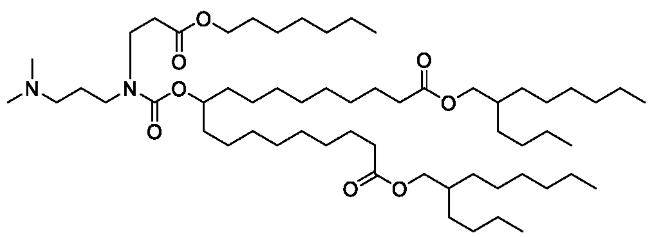 |

(continued)

| No. | Structural formula |
|---|---|
| 374 | 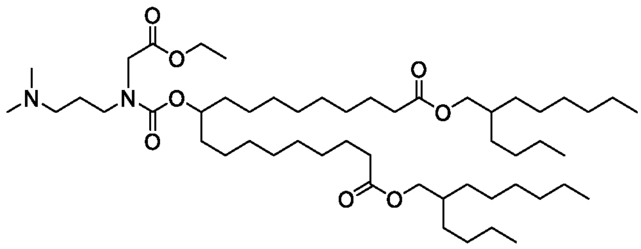 |
| 375 | 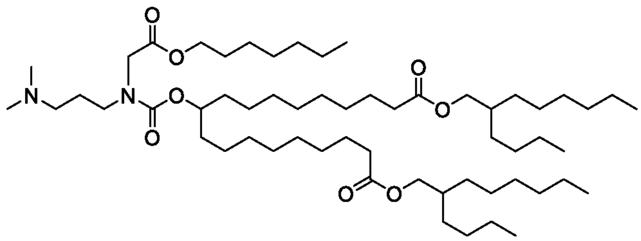 |
| 376 | 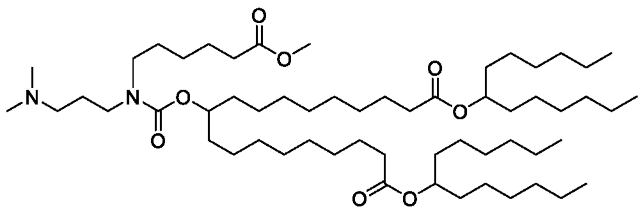 |
| 377 | 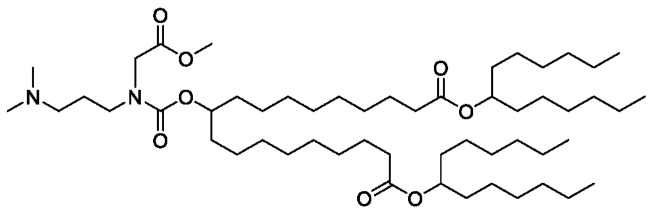 |
| 378 | 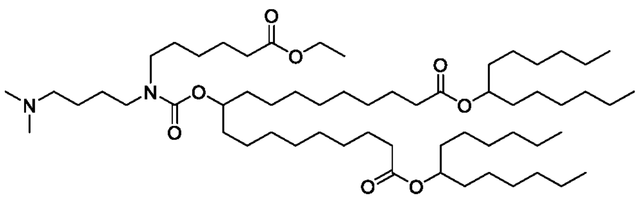 |
| 379 | 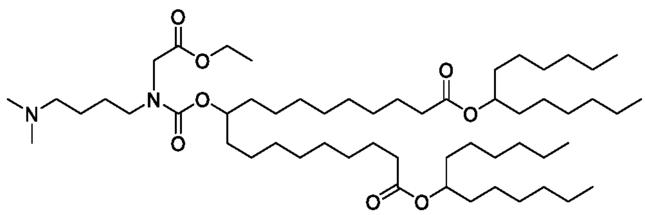 |

**57.** A lipid nanoparticle comprising the amino lipid compound of any one of claims 1 to 56.

**58.** The lipid nanoparticle according to claim 57, wherein the lipid nanoparticle further comprises one or more of a helper lipid, a structural lipid, and a PEG-lipid;
preferably, the lipid nanoparticle further comprises the helper lipid, the structural lipid, and the PEG-lipid.

**59.** The lipid nanoparticle according to claim 58, wherein the lipid nanoparticle comprises the amino lipid compound of any

one of claims 1 to 56 in an amount (molar percent) of about 25.0% to 75.0%, such as about 25.0%-28.0%, 28.0%-32.0%, 32.0%-35.0%, 35.0%-40.0%, 40.0%-42.0%, 42.0%-45.0%, 45.0%-46.3%, 46.3%-48.0%, 48.0%-49.5%, 49.5%-50.0%, 50.0%-55.0%, 55.0%-60.0%, 60.0%-65.0%, or 65.0%-75.0%, based on the total amount of the amino lipid compound of any one of claims 1 to 56, the helper lipid, the structural lipid, and the PEG-lipid.

**60.** The lipid nanoparticle according to any one of claim 58 or 59, wherein the lipid nanoparticle comprises the helper lipid in an amount (molar percent) of about 5.0% to 45.0%, such as about 5.0%-9.0%, 9.0%-9.4%, 9.4%-10.0%, 10.0%-10.5%, 10.5%-11.0%, 11.0%-15.0%, 15.0%-16.0%, 16.0%-18.0%, 18.0%-20.0%, 20.0%-25.0%, 25.0%-33.5%, 33.5%-37.0%, 37.0%-40.0%, 40.0%-42.0%, or 42.0%-45.0%, based on the total amount of the amino lipid compound of any one of claims 1 to 56, the helper lipid, the structural lipid, and the PEG-lipid.

**61.** The lipid nanoparticle according to any one of claims 58 to 60, wherein the lipid nanoparticle comprises the structural lipid in an amount (molar percent) of about 0.0% to 50.0%, such as about 0.0%-10.0%, 10.0%-15.5%, 15.5%-18.5%, 18.5%-22.5%, 22.5%-23.5%, 23.5%-28.5%, 28.5%-33.5%, 33.5%-35.0%, 35.0%-36.5%, 36.5%-38.0%, 38.0%-38.5%, 38.5%-39.0%, 39.0%-39.5%, 39.5%-40.5%, 40.5%-41.5%, 41.5%-42.5%, 42.5%-42.7%, 42.7%-43.0%, 43.0%-43.5%, 43.5%-45.0%, 45.0%-46.5%, 46.5%-48.5%, or 46.5%-50.0%, based on the total amount of the amino lipid compound of any one of claims 1 to 56, the helper lipid, the structural lipid, and the PEG-lipid.

**62.** The lipid nanoparticle according to any one of claims 58 to 61, wherein the lipid nanoparticle comprises the PEG-lipid in an amount (molar percent) of about 0.5% to 5.0%, such as about 0.5%-1.0%, 1.0%-1.5%, 1.5%-1.6%, 1.6%-2.0%, 2.0%-2.5%, 2.5%-3.0%, 3.0%-3.5%, 3.5%-4.0%, 4.0%-4.5%, or 4.5%-5.0%, based on the total amount of the amino lipid compound of any one of claims 1 to 56, the helper lipid, the structural lipid, and the PEG-lipid.

**63.** The lipid nanoparticle according to any one of claims 58 to 62, wherein the helper lipid is one or more selected from the group consisting of DSPC, DOPE, DOPC, DOPS, DSPG, DPPG, DPPC, DGTS and lysophospholipid, preferably one or more selected from the group consisting of DSPC, DOPE, DOPC and DOPS, and more preferably DSPC and/or DOPE.

**64.** The lipid nanoparticle according to any one of claims 58 to 63, wherein the structural lipid is at least one selected from the group consisting of cholesterol, cholesterol ester, sterol hormone, sterol vitamin, bile acid, cholesterin, ergosterol, β-sitosterol, and oxidized cholesterol derivative, preferably at least one selected from the group consisting of cholesterol, cholesteryl ester, sterol hormone, sterol vitamin and bile acid, more preferably cholesterol, even more preferably high purity cholesterol, especially injection grade high purity cholesterol such as CHO-HP.

**65.** The lipid nanoparticle according to any one of claims 58 to 64, wherein the PEG-lipid is selected from the group consisting of PEG-DMG and PEG-DSPE, preferably PEG-DMG;

preferably, the PEG-DMG is a polyethylene glycol (PEG) derivative of 1,2-dimyristoyl-sn-glycerol;
preferably, the PEG has an average molecular weight of about 2,000 to 5,000, preferably about 2,000.

**66.** The lipid nanoparticle according to any one of claims 58 to 65, wherein the molar ratio of the amino lipid compound of any one of claims 1 to 56: helper lipid: structural lipid: PEG-lipid is about 45 : 10 : 42.5 : 2.5, or 45 : 11 : 41.5 : 2.5, or 42.0 : 10.5 : 45.0 : 2.5, or 42.0 : 16.0 : 39.5 : 2.5, or 40.0 : 16.0 : 41.5 : 2.5, or 40.0 : 18.0 : 39.5 : 2.5, or 35.0 : 16.0 : 46.5 : 2.5, or 35.0 : 25.0 : 36.5 : 3.5, or 28.0 : 33.5 : 35.0 : 3.5, or 32.0 : 37.0 : 40.5 : 0.5, or 35.0 : 40.0 : 22.5 : 2.5, or 40.0 : 42.0 : 15.5 : 2.5, or 40.0 : 20.0 : 38.5 : 1.5, or 45.0 : 15.0 : 38.5 : 1.5, or 55.0 : 5.0 : 38.5 : 1.5, or 60.0 : 5.0 : 33.5 : 1.5, or 45.0 : 20.0 : 33.5 : 1.5, or 50.0 : 20.0 : 28.5 : 1.5, or 55.0 : 20.0 : 23.5 : 1.5, or 60.0 : 20.0 : 18.5 : 1.5, or 40.0 : 15.0 : 43.5 : 1.5, or 50.0 : 15.0 : 33.5 : 1.5, or 55.0 : 15.0 : 28.5 : 1.5, or 60.0 : 15.0 : 23.5 : 1.5, or 40.0 : 10.0 : 48.5 : 1.5, or 45.0 : 10.0 : 43.5 : 1.5, or 55.0 : 10.0 : 33.5 : 1.5, or 40.0 : 5.0 : 53.5 : 1.5, or 45.0 : 5.0 : 48.5 : 1.5, or 50.0 : 5.0 : 43.5 : 1.5.

**67.** The lipid nanoparticle according to claim 66, wherein the helper lipid is DOPE, and the structural lipid is CHO-HP.

**68.** The lipid nanoparticle according to any one of claims 58 to 65, wherein the molar ratio of the amino lipid compound of any one of claims 1 to 56: helper lipid: structural lipid: PEG-lipid is about 50.0 : 10.0 : 38.5 : 1.5, or 50.0 : 9.0 : 38.0 : 3.0, or 49.5 : 10.0 : 39.0 : 1.5, or 48.0 : 10.0 : 40.5 : 1.5, or 46.3 : 9.4 : 42.7 : 1.6, or 45.0 : 9.0 : 43.0 : 3.0, or 45.0 : 11.0 : 41.5 : 2.5, or 42.0 : 10.5 : 45.0 : 2.5, or 42.0 : 16.0 : 39.5 : 2.5, or 40.0 : 16.0 : 41.5 : 2.5, or 40.0 : 18.0 : 39.5 : 2.5, or 35.0 : 40.0 : 22.5 : 2.5, or 40.0 : 20.0 : 38.5 : 1.5, or 45.0 : 15.0 : 38.5 : 1.5, or 55.0 : 5.0 : 38.5 : 1.5, or 60.0 : 5.0 : 33.5 : 1.5, or 45.0 :

20.0 : 33.5 : 1.5, or 50.0 : 20.0 : 28.5 : 1.5, or 55.0 : 20.0 : 23.5 : 1.5, or 60.0 : 20.0 : 18.5 : 1.5, or 40.0 : 15.0 : 43.5 : 1.5, or 50.0 : 15.0 : 33.5 : 1.5, or 55.0 : 15.0 : 28.5 : 1.5, or 60.0 : 15.0 : 23.5 : 1.5, or 40.0 : 10.0 : 48.5 : 1.5, or 45.0 : 10.0 : 43.5 : 1.5, or 55.0 : 10.0 : 33.5 : 1.5, or 40.0 : 5.0 : 53.5 : 1.5, or 45.0 : 5.0 : 48.5 : 1.5, or 50.0 : 5.0 : 43.5 : 1.5.

**69.** The lipid nanoparticle according to claim 68, wherein the helper lipid is DSPC, and the structural lipid is CHO-HP.

**70.** The lipid nanoparticle according to any one of claims 57 to 69, further comprising a biologically active ingredient; preferably, the biologically active ingredient is a nucleic acid.

**71.** The lipid nanoparticle according to claim 70, wherein the mass ratio of the amino lipid compound of any one of claims 1 to 56 to the nucleic acid is about (5-30): 1, such as about (5-10): 1, (10-15): 1, (15-20): 1, (20-25): 1, or (25-30): 1, preferably about 10:1.

**72.** The lipid nanoparticle according to claim 70 or 71, wherein the nucleic acid is selected from the group consisting of RNA, antisense oligonucleotide, and DNA;

preferably, the RNA is selected from the group consisting of messenger RNA (mRNA), ribosomal RNA (rRNA), microRNA (miRNA), transfer RNA (tRNA), small interfering RNA (siRNA), small nuclear RNA (snRNA), small hairpin RNA (shRNA), single guide RNA (sgRNA), Cas9 mRNA, or a mixture thereof;
preferably, the DNA is a plasmid.

**73.** A pharmaceutical composition comprising the amino lipid compound of any one of claims 1 to 56 or the lipid nanoparticle of any one of claims 57 to 72, and a pharmaceutically acceptable carrier, diluent or excipient.

**74.** The pharmaceutical composition according to claim 73, further comprising a buffer;

preferably, the buffer is selected from phosphate buffer and Tris buffer, preferably phosphate buffer; and/or
preferably, the buffer has a concentration of about 5 mmol/L to about 30 mmol/L, preferably about 10 mmol/L; and/or
preferably, the buffer has a pH of about 6-8, preferably about 7-8, and more preferably about 7-7.5.

**75.** The pharmaceutical composition according to any one of claim 73 or 74, further comprising a cryoprotectant;

preferably, the cryoprotectant is selected from sucrose and trehalose, preferably sucrose; and/or
preferably, the cryoprotectant has a concentration of about 50 mg/ml to 100 mg/ml.

**76.** Use of the amino lipid compound of any one of claims 1 to 56 in the manufacture of a delivery vehicle for an active ingredient.

**77.** The use according to claim 76, wherein:

the delivery vehicle is the lipid nanoparticle of any one of claims 57 to 72; and/or
the active ingredient is a pharmaceutical active ingredient, preferably a nucleic acid; and/or
preferably, the nucleic acid is selected from the group consisting of RNA, antisense oligonucleotides, and DNA;
preferably, the RNA is selected from the group consisting of messenger RNA (mRNA), ribosomal RNA (rRNA), microRNA (miRNA), transfer RNA (tRNA), small interfering RNA (siRNA), small nuclear RNA (snRNA), small hairpin RNA (shRNA), single guide RNA (sgRNA), Cas9 mRNA, or a mixture thereof;
preferably, the DNA is a plasmid.

**78.** Use of the amino lipid compound of any one of claims 1 to 56, the lipid nanoparticle of any one of claims 57 to 72 or the pharmaceutical composition of any one of claims 73 to 75 in the manufacture of a medicament.

**79.** The use according to claim 78, wherein the medicament is for use in the treatment and/or prevention of a disease; preferably, the medicament is for use in gene therapy, protein replacement therapy, antisense therapy, therapy by interfering RNA, or gene vaccination.

**80.** The use according to claim 79, wherein the disease is selected from the group consisting of rare disease, infectious disease, cancer, genetic disease, autoimmune disease, diabetes, neurodegenerative disease, cardiovascular, renal

vascular disease, and metabolic disease.

**81.** The use according to claim 80, wherein the cancer is one or more selected from the group consisting of lung cancer, stomach cancer, liver cancer, esophageal cancer, colon cancer, pancreatic cancer, brain cancer, lymphoma, blood cancer, and prostate cancer; the genetic disease is one or more selected from the group consisting of hemophilia, thalassemia, and Gaucher's disease.

**82.** The use according to claim 79, wherein the gene vaccination is for use in the treatment and/or prevention of cancer, allergy, toxicity, and pathogen infection.

**83.** The use according to claim 82, wherein the pathogen is one or more selected from the group consisting of virus, bacterium, or fungi.

**84.** Use of the amino lipid compound of any one of claims 1 to 56, the lipid nanoparticle of any one of claims 57 to 72 or the pharmaceutical composition of any one of claims 73 to 75 in the manufacture of a medicament for nucleic acid transfer.

**85.** The use according to claim 84, wherein the nucleic acid is selected from the group consisting of RNA, antisense oligonucleotide, and DNA;

preferably, the RNA is selected from the group consisting of messenger RNA (mRNA), ribosomal RNA (rRNA), microRNA (miRNA), transfer RNA (tRNA), small interfering RNA (siRNA), small nuclear RNA (snRNA), small hairpin RNA (shRNA), single guide RNA (sgRNA), Cas9 mRNA, or a mixture thereof;
preferably, the DNA is a plasmid.

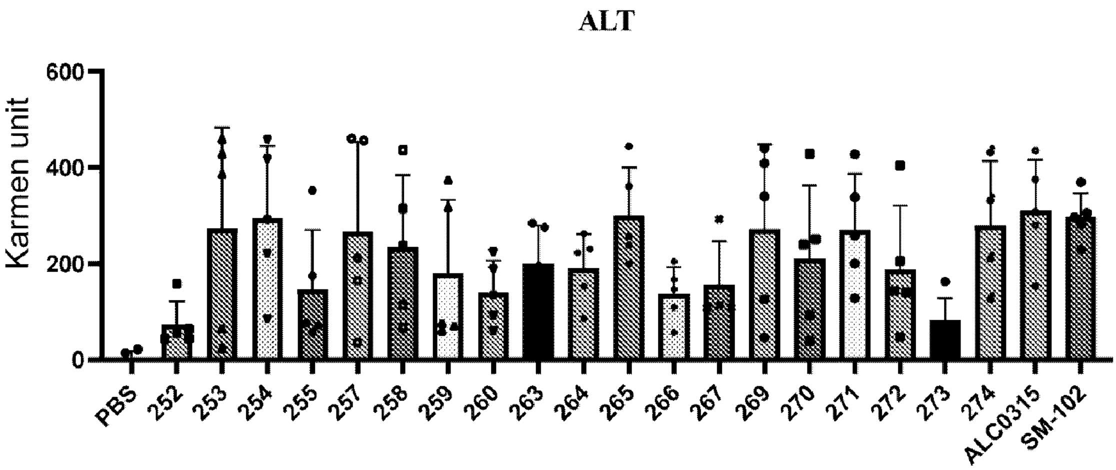
ALT
Karmen unit
600
400
200
0
PBS
252
253
254
255
257
258
259
260
263
264
265
266
267
269
270
271
272
273
274
ALC0315
SM-102

FIG. 1

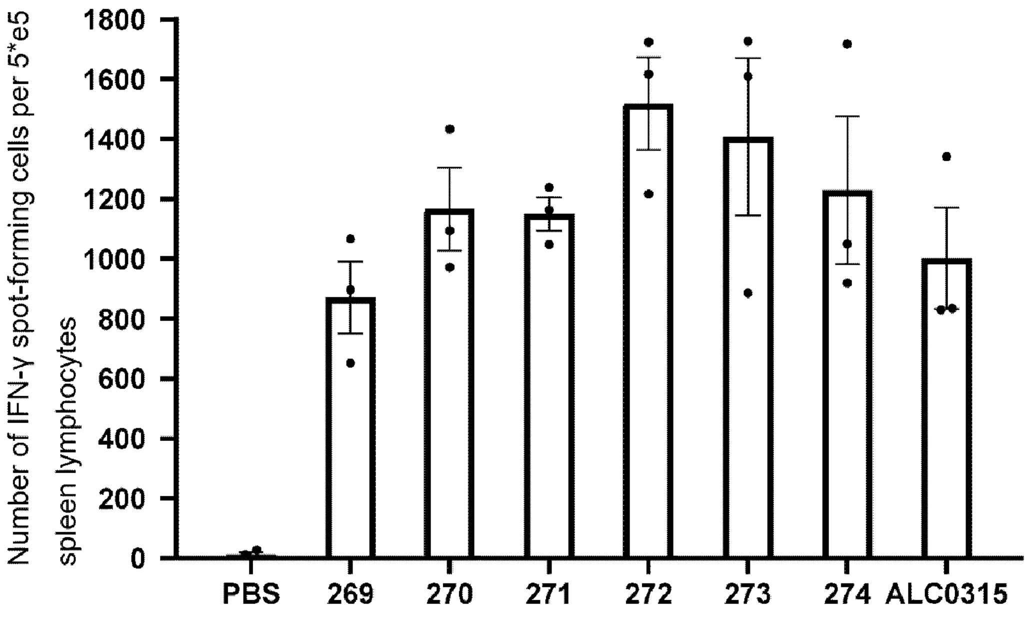
Number of IFN-γ spot-forming cells per 5*e5 spleen lymphocytes
1800
1600
1400
1200
1000
800
600
400
200
0
PBS
269
270
271
272
273
274
ALC0315

FIG. 2

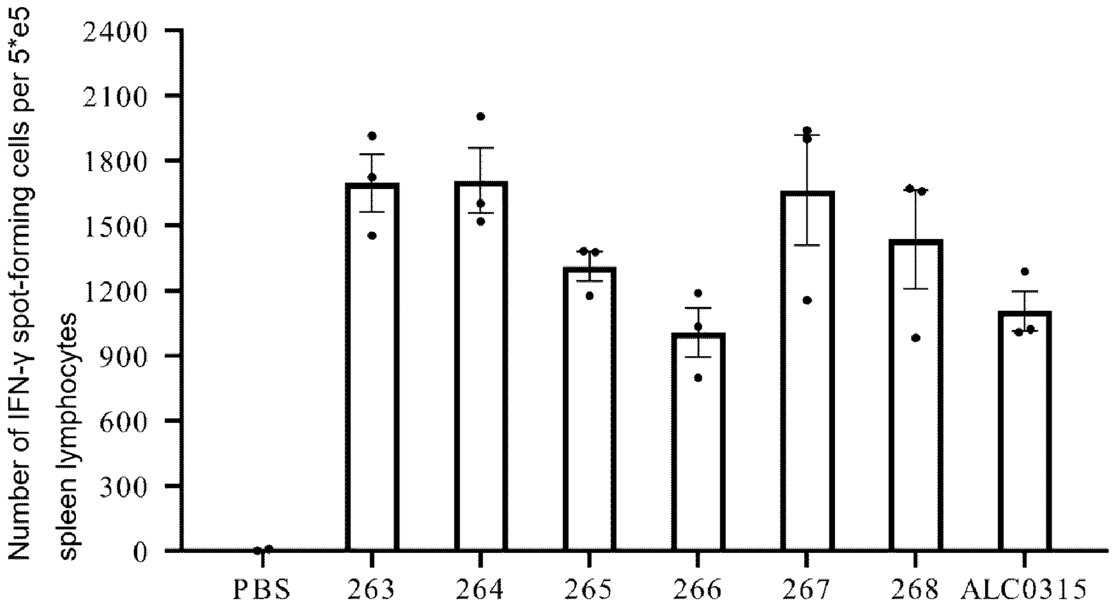
Number of IFN-γ spot-forming cells per 5*e5 spleen lymphocytes
2400
2100
1800
1500
1200
900
600
300
0
PBS
263
264
265
266
267
268
ALC0315

FIG. 3

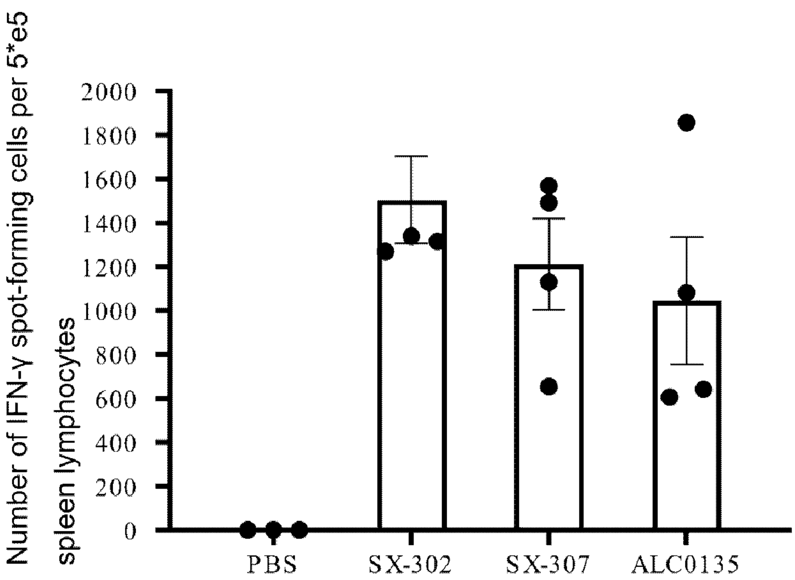
Number of IFN-γ spot-forming cells per 5*e5 spleen lymphocytes
2000
1800
1600
1400
1200
1000
800
600
400
200
0
PBS
SX-302
SX-307
ALC0135

FIG. 4

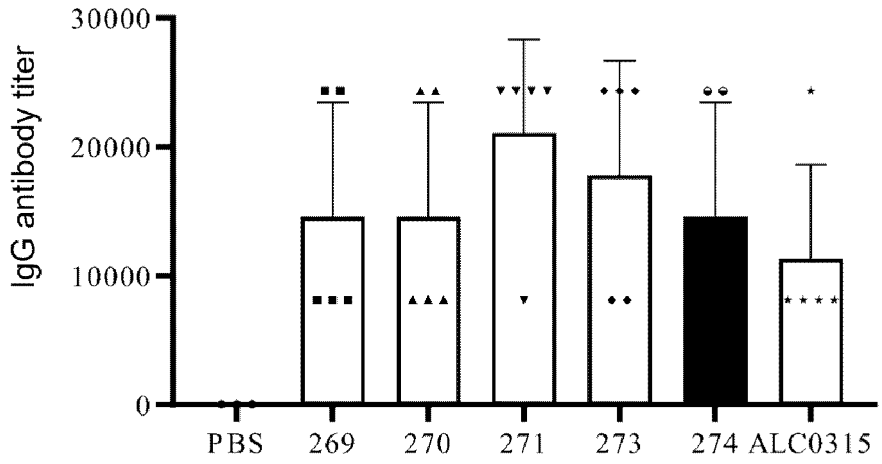
30000
20000
10000
0
IgG antibody titer
PBS
269
270
271
273
274
ALC0315

FIG. 5

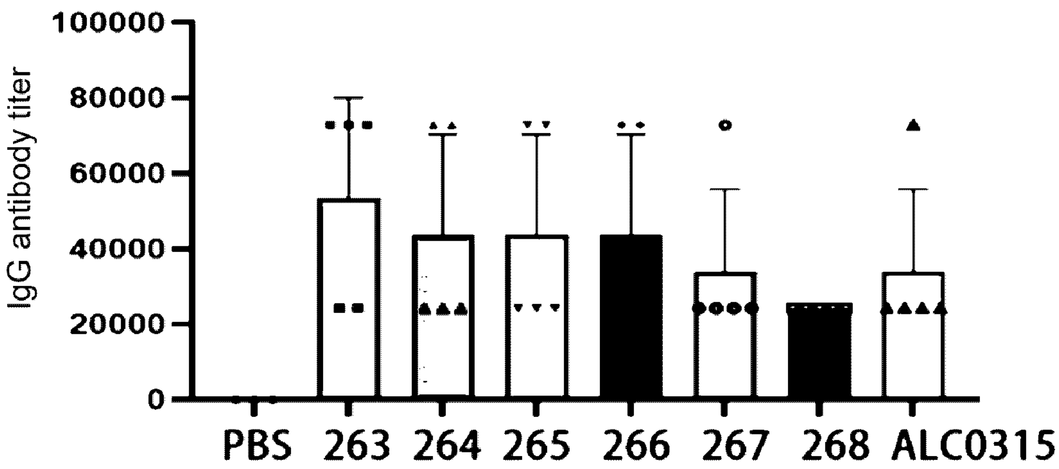
100000
80000
60000
40000
20000
0
IgG antibody titer
PBS
263
264
265
266
267
268
ALC0315

FIG. 6

**INTERNATIONAL SEARCH REPORT**

International application No. **PCT/CN2023/078449**

**A. CLASSIFICATION OF SUBJECT MATTER**

C07C271/20(2006.01)i; C07C271/16(2006.01)i; C07C271/12(2006.01)i; C07C219/16(2006.01)i; C07C229/10(2006.01)i; C07C229/12(2006.01)i; C07D207/02(2006.01)i; C07D211/04(2006.01)i; A61K9/127(2006.01)i; A61K9/51(2006.01)i; A61K47/18(2017.01)i; A61K31/7088(2006.01)i

According to International Patent Classification (IPC) or to both national classification and IPC

**B. FIELDS SEARCHED**

Minimum documentation searched (classification system followed by classification symbols)

C07C271/-; C07C219/-; C07C229/-; C07D207/-; C07D211/-; A61K9/-; A61K47/-; A61K31/-

Documentation searched other than minimum documentation to the extent that such documents are included in the fields searched

Electronic data base consulted during the international search (name of data base and, where practicable, search terms used)

CNTXT; VEN; ENTXT; WOTXT; USTXT; EPTXT; GBTXT; JPTXT; CNKI; 万方, WANFANG; 百度学术, BAIDU SCHOLAR; Web of Science; STN: 深圳深信生物科技有限公司, 李林鲜, 陈永好, 张再伟, 陈明洪, 氨基脂质, 胺基脂质, 脂质纳米颗粒, 氨基甲酸酯, 核酸, SHENZHEN SHENXIN BIOTECHNOLOGY CO. LTD., LI Linxian, CHEN Yonghao, ZHANG Zaiwei, CHEN Minghong, amino lipid, lipid nanoparticle, LNP, carbamate, nucleic acid, 式(I)结构, 式(II)结构

**C. DOCUMENTS CONSIDERED TO BE RELEVANT**

| Category* | Citation of document, with indication, where appropriate, of the relevant passages | Relevant to claim No. |
|---|---|---|
| X | US 2013123338 A1 (HEYES JAMES et al.) 16 May 2013 (2013-05-16) description, paragraphs 9-14, 25-52, 229, 247, 313, 316, 400, 409, 412, and 471-477 | 1-85 |
| X | US 2013129785 A1 (MANOHARAN MUTHIAH et al.) 23 May 2013 (2013-05-23) claim 12 | 1-11, 19, 20, 22, 28-37 |
| X | CN 104321304 A (PROTIVA BIOTHERAPEUTICS, INC.) 28 January 2015 (2015-01-28) description, paragraphs 153-155 | 1-11, 19, 20, 22, 28-30, 32-37, 39, 40 |
| Y | US 2013123338 A1 (HEYES JAMES et al.) 16 May 2013 (2013-05-16) description, paragraphs 9-14, 25-52, 229, 247, 313, 316, 400, 409, 412, and 471-477 | 1-85 |
| Y | WO 2020219876 A1 (INTELLIA THERAPEUTICS INC.) 29 October 2020 (2020-10-29) description, pages 2-3 | 1-85 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **23 May 2023** | **06 June 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2023/078449**

**C. DOCUMENTS CONSIDERED TO BE RELEVANT**

| Category* | Citation of document, with indication, where appropriate, of the relevant passages | Relevant to claim No. |
|---|---|---|
| A | CN 114073677 A (SHENZHEN SHENXIN BIOLOGICAL TECHNOLOGY CO., LTD.) 22 February 2022 (2022-02-22) entire document | 1-85 |
| A | CN 113474328 A (ACUITAS THERAPEUTICS INC.) 01 October 2021 (2021-10-01) entire document | 1-85 |
| A | US 2012172411 A1 (HEYES JAMES et al.) 05 July 2012 (2012-07-05) entire document | 1-85 |
| A | US 2014187614 A1 (DAVIDSON GARY et al.) 03 July 2014 (2014-07-03) entire document | 1-85 |

Form PCT/ISA/210 (second sheet) (July 2022)

**INTERNATIONAL SEARCH REPORT**

International application No.
**PCT/CN2023/078449**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2023/078449**

| Patent document cited in search report | Publication date (day/month/year) | Patent family member(s) | Publication date (day/month/year) |
|---|---|---|---|
| US 2013123338 A1 | 16 May 2013 | JP 2013527856 A | 04 July 2013 |
| | | CA 2799091 A1 | 17 November 2011 |
| | | EP 2569276 A1 | 20 March 2013 |
| | | EP 2569276 B1 | 24 February 2021 |
| | | JP 2020176135 A | 29 October 2020 |
| | | JP 2016222666 A | 28 December 2016 |
| | | JP 2022163090 A | 25 October 2022 |
| | | JP 2018197239 A | 13 December 2018 |
| | | WO 2011141705 A1 | 17 November 2011 |
| | | WO 2011141705 A8 | 03 January 2013 |
| | | US 2020282060 A1 | 10 September 2020 |
| | | US 2017151333 A1 | 01 June 2017 |
| US 2013129785 A1 | 23 May 2013 | US 9254327 B2 | 09 February 2016 |
| | | WO 2011143230 A1 | 17 November 2011 |
| CN 104321304 A | 28 January 2015 | ES 2898912 T3 | 09 March 2022 |
| | | HUE 041494 T2 | 28 May 2019 |
| | | RS 58077 B1 | 28 February 2019 |
| | | US 2023109183 A1 | 06 April 2023 |
| | | WO 2013126803 A1 | 29 August 2013 |
| | | NZ 700075 A | 27 May 2016 |
| | | RU 2020111326 A | 29 April 2020 |
| | | LT 2817287 T | 27 December 2018 |
| | | MX 2014010211 A | 10 August 2015 |
| | | MX 356904 B | 07 June 2018 |
| | | IL 290931 A | 01 April 2022 |
| | | PT 2817287 T | 28 December 2018 |
| | | KR 20220045089 A | 12 April 2022 |
| | | DK 2817287 T3 | 02 January 2019 |
| | | PL 2817287 T3 | 31 October 2019 |
| | | UA 120026 C2 | 25 September 2019 |
| | | JP 2021014462 A | 12 February 2021 |
| | | JP 7110297 B2 | 01 August 2022 |
| | | US 2017007702 A1 | 12 January 2017 |
| | | US 10561732 B2 | 18 February 2020 |
| | | RU 2014138476 A | 10 April 2016 |
| | | RU 2718053 C2 | 30 March 2020 |
| | | AU 2013222179 A1 | 09 October 2014 |
| | | AU 2013222179 B2 | 24 August 2017 |
| | | HK 1202855 A1 | 09 October 2015 |
| | | JP 2018039844 A | 15 March 2018 |
| | | JP 6789918 B2 | 25 November 2020 |
| | | SG 11201405157 PA | 30 October 2014 |
| | | IL 276924 A | 29 October 2020 |
| | | KR 20140129263 A | 06 November 2014 |
| | | KR 102075810 B1 | 10 February 2020 |
| | | KR 20210041135 A | 14 April 2021 |
| | | KR 102384791 B1 | 08 April 2022 |
| | | JP 2022140533 A | 26 September 2022 |
| | | JP 2015509505 A | 30 March 2015 |
| | | JP 6275655 B2 | 07 February 2018 |

Form PCT/ISA/210 (patent family annex) (July 2022)

**INTERNATIONAL SEARCH REPORT**
**Information on patent family members**

International application No.
**PCT/CN2023/078449**

| Patent document cited in search report | | | Publication date (day/month/year) | Patent family member(s) | | | Publication date (day/month/year) |
|---|---|---|---|---|---|---|---|
| | | | | EP | 3988104 | A1 | 27 April 2022 |
| | | | | BR | 112014020824 | A2 | 20 June 2017 |
| | | | | BR | 112014020824 | B1 | 04 October 2022 |
| | | | | CY | 1121232 | T1 | 29 May 2020 |
| | | | | HRP | 20182156 | T1 | 22 February 2019 |
| | | | | SI | 2817287 | T1 | 28 February 2019 |
| | | | | CA | 2865412 | A1 | 29 August 2013 |
| | | | | CA | 2865412 | C | 19 July 2022 |
| | | | | US | 2020306378 | A1 | 01 October 2020 |
| | | | | US | 11395854 | B2 | 26 July 2022 |
| | | | | KR | 20200016396 | A | 14 February 2020 |
| | | | | KR | 102239887 | B1 | 13 April 2021 |
| | | | | ZA | 201406896 | B | 25 May 2016 |
| | | | | PH | 12014501901 | B1 | 24 November 2014 |
| | | | | US | 2015064242 | A1 | 05 March 2015 |
| | | | | US | 9352042 | B2 | 31 May 2016 |
| | | | | EP | 2817287 | A1 | 31 December 2014 |
| | | | | EP | 2817287 | B1 | 03 October 2018 |
| | | | | IL | 234266 | A0 | 02 November 2014 |
| | | | | IL | 234266 | B | 30 September 2020 |
| | | | | ES | 2702874 | T3 | 06 March 2019 |
| | | | | EP | 3473611 | A1 | 24 April 2019 |
| | | | | EP | 3473611 | B1 | 20 October 2021 |
| WO | 2020219876 | A1 | 29 October 2020 | SG | 11202111154 | WA | 29 November 2021 |
| | | | | BR | 112021021313 | A2 | 18 January 2022 |
| | | | | TW | 202106662 | A | 16 February 2021 |
| | | | | EP | 3959191 | A1 | 02 March 2022 |
| | | | | CO | 2021014831 | A2 | 17 January 2022 |
| | | | | AU | 2020261419 | A1 | 04 November 2021 |
| | | | | KR | 20220005039 | A | 12 January 2022 |
| | | | | US | 2022402862 | A1 | 22 December 2022 |
| | | | | CA | 3137956 | A1 | 29 October 2020 |
| | | | | IL | 287421 | A | 01 December 2021 |
| | | | | JP | 2022530043 | A | 27 June 2022 |
| CN | 114073677 | A | 22 February 2022 | None | | | |
| CN | 113474328 | A | 01 October 2021 | US | 2020283372 | A1 | 10 September 2020 |
| | | | | US | 11453639 | B2 | 27 September 2022 |
| | | | | US | 2023123534 | A1 | 20 April 2023 |
| | | | | JP | 2022517783 | A | 10 March 2022 |
| | | | | EP | 3908568 | A1 | 17 November 2021 |
| | | | | CA | 3125485 | A1 | 16 July 2020 |
| | | | | KR | 20210138569 | A | 19 November 2021 |
| | | | | BR | 112021013654 | A2 | 14 September 2021 |
| | | | | WO | 2020146805 | A1 | 16 July 2020 |
| | | | | SG | 11202106987 | WA | 29 July 2021 |
| | | | | IL | 284535 | A | 31 August 2021 |
| | | | | AU | 2020205717 | A1 | 12 August 2021 |
| US | 2012172411 | A1 | 05 July 2012 | US | 8466122 | B2 | 18 June 2013 |
| | | | | US | 2014288146 | A1 | 25 September 2014 |

Form PCT/ISA/210 (patent family annex) (July 2022)

**INTERNATIONAL SEARCH REPORT**
**Information on patent family members**

International application No.
**PCT/CN2023/078449**

| Patent document cited in search report | | | Publication date (day/month/year) | Patent family member(s) | | | Publication date (day/month/year) |
|---|---|---|---|---|---|---|---|
| US | 2014187614 | A1 | 03 July 2014 | WO | 2012167869 | A1 | 13 December 2012 |
| | | | | EP | 2532649 | A1 | 12 December 2012 |
| | | | | EP | 2532649 | B1 | 08 April 2015 |
| | | | | US | 9511024 | B2 | 06 December 2016 |
| | | | | SG | 195093 | A1 | 30 December 2013 |
| | | | | KR | 20140041593 | A | 04 April 2014 |
| | | | | JP | 2014526437 | A | 06 October 2014 |
| | | | | JP | 5885833 | B2 | 16 March 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 107922364 A